(19) **Europäisches Patentamt / European Patent Office / Office européen des brevets**

(11) **EP 2 673 377 B1**

(12) # FASCICULE DE BREVET EUROPEEN

(45) Date de publication et mention
de la délivrance du brevet:
**10.04.2019 Bulletin 2019/15**

(21) Numéro de dépôt: **12703312.4**

(22) Date de dépôt: **09.02.2012**

(51) Int Cl.:
*C12Q 1/6876* [(2018.01)]     *C12Q 1/6883* [(2018.01)]
*C12Q 1/70* [(2006.01)]     *G01N 33/576* [(2006.01)]

(86) Numéro de dépôt international:
**PCT/EP2012/052231**

(87) Numéro de publication internationale:
**WO 2012/107528 (16.08.2012 Gazette 2012/33)**

(54) **COMBINAISON DE BIOMARQUEURS POUR LE PRONOSTIC D'UNE RÉPONSE OU NON-RÉPONSE À UN TRAITEMENT ANTI-VHC**

KOMBINATION VON BIOMARKERN ZUR PROGNOSE EINER REAKTION ODER NICHTREAKTION AUF EINE ANTI-HCV-BEHANDLUNG

COMBINATION OF BIOMARKERS FOR FORECASTING A RESPONSE OR NON-RESPONSE TO AN ANTI-HCV TREATMENT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **09.02.2011 FR 1151031
09.02.2011 US 201161440980 P
08.06.2011 FR 1155004
08.06.2011 US 201161494470 P**

(43) Date de publication de la demande:
**18.12.2013 Bulletin 2013/51**

(73) Titulaires:
• **Bio-Rad Innovations
92430 Marnes-La-Coquette (FR)**
• **Ariana Pharmaceuticals
75015 Paris (FR)**
• **Institut National de la Santé et de la Recherche Médicale
75654 Paris Cedex 13 (FR)**
• **Assistance Publique - Hôpitaux de Paris
75004 Paris (FR)**
• **Centre National de la Recherche Scientifique
75794 Paris Cedex 16 (FR)**

(72) Inventeurs:
• **BIECHE, Ivan
92150 Suresnes (FR)**
• **WATELET, Bénédicte
34980 Saint Clement De Riviere (FR)**

• **ASSELAH, Tarik
75012 Paris (FR)**
• **BATXELLI, Isabelle, Catherine
30670 Aigues-vives (FR)**
• **MATHIEU, Eve, Laure
34080 Montpellier (FR)**
• **JULLIAN, Nathalie
92120 Montrouge (FR)**
• **VIDAUD, Michel
94120 Fontenay Sous Bois (FR)**
• **MARCELLIN, Patrick
75017 Paris (FR)**
• **AFSHAR, Mohammad
75016 Paris (FR)**

(74) Mandataire: **Desaix, Anne et al
Ernest Gutmann - Yves Plasseraud S.A.S.
3, rue Auber
75009 Paris (FR)**

(56) Documents cités:
**EP-A1- 1 953 242     WO-A2-2010/076788**

• **ASSELAH T ET AL: "Liver gene expression signature to predict response to pegylated interferon plus ribavirin combination therapy in patients with chronic hepatitis C", GUT, BRITISH MEDICAL ASSOCIATION, LONDON, UK, vol. 57, no. 4, avril 2008 (2008-04), pages 516-523, XP002668777, ISSN: 0017-5749 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- EIRINI KOUTSOUNAKI ET AL: "Mannose-binding Lectin MBL2 Gene Polymorphisms and Outcome of Hepatitis C Virus-infected Patients", JOURNAL OF CLINICAL IMMUNOLOGY, KLUWER ACADEMIC PUBLISHERS-PLENUM PUBLISHERS, NE, vol. 28, no. 5, 1 juillet 2008 (2008-07-01), pages 495-500, XP019608253, ISSN: 1573-2592
- HUANG CHUNG-FENG ET AL: "Serum hs-CRP was correlated with treatment response to pegylated interferon and ribavirin combination therapy in chronic hepatitis C patients.", HEPATOLOGY INTERNATIONAL 2010 LNKD-PUBMED:21063486, vol. 4, no. 3, 2010, pages 621-627, XP002668897, ISSN: 1936-0541
- KITTL E M ET AL: "Serum protein 90K/Mac-2BP is an independent predictor of disease severity during hepatitis C virus infection'", CLINICAL CHEMISTRY AND LABORATORY MEDICINE, WALTER DE GRUYTER & CO, BERLIN, NEW YORK, vol. 38, no. 3, 1 mars 2000 (2000-03-01), pages 205-208, XP008082982, ISSN: 1434-6621, DOI: 10.1515/CCLM.2000.029
- Silvia Lee ET AL: "Decreased IP-10 and Elevated TGF[beta]1 Levels are Associated with Viral Clearance Following Therapy in Patients with Hepatitis C Virus", Disease Markers, 1 January 2010 (2010-01-01), pages 273-280, XP55262224, DOI: 10.3233/DMA-2010-0699 Retrieved from the Internet: URL:http://downloads.hindawi.com/journals/dm/2010/706984.pdf [retrieved on 2016-04-01]

## Description

L'invention est relative à des moyens qui permettent d'établir un pronostic de forte probabilité de réponse ou de non-réponse à un traitement anti-Virus de l'Hépatite C (VHC). De manière avantageuse, les moyens de l'invention permettent d'établir ce pronostic avant que le traitement anti-VHC n'ait même débuté.

## ARRIÈRE-PLAN DE L'INVENTION

Une infection par le virus de l'hépatite C (VHC) conduit, dans la grande majorité des cas, à une hépatite C chronique. L'hépatite C chronique peut évoluer vers une cirrhose hépatique, avec des complications d'hypertension portale, et vers un carcinome hépatocellulaire.

Un des objectifs du traitement contre une infection par VHC, plus particulièrement contre l'hépatite C chronique, est de parvenir à ce que les atteintes tissulaires du foie induites par l'infection virale régressent, voire soient éliminées, ou à tout le moins qu'elles ne progressent pas. Ceci permet notamment de diminuer ou d'éliminer le risque de survenue de complications et de carcinome hépatocellulaire.

Les traitements actuellement disponibles pour répondre à cet objectif sont des traitements, qui visent à éradiquer le virus. Ces traitements doivent dans un premier temps induire une diminution significative de la charge virale en VHC, de sorte à pouvoir atteindre un niveau indétectable en fin de traitement.

Les traitements anti-VHC actuels comprennent l'administration d'une combinaison d'interféron pégylé et de ribavirine. La durée de ces traitements est longue : ils sont généralement administrés sur une durée qui est d'au moins 24 semaines et qui peut aller jusqu'à 48 semaines voire plus.

Or, les traitements anti-VHC entraînent des effets secondaires majeurs pour le patient. Concernant l'interféron, les effets secondaires sont fréquents et nombreux. L'effet secondaire le plus fréquent est le syndrome pseudo-grippal (fièvre, arthralgies, céphalées, frissons). Les autres effets secondaires possibles sont : une asthénie, un amaigrissement, une perte modérée de cheveux, des troubles du sommeil, des troubles de l'humeur avec une irritabilité qui peut avoir des répercussions dans la vie quotidienne, des difficultés de concentration et une sécheresse cutanée. Certains effets secondaires rares peuvent être graves et doivent être anticipés comme les troubles psychiatriques. Une dépression peut survenir dans environ 10 % des cas. Celle ci doit être dépistée et traitée car elle peut avoir des conséquences graves (tentative de suicide). Une dysthyroïdie peut se déclarer. Le traitement par interféron est en outre contre-indiqué pendant la grossesse.

Concernant la ribavirine, le principal effet secondaire est l'anémie hémolytique. Une anémie conduit à un arrêt du traitement dans environ 5 % des cas. Une décompensation d'une coronaropathie ou d'une cardiopathie sous jacente, liée à l'anémie, peut survenir.

Une neutropénie est observée chez environ 20% des malades recevant l'association interféron pégylé et ribavirine, et représente la cause majeure de réduction de la dose d'interféron pégylé.

Le coût de ces traitements est également très élevé.

Pouvoir prédire, avant d'avoir même commencé à administrer le traitement anti-VHC, si un patient donné va ou non répondre au traitement est donc d'une importance clinique et économique majeure.

La recherche de tels moyens de prédiction a conduit à analyser différents facteurs cliniques, biologiques et viraux.

Certains facteurs cliniques propres au patient, tels que l'âge, le poids, l'ethnie et le score de fibrose hépatique, sont connus pour influencer l'efficacité du traitement anti-VHC.

Par exemple, le taux de patients répondant au traitement anti-VHC est plus faible parmi les patients dont le score de fibrose hépatique est F3 ou F4, par rapport à ceux dont le score de fibrose hépatique est F1 ou F2 (scores selon le système de scores F Metavir).

En eux-mêmes, ces facteurs cliniques ne permettent néanmoins pas de prédire de manière fiable, avant démarrage du traitement, si un patient donné va ou non répondre à un traitement anti-VHC.

En eux-mêmes, ces facteurs ne sont donc pas de bons indicateurs pronostiques pré-thérapeutiques.

Pour tenter de prédire, avant éventuelle administration du traitement, si un patient va ou non répondre à un traitement anti-VHC, ce sont des facteurs viraux qui sont actuellement utilisés.

Il a en effet été constaté que les patients qui sont infectés par un VHC de génotype 2 ou 3 répondent mieux au traitement anti-VHC que ceux qui sont infectés par un VHC de génotype 5 ou 6, qui eux-mêmes répondent mieux au traitement anti-VHC que ceux qui sont infectés par un VHC de génotype 1 ou 4.

Toutefois, la distribution des différents génotypes n'est pas homogène selon les localisations géographiques, et la seule détection du génotype viral n'apporte donc pas une solution prédictive applicable à l'ensemble des patients.

Il existe en outre des différences entre les sous-types viraux.

De fait, la connaissance de la nature du génotype viral permet essentiellement d'ajuster la posologie et/ou la durée du traitement, mais ne permet pas en soi d'établir un pronostic fiable avant démarrage du traitement.

Diverses combinaisons de facteurs biologiques et/ou cliniques et/ou viraux ont par ailleurs été testées, pour tenter de prédire, avant éventuelle administration du traitement, si un patient va ou non répondre à un traitement anti-VHC. Mais les combinaisons qui ont jusqu'ici été testées n'atteignent pas des performances satisfaisantes de prédiction.

Par exemple, Hidetsugu Saito *et al.* 2010 sont parvenus à identifier des combinaisons de facteurs biologiques, cliniques et viraux dont les performances de prédiction sont fiables lorsqu'elles sont appliquées en cours de traitement, mais n'en sont pas pour autant parvenus à établir une combinaison qui soit suffisamment fiable lorsqu'elle est appliquée avant démarrage du traitement anti-VHC.

Chen *et al.* 2005 et Chen *et al.* 2010 ont proposé une signature transcriptomique pour prédire, avant qu'un traitement anti-VHC ne soit administré, si un patient va être répondeur ou non-répondeur à ce traitement. Cette signature combine les niveaux d'expression de dix-huit gènes (G1P2, OAS2, G1P3, OAS3, RPLP2, CEB1, IFIT1, VIPERIN, RPS28, PI3KAP1, MX1, DUSP1, ATF5, LAP3, USP18, LGP1, ETEF1, STXBP5).

En outre, au moins deux de ces gènes codent pour des protéines qui sont exclusivement membranaires (G1P3 et VIPERIN) ; le produit de leur expression ne peut donc pas être détecté dans le sang circulant.

Asselah *et al.* 2008 ont analysé le niveau d'expression de cinquante-huit gènes avant application du traitement anti-VHC chez quarante patients atteints d'hépatite C chronique, parmi lesquels quatorze d'entre eux sont non-répondeurs au traitement anti-VHC. Ils ont ainsi identifié deux signatures susceptibles de permettre de prédire, avant qu'un traitement anti-VHC ne soit administré, si un patient va être non-répondeur à ce traitement.

La première signature est basée sur les niveaux d'expression de deux gènes, à savoir IFI27 et CXCL9, qui sont analysés selon la méthode KNN (méthode *k-nearest neighbour*).

La deuxième signature est basée sur les niveaux d'expression de trois gènes, à savoir IFI27, CXCL9 et IFI-6-16, qui sont analysés selon la méthode WV (méthode *weighted voting*).

Pour chacune de ces deux signatures, Asselah *et al.* 2008 indiquent que le fait d'ajouter des gènes supplémentaires ne permet pas d'améliorer l'exactitude de la classification.

Il existe donc toujours un besoin pour des moyens qui permettraient de pronostiquer, avant que l'on ait seulement commencé à administrer le traitement anti-VHC, si le patient a une forte probabilité de répondre, ou au contraire, une forte probabilité de ne pas répondre au traitement.

## RÉSUMÉ DE L'INVENTION

L'invention comme définie par les revendications 1-12 ci-jointes est relative à des moyens qui permettent d'établir un pronostic de forte probabilité de réponse ou de non-réponse à un traitement anti-VHC.

De manière avantageuse, les moyens de l'invention permettent d'établir si un sujet infecté par un ou des VHC a une forte probabilité d'être répondeur à un traitement anti-VHC qui comprendra l'administration d'interféron et l'administration de ribavirine ou d'une pro-drogue de la ribavirine, ou si, au contraire, ce sujet a une forte probabilité de ne pas être répondeur à ce traitement anti-VHC, ladite méthode comprenant les étapes suivantes :

i) procéder aux mesures suivantes :

- dans un échantillon préalablement obtenu à partir dudit sujet, doser les niveaux auxquels des gènes de mammifère choisis sont transcrits ou traduits, lesdits gènes de mammifère choisis dont on dose les niveaux de transcription ou de traduction étant la combinaison de gènes suivante :

    (a)

    - au moins un gène parmi MBL2, LGALS3BP et IL8, et
    - au moins un gène parmi G1P2, CCL21 et CXCL10, le nombre total des gènes ainsi choisis étant de 2, 3, 4 ou 5, ou

    (b)

    - au moins un gène parmi MBL2, LGALS3BP et IL8, et
    - au moins un gène parmi G1P2, CCL21 et CXCL10, et

- au moins un gène parmi AFP, CRP, CXCL11, CXCL6, CXCL9, FGF7, MDK, MMP2, SFN, TGFB2 et VEGFD, le nombre total des gènes ainsi choisis étant de 3, 4 ou 5, et
- doser les niveaux de transcription ou de traduction de 0 à 4 gènes de mammifère autres que les gènes de mammifère choisis parmi MBL2, LGALS3BP, IL8, G1P2, CXCL10, CCL21, AFP, CRP, CXCL11, CXCL6, CXCL9, FGF7, MDK, MMP2, SFN, TGFB2, VEGFD, et

ii) comparer les valeurs des mesures obtenues pour ledit sujet à l'étape i), à leurs valeurs, ou à la distribution de leurs valeurs, dans des cohortes de référence pré-établies selon leur statut de répondeur ou non-répondeur au traitement anti-VHC, pour classer ledit sujet dans celle de ces cohortes de référence vis-à-vis de laquelle il a la plus forte probabilité d'appartenance, et où l'échantillon obtenu dudit sujet est prélevé ou collecté à partir : du foie ou du parenchyme hépatique ou du sang, du sérum, du plasma ou de l'urine.

Optionnellement, les moyens de l'invention peuvent en outre mettre en oeuvre le dosage ou la mesure d'un ou plusieurs autres facteurs cliniques et/ou d'un ou plusieurs autres facteurs virologiques et/ou d'un ou plusieurs autres facteurs biologiques.

Les moyens de l'invention comprennent notamment :

- des méthodes qui comprennent le dosage ou la mesure des niveaux d'expression de gènes choisis ;
- des produits ou réactifs qui sont spécialement adaptés au dosage ou à la mesure de ces niveaux d'expression de gènes ;
- des articles manufacturés, des compositions, des compositions pharmaceutiques, des kits, des tubes, des supports solides comprenant de tels produits ou réactifs.

## BRÈVE DESCRIPTION DES FIGURES

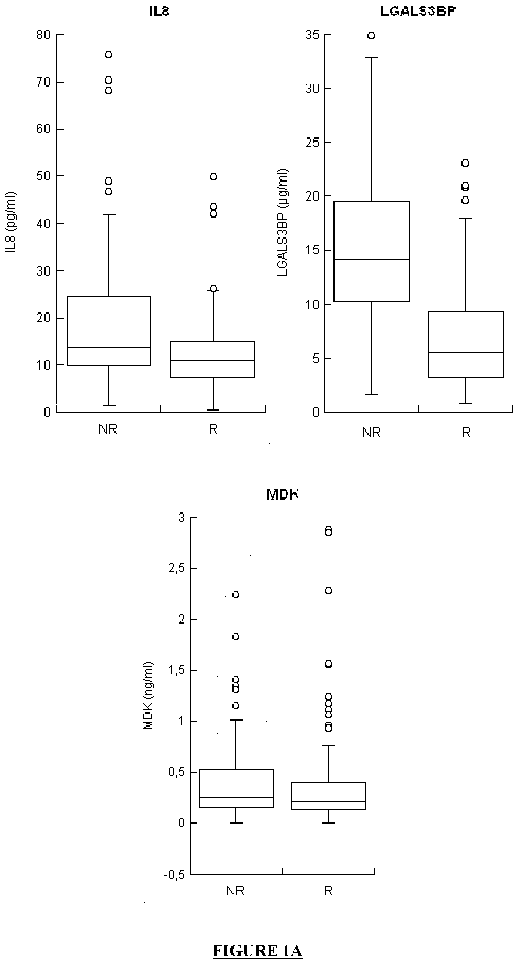

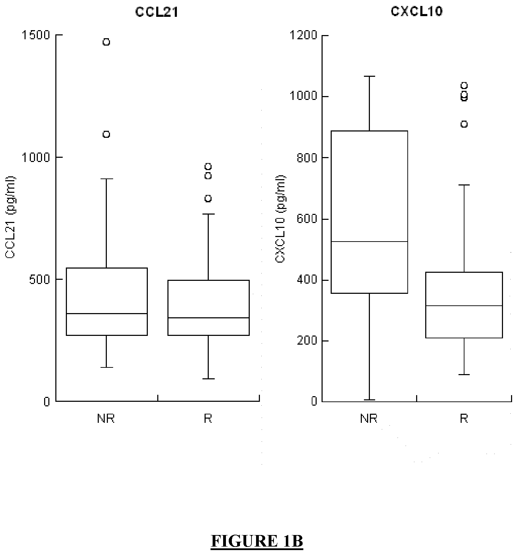

**Figures 1A et 1B :** Distribution des concentrations sériques des protéines IL8, LGALS3BP, MDK CXCL10 et CCL21 selon le statut répondeur (R) ou non-répondeur (NR) du patient.

## DESCRIPTION DÉTAILLÉE DE L'INVENTION

Le stade d'atteinte tissulaire du foie, plus particulièrement la nature et l'étendue des lésions tissulaires hépatiques, est évalué par un score de fibrose hépatique, notamment par le système de score F Metavir, qui comprend cinq stades de F0 à F4.

Lorsque le score de fibrose hépatique est d'au plus F1, le praticien peut éventuellement décider de ne pas administrer de traitement anti-VHC, alors que, lorsque le score est d'au moins F2, il est actuellement préconisé d'administrer un traitement anti-VHC, et cela quelque soit le degré d'activité nécrotico-inflammatoire.

Comme les traitements anti-VHC sont de très longue durée (généralement de 6 à 12 mois, voire plus), ils induisent des effets secondaires particulièrement graves, et sont très coûteux, l'invention propose des moyens qui sont destinés à aider à la décision d'administrer ou de ne pas administrer de traitement anti-VHC.

Les moyens de l'invention permettent un pronostic de forte probabilité de réponse ou de non-réponse au traitement anti-VHC. De manière avantageuse, les moyens de l'invention permettent d'établir ce pronostic avant que ce traitement n'ait même débuté.

Les moyens de l'invention comprennent la mesure ou le dosage des niveaux d'expression de gènes choisis. Ils concernent les sujets qui sont infectés par un ou plusieurs virus de l'hépatite, dont au moins un VHC, et plus particulièrement ceux de ces sujets qui présentent un score de fibrose hépatique d'au moins F1, plus particulièrement d'au moins F2, selon le système de score F Metavir.

Dans la description, à moins que cela ne soit autrement spécifié, ou que le contexte n'en dicte autrement, tous les termes ont leur sens habituel dans le(s) domaine(s) concerné(s).

L'expression « traitement anti-VHC », « traitement de l'hépatite C », ou une expression équivalente, ou, par voie de raccourci, le terme « traitement », signifie un traitement à visée thérapeutique, qui est destiné à induire une diminution de la charge en VHC du patient, de sorte à pouvoir atteindre, en fin de traitement, un niveau indétectable de charge en VHC, voire une éradication du ou des VHC. D'un point de vue clinique, le but thérapeutique souhaité est notamment de stopper, faire régresser, voire d'éliminer les lésions tissulaires du foie, c'est-à-dire, à tout le moins, d'empêcher que le score de fibrose hépatique n'augmente, voire de permettre que ce score diminue, de préférence qu'il diminue jusqu'à un score d'au plus F1.

Le traitement anti-VHC comprend au moins l'administration d'interféron, plus particulièrement d'interféron alpha, notamment d'interféron alpha-2a ou d'interféron alpha-2b, ou d'une pro-drogue de l'interféron.

Cet interféron est généralement une version produite par ingénierie génétique de la cytokine humaine naturelle. Cet interféron peut néanmoins être un interféron qui dérive de cellules d'ovaires de hamster chinois (cellules CHO), tel que l'interféron oméga (par exemple, l'interféron oméga qui est disponible auprès de Intarcia Therapeutics, Hayward, Californie, USA).

Cet interféron peut notamment être lié à d'autres composés, groupements ou molécules chimiques, notamment à du polyéthylène glycol (par exemple, le PEG-INTRON® commercialisé par Schering Plough Corporation, Kenilworth, New Jersey, USA, ou le PEGASYS® commercialisé par F. Hoffmann-La Roche Ltd. ; Basel, Suisse).

La forme pégylée de l'interféron a une durée de vie plus longue dans le corps humain, ce qui permet de limiter le rythme d'administration à une seule administration par semaine (en l'occurrence, à une seule injection par semaine), au lieu de trois administrations par semaine pour la forme non pégylée.

La forme pégylée de l'interféron est donc actuellement la forme préférée d'interféron.

Un interféron pégylé peut par exemple être administré :

- à une dose d'environ 1,5 g/kg/semaine pour l'interféron alpha-2b pégylé (tel que le PEG-INTRON®),
- à une concentration de 180 g/kg/semaine pour l'interféron alpha-2a pégylé (tel que le PEGASYS®).

En sus de l'interféron, un traitement anti-VHC comprend généralement l'administration d'au moins un autre agent antiviral.

En sus de l'interféron, le traitement anti-VHC actuel comprend généralement l'administration de ribavirine.

La ribavirine est un analogue nucléosidique de la guanosine.

Selon un mode particulier de l'invention, le traitement anti-VHC comprend l'administration d'interféron et l'administration de :

- ribavirine (par exemple, la ribavirine REBETOL® commercialisée par Plough Corporation, Kenilworth, New Jersey, USA, ou la ribavirine COPEGUS® commercialisée par Roche Corporation ; F. Hoffmann-La Roche Ltd. ; Basel, Suisse), ou
- d'un analogue de la ribavirine, ou
- d'une pro-drogue de la ribavirine ou d'un des ses analogues.

Les pro-drogues de la ribavirine comprennent notamment la taribavirin (par exemple, la taribavirin qui est disponible auprès de Valeant, Aliso Viejo, Californie, USA). L'administration de la ribavirine est généralement journalière.

La ribavirine peut par exemple être administrée à raison de 800 à 1200 mg/kg/jour.

Un traitement anti-VHC peut par exemple comprendre l'administration de :

- d'interféron alpha-2b pégylé (tel que PEG-INTRON®) à une dose d'environ 1,5 g/kg/semaine, et de ribavirine à une dose de 800 à 1 200 mg/kg/jour (si l'hépatopathie implique un VHC de génotype 2 ou 3, une dose d'environ 800 mg/kg/jour est généralement conseillée), ou
- d'interféron alpha-2a pégylé (tel que PEGASYS®) à une concentration de 180 g/kg/semaine et de ribavirine à raison de 1000 à 1200 mg/kg/jour.

En sus de l'interféron, ou de l'interféron et de la ribavirine, le traitement anti-VHC traitement peut en outre comprendre l'administration d'au moins un autre agent antiviral générique ou spécifique du VHC, tel que :

- au moins un inhibiteur de protéase de VHC, tel qu'un inhibiteur de la protéase NS3, et/ou
- au moins un inhibiteur de la polymérase du VHC, tel qu'un inhibiteur de la polymérase NS5B,

plus particulièrement au moins un inhibiteur de protéase de VHC, tel qu'un inhibiteur de la protéase NS3.

Ledit inhibiteur de la protéase NS3 peut par exemple être le telaprevir (VX-950 ; Vertex, Cambridge, Massachusetts, USA) ou le boceprevir (SCH-503034 ; Schering-Plough, Kenilworth, New Jersey, USA). La combinaison d'interféron (ou d'un analogue ou d'une pro-drogue de l'interféron), de ribavirine (ou d'un analogue ou d'une pro-drogue de la ribavirine) et d'un inhibiteur de protéase de VHC, tel que le telaprevir ou le boceprevir (ou d'un analogue ou d'une pro-drogue de cet inhibiteur de protéase), est une trithérapie qui est notamment envisagée pour le traitement des patients qui sont infectés par au moins un VHC de génotype 1 ou 4.

Ledit inhibiteur de la polymérase NS5B peut par exemple être un analogue nucléosidique tel que R1479, ou sa pro-drogue le R1626 (Roche, Basel, Suisse), ou l'analogue nucléosidique PSI-6130, ou sa pro-drogue le R7128 (Pharmasset, Princeton, NJ, U.S.A. ; Roche, Basel, Suisse).

En sus du ou des agents anti-viraux, le traitement anti-VHC peut en outre comprendre l'administration d'au moins un autre produit, sans activité anti-virale directe, tel qu'un adjuvant de médication, par exemple une hormone qui stimule la production d'érythrocytes et/ou de leucocytes, tel que l'érythropoïétine (EPO).

La durée du traitement anti-VHC est généralement d'au moins 24 semaines environ, très généralement d'environ 24 à 48 semaines, mais parfois plus. Par exemple, elle peut être :

- d'environ 24 semaines pour une hépatopathie à VHC de génotype 2 ou 3,
- d'environ 48 semaines pour une hépatopathie à VHC de génotype 1, 4 ou 5, ou pour un patient non-répondeur au traitement au bout de 24 semaines.

Les expressions « répondeur » ou « non-répondeur » sont entendues conformément à leur sens habituel dans le domaine médical. L'expression « répondeur » ou « non-répondeur » s'entend comme « répondeur au traitement anti-VHC » ou « non-répondeur au traitement anti-VHC », respectivement.

Il est considéré qu'un sujet est :

- un sujet répondeur au traitement (patient classé R), lorsque la charge virale de VHC est devenue indétectable dans le sang du patient à l'issue d'un traitement anti-VHC associant administration d'interféron et administration de ribavirine (ou d'une pro-drogue ou d'un analogue de ces principes actifs), et que cette charge virale reste indétectable 6 mois après l'arrêt de ce traitement ;
- un sujet non répondeur au traitement (patient classé NR), lorsque la charge virale de VHC reste détectable dans le sang du patient à l'issue de ce traitement anti-VHC ;
- un sujet répondeur-rechuteur (patient classé RR), lorsque la charge virale de VHC est devenue indétectable dans le sang du patient à l'issue de ce traitement anti-VHC, mais qu'elle est redevenue détectable 6 mois après l'arrêt de ce traitement anti-VHC.

Ce traitement anti-VHC est généralement administré :

- sur environ 24 semaines pour une hépatopathie à VHC de génotype 2 ou 3,
- sur environ 48 semaines pour une hépatopathie à VHC de génotype 1, 4, 5 ou 6.

Le traitement peut être l'un des traitements ci-dessus mentionnés, tels que notamment un traitement comprenant ou consistant en l'administration de ribavirine (ou d'une pro-drogue ou d'un analogue de ce principe actif) et d'interféron alpha-2a ou d'interféron alpha-2b, plus particulièrement d'interféron pégylé (plus particulièrement, d'interféron alpha-2a pégylé ou d'interféron alpha-2b pégylé), ou d'une pro-drogue ou d'un analogue de ce principe actif.

L'interféron est usuellement administré à un rythme d'une fois par semaine, alors que la ribavirine est usuellement administrée à un rythme de deux fois par jour.

Des exemples de traitement comprennent notamment les exemples suivants :

- traitement par administration :

   ◦ d'interféron alpha-2b pégylé (PEG-INTRON®; Schering Plough Corporation ; Kenilworth, NJ ; U.S.A.) à une dose de 1,5 g/kg/semaine, et
   ◦ de ribavirine (REBETOL® ; Schering Plough Corporation ; Kenilworth, NJ ; U.S.A.), en fonction du poids du patient et du/des génotypes de VHC, à une dose de :

      ▪ 800 à 1200 mg/kg/jour pour ceux des patients qui étaient infectés par au moins un génotype 1 et/ou 4 et/ou 5 et/ou 6 de VHC, ou à une dose de
      ▪ 800 mg/kg/jour pour ceux des patients qui étaient infectés par au moins un génotype 2 et/ou 3 de VHC,

ou
- traitement par administration :

   ◦ d'interféron alpha-2a pégylé (PEGASYS® ; Roche Corporation ; F. Hoffmann-La Roche Ltd. ; Basel, Suisse) à une dose de 180 g/kg/semaine, et
   ◦ de ribavirine (COPEGUS® ; Roche Corporation ; F. Hoffmann-La Roche Ltd. ; Basel, Suisse) à une dose de 1000 à 1200 mg/kg/jour.

L'un ou l'autre de ces deux exemples de traitement peut être administré pendant 24 semaines pour ceux des sujets qui étaient infectés par au moins un génotype 2 et/ou 3 de VHC, et pendant 48 semaines pour ceux des sujets qui étaient infectés par au moins un génotype 1 et/ou 4 et/ou 5 et/ou 6 de VHC.

La charge virale de VHC peut être considérée indétectable dans le sang du sujet, lorsque le dosage des ARN de VHC dans le sérum du sujet a conduit à une valeur inférieure à 12 Unités Internationales (UI) par mL de sérum, telle que mesurée par un test de quantification de l'ARN de VHC, par exemple telle que mesurée par un test de quantification réalisé à l'aide du kit VERSANT® HCV-RNA 3.0 (bDNA) ASSAY de la société Siemens Healthcare Diagnostics (limite de quantification = 615 - 7 690 000 UI/mL), en suivant les recommandations du fabricant de ce kit.

Les inventeurs ont identifié des gènes dont les niveaux d'expression constituent des biomarqueurs qui, lorsqu'ils sont pris en combinaison, sont pertinents pour déterminer le statut « répondeur » (R) ou « non répondeur » (NR) d'un sujet.

Les inventeurs ont en outre observé que, selon ces combinaisons de niveaux d'expression, la population des sujets répondeurs-rechuteurs (RR) ségrége très fortement avec celle des répondeurs (R) : les sujets RR sont majoritairement classés comme des R (*cf.* exemples ci-dessous).

Les gènes ainsi identifiés sont les dix-sept gènes suivants : MBL2, LGALS3BP, IL8, G1P2, CXCL10, CCL21, AFP, CRP, CXCL11, CXCL6, CXCL9, FGF7, MDK, MMP2, SFN, TGFB2, VEGFD.

De manière particulièrement avantageuse, il peut être observé que ces dix-sept gènes sont tous des gènes codant des protéines non-membranaires, c'est-à-dire des gènes qui codent pour une protéine dont la localisation est intracellulaire et/ou extracellulaire, et qui peut donc être détectée dans un fluide biologique du sujet, tel que le sang, le sérum ou le plasma.

Les inventeurs ont en outre identifié que les combinaisons les plus pertinentes comprennent :

- au moins un gène parmi MBL2, LGALS3BP, IL8, et
- au moins un gène parmi G1P2, CCL21, CXCL10, et
- optionnellement, au moins un gène parmi AFP, CRP, CXCL11, CXCL6, CXCL9, FGF7, MDK, MMP2, SFN, TGFB2, VEGFD.

Chacun de ces gènes est individuellement connu de la personne du métier, et est entendu conformément à sa signification dans le domaine. Un rappel de leurs identités respectives est présenté à titre indicatif dans le tableau 1 ci-dessous.

**Tableau 1 : identité des gènes**

| Symbole | Nom (en français) de la protéine codée | Nom (en anglais) de la protéine codée | Alias | Numéro d'accession NM |
|---|---|---|---|---|
| MBL2 | lectine 2 se liant au mannose | mannose-binding lectin 2 | | NM_000242 |
| G1P2 | protéine inductible par l'interféron alpha (clone IFI-15K) | interferon alpha inducible protein (clone IFI-15K) | ISG15, IFI15 | NM_ 005101.3 |
| MDK | midkine | midkine | NEGF2 | NM_ 001012334 |
| LGALS3BP | protéine se liant à LGALS3 (lectine, se liant à la galactosidase, soluble, 3) | lectin, galactosidase-binding, soluble, 3 binding protein | 90K, MAC-2-BP | NM_005567.3 |
| CXCL10 | ligand 10 à chémokine (motif CXC) | chemokine (CXC motif) ligand 10 | C7, IFI10, INP10, IP-10, SCYB10, crg-2, gIP-10, mob-1 | NM_001565 |
| FGF7 | facteur de croissance de fibroblastes 7 | fibroblast growth factor 7 | HBGF7, KGF | NM_002009 |
| IL8 | interleukine 8 | interleukin 8 | CXCL8, GCP-1, GCP1, LECT, LUCT, LYNAP, MDNCF, MONAP, NAF, NAP-1, NAP1 | NM_000584 |
| TGFB2 | facteur de croissance transformant beta 2 | transforming growth factor beta 2 | | NM_ 001135599.1 |
| CCL21 | ligand 21 à chémokine (motif C-C) | chemokine (C-C motif) ligand 21 | ECL, SLC, SCYA21 | NM_002989 |
| CXCL6 | ligand 6 à chémokine (motif CXC) | chemokine (CXC motif) ligand 6 | CKA-3, GCP-2, GCP2, SCYB6 | NM_002993 |
| MMP2 | métallopeptidase 2 de matrice | matrix metallopeptidase2 | CLG4, MONA TBE1 | NM_004530 |
| SFN | stratifine | stratifin | YWHAS | NM_006142.3 |

(suite)

| Symbole | Nom (en français) de la protéine codée | Nom (en anglais) de la protéine codée | Alias | Numéro d'accession NM |
|---|---|---|---|---|
| CXCL11 | ligand 11 à chémokine (motif CXC) | chemokine (CXC motif) ligand 11 | IP9, SCYB11 | NM_ 005409.3 |
| AFP | alphafétoprotéine | alphafetoprotein | FETA, HPFAP | NM_001134 |
| VEGFD | facteur de croissance induit par C-Fos | C-Fos induced growth factor | FIGF | NM_004469.2 |
| CRP | protéine C-réactive apparentée à la pentaxine | C-reactive protein pentaxin-related | PTX1 | NM_000567.2 |
| CXCL9 | ligand 9 à chémokine (motif CXC) | chemokine (CXC motif) ligand 9 | CMK, Myg, SCYB9 | NM_002416.1 |
| | | | | |
| RPLP0 | phosphoprotéine ribosomale acide P0 humaine | human acidic ribosomal phosphoprotein P0 | 36B4 | NM_ 001002 |
| TBP | protéine se liant à une boîte TATA | TATA box binding protein | | NM_003194 |

Aucun de ces gènes n'est un gène de virus d'hépatite. Il s'agit de gènes de mammifères, plus particulièrement de gènes humains.

Chacun de ces gènes code pour une protéine non-membranaire, c'est-à-dire une protéine qui n'est pas ancrée dans une membrane cellulaire. La localisation *in vivo* de ces protéines est donc intracellulaire et/ou extracellulaire. Ces protéines sont présentes dans un fluide biologique du sujet, tel que par exemple dans le sang, le sérum, le plasma ou l'urine, notamment dans le sang ou le sérum ou le plasma.

En sus des niveaux d'expression de gènes choisis parmi la liste de dix-sept gènes de l'invention (MBL2, LGALS3BP, IL8, G1P2, CXCL10, CCL21, AFP, CRP, CXCL11, CXCL6, CXCL9, FGF7, MDK, MMP2, SFN, TGFB2, VEGFD), les moyens de l'invention peuvent en outre comprendre la mesure d'autres facteurs, notamment d'un ou plusieurs facteurs cliniques et/ou d'un ou plusieurs facteurs virologiques et/ou d'un ou plusieurs facteurs biologiques autres que le niveau d'expression de gènes choisis parmi ladite liste de dix-sept gènes.

Plus particulièrement, en sus des niveaux d'expression de gènes choisis parmi ladite liste de dix-sept gènes de l'invention, les moyens de l'invention peuvent optionnellement comprendre (*cf.* exemples 2c) et 3b) ci-dessous) :

- le dosage du niveau d'expression de gènes de mammifères (plus particulièrement de gènes humains) autres que ceux de ladite liste de dix-sept gènes, par exemple pour doser le niveau de transcription de gènes qui sont ci-dessous listés en tant que « autres facteurs biologiques », tels que le gène codant la gamma glutamyl transpeptidase (GGT) et/ou le gène codant la phosphatase alcaline (PAL), et/ou
- le dosage de métabolites intracorporels (par exemple, le cholestérol), et/ou
- le dosage d'éléments figurés du sang (par exemple les plaquettes), et/ou
- le dosage de la quantité de fer circulant.

Il s'agit toutefois de dosages optionnels.

Le nombre de gènes de mammifères (plus particulièrement de gènes humains), dont le niveau d'expression est dosé, et qui ne sont pas des gènes choisis parmi ladite liste de dix-sept gènes de l'invention (par exemple GGT et/ou PAL), peut être de préférence au maximum de 18, plus particulièrement de 14 ou moins, plus particulièrement de 11 ou moins, plus particulièrement de 6 ou moins, plus particulièrement de 4 ou 3 ou 2 ou 1 ou 0, plus particulièrement de 3 ou 2 ou 1 ou 0, notamment de 2 ou 1 ou 0.

Par suite, en comptabilisant ces « autres » gènes de mammifères (plus particulièrement ces « autres » gènes humains), dont le niveau d'expression peut être éventuellement dosé, ainsi que le nombre maximal des dix-sept gènes qui peuvent être des gènes choisis conformément à l'invention, le nombre total de gènes dont le niveau d'expression est dosé dans une méthode conforme à l'invention, peut être de préférence de 2 à 35 gènes, plus particulièrement de 2 à 31, plus particulièrement de 2 à 28, plus particulièrement de 2 à 23, plus particulièrement de 2 à 21, plus particulièrement de 2 à 20, plus particulièrement de 2 à 19, plus particulièrement de 2 à 18, notamment de 2 à 17, plus particulièrement de 2 à 16, plus particulièrement de 2 à 15, plus particulièrement de 2 à 14, plus particulièrement de 2 à 13, plus particulièrement de 2 à 12, plus particulièrement de 2 à 11, plus particulièrement de 2 à 10, plus particulièrement de 2 à 9, plus particulièrement de 2 à 8, plus particulièrement de 2 à 7, plus particulièrement de 2 à 6 (par exemple de 2, 3, 4, 5 ou 6), plus particulièrement de 2 à 5 (par exemple de 2, 3, 4 ou 5).

En outre, comme cela est présenté plus en détail ci-dessous, et comme cela est illustré en exemples, le nombre de gènes choisis parmi la liste de dix-sept gènes de l'invention (MBL2, LGALS3BP, IL8, G1P2, CXCL10, CCL21, AFP, CRP, CXCL11, CXCL6, CXCL9, FGF7, MDK, MMP2, SFN, TGFB2, VEGFD) peut avantageusement être de 2, 3, 4 ou 5.

Selon l'invention, le nombre total de gènes dont le niveau d'expression est dosé, est de :

- 2, 3, 4 ou 5 gènes choisis parmi ladite liste de dix-sept gènes de l'invention, et
- 0, 1, 2, 3 ou 4 « autres » gènes de mammifère, plus particulièrement de 0, 1, 2 ou 3 « autres » gènes de mammifère, plus particulièrement de 0, 1 ou 2 « autres » gènes de mammifère.

Selon l'invention, le nombre total de gènes de mammifère dont le niveau d'expression est dosé dans une méthode conforme à l'invention, est donc de 2 à 9, plus particulièrement de 2 à 8, plus particulièrement de 2 à 7, plus particulièrement de 2 à 6, plus particulièrement de 2 à 5, plus particulièrement de 2 à 4.

Les moyens de l'invention peuvent optionnellement comprendre le dosage du produit d'expression (ARN ou protéique) d'un ou plusieurs gènes non humains, plus particulièrement d'un ou plusieurs gènes viraux, plus particulièrement d'un ou plusieurs gènes de virus d'hépatite, plus particulièrement d'un ou plusieurs gènes de VHC.

Les moyens de l'invention peuvent optionnellement comprendre la détermination du ou des génotypes du ou des VHC dont le sujet est infecté.

Les moyens de l'invention peuvent optionnellement comprendre la détermination d'un ou plusieurs facteurs cliniques dudit sujet tels que la charge virale avant traitement (CVavantTTT dans les exemples ci-dessous).

Une caractéristique des moyens de l'invention est qu'ils comprennent le fait de doser (ou mesurer) le niveau auquel des gènes choisis s'expriment dans l'organisme dudit sujet. L'expression « niveau d'expression d'un gène » ou expression équivalente désigne ici tant le niveau auquel ce gène est transcrit en ARN, plus particulièrement en ARNm, que le niveau auquel est exprimée une protéine codée par ce gène.

Le terme « doser » ou « mesurer » ou terme équivalent est entendu selon son sens commun dans le domaine, et fait référence au fait de quantifier.

On peut doser le niveau de transcription (ARN) de chacun desdits gènes, ou doser le niveau de traduction (protéine) de chacun desdits gènes, ou bien encore doser le niveau de transcription pour certains desdits gènes choisis et le niveau de traduction pour les autres de ces gènes choisis. Selon un mode de réalisation de l'invention, on dose soit le niveau de transcription soit le niveau de traduction de chacun desdits gènes choisis.

Le fait de doser (ou mesurer) le niveau de transcription d'un gène comprend le fait de quantifier des ARN transcrits de ce gène, plus particulièrement de déterminer la concentration d'ARN transcrits par ce gène (par exemple, la quantité de ces ARN rapportée à la quantité totale d'ARN initialement présente dans l'échantillon, telle que par exemple une valeur de Ct normalisée par la méthode du $2^{-\Delta Ct}$ ; *cf.* ci-dessous).

Le fait de doser (ou mesurer) le niveau de traduction d'un gène comprend le fait de quantifier des protéines codées par ce gène, plus particulièrement de déterminer la concentration en protéines codées par ce gène (par exemple, la quantité de cette protéine par volume de fluide biologique).

Certaines protéines codées par un gène de mammifère, notamment un gène humain, peuvent parfois subir des modifications post-traductionnelles telles que, par exemple, un clivage en polypeptides et/ou peptides. Le cas échéant, le fait de doser (ou mesurer) le niveau de traduction d'un gène peut alors comprendre le fait de quantifier ou de déterminer la concentration non pas de la ou des protéines elles-mêmes, mais d'une ou de formes post-traductionnelles de cette ou ces protéines, telles que par exemple, des polypeptides et/ou peptides qui sont des fragments spécifiques de cette ou ces protéines. Pour doser ou mesurer le niveau d'expression d'un gène, l'on peut donc quantifier :

- des ARN transcrits de ce gène, ou
- des protéines exprimées par ce gène, ou des formes post-traductionnelles de telles protéines, telles que par exemple des polypeptides ou peptides qui sont des fragments spécifiques de ces protéines.

L'invention est relative aux objets définis dans les revendications.

L'invention est notamment relative à des moyens qui permettent de pronostiquer si un sujet infecté par un ou des VHC a une forte probabilité de répondre à un traitement anti-VHC qui comprendra l'administration d'interféron et de ribavirine, ou si, au contraire, ce sujet a une forte probabilité de ne pas répondre à ce traitement anti-VHC.

Les moyens de l'invention comprennent notamment :

- des méthodes qui comprennent le dosage ou la mesure des niveaux d'expression de gènes choisis (niveaux de transcription ou de traduction);
- des produits ou réactifs qui sont spécialement adaptés au dosage ou à la mesure de ces niveaux d'expression de gènes ;
- des articles manufacturés, des compositions, des compositions pharmaceutiques, des kits, des tubes, des supports solides comprenant de tels produits ou réactifs.

Les moyens de l'invention, plus particulièrement la méthode de l'invention, sont mis en oeuvre avant traitement de l'infection à VHC, et, de manière avantageuse, peuvent être mis en oeuvre avant que le traitement anti-VHC n'ait commencé, plus particulièrement avant que tout traitement anti-VHC n'ait commencé.

L'invention est ainsi relative à une méthode, plus particulièrement une méthode *in vitro*, pour pronostiquer si un sujet infecté par un ou des VHC a une forte probabilité de répondre à un traitement anti-VHC qui comprendra l'administration d'interféron et de ribavirine, ou si, au contraire, ce sujet a une forte probabilité de ne pas répondre à ce traitement anti-VHC.

La méthode comprend le fait de doser les niveaux auxquels des gènes choisis sont transcrits ou traduits, lesdits gènes choisis étant des gènes choisis parmi la liste de gènes suivants: MBL2, LGALS3BP, IL8, G1P2, CXCL10, CCL21, AFP, CRP, CXCL11, CXCL6, CXCL9, FGF7, MDK, MMP2, SFN, TGFB2, VEGFD.

Plus particulièrement, la méthode de pronostic de l'invention comprend le fait de doser les niveaux auxquels des gènes choisis sont transcrits ou traduits, lesdits gènes choisis étant :

- au moins un gène parmi MBL2, LGALS3BP, IL8, et
- au moins un gène parmi G1P2, CCL21, CXCL10, et
- optionnellement, au moins un gène parmi AFP, CRP, CXCL11, CXCL6, CXCL9, FGF7, MDK, MMP2, SFN, TGFB2, VEGFD.

Ces dosages peuvent être faits dans un échantillon préalablement obtenu à partir dudit sujet.

Dans la méthode pronostique de l'invention, le nombre total des gènes ainsi choisis est de 2, 3, 4 ou 5.

Ceci étant, comme cela est présenté et illustré plus en détail ci-dessous, la méthode pronostique de l'invention peut en outre comprendre le dosage ou la mesure d'un ou plusieurs autres facteurs, notamment d'un ou plusieurs facteurs virologiques et/ou d'un ou plusieurs facteurs cliniques et/ou d'un ou plusieurs facteurs biologiques autres que les niveaux d'expression de gènes choisis parmi MBL2, LGALS3BP, IL8, G1P2, CXCL10, CCL21, AFP, CRP, CXCL11, CXCL6, CXCL9, FGF7, MDK, MMP2, SFN, TGFB2, VEGFD.

Une méthode pronostique de l'invention peut donc être définie par le fait qu'elle comprend l'étape de procéder à des mesures, qui comprennent ou sont constituées des mesures suivantes :

- dans un échantillon préalablement obtenu à partir dudit sujet, doser les niveaux auxquels des gènes choisis sont transcrits ou traduits, lesdits gènes choisis étant :

  - au moins un gène parmi MBL2, LGALS3BP, IL8, et
  - au moins un gène parmi G1P2, CCL21, CXCL10, et
  - optionnellement, au moins un gène parmi AFP, CRP, CXCL11, CXCL6, CXCL9, FGF7, MDK, MMP2, SFN, TGFB2, VEGFD,
    le nombre total des gènes ainsi choisis étant de 2, 3, 4 ou 5,

- optionnellement, mesurer ou déterminer, pour ledit sujet, la valeur d'un ou plusieurs facteurs cliniques et/ou d'un ou plusieurs facteurs virologiques et/ou d'un ou plusieurs facteurs biologiques autres que les niveaux d'expression de gènes choisis parmi MBL2, LGALS3BP, IL8, G1P2, CXCL10, CCL21, AFP, CRP, CXCL11, CXCL6, CXCL9, FGF7, MDK, MMP2, SFN, TGFB2, VEGFD.

L'invention peut être considérée comme une méthode de thérapie anti-VHC, qui comprend le fait de pronostiquer la réponse d'un sujet à un traitement anti-VHC à l'aide de la méthode pronostique de l'invention. Si ledit sujet est pronostiqué non-répondeur, le praticien peut choisir de ne pas lui administrer un traitement qui comprend (plus particulièrement qui est essentiellement constitué de) administration d'interféron et administration de ribavirine (ou de leurs pro-drogues), plus particulièrement de ne pas lui administrer un tel traitement à titre de traitement de première intention. Dans une telle situation, le praticien peut par exemple choisir d'administrer un traitement anti-VHC qui ne comprend pas (ou qui n'est pas essentiellement constitué de) administration d'interféron et administration de ribavirine (ou de leurs pro-drogues), plus particulièrement de lui administrer un tel traitement à titre de traitement de première intention. Le praticien peut alternativement choisir de ne pas lui administrer de traitement anti-VHC, à tout le moins en première intention. Si ledit sujet est pronostiqué répondeur, le praticien peut choisir d'administrer un traitement anti-VHC, notamment un traitement qui comprend (plus particulièrement qui est essentiellement constitué de) administration d'interféron et administration de ribavirine (ou de leurs pro-drogues), plus particulièrement d'administrer en première intention un traitement qui comprend (plus particulièrement qui est essentiellement constitué de) administration d'interféron et administration de ribavirine (ou de leurs pro-drogues).

Le dosage (ou la mesure) du niveau d'expression desdits gènes choisis peut être fait dans un échantillon préalablement obtenu à partir dudit sujet, tel que :

- un échantillon biologique prélevé ou collecté dudit sujet, ou
- un échantillon comprenant des acides nucléiques (notamment des ARN) et/ou protéines et/ou polypeptides et/ou peptides dudit échantillon biologique, notamment un échantillon comprenant des acides nucléiques et/ou protéines et/ou polypeptides et/ou peptides qui ont été, ou sont susceptibles d'avoir été extraits et/ou purifiés dudit échantillon biologique, ou
- un échantillon comprenant des ADNc qui ont été, ou sont susceptibles d'avoir été obtenus par transcription inverse desdits ARN.

Un échantillon biologique collecté ou prélevé dudit sujet peut par exemple être un échantillon prélevé ou collecté, ou susceptible d'être prélevé ou collecté, à partir :

- d'un organe ou tissu interne dudit sujet, notamment de son foie ou de son parenchyme hépatique, ou
- d'un fluide biologique dudit sujet, notamment un fluide intracorporel tel que le sang, le sérum, le plasma, ou de l'urine.

Un échantillon biologique collecté ou prélevé dudit sujet peut par exemple être un échantillon comprenant une portion de tissu dudit sujet, notamment une portion de tissu hépatique, plus particulièrement une portion de parenchyme hépatique.

Un échantillon biologique collecté ou prélevé dudit sujet peut par exemple être un échantillon comprenant des cellules qui ont été, ou sont susceptibles d'être, prélevées ou collectées d'un tissu dudit sujet, notamment d'un tissu hépatique, plus particulièrement des cellules hépatiques.

Un échantillon biologique collecté ou prélevé dudit sujet peut par exemple être un échantillon de fluide biologique, tel qu'un échantillon de sang, de sérum, de plasma ou d'urine, plus particulièrement un échantillon de fluide intracorporel tel qu'un échantillon de sang ou de sérum ou de plasma. En effet, les dix-sept gènes de ladite liste de l'invention codent tous pour des protéines non-membranaires, et le produit de leur expression a notamment une localisation extracellulaire.

Selon un mode de réalisation avantageux de l'invention, ledit échantillon biologique est donc un échantillon de fluide biologique dudit sujet, tel qu'un échantillon de fluide intracorporel, tel que du sang, du sérum, du plasma, ou échantillon d'urine, et les niveaux d'expression desdits gènes choisis qui sont dosés peuvent être des niveaux de traduction protéique.

Le prélèvement ou la collecte dudit échantillon biologique peut être fait par insertion d'un instrument de prélèvement, notamment par insertion d'une aiguille ou d'un cathéter, dans le corps dudit sujet. Cet instrument peut par exemple être inséré :

- dans un organe ou tissu interne dudit sujet, notamment dans son foie ou dans parenchyme hépatique, par exemple,

    - pour prélever un échantillon de foie ou de parenchyme hépatique, ce prélèvement pouvant par exemple être fait par ponction biopsique hépatique (PBH), plus particulièrement par PBH par voie transjugulaire ou par voie transpariétale, ou
    - pour prélever ou collecter des cellules à partir du compartiment hépatique (prélèvement de cellules, et non de tissu), plus particulièrement à partir du parenchyme hépatique, notamment pour prélever des cellules hépatiques, ce prélèvement ou cette collecte pouvant par exemple être fait par cyto-ponction hépatique ;

et/ou
- dans une veine, une artère ou un vaisseau dudit sujet pour prélever un fluide biologique dudit sujet, tel que du sang.

Les moyens de l'invention ne sont pas limités à une mise en oeuvre sur une biopsie de tissu, notamment de tissu hépatique. Ils peuvent être mis en oeuvre sur un échantillon obtenu, ou susceptible d'être obtenu par un prélèvement de taille ou volume nettement plus réduit qu'un échantillon de tissu, à savoir un échantillon qui se limite à quelques cellules. Les moyens de l'invention peuvent notamment être mis en oeuvre sur un échantillon obtenu, ou susceptible d'être obtenu par cyto-ponction hépatique.

La quantité ou le volume de matière prélevé par cyto-ponction hépatique est beaucoup plus réduit que celui prélevé par PBH. Outre le gain immédiat pour le patient en termes de diminution d'invasivité de la technique de prélèvement et de diminution de la morbidité associée, la cyto-ponction hépatique présente l'avantage de pouvoir être répétée à des temps distincts sur le même patient (par exemple, pour déterminer l'évolution de la fibrose hépatique entre deux instants), alors que la PBH ne peut être raisonnablement répétée sur le même patient. La cyto-ponction hépatique présente donc, contrairement à la PBH, l'avantage de permettre de suivre l'évolution clinique du patient.

Ainsi, conformément à l'invention, ledit échantillon biologique peut avantageusement être :

- un prélèvement ou une collecte de cellules à partir du compartiment hépatique (prélèvement ou collecte de cellules, et non de tissu), plus particulièrement à partir du parenchyme hépatique, c'est-à-dire un échantillon biologique obtenu, ou susceptible d'être obtenu par cyto-ponction hépatique ; et/ou
- un prélèvement ou une collecte de fluide biologique dudit sujet, tel que du sang ou de l'urine, notamment du sang.

Le dosage (ou la mesure) peut être fait dans un échantillon biologique qui a été collecté ou prélevé dudit sujet, et qui a été plus avant transformé, par exemple :

- par extraction et/ou purification des acides nucléiques, notamment des ARN, plus particulièrement des ARNm, et/ou par transcription inverse de ces ARN, notamment de ces ARNm, ou
- par extraction et/ou purification des protéines et/ou polypeptides et/ou peptides, ou par extraction et/ou purification d'une fraction protéique, telle que le sérum ou le plasma extrait du sang.

Par exemple, lorsque l'échantillon biologique collecté ou prélevé est un fluide biologique tel que du sang ou de l'urine, cet échantillon peut, avant de réaliser le dosage ou la mesure, être transformé :

- par extraction d'acides nucléiques, notamment d'ARN, plus particulièrement d'ARNm, et/ou par transcription inverse de ces ARN, notamment de ces ARNm (le plus généralement, par extraction des ARN et transcription inverse de ces ARN), ou
- par séparation et/ou extraction de la fraction sérique, ou par extraction ou purification des protéines et/ou polypeptides et/ou peptides sériques.

Ainsi, selon un mode de réalisation de l'invention, ledit échantillon obtenu à partir dudit sujet comprend (par exemple dans une solution), ou est, un échantillon de fluide biologique dudit sujet, tel qu'un échantillon de sang, de sérum, de plasma ou d'urine, et/ou est un échantillon qui comprend (par exemple dans une solution) :

- des ARN, notamment des ARNm, susceptibles d'avoir été extraits ou purifiés d'un fluide biologique, tel que du sang ou de l'urine, notamment du sang ; et/ou des ADNc susceptibles d'avoir été obtenus par transcription inverse de tels ARN ; et/ou
- des protéines et/ou polypeptides et/ou peptides susceptibles d'avoir été extraits ou purifiés d'un fluide biologique, tel que du sang ou de l'urine, notamment du sang, et/ou susceptibles d'avoir été codés par de tels ARN, de préférence,
- des protéines et/ou polypeptides et/ou peptides susceptibles d'avoir été extraits ou purifiés d'un fluide biologique, tel que du sang ou de l'urine, notamment du sang, et/ou susceptibles d'avoir été codés par de tels ARN.

Lorsque ledit échantillon obtenu à partir dudit sujet comprend un échantillon biologique obtenu, ou susceptible d'être obtenu par prélèvement d'un fluide biologique, tel que du sang ou de l'urine, ou lorsque ledit échantillon obtenu à partir dudit sujet est issu, ou susceptible d'être issu d'un tel échantillon biologique par extraction et/ou purification de molécules contenues dans cet échantillon biologique, le dosage est de préférence un dosage de protéines et/ou polypeptides et/ou peptides, plutôt qu'un dosage d'acides nucléiques.

Lorsque l'échantillon biologique collecté ou prélevé est un échantillon comprenant une portion de tissu, notamment une portion de tissu hépatique, plus particulièrement une portion de parenchyme hépatique, tel que par exemple un échantillon biologique prélevé ou susceptible d'être prélevé par ponction biopsique hépatique (PBH), ou lorsque l'échantillon biologique collecté ou prélevé est un échantillon comprenant des cellules obtenues, ou susceptibles d'être obtenues à partir d'un tel tissu, tel que par exemple un échantillon collecté, ou susceptible d'être collecté, par cyto-ponction hépatique, cet échantillon biologique peut être transformé :

- par extraction d'acides nucléiques, notamment d'ARN, plus particulièrement d'ARNm, et/ou par transcription inverse de ces ARN, notamment de ces ARNm (le plus généralement, par extraction de ces ARN et transcription inverse de ces ARN), ou

- par séparation et/ou extraction des protéines et/ou polypeptides et/ou peptides.

Une étape de lyse des cellules, notamment de lyse des cellules hépatiques, contenues dans ledit échantillon biologique, peut être préalablement réalisée de sorte à rendre acides nucléiques, ou le cas échéant, protéines et/ou polypeptides et/ou peptides, directement accessibles à l'analyse.

Ainsi, selon un mode de réalisation de l'invention, ledit échantillon obtenu à partir dudit sujet est un échantillon de tissu dudit sujet, notamment de tissu hépatique, plus particulièrement de parenchyme hépatique, ou est un échantillon de cellules d'un tel tissu, et/ou est un échantillon qui comprend (par exemple dans une solution):

- des cellules hépatiques, plus particulièrement des cellules du parenchyme hépatique, par exemple des cellules obtenues, ou susceptibles d'être obtenues par dissociation des cellules d'une biopsie de tissu hépatique ou par cyto-ponction hépatique; et/ou
- des ARN, notamment des ARNm, susceptibles d'avoir été extraits ou purifiés de telles cellules ; et/ou
- des ADNc susceptibles d'avoir été obtenus par transcription inverse de tels ARN ; et/ou
- des protéines et/ou polypeptides et/ou peptides susceptibles d'avoir été extraits ou purifiés de telles cellules, et/ou susceptibles d'avoir été codés par de tels ARN.

Conformément à l'invention, ledit sujet est un humain ou un animal non-humain, notamment un humain ou un mammifère non-humain, plus particulièrement un humain.

Du fait de la sélection particulière de gènes proposée par l'invention, le statut de répondeur ou de non-répondeur dudit sujet peut être déduit, ou déterminé à partir, des valeurs des mesures obtenues pour ledit sujet, notamment par induction statistique et/ou classification statistique, par exemple vis-à-vis de cohortes de référence (pré-)établies selon leur statut de répondeur ou de non-répondeur.

En sus du fait de doser (ou mesurer) le niveau auquel des gènes choisis s'expriment dans l'organisme dudit sujet, une méthode de l'invention peut donc en outre comprendre une étape de déduction ou détermination du statut de répondeur ou de non-répondeur dudit sujet à partir des valeurs des mesures obtenues pour ledit sujet. Cette étape de déduction ou détermination est une étape par laquelle les valeurs des mesures obtenues pour ledit sujet sont analysées pour en induire le statut de répondeur ou de non-répondeur dudit sujet.

La déduction ou détermination du statut de répondeur ou de non-répondeur dudit sujet peut être faite en comparant les valeurs des mesures obtenues pour ledit sujet à leurs valeurs, ou à la distribution de leurs valeurs, dans des cohortes de référence pré-établies selon leur statut de répondeur ou non-répondeur au traitement anti-VHC, pour classer ledit sujet dans celle de ces cohortes de référence vis-à-vis de laquelle il a la plus forte probabilité d'appartenance (c'est-à-dire pour attribuer audit sujet son statut de répondeur ou de non-répondeur). Les individus qui composent ces cohortes sont des individus, pour lesquels il a été établi, par application du traitement anti-VHC, qu'ils sont répondeurs ou non-répondeurs à ce traitement.

Les dosages effectués sur ledit sujet et sur les individus des cohortes ou sous-populations de référence sont des dosages de niveaux d'expression (transcription ou traduction) de gènes.

Pour doser le niveau de transcription d'un gène, on dose son niveau de transcription en ARN. Un tel dosage peut par exemple comprendre la mesure de la concentration en ARN transcrits de chacun desdits gènes choisis, soit par mesure de la concentration de ces ARN, soit par mesure de la concentration des ADNc obtenus par transcription inverse de ces ARN. Le dosage d'acides nucléiques est bien connu de la personne du métier. Par exemple, le dosage d'ARN ou des ADNc correspondants peut se faire par amplification d'acide nucléique, notamment par PCR. Des réactifs sont décrits ci-dessous à cet effet (*cf*. exemple 1 ci-dessous). Des exemples d'amorces et sondes appropriés en sont également donnés (*cf*. par exemple tableau 32 ci-dessous). Les conditions d'amplification d'acides nucléiques peuvent être choisies par la personne du métier. Des exemples de conditions d'amplification sont donnés dans la partie « exemples » qui suit (*cf*. exemple 1 ci-dessous).

Pour doser le niveau de traduction d'un gène, on dose son niveau de traduction en protéines. Un tel dosage peut par exemple comprendre la mesure de la concentration en protéines traduites à partir de chacun desdits gènes choisis (par exemple, dosage des protéines dans la circulation générale, notamment dans le sérum). Le dosage de protéines est bien connu de la personne du métier. Par exemple, les protéines (et/ou polypeptides et/ou peptides) peuvent être dosées par ELISA ou toute autre méthode immunométrique connue de la personne du métier, ou par une méthode utilisant la spectrométrie de masse connue de la personne du métier.

Les valeurs de dosage sont des valeurs de concentration ou de proportion, ou des valeurs qui représentent une concentration ou une proportion. L'objectif est qu'au sein d'une même combinaison, les valeurs de dosage des niveaux d'expression de chacun desdits gènes choisis reflètent le plus précisément possible, à tout le moins l'une par rapport à l'autre, le degré auquel chacun de ces gènes est exprimé (degré de transcription ou degré de traduction), notamment en étant proportionnelles à ces degrés respectifs.

Par exemple, dans le cas du dosage du niveau d'expression d'un gène par dosage des ARN transcrits, c'est-à-dire dans le cas du dosage du niveau de transcription de ce gène, le dosage se fait généralement par amplification des ARN par transcription inverse et PCR (RT-PCR), et par mesure de valeurs de Ct (*Cycle threshold*).

Une valeur de Ct donne une mesure de la quantité initiale des ARN amplifiés (plus la valeur de Ct est faible, plus la quantité de ces acides nucléiques est élevée). Les valeurs de Ct mesurées pour un ARN cible ($Ct_{cible}$) sont généralement rapportées à la quantité d'ARN total initialement présente dans l'échantillon, par exemple en déduisant de ce $Ct_{cible}$ la valeur d'un Ct de référence ($Ct_{référence}$), telle que la valeur de Ct qui a été mesurée dans les mêmes conditions opératoires pour l'ARN d'un gène contrôle endogène dont le niveau d'expression est stable (par exemple un gène impliqué dans une cascade métabolique cellulaire, tel que RPLPO ou TBP ; *cf.* exemple 1 ci-dessous).

Selon un mode de réalisation de l'invention, la différence ($Ct_{cible}$ - $Ct_{référence}$), ou $\Delta Ct$, peut en outre être exploitée par la méthode connue sous le nom de méthode du $2^{-\Delta Ct}$ (Livak et Schmittgen 2001 ; Schmittgen et Livak 2008), sous la forme :

$$2^{-\Delta Ct} = 2^{-(Ct\ cible\ -\ Ct\ référence)}.$$

Ainsi, selon un mode de réalisation de l'invention, le dosage des niveaux auxquels chacun desdits gènes choisis sont transcrits est réalisée par :

- amplification, d'un fragment des ARN transcrits par chacun desdits gènes choisis, par exemple par transcription inverse et PCR de ces fragments d'ARN, pour obtenir les valeurs de Ct de chacun de ces ARN,
- optionnellement, normalisation de chacune de ces valeurs de Ct par rapport à la valeur de Ct obtenue pour l'ARN d'un gène contrôle endogène, tel que RPLPO ou TBP, par exemple par la méthode du $2^{-\Delta Ct}$
- optionnellement, transformation Box-Cox desdites valeurs normalisées de Ct.

Dans le cas du dosage du niveau d'expression d'un gène par dosage des protéines exprimées par ce gène, c'est-à-dire dans le cas du dosage du niveau de traduction de ce gène, le dosage se fait généralement par une méthode immunométrique à l'aide d'anticorps spécifiques, et par expression des mesures ainsi faites en quantités pondérales ou en unités internationales, à l'aide d'une gamme étalon. Des exemples d'anticorps spécifiques sont indiqués dans le tableau 29 ci-dessous. Des exemples de moyens de dosage protéique sont donnés en tableau 44 ci-dessous. Une valeur de dosage du niveau de traduction d'un gène peut par exemple être exprimée en quantité de cette protéine par volume de fluide biologique, par exemple, par volume de sérum (par exemple, en mg/mL ou en $\mu$g/mL ou en ng/mL ou en pg/mL).

Si souhaité ou requis, la distribution des valeurs de dosage obtenues sur les individus d'une cohorte peut être lissée, de sorte à la rapprocher d'une loi gaussienne.

A cet effet, les valeurs de dosage obtenues sur les individus de cette cohorte, par exemple les valeurs obtenues par la méthode du $2^{-\Delta Ct}$, peuvent être transformées par une transformation de type Box-Cox (Box et Cox, 1964 ; *cf.* tableaux 5, 10, 15, 19, 23 et 27 ci-dessous ; *cf.* exemples 2 à 4 ci-dessous).

L'invention est donc notamment relative à une méthode *in vitro* pour pronostiquer si un sujet infecté par un ou des VHC a une forte probabilité d'être répondeur à un traitement anti-VHC qui comprendra l'administration d'interféron et de ribavirine, ou si, au contraire, ce sujet a une forte probabilité de ne pas être répondeur à ce traitement anti-VHC, ladite méthode comprenant les étapes suivantes :

i) procéder aux mesures suivantes :

- dans un échantillon préalablement obtenu à partir dudit sujet, doser les niveaux auxquels des gènes de mammifère choisis sont transcrits ou traduits, lesdits gènes choisis étant :

  - au moins un gène parmi MBL2, LGALS3BP, IL8, et
  - au moins un gène parmi G1P2, CCL21, CXCL10, et
  - optionnellement, au moins un gène parmi AFP, CRP, CXCL11, CXCL6, CXCL9, FGF7, MDK, MMP2, SFN, TGFB2, VEGFD,
    le nombre total des gènes ainsi choisis étant de 2, 3, 4 ou 5,

- doser les niveaux de transcription ou de traduction de 0 à 4 gènes de mammifère autres que les gènes de mammifère choisis parmi MBL2, LGALS3BP, IL8, G1P2, CXCL10, CCL21, AFP, CRP, CXCL11, CXCL6, CXCL9, FGF7, MDK, MMP2, SFN, TGFB2, VEGFD,
- optionnellement, mesurer ou déterminer, pour ledit sujet, la valeur d'un ou plusieurs facteurs cliniques et/ou d'un ou plusieurs facteurs virologiques et/ou d'un ou plusieurs facteurs biologiques autres que les niveaux d'expression de gènes choisis parmi MBL2, LGALS3BP, IL8, G1P2, CXCL10, CCL21, AFP, CRP, CXCL11, CXCL6, CXCL9, FGF7, MDK, MMP2, SFN, TGFB2, VEGFD,

ii) comparer les valeurs des mesures obtenues pour ledit sujet à l'étape i), à leurs valeurs, ou à la distribution de leurs valeurs, dans des cohortes de référence pré-établies selon leur statut de répondeur ou non-répondeur au traitement anti-VHC, pour classer ledit sujet dans celle de ces cohortes de référence vis-à-vis de laquelle il a la plus forte probabilité d'appartenance.

La comparaison de l'étape ii) peut notamment être faite en combinant les valeurs de dosage (ou de mesure) obtenues pour ledit sujet dans un modèle de classification multivariée.

Un tel modèle de classification multivariée compare (de manière combinée) les valeurs des mesures obtenues pour ledit sujet à leurs valeurs, ou à la distribution de leurs valeurs, dans des cohortes de référence pré-établies selon leur statut de répondeur ou non-répondeur au traitement anti-VHC, pour classer ledit sujet dans celle de ces cohortes de référence vis-à-vis de laquelle il a la plus forte probabilité d'appartenance, par exemple en lui attribuant une valeur de sortie indicatrice du statut de répondeur ou de non-répondeur dudit sujet.

Un tel modèle de classification multivariée peut être construit, notamment préalablement construit, en faisant une comparaison inter-cohorte des valeurs de mesures obtenues pour lesdites cohortes de référence ou des distributions de ces valeurs de mesures.

Plus particulièrement, un tel modèle de classification multivariée peut être construit, notamment préalablement construit, en dosant ou mesurant les niveaux d'expression desdits gènes choisis dans des cohortes de référence pré-établies selon leur statut de répondeur ou non-répondeur au traitement anti-VHC, et en analysant ces valeurs de dosage, ou leur distribution, par une méthode statistique multivariée pour construire un modèle de classification multivariée qui induit ou détermine un statut de répondeur ou non-répondeur au traitement anti-VHC à partir des valeurs de niveaux d'expression desdits gènes choisis.

Si, en sus des valeurs de dosage des niveaux de transcription ou de traduction desdits gènes choisis, les valeurs dosées pour ledit sujet comprennent la ou les valeurs d'un ou plusieurs autres facteurs, tels qu'un ou plusieurs facteurs virologiques et/ou un ou plusieurs facteurs cliniques et/ou un ou plusieurs des autres facteurs biologiques (*cf*. ci-dessous et dans les exemples), le modèle de classification est bien sûr construit, notamment préalablement construit, en dosant ou mesurant les mêmes valeurs dans des cohortes de référence pré-établies selon leur statut de répondeur ou non-répondeur au traitement anti-VHC, et en analysant ces valeurs, ou leur distribution, par une méthode statistique multivariée pour construire un modèle de classification multivariée qui induit ou détermine un statut de répondeur ou non-répondeur au traitement anti-VHC à partir de ces valeurs.

Par exemple, un modèle peut être construit par une fonction mathématique, une technique non paramétrique, une procédure de classification heuristique ou encore une approche de prédiction probabiliste. Un exemple typique de classification fondée sur la quantification du niveau d'expression de biomarqueurs consiste en la discrimination de sujets "sains" versus "malades". La formalisation de ce problème consiste en m échantillons indépendants, décrits par $n$ variables aléatoires. Chaque individu $i$ ($i=1,...,m$) est caractérisé par un vecteur $x_i$ décrivant les $n$ valeurs caractéristiques : $x_{ij}$, $i=1,...m$ $j=1,...n$

Ces valeurs caractéristiques peuvent par exemple représenter des valeurs d'expression génique et/ou des intensités de données protéiques et/ou des intensités de données métaboliques et/ou des données cliniques.

Chaque échantillon $x_i$ est associé à une valeur discrète $y_i$, représentant le statut clinique de l'individu $i$. A titre d'exemple, $y_i$ = 0 si le patient $i$ a un statut de non-répondeur au traitement anti-VHC, $y_i$ = 1 si le patient $i$ a un statut de répondeur au traitement anti-VHC. Un modèle offre une règle décisionnelle (par exemple, une fonction mathématique, un algorithme ou une procédure), qui utilise l'information disponible depuis $x_i$ pour prédire $y_j$ dans chaque échantillon observé. L'objectif est d'utiliser ce modèle afin de prédire le statut clinique du patient $p$, à savoir $y_p$, à partir des valeurs biologiques et/ou cliniques disponibles, à savoir $x_p$.

Différents modèles de classification multivariée sont connus de la personne du métier (*cf*. Hastie, Tibishirani et Friedman, 2009 ; Falissard, 2005 ; Theodoridis et Koutroumbos 2009).

Ils sont généralement construits par traitement et interprétation des données selon, par exemple :

- une méthode d'analyse statistique multivariée, par exemple :

    ◦ une fonction mathématique linéaire ou non linéaire, notamment une fonction mathématique linéaire, tel qu'une fonction générée par la méthode mROC (méthode ROC multivariée), ou
    ◦ une méthode ROC (*Receiver Operating Characteristics*) ;
    ◦ une méthode de régression, linéaire ou non linéaire, comme par exemple la régression logistique ;
    ◦ une méthode PLS-DA (*Partial Least Squares - Discriminant Analysis*) ;
    ◦ une méthode LDA (*Linear Discriminant Analysis*) ;

- une méthode par apprentissage ou intelligence artificielle, par exemple, un algorithme d'apprentissage ou d'intelligence artificielle, une méthode de classification non paramétrique, ou heuristique, ou de prédiction probabiliste, telle que :

  ◦ un arbre décisionnel ; ou
  ◦ une méthode de type *boosting,* fondée sur des classifieurs binaires (ex : *Adaboost*) ou une méthode liée au *boosting* (*bagging*) ; ou
  ◦ une méthode des k plus proches voisins (*k-nearest neighbors* ou KNN), ou plus généralement la méthode des k plus proches voisins pondérés (*weighed k-nearest neighbors* ou WKNN), ou
  ◦ une méthode (par exemple, un algorithme) Machines à Vecteurs de support (*Support Vector Machine* ou SVM) ; ou
  ◦ une forêt aléatoire (*Random Forest* ou RF); ou
  ◦ un réseau bayésien ; ou
  ◦ un réseau de neurones (*Neural Network*) ; ou
  ◦ un treillis de Galois (ou *Formal Concept Analysis*).

Les règles décisionnelles des modèles de classification multivariée peuvent par exemple être fondées sur une formule mathématique du type $y = f(x_1, x_2, ... x_n)$ où $f$ est une fonction mathématique linéaire ou non linéaire (régression logistique, mROC, par exemple), ou sur un algorithme d'apprentissage automatique ou d'intelligence artificielle dont les caractéristiques consistent en une série de paramètres de réglage identifiés comme étant les plus efficaces pour la discrimination des sujets (par exemple, KNN, WKNN, SVM, RF).

La méthode ROC multivariée (mROC) est une généralisation de la méthode ROC (*Receiver Operating Characteristic*) (*cf*. Reiser et Faraggi 1997 ; Su et Liu 1993, Shapiro, 1999). Elle calcule l'aire sous la courbe ROC (*Area Under the Curve* ou AUC) relative à une combinaison linéaire de biomarqueurs et/ou de transformations de biomarqueurs (dans le cas d'une normalisation), sous l'hypothèse d'une distribution normale multivariée. La méthode mROC a notamment été décrite dans Kramar *et al.* 1999 et Kramar *et al.* 2001. Référence est également faite aux exemples ci-dessous, notamment au point 2 de l'exemple 1 ci-dessous (modèle mROC).

Le logiciel mROC version 1.0, disponible commercialement auprès des concepteurs (A. Kramar, A. Fortune, D. Farragi et B. Reiser) peut par exemple être utilisé pour construire un modèle mROC.

Andrew Kramar et Antoine Fortune peuvent être contactés auprès de, ou via, l'Unité de Biostatistique du Centre Régional de Lutte contre le Cancer (CRLC) Val d'Aurelle - Paul Lamarque (208, rue des Apothicaires ; Parc Euromédecine ; 34298 Montpellier Cedex 5 ; France).

David Faraggi et Benjamin Reiser peuvent être contactés auprès du, ou via le, Département de Statistique de l'Université de Haifa (Mount Carmel ; Haifa 31905 ; Israël).

La famille des méthodes d'intelligence artificielle ou d'apprentissage automatique est une famille d'algorithmes qui, au lieu de procéder à une généralisation explicite, comparent les exemples d'un nouveau problème avec les exemples considérés en apprentissage et qui ont été stockés en mémoire. Ces algorithmes construisent directement les hypothèses à partir des exemples d'apprentissage eux-mêmes. Un exemple simple de ce type d'algorithme est l'algorithme des *k* plus proches voisins (*k-nearest neighbors* ou KNN), et une de ses extensions possibles connue sous le nom d'algorithme des k plus proches voisins pondérés (*weighted k nearest neighbors* ou WKNN) (Hechenbichler et Schliep, 2004).

Dans le contexte de classification d'une nouvelle observation x, l'idée fondatrice simple est de faire voter les plus proches voisins de cette observation. La classe (ou statut clinique) de *x* est déterminée en fonction de la classe majoritaire parmi les *k* plus proches voisins de l'observation *x*.

Des bibliothèques de fonctions spécifiques KKNN sont disponibles par exemple dans le logiciel R (http://www. R-project.org/). Le logiciel R a été initialement développé par John Chambers et les laboratoires Bell (*cf*. Chambers 2008). La version actuelle de cette suite logicielle est la version 2.11.1. Le code source est disponible librement sous les termes de la licence publique générale « Free Software Foundation's GNU », sur le site http://www. R-project.org/. Ce logiciel peut être utilisé pour construire un modèle WKNN. Référence est également faite aux exemples ci-dessous, notamment au point 2 de l'exemple 1 ci-dessous (modèle WKNN).

Une Forêt Aléatoire (*Random Forest* ou RF) est constituée d'un ensemble d'arbres simples de prévision, chacun étant capable de produire une réponse lorsqu'on lui présente un sous-ensemble de prédicteurs (Breiman 2001 ; Liaw et Wiener 2002). Les calculs sont réalisés avec le logiciel R. Ce logiciel peut être utilisé pour construire des modèles RF. Référence est également faite aux exemples ci-dessous, notamment au point 2 de l'exemple 1 ci-dessous (modèle RF).

Un réseau de neurones est constitué d'un graphe pondéré orienté dont les noeuds symbolisent les neurones. Le réseau est construit à partir d'exemples de chaque classe (par exemple, F2 versus F1), et est ensuite utilisé pour déterminer à quelle classe appartient un nouvel élément ; *cf*. Intrator et Intrator 1993, Riedmiller et Braun 1993, Riedmiller 1994, Anastasiadis *et. al.* 2005 ; *cf*. http://cran.r-project.org/web/packages/neuralnet/index.html. Le logiciel R librement disponible sur http://www.r-project.org/, (version 1.3 de *Neuralnet,* écrit par Stefan Fritsch et Frauke Guenther, suivant le travail de Marc Suling) peut par exemple être utilisé pour construire un réseau de neurones.

Référence est également faite aux exemples ci-dessous, notamment au point 2 de l'exemple 1 ci-dessous (modèle NN).

La comparaison de ladite étape ii) peut donc être notamment réalisée en suivant une méthode, et/ou en utilisant un algorithme ou un logiciel :

- mROC,
- KNN, WKNN, plus particulièrement WKNN,
- RF, ou
- NN,

plus particulièrement mROC.

Chacun de ces algorithmes, logiciels ou méthodes permet de construire un modèle de classification multivariée, à partir des valeurs de mesures de chacune desdites cohortes de référence, et de combiner les valeurs de mesures obtenues sur ledit sujet dans ce modèle pour en induire son statut de répondeur ou de non-répondeur.

Selon un mode de réalisation de l'invention, le modèle de classification multivariée mis en oeuvre dans la méthode de l'invention est exprimé par une fonction mathématique, linéaire ou non linéaire, plus particulièrement une fonction linéaire (par exemple, un modèle mROC). Le statut de répondeur ou de non-répondeur dudit sujet est alors induit par combinaison desdites valeurs de mesures obtenues pour ledit sujet dans cette fonction mathématique, notamment une fonction linéaire ou non linéaire, pour obtenir une valeur de sortie, plus particulièrement une valeur numérique de sortie, indicatrice du statut de répondeur ou de non-répondeur dudit sujet.

Selon un mode de réalisation de l'invention, le modèle de classification multivariée mis en oeuvre dans la méthode de l'invention est un modèle par apprentissage ou intelligence artificielle, un modèle de classification non paramétrique, ou heuristique, ou de prédiction probabiliste (par exemple, un modèle WKNN, RF ou NN). Le statut de répondeur ou de non-répondeur dudit sujet est alors induit par combinaison desdites valeurs de mesures obtenues pour ledit sujet dans un modèle de classification non paramétrique, ou heuristique, ou de prédiction probabiliste (par exemple, un modèle WKNN, RF ou NN), pour obtenir une valeur de sortie, plus particulièrement une étiquette de sortie, indicatrice du statut de répondeur ou de non-répondeur dudit sujet.

De manière alternative ou complémentaire, ladite comparaison de l'étape ii) peut comprendre le fait de comparer les valeurs des mesures obtenues pour ledit sujet, à au moins une valeur de référence qui discrimine entre un statut de répondeur ou de non-répondeur, pour classer ledit sujet dans le groupe des individus répondeurs ou dans le groupes des individus non-répondeurs.

Par exemple, les valeurs des mesures peuvent être comparées à leurs valeurs de référence dans :

- une sous-population d'individus qui sont de la même espèce que ledit sujet, qui sont infectés par le VHC, auxquels le traitement anti-VHC a été administré, et qui se sont montrés répondeurs à ce traitement, et/ou
- une sous-population d'individus de sujets qui sont de la même espèce que ledit sujet, qui sont infectés par le VHC, auxquels le traitement anti-VHC a été administré, et qui se sont montrés non-répondeurs à ce traitement,

ou à une valeur de référence qui représente la combinaison de ces valeurs de référence. Une valeur de référence peut par exemple être :

- la valeur du dosage du niveau d'expression de chacun desdits gènes choisis dans chacun des individus pour chacune des sous-populations ou cohortes de référence, ou
- un critère de position, par exemple la moyenne ou la médiane, ou un quartile, ou le minimum, ou le maximum de ces valeurs dans chacune de ces sous-populations ou cohortes de référence, ou
- une combinaison de ces valeurs ou moyennes. ou médiane, ou quartile, ou minimum, ou maximum.

La ou les valeurs de référence utilisées doivent permettre de discriminer le statut de répondeur de celui de non-répondeur.

Il peut par exemple s'agir d'un seuil de décision ou de prédiction, établi en fonction de la distribution des valeurs des mesures dans chacune desdites sous-populations ou cohortes, et en fonction des niveaux de sensibilité (Se) et de spécificité (Spe) fixés par l'utilisateur (Se = VP / (VP + FN) et Sp = VN / (VN + FP), avec VP = le nombre de vrais positifs, FN = le nombre de faux négatifs, VN = le nombre de vrais négatifs, FP = le nombre de faux positifs). Ce seuil de décision ou prédiction peut notamment être un seuil optimal qui attribue un poids équitable à la sensibilité (Se) et à la spécificité (Spe), tel que le seuil maximisant l'indice de Youden (J) défini par J = Se + Spe -1.

Alternativement ou complémentairement, il peut y avoir comparaison à plusieurs valeurs de référence. C'est notamment le cas lorsque les valeurs des mesures obtenues pour ledit sujet sont comparées à leurs valeurs dans chacune desdites sous-populations ou cohortes de référence, par exemple à l'aide d'une méthode de classification par apprentissage automatique ou par intelligence artificielle.

Ainsi, la comparaison de l'étape ii) peut par exemple être faite comme suit :

- choisir les niveaux de sensibilité (Se) et spécificité (Spe) que l'on souhaite donner à la méthode,
- établir une fonction mathématique, linéaire ou non linéaire, notamment une fonction mathématique linéaire (par exemple, par la méthode mROC), à partir des valeurs de dosage desdits gènes dans chacune desdites sous-populations ou cohortes, et calculer le seuil de décision ou prédiction associé à cette fonction du fait des choix de niveaux de sensibilité (Se) et spécificité (Spe) faits (par exemple, en calculant le seuil maximisant l'indice de Youden),
- combiner les valeurs de dosage obtenues pour ledit sujet dans cette fonction mathématique, pour obtenir une valeur de sortie, qui, comparée audit seuil de décision ou prédiction, permet d'attribuer un statut de répondeur ou un statut de non-répondeur audit sujet, c'est-à-dire de classer ledit sujet dans celle de ces sous-populations ou cohortes de référence, vis-à-vis de laquelle il a la plus forte probabilité d'appartenance.

L'invention repose notamment sur la démonstration que, lorsqu'ils sont pris en combinaison, les niveaux d'expression de :

- au moins un gène parmi MBL2, LGALS3BP, IL8, et
- au moins un gène parmi G1P2, CCL21, CXCL10, et
- optionnellement, au moins un gène parmi AFP, CRP, CXCL11, CXCL6, CXCL9, FGF7, MDK, MMP2, SFN, TGFB2, VEGFD,

sont des biomarqueurs qui donnent une « signature » prédictive du statut de répondeur ou de non-répondeur.

La personne du métier disposant d'une combinaison de gènes décrite par l'invention est en mesure de construire un modèle de classification multivariée, notamment un modèle d'analyse statistique multivariée (par exemple une fonction mathématique linéaire ou non linéaire) ou un modèle d'apprentissage ou d'intelligence artificielle (par exemple, un algorithme d'apprentissage ou d'intelligence artificielle), à l'aide de ses connaissances générales dans le domaine des techniques et moyens statistiques, notamment dans le domaine du traitement et de l'interprétation statistiques de données, plus particulièrement de données biologiques.

Un modèle de classification multivariée peut par exemple être construit, notamment préalablement construit, comme suit :

a) pour une population d'individus qui sont de la même espèce que ledit sujet, et qui sont infectés par un ou des VHC, déterminer pour chacun de ces individus, s'il répond ou ne répond pas à un traitement anti-VHC qui comprend l'administration d'interféron et de ribavirine, et classer ces individus en sous-populations distinctes selon qu'ils se sont montrés répondeurs ou non-répondeurs à ce traitement, constituant ainsi des cohortes de référence établies selon la réponse ou non-réponse de ces individus au traitement anti-VHC ;
b) dans au moins un échantillon qui a été préalablement obtenu à partir de chacun desdits individus, dont la nature est identique à celle de l'échantillon dudit sujet, procéder aux mêmes mesures que celles faites pour ledit sujet à ladite étape i) ;
c) faire une comparaison inter-cohorte des valeurs des mesures obtenues à l'étape b), ou de la distribution de ces valeurs, pour construire un modèle de classification multivariée, qui induit un statut de répondeur au traitement anti-VHC ou un statut de non-répondeur à ce traitement, à partir de la combinaison desdites valeurs de mesures obtenues à l'étape b).

Si ledit ou lesdits sujets dont on cherche à déterminer le statut de répondeur ou de non-répondeur présentent cette fibrose du fait d'une affection chronique hépatique particulière connue, par exemple, du fait d'une infection par un génotype particulier de VHC, on utilise avantageusement des individus de situation clinique comparable. Les individus sont par ailleurs choisis de sorte à constituer une cohorte statistiquement acceptable, ne présentant pas de biais particulier, notamment pas de biais clinique particulier. L'objectif est de construire un modèle de classification multivariée qui soit le plus pertinent possible d'un point de vue statistique.

De préférence, les cohortes ou sous-populations d'individus comprennent le plus d'individus possibles. Si le nombre d'individus est trop faible, la comparaison ou le modèle construit peut ne pas être suffisamment fiable et généralisable en vue des applications médicales visées.

Notamment, on utilisera des cohortes ou sous-populations qui comprennent chacune au moins 30 individus, par exemple au moins 40 individus, de préférence au moins 50 individus, plus particulièrement au moins 70 individus, de manière encore plus particulière au moins 100 individus.

De préférence, un nombre comparable d'individus est présent dans chaque cohorte ou sous-population. Par exemple, le nombre d'individus d'une cohorte ou sous-population ne dépasse pas le seuil de 3 fois le nombre d'individus d'une autre cohorte, plus particulièrement le seuil de 2,5 fois le nombre d'individus d'une autre cohorte.

Lorsque l'analyse statistique menée utilise une fonction mathématique, telle que par exemple dans le cas d'une méthode mROC, le nombre d'individus requis par cohorte peut éventuellement être de l'ordre de 20 à 40 individus par cohorte de référence. Dans le cas d'une méthode d'analyse par apprentissage automatique, telle qu'une méthode KNN, WKNN, RF ou NN, il est préférable d'avoir au moins 30 individus par cohorte, de préférence au moins 70 individus, de manière encore plus particulière au moins 100 individus. Dans les exemples qui suivent, le nombre total d'individus compris dans l'ensemble des cohortes est de plus de 120.

Les individus qui composent les cohortes de référence sont des individus qui ont reçu un traitement anti-VHC, et dont le statut de répondeur ou de non-répondeur a été déterminé après application de ce traitement, notamment par mesure de la charge en VHC de ces individus à l'issue du traitement, et si cette charge est alors devenue indétectable, 6 mois après traitement.

Pour déterminer le statut de répondeur ou de non-répondeur d'un individu, et par suite, affecter cet individu à une cohorte de référence, la personne du métier peut mettre en oeuvre tout moyen qu'elle juge approprié. Le test de quantification de l'ARN de VHC VERSANT® HCV-RNA 3.0 (bDNA) ASSAY de la société Siemens Healthcare Diagnostics (limite de quantification = 615 - 7 690 000 UI/mL) est un exemple de moyen permettant de mesurer la charge virale, et de déterminer si cette charge est devenue indétectable à l'issue du traitement et le reste 6 mois après traitement (individus répondeurs), ou si cette charge est toujours détectable à l'issue du traitement (individus non-répondeurs).

Bien qu'il faille bien sûr limiter le nombre d'échantillons pris sur un même individu, notamment en cas de ponction biopsie hépatique, plusieurs échantillons peuvent être collectés sur un même individu. Dans ce cas, les résultats de dosage des différents échantillons d'un même individu sont considérés dans leur moyenne résultante ; il n'est pas considéré qu'ils puissent être équivalents à des valeurs de dosage obtenues sur des individus distincts.

La comparaison des valeurs des mesures dans chacune desdites cohortes peut se faire par tout moyen connu de la personne du métier. Elle se fait généralement par traitement et interprétation statistiques de ces valeurs. Cette comparaison statistique multivariée permet de construire un modèle de classification multivariée qui induit une valeur de statut de répondeur ou de non-répondeur à partir de la combinaison de ces valeurs.

Une fois que ledit modèle de classification multivariée est construit, il peut être utilisé pour l'analyse des valeurs des mesures obtenues pour ledit sujet, et surtout être ré-utilisé pour l'analyse des valeurs des mesures d'autres sujets. Ainsi, le ledit modèle de classification multivariée peut être établi de manière indépendante des mesures effectuées pour ledit ou lesdits sujets, et peut être préalablement construit.

Si besoin en était, plutôt que de constituer des cohortes et réunir les données des individus qui les constituent, la personne du métier peut, pour construire des exemples de modèles de classification multivariée conformes à l'invention, utiliser, à titre d'individus de cohortes, les sujets qui sont décrits en partie exemples ci-dessous, et peut, à titre de données d'individus de cohortes, utiliser les données qui sont présentées pour ces sujets dans les exemples ci-dessous, plus particulièrement en tableaux 34 à 36 ci-dessous.

De préférence, ledit modèle de classification multivariée est un système particulièrement discriminant. Avantageusement, ledit modèle de classification multivariée présente une aire sous la courbe ROC (ou AUC) et/ou une valeur d'erreur LOOCV particulière(s). L'acronyme « AUC » désigne *Area Under the Curve,* c'est-à-dire l'aire sous la courbe ROC (*Receiver Operating Characteristic*). L'acronyme « LOOCV » désigne *Leave-One-Out-Cross-Validation, cf.* Hastie, Tibishirani et Friedman, 2009.

La caractéristique d'AUC vient notamment s'appliquer aux modèles de classification multivariée, qui sont définis par une fonction mathématique, comme par exemple les modèles utilisant une méthode mROC de classification.

Les modèles d'intelligence artificielle ou d'apprentissage automatique multivariés ne sont quant à eux pas à proprement parler définis par une fonction mathématique. Néanmoins, dès lors qu'ils impliquent un seuil décisionnel (*threshold*), ils peuvent être appréhendés au moyen d'une courbe ROC, et donc d'un calcul d'AUC. C'est par exemple le cas des modèles utilisant une méthode RF (*Random Forest*). En effet, dans le cas de la méthode RF, une courbe ROC peut être calculée à partir des prédictions des échantillons OOB (*Out-Of-Bag*, ou « hors-du-sac » selon la traduction en langue française).

Ceux des modèles d'intelligence artificielle ou d'apprentissage automatique multivariés, qui ne pourraient être caractérisés par une valeur d'AUC, peuvent par contre, comme tous les autres modèles d'intelligence artificielle ou d'apprentissage automatique multivariés, être caractérisés par la valeur du paramètre « erreur de classification » qui leur est associée, telle que par exemple la valeur de l'erreur LOOCV.

Ladite valeur particulière d'AUC peut notamment être d'au moins 0,76, d'au moins 0,77, d'au moins 0,78, plus particulièrement d'au moins 0,79, encore plus particulièrement d'au moins 0,80, d'au moins 0,81, d'au moins 0,82, d'au moins 0,83, d'au moins 0,84, plus particulièrement d'au moins 0,85, encore plus particulièrement d'au moins 0,86, encore plus particulièrement d'au moins 0,87, par exemple d'au moins 0,88, 0,89 ou 0,90 (de préférence, avec un intervalle de confiance à 95% à au plus ± 11%, plus particulièrement à moins de ± 10,5%, encore plus particulièrement à moins de

$\pm$ 9,5%, notamment à moins de $\pm$ 8,5%) ; *cf.* par exemple, combinaisons n°1 à n°43 en tableaux 6, 11, 16, 20, 24, 28 ci-dessous.

Avantageusement, ladite valeur particulière d'erreur LOOCV est d'au plus 18%, d'au plus 17%, d'au plus 16%, d'au plus 15%, d'au plus 14%, d'au plus 13%, d'au plus 12%, d'au plus 11%, au plus 10%, au plus 9%, au plus 8%, au plus 7%, au plus 6%, au plus 5%, au plus 4%, au plus 3%, au plus 2%, au plus 1% (*cf.* par exemple, combinaisons n°1 à 8, 10 à 14, 16 à 29 en tableau 13 ci-dessous).

Les performances diagnostiques d'un biomarqueur sont généralement caractérisées selon au moins un des deux indices suivants :

- la sensibilité (Se), qui représente sa capacité à détecter la population dite "pathologique" constituée d'individus dits "cas" (en l'occurrence, les patients qui ont un statut de non-répondeurs) ;
- la spécificité (Sp ou Spe), qui représente sa capacité à détecter la population dite "saine", constituée des patients dits "contrôles" (en l'occurrence, les patients qui ont un statut de répondeurs).

Lorsqu'un biomarqueur génère des valeurs continues (par exemple, des valeurs de concentration), différentes positions de seuil prédictif (*Prediction Threshold* ou PT) peuvent être définies afin d'assigner un échantillon à la classe positive (test positif : $y$ = 1). La comparaison de la concentration du biomarqueur à la valeur PT permet de classer le sujet dans la cohorte à laquelle il a la plus forte probabilité d'appartenance.

Par exemple, si l'on considère une cohorte d'individus qui présentent un statut de répondeurs et une cohorte d'individus qui présentent un statut de non-répondeurs, et si l'on considère un sujet ou patient p, dont le statut doit être déterminé, pour lequel la valeur de la combinaison des mesures est V (V étant égal à Z dans le cas des modèles mROC), la règle décisionnelle est la suivante :

- lorsque la valeur moyenne de la combinaison des mesures dans la cohorte des individus "répondeurs" est inférieure à celle de la cohorte des individus "non-répondeurs" :

  - si V $\geq$ PT : le test est positif, un statut de non-répondeur est assigné audit patient p,
  - si V < PT : le test est négatif, un statut de répondeur est assigné audit patient p, ou

- lorsque la valeur moyenne de la combinaison des mesures dans la cohorte des individus "répondeurs" est supérieure à celle de la cohorte des individus "non-répondeurs" :

  - si V $\geq$ PT : le test est négatif, un statut de répondeur est assigné audit patient p,
  - si V < PT : le test est positif, un statut de non-répondeur est assigné audit patient p.

Comme la combinaison de biomarqueurs de l'invention est effectivement discriminante, les distributions, supposées gaussiennes, de la combinaison de biomarqueurs dans chaque population d'intérêt se différencient nettement. Il convient alors de définir la valeur seuil optimale qui conférera à cette combinaison de biomarqueurs les meilleures performances diagnostiques.

En effet, pour un seuil donné PT, les valeurs suivantes peuvent être calculées :

- le nombre de vrais positifs : VP ;
- le nombre de faux négatifs : FN ;
- le nombre de faux positifs : FP ;
- le nombre de vrais négatifs : VN.

Les calculs des paramètres de sensibilité (Se) et spécificité (Sp) se déduisent des formules suivantes :

$$Se = VP / (VP + FN) ;$$

$$Sp = VN / (VN + FP).$$

La sensibilité peut donc être considérée comme étant la probabilité que le test soit positif sachant que le statut du sujet est un statut de non-répondeur ; et la spécificité peut être considérée comme étant la probabilité que le test soit négatif sachant que le statut du sujet est un statut de répondeur.

Une courbe ROC peut être utilisée pour visualiser le pouvoir prédictif du biomarqueur (ou pour l'approche multivariée, le pouvoir prédictif de la combinaison de biomarqueurs intégrés dans le modèle) pour différentes valeurs PT (Swets 1988). Chaque point de la courbe représente la Sensibilité versus (1-Spécificité) pour une valeur spécifique PT.

Par exemple, si les concentrations du biomarqueur d'intérêt varient de 0 à 35, différentes valeurs PT peuvent être successivement positionnées à 0,5 ; 1 ; 1,5 ; ... ; 35. Ainsi, pour chaque valeur PT, les échantillons testés sont classifiés, la sensibilité et la spécificité sont calculées et les points résultants sont reportés sur un graphique.

Plus la courbe ROC est proche de la première diagonale (droite reliant le coin bas gauche au coin haut droit), plus les performances discriminantes du modèle sont pauvres. Un test à fort pouvoir discriminant occupera la partie supérieure gauche du graphique. Un test peu discriminant avoisinera la première diagonale du graphique. L'aire sous la courbe ROC (AUC) est un bon indicateur de performance diagnostique. Celle-ci varie de 0,5 (biomarqueur non discriminant) à 1 (biomarqueur parfaitement discriminant). La valeur 0,76 induit un biomarqueur discriminant.

Une courbe ROC peut être approximée par deux principales techniques : paramétrique et non paramétrique (Shapiro 1999). Dans le premier cas, les données sont supposées suivre une distribution statistique spécifique (par exemple, gaussienne), qui est alors ajustée aux données observées pour produire une courbe ROC lissée. Des approches non paramétriques considèrent l'estimation de Se et (1-Sp) à partir des données observées. La courbe ROC empirique résultante n'est pas une fonction mathématique lissée, mais une courbe constante par morceaux.

Le choix du seuil ou du seuil optimal, noté $\delta$ (delta), dépend des priorités de l'utilisateur en termes de sensibilité et spécificité. Dans le cas de poids équitables attribués aux sensibilité et spécificité, ce dernier peut être défini comme le seuil maximisant l'indice de Youden (J = Se + Sp - 1).

Avantageusement, les moyens de l'invention permettent d'atteindre :

- une sensibilité [Se = VP / (VP + FN)] d'au moins 70% (ou plus), et/ou
- une spécificité [Sp = VN / (VN + FP)] d'au moins 70% (ou plus).

Conformément à l'invention, la sensibilité peut être d'au moins 70%, d'au moins 71%, d'au moins 72%, d'au moins 73%, ou d'au moins 74%, ou d'au moins 75%, ou d'au moins 76%, ou d'au moins 77%, ou d'au moins 78%, au d'au moins 79%, ou d'au moins 80%, ou d'au moins 81%, ou d'au moins 82% (*cf.*, par exemple, combinaisons n° 1 à 43 des tableaux 5, 7 et 12 ci-dessous, plus particulièrement les caractéristiques de sensibilité des combinaisons des niveaux de transcription ou de traduction de ces combinaisons présentées en tableaux 3, 8, 13, 17, 21, 25 ci-dessous).

Plus particulièrement, la sensibilité peut être d'au moins 72%, d'au moins 73%, ou d'au moins 74%, ou d'au moins 75%, ou d'au moins 76%, ou d'au moins 77%, ou d'au moins 78%, au d'au moins 79%, ou d'au moins 80%, ou d'au moins 81%, ou d'au moins 82% ou un seuil plus élevé (*cf.*, par exemple, combinaisons n° 1 à 26, 30, 33 à 35 et 37 à 39 des tableaux 5, 7 et 12 ci-dessous, plus particulièrement les caractéristiques de sensibilité des combinaisons n° 1 à 26, 30, 33 à 35 et 37 à 39 présentées en tableaux 3, 8, 13, 17, 21, 25 ci-dessous).

Selon un mode de réalisation particulier de l'invention, les niveaux d'expression dosés pour les gènes choisis parmi ladite liste de dix-sept gènes de l'invention sont des niveaux d'expression protéique (l'échantillon biologique étant alors avantageusement un échantillon de fluide biologique, notamment un échantillon de fluide intracorporel, tel que sang, sérum, plasma), et la sensibilité de la combinaison de niveaux d'expression ainsi dosée est d'au moins 79%, plus particulièrement d'au moins 80%, plus particulièrement d'au moins 81%, notamment de 82% ou plus (*cf.* par exemple, combinaison n°15 en tableau 3 ci-dessous, combinaison n°9 en tableau 8 ci-dessous, combinaison n°24 en tableau 17 ci-dessous, combinaison n°24 en outre combinée à deux autres facteurs biologiques (en l'occurrence, GGT et/ou PAL) et à un facteur virologique, en tableau 25 ci-dessous).

Alternativement ou complémentairement, la spécificité peut être d'au moins 70%, d'au moins 71%, d'au moins 72%, d'au moins 73%, ou d'au moins 74%, ou d'au moins 75%, ou d'au moins 76%, ou d'au moins 77%, ou d'au moins 78%, ou d'au moins 79%, ou d'au moins 80%, ou d'au moins 81%, ou d'au moins 82%, ou d'au moins 83%, ou d'au moins 84%, ou d'au moins 85%, ou d'au moins 86%, ou d'au moins 87%, ou d'au moins 88%, ou d'au moins 89%, ou d'au moins 90%, ou d'au moins 91%, ou d'au moins 92%, ou d'au moins 92% (*cf.*, par exemple, combinaisons n° 1 à 43 des tableaux 5, 7 et 12 ci-dessous, plus particulièrement les caractéristiques de spécificité des combinaisons des niveaux de transcription ou de traduction de ces combinaisons présentées en tableaux 3, 8, 13, 17, 21, 25 ci-dessous).

Plus particulièrement, la spécificité peut être d'au moins 82%, ou d'au moins 83%, ou d'au moins 84%, ou d'au moins 85%, ou d'au moins 86%, ou d'au moins 87%, ou d'au moins 88%, ou d'au moins 89%, ou d'au moins 90%, ou d'au moins 91%, ou d'au moins 92%, ou d'au moins 92% (*cf.* par exemple, combinaisons n°1 à 5, 7 à 13, 16 à 22, et 27 en tableau 13 ci-dessous).

Toutes les combinaisons de ces seuils de sensibilité et de ces seuils de spécificité sont explicitement incluses dans le contenu de la description de l'invention (*cf.*, par exemple, combinaisons n° 1 à 43 des tableaux 5, 7 et 12 ci-dessous).

Plus particulièrement, toutes les combinaisons comprenant au moins la combinaison d'un seuil de sensibilité et d'un seuil de spécificité sont explicitement incluses dans le contenu de la description de l'invention.

Alternativement ou complémentairement à ces caractéristiques de sensibilité et/ou spécificité, les Valeurs de Prédiction Négative (VPN) atteintes, ou susceptibles d'être atteintes, par les moyens de l'invention sont particulièrement élevées.

La VPN est égale à VN / (VN+FN), avec VN = Vrais Négatifs et FN = Faux Négatifs, et représente donc la probabilité que le sujet testé soit répondeur au traitement anti-VHC, sachant que le test de l'invention est négatif.

Conformément à l'invention, la VPN peut être d'au moins 78%, ou d'au moins 79%, ou d'au moins 80%, ou d'au moins 81%, ou d'au moins 82%, ou d'au moins 83%, ou d'au moins 84%, ou d'au moins 85%, ou d'au moins 86%, ou d'au moins 87%, ou d'au moins 88% (*cf.*, par exemple, combinaisons n° 1 à 43 des tableaux 5, 7 et 12 ci-dessous, plus particulièrement les caractéristiques de VPN des combinaisons des niveaux de transcription ou de traduction de ces combinaisons présentées en tableaux 3, 8, 13, 17, 21, 25 ci-dessous).

Toutes les combinaisons de seuils de VPN et/ou de seuils de sensibilité et/ou de seuils de spécificité sont explicitement incluses dans le contenu de la description de l'invention. Plus particulièrement, toutes les combinaisons comprenant au moins la combinaison d'un seuil de sensibilité et d'un seuil de VPN sont explicitement incluses dans le contenu de la description de l'invention.

Alternativement ou complémentairement à ces caractéristiques de sensibilité et/ou spécificité et/ou de VPN, les Valeurs de Prédiction Positive (VPP) atteintes, ou susceptibles d'être atteintes, par les moyens de l'invention sont particulièrement élevées. La VPP est égale à VP / (VP + FP) avec VP = les Vrais Positifs et FP = Faux Positifs, et représente donc la probabilité que le sujet testé soit non-répondeur, sachant que le test de l'invention est positif.

Conformément à l'invention, la VPP peut être d'au moins 63%, ou d'au moins 64%, ou d'au moins 65%, ou d'au moins 66%, ou d'au moins 67%, ou d'au moins 68%, ou d'au moins 69%, ou d'au moins 70%, ou d'au moins 71%, ou d'au moins 72%, ou d'au moins 73%, ou d'au moins 74%, ou d'au moins 75%, ou d'au moins 76%, ou d'au moins 77%, ou d'au moins 78%, ou d'au moins 79%, ou d'au moins 80%, ou d'au moins 81%, ou d'au moins 82%, ou d'au moins 83%, ou d'au moins 84%, ou d'au moins 85%, ou d'au moins 86%, ou d'au moins 87%, ou d'au moins 88%, ou d'au moins 89%, ou d'au moins 90%, ou d'au moins 91%, ou d'au moins 92%, ou d'au moins 93%, ou d'au moins 94% (*cf.*, par exemple, combinaisons n° 1 à 43 des tableaux 5, 7 et 12 ci-dessous, plus particulièrement les caractéristiques de VPP des combinaisons des niveaux de transcription ou de traduction de ces combinaisons présentées en tableaux 3, 8, 13, 17, 21, 25 ci-dessous). Plus particulièrement, la VPP peut être d'au moins d'au moins 78%, ou d'au moins 79%, ou d'au moins 80%, ou d'au moins 81%, ou d'au moins 82%, ou d'au moins 83%, ou d'au moins 84%, ou d'au moins 85%, ou d'au moins 86%, ou d'au moins 87%, ou d'au moins 88%, ou d'au moins 89%, ou d'au moins 90%, ou d'au moins 91%, ou d'au moins 92%, ou d'au moins 93%, ou d'au moins 94% (*cf.* par exemple, combinaisons n°1 à 5, 7 à 8, 10 à 14, 16 à 22, 27 et 30 en tableau 13 ci-dessous).

Toutes les combinaisons de seuils de VPP et/ou VPN et/ou de seuils de sensibilité et/ou de seuils de spécificité sont explicitement incluses dans le contenu de la description de l'invention.

Plus particulièrement, toutes les combinaisons comprenant au moins la combinaison d'un seuil de sensibilité et d'un seuil de VPP sont explicitement incluses dans le contenu de la description de l'invention.

Plus particulièrement, toutes les combinaisons comprenant au moins un desdits seuils de VPN et/ou au moins un desdits seuils de sensibilité, plus particulièrement au moins un desdits seuils de VPN et un desdits seuils de sensibilité, plus particulièrement au moins un desdits seuils de VPN et un desdits seuils de sensibilité et un desdits seuils de spécificité, sont incluses dans la description de l'invention.

Les combinaisons prédictives de l'invention comprennent des combinaisons des niveaux d'expression de gènes, choisis comme ci-dessus indiqué.

Comme indiqué plus en détail ci-dessous, et comme illustré dans les exemples ci-dessous (*cf.* exemples 2c) et 3b) ci-dessous), il peut néanmoins être choisi de faire intervenir dans ces combinaisons un ou plusieurs facteurs autres que les niveaux d'expression de ces gènes, pour combiner cet ou ces autres facteurs et les niveaux d'expression des gènes choisis en une règle décisionnelle.

Cet ou ces autres facteurs sont préférablement choisis de sorte à construire un modèle de classification dont le pouvoir prédictif est encore amélioré par rapport au modèle qui ne comprendrait pas cet ou ces autres facteurs.

En sus du niveau d'expression desdits gènes choisis, on peut donc également mesurer ou doser un ou plusieurs autres facteurs, tels qu'un ou plusieurs facteurs cliniques et/ou un ou plusieurs facteurs virologiques et/ou un ou plusieurs facteurs biologiques autres que le niveau d'expression desdits gènes choisis (*cf.* par exemple, les tableaux 21 à 24 et 25 à 28 ci-dessous, qui en présentent des exemples pour la combinaison n°24 dosée en niveaux de transcriptions ou en niveaux de traductions).

La(les) valeur(s) de cet(ces) autre(s) facteur(s) peuvent alors être prises en compte pour construire le modèle de classification multivariée, et peuvent ainsi conduire à des performances de classification encore améliorées, plus particulièrement à des caractéristiques de sensibilité et/ou spécificité et/ou VPN et/ou VPP augmentées.

Par exemple, si l'on compare les valeurs présentées pour la combinaison n°24 en tableaux 13, 16 et 21, 24 ci-dessous, on constate que les valeurs d'AUC, Se et VPN augmentent lorsqu'à la combinaison des niveaux de transcription desdits gènes choisis, sont outre combinés d'autres facteurs, notamment au moins un autre facteur biologique et au moins un facteur virologique (en l'occurrence, PAL et CVavantTTT).

De même, si l'on compare les valeurs présentées pour la combinaison n°24 en tableaux 17, 20 et 25, 28 ci-dessous, on constate que plusieurs des valeurs d'AUC, Spe et VPP augmentent lorsqu'à la combinaison des niveaux de traduction desdits gènes choisis, sont en outre combinés d'autres facteurs, notamment au moins deux autres facteurs biologiques et au moins un facteur virologique (en l'occurrence, GGT, PAL et CVavantTTT).

Avantageusement, lorsqu'un ou plusieurs autres facteurs sont combinés à une combinaison de gènes choisis parmi ladite liste de dix-sept gènes de l'invention, au moins l'une des caractéristiques d'AUC (le cas échéant, d'erreur LOOCV), de sensibilité, de spécificité, de VPN et de VPP, s'en trouve améliorée.

Comme précédemment indiqué, et comme illustré ci-dessous, les moyens de l'invention font intervenir le dosage du niveau d'expression de :

- au moins un gène parmi MBL2, LGALS3BP, IL8, et
- au moins un gène parmi G1P2, CCL21, CXCL10, et
- optionnellement, au moins un gène parmi AFP, CRP, CXCL11, CXCL6, CXCL9, FGF7, MDK, MMP2, SFN, TGFB2, VEGFD.

Avantageusement, le nombre total des gènes ainsi choisis est de 2, 3, 4 ou 5.

Le choix des gènes est fait selon les exigences ou souhaits de performances à atteindre, par exemple en fonction de la sensibilité et/ou spécificité et/ou VPN et/ou VPP que l'on souhaite ou désire atteindre. Bien entendu, plus le nombre de gènes choisis est faible, plus la mise en oeuvre des moyens de l'invention est simple.

Tous les choix possibles de gènes sont explicitement inclus dans la description de l'invention.

De manière similaire à ce qui a été indiqué ci-dessus pour les seuils de sensibilité, les seuils de spécificité, les seuils de VPN, les seuils de VPP, et le nombre total de gènes choisis, toutes les combinaisons de gènes choisis dans chacune des listes de gènes et/ou de nombres totaux de gènes choisis et/ou de seuils de sensibilité et/ou de seuils de spécificité et/ou de seuils de VPN et/ou de seuils de VPP sont explicitement incluses dans le contenu de la description de l'invention.

Quarante-trois exemples de combinaisons de gènes conformes à l'invention sont présentés dans les tableaux 2, 7, 12 ci-dessous.

Par exemple, lesdits gènes choisis parmi ladite liste de dix-sept gènes de l'invention sont :

- LGALS3BP et CXCL10 (combinaison n°15) ; ou
- LGALS3BP, CXCL10 et MDK (combinaison n°9) ; ou
- LGALS3BP, IL8, CXCL10, CCL21 et MDK (combinaison n°24) ; ou
- CRP, G1P2, LGALS3BP, MBL2, TGFB2 (combinaison n°1) ; ou
- AFP, CXCL6, CXCL9, G1P2, MBL2 (combinaison n°2) ; ou
- AFP, FGF7, G1P2, MBL2, MMP2 (combinaison n°3) ; ou
- CXCL11, G1P2, IL8, MBL2, TGFB2 (combinaison n°4) ; ou
- G1P2, IL8, MBL2, SFN, TGFB2 (combinaison n°5) ; ou
- CCL21, FGF7, IL8, LGALS3BP, MBL2 (combinaison n°6) ; ou
- G1P2, LGALS3BP, MBL2, MDK, TGFB2 (combinaison n°7) ; ou
- G1P2, LGALS3BP, MBL2, MMP2, TGFB2 (combinaison n°8) ; ou
- G1P2, LGALS3BP, MBL2, SFN, TGFB2 (combinaison n°10) ; ou
- CXCL6, CXCL10, G1P2, MBL2, MMP2 (combinaison n°11) ; ou
- CXCL6, CXCL11, G1P2, MBL2, MMP2 (combinaison n°12) ; ou
- FGF7, G1P2, LGALS3BP, MBL2, TGFB2 (combinaison n°13) ; ou
- AFP, CXCL6, G1P2, IL8, MDK (combinaison n°14) ; ou
- CCL21, G1P2, LGALS3BP, MBL2, SFN (combinaison n°16) ; ou
- CXCL10, G1P2, LGALS3BP, MBL2, TGFB2 (combinaison n°17) ; ou
- CRP, CXCL6, G1P2, MBL2, SFN (combinaison n°18) ; ou
- CXCL10, CXCL11, G1P2, MBL2, MMP2 (combinaison n°19) ; ou
- CXCL11, G1P2, LGALS3BP, MBL2, MDK (combinaison n°20) ; ou
- G1P2, IL8, LGALS3BP, MBL2, TGFB2 (combinaison n°21) ; ou
- FGF7, G1P2, IL8, MDK, SFN (combinaison n°22) ; ou
- CCL21, FGF7, LGALS3BP, MBL2, MDK (combinaison n°23) ; ou
- CCL21, CXCL6, IL8, LGALS3BP, MDK (combinaison n°25) ; ou
- CCL21, FGF7, MBL2, MDK, VEGFD (combinaison n°26) ; ou
- CXCL6, IL8, CCL21, GIP2, MDK (combinaison n°30) ; ou
- CXCL6, IL8, CXCL10, GIP2, MDK (combinaison n°33) ; ou
- CCL21, CXCL10, GIP2, LGALS3BP, MDK (combinaison n°34) ; ou

- CXCL6, IL8, CCL21, GIP2, LGALS3BP (combinaison n°35) ; ou
- IL8, CCL21, CXCL10, GIP2, LGALS3BP (combinaison n°37) ; ou
- IL8, CXCL10, GIP2, LGALS3BP, MDK (combinaison n°38) ; ou
- CXCL6, IL8, GIP2, LGALS3BP, MDK (combinaison n°39) ; ou
- FGF7, G1P2, LGALS3BP, MBL2, MDK (combinaison n°27) ; ou
- CXCL10, FGF7, IL8, MDK, VEGFD (combinaison n°28) ; ou
- CCL21, CXCL6, CXCL10, LGALS3BP, MDK (combinaison n°29) ; ou
- IL8, CCL21, GIP2, LGALS3BP, MDK (combinaison n°31) ; ou
- IL8, CCL21, CXCL10, GIP2, MDK (combinaison n°32) ; ou
- CXCL6, IL8, CXCL10, GIP2, LGALS3BP (combinaison n°36) ; ou
- CXCL6, IL8, CCL21, CXCL10, GIP2 (combinaison n°40) ; ou
- CXCL6, CXCL10, GIP2, LGALS3BP, MDK (combinaison n°41) ; ou
- CXCL6, IL8, CCL21, CXCL10, LGALS3BP (combinaison n°42) ; ou
- CXCL6, CCL21, CXCL10, GIP2, LGALS3BP (combinaison n°43).

Selon un aspect de l'invention, lesdits gènes choisis à l'étape i) ne sont pas :

- FGF7, G1P2, LGALS3BP, MBL2, MDK (combinaison n°27) ;
- CXCL10, FGF7, IL8, MDK, VEGFD (combinaison n°28) ;
- CCL21, CXCL6, CXCL10, LGALS3BP, MDK (combinaison n°29) ;
- IL8, CCL21, GIP2, LGALS3BP, MDK (combinaison n°31) ;
- IL8, CCL21, CXCL10, GIP2, MDK (combinaison n°32) ;
- CXCL6, IL8, CXCL10, GIP2, LGALS3BP (combinaison n°36) ;
- CXCL6, IL8, CCL21, CXCL10, GIP2 (combinaison n°40) ;
- CXCL6, CXCL10, GIP2, LGALS3BP, MDK (combinaison n°41) ;
- CXCL6, IL8, CCL21, CXCL10, LGALS3BP (combinaison n°42) ;
- CXCL6, CCL21, CXCL10, GIP2, LGALS3BP (combinaison n°43).

Selon un aspect de l'invention, lesdits gènes choisis à l'étape i) sont :

- LGALS3BP et CXCL10 (combinaison n°15) ; ou
- LGALS3BP, CXCL10 et MDK (combinaison n°9) ; ou
- LGALS3BP, IL8, CXCL10, CCL21 et MDK (combinaison n°24) ; ou
- CRP, G1P2, LGALS3BP, MBL2, TGFB2 (combinaison n°1) ; ou
- AFP, CXCL6, CXCL9, G1P2, MBL2 (combinaison n°2) ; ou
- AFP, FGF7, G1P2, MBL2, MMP2 (combinaison n°3) ; ou
- CXCL11, G1P2, IL8, MBL2, TGFB2 (combinaison n°4) ; ou
- G1P2, IL8, MBL2, SFN, TGFB2 (combinaison n°5) ; ou
- CCL21, FGF7, IL8, LGALS3BP, MBL2 (combinaison n°6) ; ou
- G1P2, LGALS3BP, MBL2, MDK, TGFB2 (combinaison n°7) ; ou
- G1P2, LGALS3BP, MBL2, MMP2, TGFB2 (combinaison n°8) ; ou
- G1P2, LGALS3BP, MBL2, SFN, TGFB2 (combinaison n°10) ; ou
- CXCL6, CXCL10, G1P2, MBL2, MMP2 (combinaison n°11) ; ou
- CXCL6, CXCL11, G1P2, MBL2, MMP2 (combinaison n°12) ; ou
- FGF7, G1P2, LGALS3BP, MBL2, TGFB2 (combinaison n°13) ; ou
- AFP, CXCL6, G1P2, IL8, MDK (combinaison n°14) ; ou
- CCL21, G1P2, LGALS3BP, MBL2, SFN (combinaison n°16) ; ou
- CXCL10, G1P2, LGALS3BP, MBL2, TGFB2 (combinaison n°17) ; ou
- CRP, CXCL6, G1P2, MBL2, SFN (combinaison n°18) ; ou
- CXCL10, CXCL11, G1P2, MBL2, MMP2 (combinaison n°19) ; ou
- CXCL11, G1P2, LGALS3BP, MBL2, MDK (combinaison n°20) ; ou
- G1P2, IL8, LGALS3BP, MBL2, TGFB2 (combinaison n°21) ; ou
- FGF7, G1P2, IL8, MDK, SFN (combinaison n°22) ; ou
- CCL21, FGF7, LGALS3BP, MBL2, MDK (combinaison n°23) ; ou
- CCL21, CXCL6, IL8, LGALS3BP, MDK (combinaison n°25) ; ou
- CCL21, FGF7, MBL2, MDK, VEGFD (combinaison n°26) ; ou
- CXCL6, IL8, CCL21, GIP2, MDK (combinaison n°30) ; ou
- CXCL6, IL8, CXCL10, GIP2, MDK (combinaison n°33) ; ou

- CCL21, CXCL10, GIP2, LGALS3BP, MDK (combinaison n°34) ; ou
- CXCL6, IL8, CCL21, GIP2, LGALS3BP (combinaison n°35) ; ou
- IL8, CCL21, CXCL10, GIP2, LGALS3BP (combinaison n°37) ; ou
- IL8, CXCL10, GIP2, LGALS3BP, MDK (combinaison n°38) ; ou
- CXCL6, IL8, GIP2, LGALS3BP, MDK (combinaison n°39).

Des exemples de modèles de classification multivariée ont été construits pour chacune de ces combinaisons de gènes. Les tableaux 2 à 28 ci-dessous en présentent des exemples. Les tableaux 2 à 6 (combinaison n°15) illustrent les performances de la combinaison des niveaux d'expression de deux gènes (en l'occurrence, concentrations sériques de deux protéines).

Les tableaux 7 à 11 (combinaison n°9) illustrent les performances de la combinaison des niveaux d'expression de trois gènes (en l'occurrence, concentrations sériques de trois protéines).

Les tableaux 12 à 16 (combinaison n°1 à 8, 10 à 14, et 16 à 43) illustrent les performances de la combinaison des niveaux de transcription de cinq gènes (en l'occurrence, valeur de Ct qui a été mesurée pour les transcrits ARN de ce gène et qui a été normalisée par la méthode du $2^{-\Delta Ct}$).

Les tableaux 17 à 20 (combinaison n°24) illustrent les performances de la combinaison des niveaux de traduction de cinq gènes (en l'occurrence, concentrations sériques de cinq protéines).

**Tableau 2 : exemple de combinaison des niveaux d'expression de deux gènes**

| n° de la combinaison | Gènes choisis | |
|---|---|---|
| 15 | CXCL10 | LGALS3BP |

**Tableau 3 : valeurs de sensibilité (Se), spécificité (Spe), Valeur Prédictive Négative (VPN), Valeur Prédictive Positive (VPP), et d'erreur LOOCV qui peuvent être associées aux combinaisons des niveaux de traduction (plus particulièrement des niveaux de traduction en protéines sériques) de gènes choisis conformément à l'invention**

| n° de la combinaison (*cf.* tableau 2) | Modèle de classification utilisé | Se | Spe | VPN | VPP | Erreur LOOCV |
|---|---|---|---|---|---|---|
| 15 | mROC (*) | 82 | 72 | 82 | 72 | ND |

(*) : les valeurs de Se, Spe, VPN, VPP et Erreur LOOCV indiquées sont celles de la fonction mROC correspondante du tableau 4 ci-dessous Erreur LOOCV = erreur calculée par validation croisée ou « Leave-One-Out Cross Validation » ; ND = non déterminée

**Tableau 4 : exemples de modèles mROC (fonction Z) combinant les niveaux de traduction protéique (plus particulièrement en protéines sériques) de gènes choisis conformément à l'invention, et exemple de seuil PT pour ces fonctions (en l'occurrence, seuil maximisant l'indice de Youden $\delta$)**

| n° de combinaison (*cf.* tableau 2 ci-dessus) | Fonction Z combinant les niveaux de traduction (protéines sériques) des gènes choisis | Nom de la fonction | Seuil PT (seuil $\delta$) |
|---|---|---|---|
| 15 | Z = 0,030 x CXCL10$^t$ + 0,447 x LGALS3BP$^t$ | **Z15PROT** | 2,169 |

**Tableau 5 : liste des gènes, pour lesquels il est conseillé de normaliser les valeurs de dosage mesurées (notamment, les valeurs de dosage des niveaux de traduction protéique, plus particulièrement lorsque ces protéines sont des protéines sériques), par exemple par une normalisation Box-Cox, et exemple de valeurs de paramètre Box-Cox ($\lambda$) utilisables dans les fonctions Z indiquées au tableau 4 ci-dessus.**

| Gènes, pour lesquels il est conseillé de normaliser la valeur du niveau de traduction (protéine) | Exemple de valeurs de paramètre Box-Cox ($\lambda$) utilisable pour les fonctions Z du tableau 4 ci-dessus (*) |
|---|---|
| CXCL10 | 0,41 |

(suite)

| Gènes, pour lesquels il est conseillé de normaliser la valeur du niveau de traduction (protéine) | Exemple de valeurs de paramètre Box- Cox ($\lambda$) utilisable pour les fonctions Z du tableau 4 ci-dessus (*) |
|---|---|
| LGALS3BP | 0,33 |
| (*) : paramètre lambda des transformations Box-Cox [BMQ$^t$ = (BMQ$^\lambda$-1)/$\lambda$] | |

**Tableau 6 : AUC des fonctions Z du tableau 4**

| n° de combinaison (*cf.* tableau 2 ci-dessus) | Nom de la fonction (*cf.* tableau 4 ci-dessus) | AUC | AUC limite inférieure | AUC limite supérieure |
|---|---|---|---|---|
| **15** | **Z15PROT** | 0,831 | 0,760 | 0,885 |

**Tableau 7 : exemple de combinaison des niveaux d'expression de trois gènes**

| n° de la combinaison | Gènes choisis | | |
|---|---|---|---|
| **9** | LGALS3BP | CXCL10 | MDK |

**Tableau 8 : valeurs de sensibilité (Se), spécificité (Spe), Valeur Prédictive Négative (VPN), Valeur Prédictive Positive (VPP), et d'erreur LOOCV qui peuvent être associées aux combinaisons des niveaux de traduction (plus particulièrement des niveaux de traduction en protéines sériques) de gènes choisis conformément à l'invention**

| n° de la combinaison (*cf.* tableau 7) | Modèle de classification utilisé | Se | Spe | VPN | VPP | Erreur LOOCV |
|---|---|---|---|---|---|---|
| 9 | mROC (*) | 82 | 74 | 83 | 73 | ND |
| (*) : les valeurs de Se, Spe, VPN, VPP et Erreur LOOCV indiquées sont celles de la fonction mROC correspondante du tableau 9 ci-dessous Erreur LOOCV = erreur calculée par validation croisée ou « Leave-One-Out Cross Validation » ; ND = non déterminée | | | | | | |

**Tableau 9 : exemples de modèles mROC (fonction Z) combinant les niveaux de traduction protéique (plus particulièrement en protéines sériques) de gènes choisis conformément à l'invention, et exemple de seuil PT pour ces fonctions (en l'occurrence, seuil maximisant l'indice de Youden $\delta$)**

| n° de combinaison (*cf.* tableau 7 ci-dessus) | Fonction Z combinant les niveaux de traduction (protéines sériques) des gènes choisis | Nom de la fonction | Seuil PT (seuil $\delta$) |
|---|---|---|---|
| **9** | Z = 0,029 x CXCL10$^t$ + 0,472 x LGALS3BP$^t$- 0,319 x MDK | Z9PROT | 2,164 |

**Tableau 10 : liste des gènes, pour lesquels il est conseillé de normaliser les valeurs de dosage mesurées (notamment, les valeurs de dosage des niveaux de traduction protéique, plus particulièrement lorsque ces protéines sont des protéines sériques), par exemple par une normalisation Box-Cox, et exemple de valeurs de paramètre Box-Cox ($\lambda$) utilisables dans les fonctions Z indiquées au tableau 9 ci-dessus.**

| Gènes, pour lesquels il est conseillé de normaliser la valeur du niveau de traduction (protéine) | Exemple de valeurs de paramètre Box- Cox ($\lambda$) utilisable pour les fonctions Z du tableau 9 ci-dessus (*) |
|---|---|
| CXCL10 | 0,41 |

(suite)

| Gènes, pour lesquels il est conseillé de normaliser la valeur du niveau de traduction (protéine) | Exemple de valeurs de paramètre Box- Cox ($\lambda$) utilisable pour les fonctions Z du tableau 9 ci-dessus (*) |
|---|---|
| LGALS3BP | 0,33 |
| (*) : paramètre lambda des transformations Box-Cox [$BMQ^t = (BMQ^\lambda-1)/\lambda$] | |

**Tableau 11 : AUC des fonctions Z du tableau 9**

| n° de combinaison (*cf.* tableau 7 ci-dessus) | **Nom** de la fonction (*cf.* tableau 9 ci-dessus) | **AUC** | **AUC limite inférieure** | **AUC limite supérieure** |
|---|---|---|---|---|
| **9** | **Z9PROT** | 0,836 | 0,766 | 0,888 |

**Tableau 12 : exemples de combinaisons des niveaux d'expression de cinq gènes**

| n° de la combinaison | Gènes choisis | | | | |
|---|---|---|---|---|---|
| 1 | CRP | G1P2 | LGALS3BP | MBL2 | TGFB2 |
| 2 | AFP | CXCL6 | CXCL9 | G1P2 | MBL2 |
| 3 | AFP | FGF7 | G1P2 | MBL2 | MMP2 |
| 4 | CXCL11 | G1P2 | IL8 | MBL2 | TGFB2 |
| 5 | G1P2 | IL8 | MBL2 | SFN | TGFB2 |
| 6 | CCL21 | FGF7 | IL8 | LGALS3BP | MBL2 |
| 7 | G1P2 | LGALS3BP | MBL2 | MDK | TGFB2 |
| 8 | G1P2 | LGALS3BP | MBL2 | MMP2 | TGFB2 |
| 10 | G1P2 | LGALS3BP | MBL2 | SFN | TGFB2 |
| 11 | CXCL6 | CXCL10 | G1P2 | MBL2 | MMP2 |
| 12 | CXCL6 | CXCL11 | G1P2 | MBL2 | MMP2 |
| 13 | FGF7 | G1P2 | LGALS3BP | MBL2 | TGFB2 |
| 14 | AFP | CXCL6 | G1P2 | IL8 | MDK |
| 16 | CCL21 | G1P2 | LGALS3BP | MBL2 | SFN |
| 17 | CXCL10 | G1P2 | LGALS3BP | MBL2 | TGFB2 |
| 18 | CRP | CXCL6 | G1P2 | MBL2 | SFN |
| 19 | CXCL10 | CXCL11 | G1P2 | MBL2 | MMP2 |
| 20 | CXCL11 | G1P2 | LGALS3BP | MBL2 | MDK |
| 21 | G1P2 | IL8 | LGALS3BP | MBL2 | TGFB2 |
| 22 | FGF7 | G1P2 | IL8 | MDK | SFN |
| 23 | CCL21 | FGF7 | LGALS3BP | MBL2 | MDK |
| 24 | CCL21 | CXCL10 | IL8 | LGALS3BP | MDK |
| 25 | CCL21 | CXCL6 | IL8 | LGALS3BP | MDK |
| 26 | CCL21 | FGF7 | MBL2 | MDK | VEGFD |
| 27 | FGF7 | G1P2 | LGALS3BP | MBL2 | MDK |
| 28 | CXCL10 | FGF7 | IL8 | MDK | VEGFD |
| 29 | CCL21 | CXCL6 | CXCL10 | LGALS3BP | MDK |

(suite)

| n° de la combinaison | Gènes Choisis | | | | |
|---|---|---|---|---|---|
| 30 | CXCL6 | IL8 | CCL21 | GIP2 | MDK |
| 31 | IL8 | CCL21 | GIP2 | LGALS3BP | MDK |
| 32 | IL8 | CCL21 | CXCL10 | GIP2 | MDK |
| 33 | CXCL6 | IL8 | CXCL10 | GIP2 | MDK |
| 34 | CCL21 | CXCL10 | GIP2 | LGALS3BP | MDK |
| 35 | CXCL6 | IL8 | CCL21 | GIP2 | LGALS3BP |
| 36 | CXCL6 | IL8 | CXCL10 | GIP2 | LGALS3BP |
| 37 | IL8 | CCL21 | CXCL10 | GIP2 | LGALS3BP |
| 38 | IL8 | CXCL10 | GIP2 | LGALS3BP | MDK |
| 39 | CXCL6 | IL8 | GIP2 | LGALS3BP | MDK |
| 40 | CXCL6 | IL8 | CCL21 | CXCL10 | GIP2 |
| 41 | CXCL6 | CXCL10 | GIP2 | LGALS3BP | MDK |
| 42 | CXCL6 | IL8 | CCL21 | CXCL10 | LGALS3BP |
| 43 | CXCL6 | CCL21 | CXCL10 | GIP2 | LGALS3BP |

**Tableau 13 : valeurs de sensibilité (Se), spécificité (Spe), Valeur Prédictive Négative (VPN), Valeur Prédictive Positive (VPP), et d'erreur LOOCV qui peuvent être associées aux combinaisons des niveaux de transcription de cinq gènes choisis conformément à l'invention (transcrits ARN, plus particulièrement ARN d'un prélèvement de tissu ou cellules hépatiques)**

| n° de la combinaison (cf. tableau 12) | Modèle de classification utilisé | Se | Spe | VPN | VPP | Erreur LOOCV |
|---|---|---|---|---|---|---|
| 1 | WKNN | 82 | 92 | 88 | 88 | 12 |
| 2 | RF | 80 | 92 | 87 | 88 | 13 |
| 3 | RF | 80 | 84 | 85 | 78 | 18 |
| 4 | RF | 80 | 84 | 85 | 78 | 18 |
| 5 | RF | 80 | 84 | 85 | 78 | 18 |
| 6 | mROC (*) | 80 | 70 | 83 | 65 | ND |
| 7 | WKNN | 77 | 90 | 85 | 85 | 15 |
| 8 | WKNN | 77 | 90 | 85 | 85 | 15 |
| 10 | WKNN | 75 | 89 | 80 | 94 | 16 |
| 11 | WKNN | 75 | 84 | 84 | 82 | 17 |
| 12 | WKNN | 75 | 84 | 81 | 88 | 17 |
| 13 | WKNN | 75 | 83 | 82 | 86 | 17 |
| 14 | WKNN | 75 | 71 | 81 | 86 | 18 |
| 16 | WKNN | 73 | 92 | 79 | 90 | 18 |
| 17 | WKNN | 73 | 89 | 82 | 82 | 18 |
| 18 | WKNN | 73 | 87 | 80 | 88 | 18 |
| 19 | WKNN | 73 | 86 | 85 | 78 | 18 |

(suite)

| n° de la combinaison (*cf.* tableau 12) | Modèle de classification utilisé | Se | Spe | VPN | VPP | Erreur LOOCV |
|---|---|---|---|---|---|---|
| 20 | WKNN | 73 | 86 | 78 | 93 | 18 |
| 21 | WKNN | 73 | 83 | 80 | 88 | 18 |
| 22 | WKNN | 73 | 83 | 79 | 90 | 18 |
| 23 | mROC (*) | 73 | 75 | 80 | 66 | ND |
| 24 | mROC (*) | 73 | 74 | 80 | 66 | ND |
| 25 | mROC (*) | 73 | 73 | 79 | 65 | ND |
| 26 | mROC (*) | 73 | 70 | 79 | 63 | ND |
| 27 | WKNN | 70 | 90 | 81 | 84 | 18 |
| 28 | mROC (*) | 70 | 78 | 79 | 69 | ND |
| 29 | mROC (*) | 70 | 74 | 78 | 65 | ND |
| 30 | mROC (*) | 73 | 73 | 79 | 79 | ND |
| 31 | mROC (*) | 71 | 76 | 80 | 65 | ND |
| 32 | mROC (*) | 71 | 76 | 79 | 67 | ND |
| 33 | mROC (*) | 73 | 73 | 79 | 65 | ND |
| 34 | mROC (*) | 73 | 73 | 79 | 65 | ND |
| 35 | mROC (*) | 73 | 73 | 79 | 65 | ND |
| 36 | mROC (*) | 71 | 75 | 78 | 66 | ND |
| 37 | mROC (*) | 73 | 76 | 80 | 68 | ND |
| 38 | mROC (*) | 73 | 73 | 79 | 65 | ND |
| 39 | mROC (*) | 73 | 73 | 79 | 64 | ND |
| 40 | mROC (*) | 71 | 75 | 78 | 66 | ND |
| 41 | mROC (*) | 71 | 75 | 78 | 66 | ND |
| 42 | mROC (*) | 71 | 73 | 78 | 65 | ND |
| 43 | mROC (*) | 71 | 73 | 78 | 65 | ND |

(*) : les valeurs de Se, Spe, VPN, VPP et Erreur LOOCV indiquées sont celles de la fonction mROC correspondante du tableau 14 ci-dessous Erreur LOOCV = erreur calculée par validation croisée ou « Leave-One-Out Cross Validation » (Hastie, Tibishirani et Friedman, 2009) ; ND = non déterminée

**Tableau 14 : exemples de modèles mROC (fonction Z) combinant les niveaux de transcription de cinq gènes choisis (transcrits ARN, plus particulièrement ARN d'un prélèvement de tissu ou cellules hépatiques), et exemple de seuil PT pour ces fonctions (en l'occurrence, seuil maximisant l'indice de Youden δ)**

| n° de combinaison (*cf.* tableau 12 ci-dessus) | Fonction Z combinant les niveaux de transcription (ARN) des gènes choisis | Nom de la fonction | Seuil PT (seuil δ) |
|---|---|---|---|
| 6 | $Z = 0,428 \times CCL21^t + 0,543 \times FGF7 + 0,029 \times IL8 + 0,281 \times LGALS3BP^t + 0,108 \times (-MBL2)$ | **Z6ARN** | -2,884 |
| 23 | $Z = 0,417 \times CCL21^t + 0,55 \times FGF7 + 0,198 \times LGALS3BP^t + 0,099 \times (-MBL2) + 0,147 \times MDK^t$ | **Z23ARN** | -2,842 |

(suite)

| n° de combinaison (cf. tableau 12 ci-dessus) | Fonction Z combinant les niveaux de transcription (ARN) des gènes choisis | Nom de la fonction | Seuil PT (seuil $\delta$) |
|---|---|---|---|
| **24** | **Z** = 0,359 x CCL21$^t$ + 0,028 x CXCL10$^t$ + 0,055 x IL8 + 0,107 x LGALS3BP$^t$ + 0,22 x MDK$^t$ | **Z24ARN** | -2,309 |
| **25** | **Z** = 0,374 x (CCL21$^t$) - 0,105 x CXCL6 + 0,068 x IL8 + 0,11 x LGALS3BP$^t$ + 0,225 x MDK$^t$ | **Z25ARN** | -2,331 |
| **26** | **Z** = 0,516 x CCL21$^t$ + 0,554 x FGF7 + 0,07 x (-MBL2) + 0,276 x MDK$^t$ - 0,092 x VEGFD | **Z26ARN** | -2,868 |
| **28** | **Z** = 0,321x CXCL10$^t$ + 0,623 xFGF7- 0,018 x IL8 + 0,352 x MDK$^t$ - 0,067 x VEGFD | **Z28ARN** | -1,363 |
| **29** | **Z** = 0,356 x CCL21$^t$ + 0,116 x CXCL6 + 0,072 x CXCL10$^t$ + 0,087 x LGALS3BP$^t$ + 0,244 x MDK$^t$ | **Z29ARN** | -2,417 |
| **30** | Z = 0,283*CCL21$^t$ -0,108*CXCL6 + 0,162*GIP2$^t$ + 0,077 * IL8 + 0,195 * MDK$^t$ | **Z30ARN** | -1,504 |
| **31** | Z = 0,266*CCL21$^t$ + 0,155*GIP2$^t$ + 0,068*IL8 + 0,050*LGALS3BP$^t$ + 0,160*MDK$^t$ | **Z31ARN** | -1,509 |
| **32** | Z = 0,304 * CCL21$^t$ - 0,034 * CXCL10$^t$ + 0,168 * GIP2$^t$ + 0,069 * IL8 + 0,186 * MDK$^t$ | **Z32ARN** | -1,473 |
| **33** | Z = -0,125 * CXCL6 + 0,074 * CXCL10$^t$ + 0,198 * GIP2$^t$ + 0,080 * IL8 + 0,241 * MDK$^t$ | **Z33ARN** | -0,554 |
| **34** | Z = 0,290 * CCL21$^t$ + 0,035 * CXCL10$^t$ + 0,137 * GIP2$^t$ + 0,029 * LGALS3BP$^t$ + 0,214 * MDK$^t$ | **Z34ARN** | -1,782 |
| **35** | Z = 0,276 * CCL21$^t$ - 0,039 * CXCL6 + 0,175 * GIP2$^t$ + 0,085 * IL8 + 0,126 * LGALS3BP$^t$ | **Z35ARN** | -1,405 |
| **36** | Z = -0,047 * CXCL6 + 0,026 * CXCL10$^t$ + 0,211 * GIP2$^t$ + 0,095 * IL8 + 0,181 * LGALS3BP$^t$ | **Z36ARN** | -0,407 |
| **37** | Z = 0,311 * CCL21$^t$ - 0,072 * CXCL10$^t$ + 0,182 * GIP2$^t$ + 0,087 * IL8 + 0,129 * LGALS3BP$^t$ | **Z37ARN** | -1,386 |
| **38** | Z = 0,050 * CXCL10$^t$ + 0,183 * GIP2$^t$ + 0,073 * IL8 + 0,091 * LGALS3BP$^t$ + 0,177 * MDK$^t$ | **Z38ARN** | -0,642 |
| **39** | Z = -0,119 * CXCL6 + 0,193 * GIP2$^t$ + 0,091 * IL8 + 0,1 * LGALS3BP$^t$ + 0,179 * MDK$^t$ | **Z39ARN** | -0,576 |
| **40** | Z = 0,379 * CCL21$^t$ - 0,019 * CXCL6 - 0,059 * CXCL10$^t$ + 0,222 * GIP2$^t$ + 0,09 * IL8 | **Z40ARN** | -1,282 |

(suite)

| n° de combinaison (*cf.* tableau 12 ci-dessus) | Fonction Z combinant les niveaux de transcription (ARN) des gènes choisis | Nom de la fonction | Seuil PT (seuil $\delta$) |
|---|---|---|---|
| 41 | Z = 0,162 * CXCL6 + 0,114 * CXCL10$^t$ + 0,166 * GIP2$^t$ + 0,073 * LGALS3BP$^t$ + 0,214 * MDK$^t$ | **Z41ARN** | -0,802 |
| 42 | Z = 0,409 * CCL21$^t$ - 0,014 * CXCL6 + 0,0004 * CXCL10$^t$ + 0,076 * IL8 + 0,231 * LGALS3BP$^t$ | **Z42ARN** | -2,33 |
| 43 | Z = 0,322 * CCL21$^t$ + 0,278 * CXCL6 - 0,007 * CXCL10$^t$ + 0,167 * GIP2$^t$ + 0,125 * LGALS3BP$^t$ | **Z43ARN** | -1,62 |

**Tableau 15 : liste des gènes, pour lesquels il est conseillé de normaliser les valeurs de dosage mesurées (notamment, les valeurs de dosage des niveaux de transcription ARN, plus particulièrement lorsque ces ARN proviennent d'un prélèvement de tissu ou cellules hépatiques), par exemple par une normalisation Box-Cox, et exemple de valeurs de paramètre Box-Cox ($\lambda$) utilisables dans les fonctions Z indiquées tableau 14 ci-dessus.**

| Gènes, pour lesquels il est conseillé de normaliser la valeur du niveau de transcription (ARN) | Exemple de valeur de paramètre Box-Cox ($\lambda$) utilisable pour les fonctions Z du tableau 14 ci-dessus |
|---|---|
| CCL21 | 0,02 |
| MDK | 0,12 |
| LGALS3BP | -0,06 |
| CXCL10 | 0,18 |
| G1P2 | 0,07 |

**Tableau 16 : AUC des fonctions Z du tableau 14**

| n° de combinaison (*cf.* tableau 12 ci-dessus) | Nom de la fonction (*cf.* tableau 14 ci-dessus) | AUC | AUC limite inférieure | AUC limite supérieure |
|---|---|---|---|---|
| 6 | **Z6ARN** | 0,805 | 0,709 | 0,875 |
| 23 | **Z23ARN** | 0,801 | 0,704 | 0,872 |
| 24 | **Z24ARN** | 0,771 | 0,665 | 0,851 |
| 25 | **Z25ARN** | 0,771 | 0,665 | 0,851 |
| 26 | **Z26ARN** | 0,794 | 0,696 | 0,867 |
| 28 | **Z28ARN** | 0,795 | 0,693 | 0,869 |
| 29 | **Z29ARN** | 0,767 | 0,629 | 0,834 |
| 30 | **Z30ARN** | 0,784 | 0,667 | 862 |
| 31 | **Z31ARN** | 0,783 | 0,676 | 0,861 |
| 32 | **Z32ARN** | 0,782 | 0,676 | 0,861 |
| 33 | **Z33ARN** | 0,78 | 0,674 | 0,859 |
| 34 | **Z34ARN** | 0,779 | 0,671 | 0,859 |
| 35 | **Z35ARN** | 0,778 | 0,672 | 0,857 |
| 36 | **Z36ARN** | 0,778 | 0,673 | 0,857 |

(suite)

| n° de combinaison (*cf.* tableau 12 ci-dessus) | Nom de la fonction (*cf.* tableau 14 ci-dessus) | AUC | AUC limite inférieure | AUC limite supérieure |
|---|---|---|---|---|
| 37 | **Z37ARN** | 0,778 | 0,672 | 0,857 |
| 38 | **Z38ARN** | 0,778 | 0,67 | 0,858 |
| 39 | **Z39ARN** | 0,777 | 0,669 | 0,857 |
| 40 | **Z40ARN** | 0,775 | 0,671 | 0,854 |
| 41 | **Z41ARN** | 0,773 | 0,664 | 0,855 |
| 42 | **Z42ARN** | 0,768 | 0,662 | 0,848 |
| 43 | **Z43ARN** | 0,765 | 0,654 | 0,848 |

**Tableau 17 : valeurs de sensibilité (Se), spécificité (Spe), Valeur Prédictive Négative (VPN), Valeur Prédictive Positive (VPP), et d'erreur LOOCV qui peuvent être associées aux combinaisons des niveaux de traduction (plus particulièrement des niveaux de traduction en protéines sériques) de cinq gènes choisis conformément à l'invention**

| n° de la combinaison (*cf.* tableau 12) | Modèle de classification utilisé | Se | Spe | VPN | VPP | Erreur LOOCV |
|---|---|---|---|---|---|---|
| 24 | mROC (*) | 82 | 74 | 83 | 73 | ND |
| (*) : les valeurs de Se, Spe, VPN, VPP et Erreur LOOCV indiquées sont celles de la fonction mROC correspondante du tableau 18 ci-dessous Erreur LOOCV = erreur calculée par validation croisée ou « Leave-One-Out Cross Validation » | | | | | | |

**Tableau 18 : exemples de modèles de modèles mROC (fonction Z) combinant les niveaux de traduction protéique (plus particulièrement en protéines sériques) de cinq gènes choisis conformément à l'invention, et exemple de seuil PT pour ces fonctions (en l'occurrence, seuil maximisant l'indice de Youden $\delta$)**

| n° de combinaison (*cf.* tableau 12 ci-dessus) | Fonction Z combinant les niveaux de traduction (protéines sériques) des gènes choisis | Nom de la fonction | Seuil PT (seuil $\delta$) |
|---|---|---|---|
| 24 | $Z = 0,025 \times CXCL10^t + 0,071 \times IL8^t + 0,465 \times LGALS3BP^t - 0,341 \times MDK - 0,001 \times CCL21^t$ | **Z24PROT** | 2,231 |

**Tableau 19 : liste des gènes, pour lesquels il est conseillé de normaliser les valeurs de dosage mesurées (notamment, les valeurs de dosage des niveaux de traduction protéique, plus particulièrement lorsque ces protéines sont des protéines sériques), par exemple par une normalisation Box-Cox, et exemple de valeurs de paramètre Box-Cox ($\lambda$) utilisables dans les fonctions Z indiquées au tableau 18 ci-dessus.**

| Gènes, pour lesquels il est conseillé de normaliser la valeur du niveau de traduction (protéine) | Exemple de valeurs de paramètre Box-Cox ($\lambda$) utilisable pour les fonctions Z du tableau 18 ci-dessus (*) |
|---|---|
| IL8 | 0,23 |
| LGALS3BP | 0,33 |
| CXCL10 | 0,41 |
| CCL21 | -0,01 |
| (*) : paramètre lambda des transformations Box-Cox [$BMQ^t = (BMQ^\lambda - 1)/\lambda$] | |

**Tableau 20 : AUC des fonctions Z du tableau 18**

| n° de combinaison (*cf.* tableau 12 ci-dessus) | Nom de la fonction (*cf.* tableau 18 ci-dessus) | AUC | AUC limite inférieure | AUC limite supérieure |
|---|---|---|---|---|
| 24 | Z24PROT | 0,838 | 0,769 | 0,890 |

En sus des niveaux d'expression desdits gènes choisis parmi la liste de dix-sept gènes de l'invention, les moyens de l'invention peuvent en outre comprendre la combinaison d'un ou plusieurs autres facteurs, tels que :

- un ou plusieurs facteurs cliniques, tels que :

  ◦ sexe (féminin F ou masculin M),
  ◦ âge à la date du prélèvement (Age), par exemple, âge à la date de la PBH, âge à la date de la cytoponction hépatique, âge à la date du prélèvement de sang, de sérum, de plasma ou d'urine,
  ◦ âge du patient à la date de la contamination,
  ◦ âge du patient au début du traitement,
  ◦ indice de masse corporelle (IMC),
  ◦ indice de sensibilité à l'insuline (HOMA),
  ◦ diabète,
  ◦ consommation d'alcool,
  ◦ degré de stéatose,
  ◦ mode de contamination,
  ◦ activité Metavir,
  ◦ score de fibrose hépatique mesuré selon le système Metavir (score F Metavir) ou selon le système Ishak ;

  et/ou
- un ou plusieurs facteurs virologiques, tels que :

  ◦ génotype viral, plus particulièrement génotype du ou des VHC,
  ◦ durée de l'infection,
  ◦ charge virale avant traitement, plus particulièrement charge en VHC avant traitement (CVavantTTT),
  ◦ charge virale, plus particulièrement charge en VHC, mesurée chez le patient à la date du début du traitement (charge virale à J0),
  ◦ charge virale, plus particulièrement charge en VHC, mesurée chez le patient à la date du prélèvement (charge virale à PBH, charge virale à la date de la cytoponction hépatique, charge virale à la date du prélèvement de sang, de sérum, de plasma ou d'urine) ;

  et/ou
- un ou plusieurs facteurs biologiques autres que les niveaux d'expression desdits gènes choisis, qui peuvent notamment être choisis parmi des concentrations, teneurs ou taux en protéines intracorporelles, des concentrations, teneurs ou taux en métabolites intracorporels, des concentrations, teneurs ou taux en éléments figurés du sang, des mesures représentatives de la quantité de fer circulant, de tels que :

  ◦ concentration en haptoglobine (Hapto),
  ◦ concentration en apolipoprotéine A1 (ApoA1),
  ◦ teneur en bilirubine totale (BLT),
  ◦ concentration en gamma glutamyl transpeptidase (GGT),
  ◦ concentration en aspartate aminotransférase (AST),
  ◦ concentration en alanine aminotransférase (ALT),
  ◦ teneur en plaquettes (PLQ),
  ◦ taux de prothrombine (TP),
  ◦ teneur en cholestérol HDL (Chol-HDL),
  ◦ teneur en cholestérol total,
  ◦ concentration en ferritine (Ferritine),
  ◦ teneur glycémique (glycémie),
  ◦ concentration en peptide C,

○ teneur en insuline (insulinémie),
○ concentration en triglycérides (TG),
○ teneur en albumine,
○ coefficient de saturation en fer de la transferrine (CS),
○ concentration en phosphatase alcaline (PAL).

Les tableaux 21 à 24 (combinaison n°24) illustrent les performances de la combinaison des niveaux de transcription de gènes (en l'occurrence, valeur de Ct qui a été mesurée pour les transcrits ARN de ce gène et qui a été normalisée par la méthode du $2^{-\Delta Ct}$), combinée en outre à un ou plusieurs autres facteurs biologiques, virologiques, cliniques (en l'occurrence, CVavantTTT et PAL).

Les tableaux 25 à 28 (combinaison n°24) illustrent les performances de la combinaison des niveaux de traduction de gènes (en l'occurrence, concentrations sériques de deux à cinq protéines), combinée en outre à un ou plusieurs autres facteurs biologiques, virologiques, cliniques (en l'occurrence, CVavantTTT, GGT et PAL ; ou Age, CVavantTTT et ALT ; ou Age, CVavantTTT et GGT ; ou CVavantTTT, AST et PAL ; ou IMC).

Cet ou ces autres facteurs peuvent être mesurés sur un échantillon de nature différente de celui utilisé pour mesurer les niveaux d'expression desdits gènes choisis. Par exemple, l'échantillon biologique pour la mesure des niveaux d'expression desdits gènes choisis parmi ladite liste de dix-sept gènes de l'invention peut être un échantillon de PBH ou de cytoponction hépatique, et l'échantillon biologique pour la mesure des valeurs desdits autres facteurs peut être un échantillon de fluide biologique, tel que sang, plasma ou sérum ou urine. De même, la nature du niveau d'expression mesuré peut être différente ; par exemple, pour la mesure du niveau d'expression desdits gènes choisis, on peut mesurer les niveaux de leur transcription en ARN, alors que, pour ceux desdits autres facteurs qui sont des facteurs biologiques, le niveau d'expression mesuré sera généralement une concentration protéique. Le dosage ou la mesure de certains de ces facteurs pourrait parfois être considéré comme le dosage du niveau de traduction (mesure de la concentration protéique) d'un gène autre qu'un gène choisi conformément à l'invention (par exemple GGT et/ou PAL et/ou ALT et/ou AST ; *cf.* tableaux 21 à 24 et 25 à 28 ci-dessous ; *cf.* exemples 2c) et 3b) ci-dessous).

Le nombre de gènes dont le niveau d'expression est dosé, et qui ne sont pas des gènes choisis parmi ladite liste de dix-sept gènes de l'invention (par exemple GGT et/ou PAL et/ou ALT et/ou AST), est de préférence au maximum de 18, plus particulièrement de 14 ou moins, plus particulièrement de 11 ou moins, plus particulièrement de 6 ou moins, plus particulièrement de 4 ou 3 ou 2 ou 1 ou 0, plus particulièrement de 3 ou 2 ou 1 ou 0, notamment de 2 ou 1 ou 0.

Avantageusement, cet ou ces autres facteurs sont ou comprennent un ou plusieurs facteurs biologiques, notamment un ou plusieurs facteurs parmi les facteurs biologiques suivants :

○ concentration en phosphatase alcaline (PAL), et/ou
○ concentration en gamma glutamyl transpeptidase (GGT),
plus particulièrement au moins PAL,
○ concentration en alanine aminotransférase (ALT) et/ou
○ concentration en aspartate aminotransférase (AST).

Les exemples 2c), 3b) et 6a) à 6c) ci-dessus donnent une illustration de telles combinaisons. Alternativement ou complémentairement, cet ou ces facteurs peuvent plus particulièrement être ou comprendre un ou plusieurs facteurs parmi les facteurs virologiques suivants :

○ charge virale avant traitement (CVavantTTT) ; et/ou
○ génotype du ou des VHC.

Les exemples 2c), 3b) et 6a) à 6c) ci-dessus donnent une illustration de telles combinaisons. Alternativement ou complémentairement, ce ou ces facteurs peuvent plus particulièrement être ou comprendre un ou plusieurs facteurs cliniques, notamment le facteur clinique score de fibrose hépatique, qui peut être mesuré selon le système Metavir (score F Metavir) ou selon le système Ishak, et/ou âge à la date du prélèvementTTT (Age), par exemple, âge à la date de la PBH, âge à la date de la cytoponction hépatique, âge à la date du prélèvement de sang, de sérum, de plasma ou d'urine, et/ou indice de masse corporelle (IMC).

L'exemple 6d) ci-dessus donne une illustration de telles combinaisons.

Selon un mode de réalisation particulier de l'invention, en sus du dosage des niveaux d'expression, plus particulièrement des niveaux de traduction, de gènes choisis parmi ladite liste de dix-sept gènes de l'invention, les moyens de l'invention peuvent en outre comprendre le dosage ou la mesure des autres facteurs suivants :

- un ou plusieurs facteurs cliniques qui est ou comprennent le score de fibrose hépatique (qui peut être mesuré selon le système Metavir (score F Metavir) ou selon le système Ishak), et/ou âge à la date du prélèvement (Age), par exemple, âge à la date de la PBH, âge à la date de la cytoponction hépatique, âge à la date du prélèvement de sang, de sérum, de plasma ou d'urine, et/ou indice de masse corporelle (IMC) ; et/ou

- un ou plusieurs facteurs virologiques qui est ou comprennent le génotype du ou des VHC et/ou la charge en VHC avant traitement ; et/ou
- un ou plusieurs facteurs biologiques autres que les niveaux d'expression de gènes choisis parmi MBL2, LGALS3BP, IL8, G1P2, CXCL10, CCL21, AFP, CRP, CXCL11, CXCL6, CXCL9, FGF7, MDK, MMP2, SFN, TGFB2, VEGFD, qui est ou comprennent la concentration en gamma glutamyl transpeptidase (GGP) et/ou la concentration en phosphatase alcaline (PAL) et/ou concentration en alanine aminotransférase (ALT) et/ou concentration en aspartate aminotransférase (AST).

Alternativement ou complémentairement, en sus du dosage des niveaux d'expression, plus particulièrement des niveaux de traduction, de gènes choisis parmi ladite liste de dix-sept gènes de l'invention, les moyens de l'invention peuvent en outre comprendre :

- le fait de déterminer le score de fibrose hépatique dudit sujet, plus particulièrement le fait de déterminer si le score de fibrose hépatique dudit sujet est un score qui, selon le système de score Metavir, est d'au plus F1 ou d'au moins F2, plus particulièrement d'au moins F2 ; et/ou
- le fait de déterminer si le ou les VHC dont est infecté ledit sujet comprennent un VHC de génotype 1, 4, 5 ou 6, plus particulièrement de génotype 1 ou 4, plus particulièrement de génotype 1.

Ces déterminations peuvent être faites lors de l'étape i), ou être faites indépendamment de l'étape i).

**Tableau 21 : valeurs de sensibilité (Se), spécificité (Spe), Valeur Prédictive Négative (VPN), Valeur Prédictive Positive (VPP), et d'erreur LOOCV qui peuvent être associées à une combinaison des niveaux de transcription de gènes choisis conformément à l'invention (transcrits ARN, plus particulièrement lorsque ces ARN sont issus d'un prélèvement de tissu ou cellules hépatiques), combinée en outre à d'autres facteurs biologiques et/ou à des facteurs cliniques et/ou à des facteurs virologiques**

| n° de la combinaison de gènes choisis (*cf.* tableau 12) | Autres facteurs biologiques et/ou facteurs cliniques et/ou facteurs virologiques, combinés aux niveaux d'expression de la combinaison de gènes choisis | Modèle de classification utilisé | Se | Spe | VPN | VPP | Erreur LOOCV |
|---|---|---|---|---|---|---|---|
| **24** ARN(§) | charge virale avant traitement (CVavantTTT) ; concentration en phosphatase alcaline (PAL) | mROC (*) | 81 | 71 | 83 | 68 | ND |
| (*) : les valeurs de Se, Spe, VPN, VPP et Erreur LOOCV indiquées sont celles de la fonction du tableau 22 ci-dessous<br>(§) : plus particulièrement, ARN issus d'un prélèvement de tissu ou cellules hépatiques<br>ND = non déterminée | | | | | | | |

**Tableau 22 : exemple de modèle mROC (fonction Z) pour une combinaison de gènes choisis conformément à l'invention (dosage de leurs niveaux de transcription en ARN), combinée en outre à d'autres facteurs** (facteurs biologiques autres que les niveaux d'expression de gènes choisis conformément à l'invention et/ou facteurs cliniques et/ou facteurs virologiques), et **exemple de seuil PT pour cette fonction (en l'occurrence, seuil maximisant l'indice de Youden $\delta$),**

| n° de la combinaison de gènes choisis (*cf.* tableau 12) | Autres facteurs | Exemple de fonction Z (modèle mROC) | Nom de la fonction | Seuil PT (seuil $\delta$) |
|---|---|---|---|---|
| **24** ARN(§) | charge virale avant traitement (CVavantTTT) ; concentration en phosphatase alcaline (PAL) | $Z = -0,051 \times CXCL10^t + 0,032 \times IL8 + 0,357 \times CCL21^t + 0,189 \times MDK^t + 0,182 \times LGALS3BP^t + 0,052 \times CVavantTTT^t + 2,644 \times PAL^t$ | **Z24ARNsupp** | 5,454 |
| (§) : plus particulièrement, ARN issus d'un prélèvement de tissu ou cellules hépatiques | | | | |

**Tableau 23 : exemple de valeurs de paramètre Box-Cox ($\lambda$) utilisables dans la fonction Z indiquée au tableau 23 ci-dessus.**

| Gènes, pour lesquels il est conseillé de normaliser la valeur du niveau de transcription (ARN) | Exemple de valeur du paramètre lambda utilisable pour les fonctions Z du tableau 22 ci-dessus (*) |
|---|---|
| **CXCL10** | 0,04 |
| **CCL21** | 0,02 |
| **LGALS3BP** | -0,07 |
| **MDK** | 0,15 |
| **CVavantTTT** | 0,2 |
| **PAL** | -0,26 |
| (*) : paramètre lambda des transformations Box-Cox [$BMQ^t = (BMQ^\lambda - 1)/\lambda$] | |

**Tableau 24 : pour la fonction du tableau 22, valeur d'AUC**

| Nom de la fonction | AUC | AUC limite inférieure | AUC limite supérieure |
|---|---|---|---|
| **Z24ARNsupp** (*cf.* tableau 22) | 0,827 | 0,730 | 0,894 |

**Tableau 25 : valeurs de sensibilité (Se), spécificité (Spe), Valeur Prédictive Négative (VPN), Valeur Prédictive Positive (VPP), et d'erreur LOOCV qui peuvent être associées à une combinaison des niveaux de traduction protéique (plus particulièrement en protéines sériques) de gènes choisis conformément à l'invention, combinée en outre à d'autres facteurs biologiques et/ou à des facteurs cliniques et/ou à des facteurs virologiques**

| n° de la combinaison de gènes choisis (*cf.* tableau 12) | Autres facteurs biologiques et/ou facteurs cliniques et/ou facteurs virologiques, combinés aux niveaux d'expression de la combinaison de gènes choisis | Modèle de classification utilisé | Se | Spe | VPN | VPP | Erreur LOOCV |
|---|---|---|---|---|---|---|---|
| **24 protéines ($)** | charge virale avant traitement (CVavantTTT) ; concentration en gamma glutamyl transpeptidase (GGT) ; concentration en phosphatase alcaline (PAL) | mROC (*) | 82 | 77 | 83 | 75 | ND |
| **15** protéines1($) | âge à la date du prélèvement (Age) ; charge virale avant traitement (CVavantTTT) ; concentration en alanine aminotransférase (ALT) | mROC (*) | 82 | 77 | 83 | 75 | ND |
| **15** protéines2($) | âge à la date du prélèvement (Age) ; charge virale avant traitement (CVavantTTT) ; concentration en gamma glutamyl transpeptidase (GGT) | mROC (*) | 83 | 74 | 84 | 74 | ND |
| **15** protéines3($) | charge virale avant traitement (CVavantTTT) ; concentration en aspartate aminotransférase (AST) ; concentration en phosphatase alcaline (IMC) | mROC (*) | 86 | 77 | 86 | 76 | ND |
| **15** protéines4($) | indice de masse corporelle (IMC) | mROC (*) | 81 | 78 | 82 | 76 | ND |
| (*) : les valeurs de Se, Spe, VPN, VPP et Erreur LOOCV indiquées sont celles de la fonction du tableau 26 ci-dessous<br>($) : plus particulièrement, protéines contenues dans un prélèvement sanguin et/ou dans la partie sérique de ce prélèvement<br>ND = non déterminée | | | | | | | |

**Tableau 26 : exemple de modèle mROC (fonction Z) pour une combinaison de gènes choisis conforme à l'invention (dosage de leurs niveaux de traduction, plus particulièrement en protéines sériques), combinée en outre à d'autres facteurs (facteurs biologiques autres que les niveaux d'expression de cinq gènes choisis conformément à l'invention et/ou facteurs cliniques et/ou facteurs virologiques), et exemple de seuil PT pour cette fonction (en l'occurrence, seuil maximisant l'indice de Youden $\delta$)**

| n° de la combinaison de gènes choisis (*cf.* tableau 12) | Autres facteurs | Exemple de modèle de classification multivariée (modèle mROC) | Nom de la fonction | Seuil PT (seuil $\delta$) |
|---|---|---|---|---|
| **24** protéines($) | charge virale avant traitement (CVavantTTT) ; concentration en gamma glutamyl transpeptidase (GGT) ; concentration en phosphatase alcaline (PAL) | $Z = -0,353 \times MDK + 0,059 \times IL8^t + 0,456 \times LGALS3BP^t + 0,010 \times CXCL10^t - 0,118 \times CCL21^t + 0,058 \times CVavantTTT^t + 0,227 \times GGG^t + 0,408 \times PAL^t$ | **Z24PROTsupp** | 4,516 |
| **15** protéines1($) | âge à la date du prélèvement (Age) ; charge virale avant traitement (CVavantTTT) ; concentration en alanine aminotransférase (ALT) | $Z = 0,569 \times LGALS3BPt + 0,033 \times CXCL10^t + 0,059 \times CVavantTTT^t - 0,899 \times Age^t - 0,538 \times ALT^t$ | **Z15PROTsupp1** | -2,345 |
| **15** protéines2($) | âge à la date du prélèvement (Age) ; charge virale avant traitement (CVavantTTT) ; concentration en gamma glutamyl transpeptidase (GGT) | $Z = 0,492 \times LGALS3BP^t + 0,018 \times CXCL10^t - 0,701 \times Age^t + 0,058 \times CVavantTTT^t + 0,202 \times GGT^t$ | **Z15PROTsupp2** | 0,696 |
| **15** protéines3($) | charge virale avant traitement (CVavantTTT) ; concentration en aspartate aminotransférase (AST) ; concentration en phosphatase alcaline (IMC) | $Z = 0,499 \times LGALS3BP^t + 0,028 \times CXCL10^t + 0,06 \times CVavantTTT^t - 1,147 \times AST^t + 0,931 \times PAL^t$ | **Z15PROTsupp3** | 3,862 |
| **15** protéines4($) | indice de masse corporelle (IMC) | $Z = 0,451 \times LGALS3BP^t + 0,033 \times CXCL10^t - 0,535 \times IMC^t$ | **Z15PROTsupp4** | 0,375 |
| ($) : plus particulièrement, protéines contenues dans un prélèvement sanguin et/ou dans la partie sérique de ce prélèvement | | | | |

**Tableau 27 : exemple de valeurs de paramètre Box-Cox ($\lambda$) utilisables dans les fonctions Z indiquées au tableau 26 ci-dessus**

| Gènes, pour lesquels il est conseillé de normaliser la valeur du niveau de transcription (ARN) | Exemple de valeur du paramètre lambda utilisable pour les fonctions Z du tableau 26 ci-dessus (*) |
|---|---|
| IL8 | 0,23 |
| LGALS3BP | 0,33 |
| CXCL10 | 0,41 |
| CCL21 | -0,01 |
| CVavantTTT | 0,20 |
| GGT | -0,01 |

(suite)

| Gènes, pour lesquels il est conseillé de normaliser la valeur du niveau de transcription (ARN) | Exemple de valeur du paramètre lambda utilisable pour les fonctions Z du tableau 26 ci-dessus (*) |
|---|---|
| PAL | -0,11 |
| ALT | -0,09 |
| AST | -0,30 |
| Age | 0,09 |
| IMC | 0,08 |
| (*) : paramètre lambda des transformations Box-Cox [$BMQ^t = (BMQ^\lambda-1)/\lambda$] | |

**Tableau 28 : pour les fonctions Z du tableau 26, valeur d'AUC**

| Nom de la fonction | AUC | AUC limite inférieure | AUC limite supérieure |
|---|---|---|---|
| **Z24PROTsupp** | **0,872** | 0,812 | 0,916 |
| **Z15PROTsupp1** | **0,877** | 0,817 | 0,920 |
| **Z15PROTsupp2** | **0,872** | 0,810 | 0,916 |
| **Z15PROTsupp3** | **0,869** | 0,806 | 0,913 |
| **Z15PROTsupp4** | **0,834** | 0,763 | 0,887 |

Selon un mode de réalisation de l'invention, parmi ladite liste optionnelle de onze gènes (AFP, CRP, CXCL11, CXCL6, CXCL9, FGF7, MDK, MMP2, SFN, TGFB2, VEGFD), on choisit 0, 1 ou 2 gènes parmi MDK, TGFB2, FGF7, CXCL6, MMP2, SFN, plus particulièrement parmi MDK, TGFB2, FGF7, CXCL6, parmi MDK, TGFB2, FGF7, CXCL6, plus particulièrement MDK ou au moins MDK.

Selon un mode de réalisation de l'invention, le nombre de ces gènes choisis conformément à l'invention (parmi ladite liste de dix-sept gènes de l'invention) est de 3, 4 ou 5, et :

- parmi MBL2, LGALS3BP, IL8, on choisit au moins MBL2 et/ou IL8 ;
- parmi CXCL10, G1P2, CCL21, on choisit au moins G1P2 et/ou au moins CCL21 ;
- parmi ladite liste optionnelle de onze gènes (AFP, CRP, CXCL11, CXCL6, CXCL9, FGF7, MDK, MMP2, SFN, TGFB2, VEGFD), on choisit au moins 1 gène, notamment 1, 2, ou 3 gènes ; plus particulièrement on choisit 0, 1 ou 2 gènes parmi MDK, TGFB2, FGF7, CXCL6, MMP2, SFN, plus particulièrement parmi MDK, TGFB2, FGF7, CXCL6, plus particulièrement au moins MDK.

Selon un mode particulier de réalisation, les niveaux d'expression dosés pour les gènes ainsi choisis sont des niveaux de transcription.

Selon un mode de réalisation de l'invention, le nombre de gènes choisis conformément à l'invention (parmi ladite liste de dix-sept gènes de l'invention) est de 2, 3 ou 4. Avantageusement, l'échantillon biologique est un échantillon de fluide biologique notamment un échantillon de fluide intracorporel tel que du sang, du sérum, du plasma, ou un échantillon d'urine. Les niveaux d'expression dosés pour ces gènes peuvent alors être des niveaux de traductions.

Parmi ladite liste optionnelle de onze gènes (AFP, CRP, CXCL11, CXCL6, CXCL9, FGF7, MDK, MMP2, SFN, TGFB2, VEGFD), on peut alors choisir 0, 1 ou 2 gènes parmi MDK, TGFB2, FGF7, CXCL6, MMP2, SFN, plus particulièrement parmi MDK, TGFB2, FGF7, CXCL6, plus particulièrement MDK ou au moins MDK.

On peut alors notamment choisir :

- au moins LGALS3BP parmi MBL2, LGALS3BP, IL8,
- au moins CXCL10 parmi CXCL10, G1P2, CCL21, et
- 0, 1 ou 2 gènes parmi ladite liste optionnelle de onze gènes, plus particulièrement 0, 1 ou 2 gènes parmi MDK, TGFB2, FGF7, CXCL6, MMP2, SFN, plus particulièrement 0, 1 ou 2 gènes parmi MDK, TGFB2, FGF7, CXCL6, plus particulièrement MDK ou au moins MDK ;

*cf.* par exemple, la combinaison n°24 dosée en niveaux de traductions en tableaux 17 à 20 ci-dessus.

Comme ci-dessus indiqué et ci-dessous illustré, en sus des niveaux d'expression, plus particulièrement de traductions, de ces gènes choisis, on peut doser ou mesurer un ou plusieurs facteurs virologiques (tels que CVavantTTT et/ou génotype(s) du ou des VHC) et/ou un ou plusieurs facteurs biologiques autres (tels que GGT et/ou PAL) et/ou un ou plusieurs facteurs cliniques (tels que score de fibrose hépatique) ; *cf.* par exemple, la combinaison n°24 dosée en niveaux de traductions et combinée aux facteurs CVavantTTT, GGT et PAL, en tableaux 25 à 28 ci-dessus.

On peut remarquer que la combinaison n°24 présente de meilleures sensibilité et spécificité lorsqu'elle est dosée en niveaux de traductions que lorsqu'elle est dosée en niveaux de transcriptions (sensibilité de 82% et spécificité de 74% pour le dosage des protéines sériques, *versus* sensibilité de 73% et spécificité de 74% pour le dosage des ARN hépatiques ; *cf.* tableaux 17 et 13 ci-dessous).

Selon un mode de réalisation de l'invention, la combinaison de gènes choisis parmi ladite liste de dix-sept gènes de l'invention est la combinaison n°15, ou la n°9, ou la n°24. Avantageusement, les niveaux d'expression de ces gènes choisis sont des niveaux de traductions.

Comme ci-dessus indiqué et ci-dessous illustré, en sus des niveaux d'expression de ces gènes choisis, on peut doser ou mesurer un ou plusieurs facteurs virologiques (tels que CVavantTTT et/ou génotype(s) du ou des VHC) et/ou un plusieurs facteurs biologiques autres (tels que GGT et/ou PAL et/ou ALT et/ou AST) et/ou un ou plusieurs facteurs cliniques (tels que score de fibrose hépatique et/ou Age et/ou IMC).

Selon un aspect complémentaire de l'invention, l'invention est relative à des produits ou réactifs pour la détection et/ou la détermination et/ou le dosage desdites mesures, plus particulièrement pour la détection et/ou le dosage des niveaux d'expression desdits gènes choisis, et à des articles manufacturés, des compositions, des compositions pharmaceutiques, des kits, des tubes, des supports solides comprenant de tels réactifs, ainsi qu'à des systèmes informatiques (notamment, produit programme d'ordinateur et dispositif informatique), qui sont spécialement adaptés à la mise en oeuvre d'une méthode de l'invention.

L'invention peut être mise en oeuvre via notamment un **réactif** qui détecte de manière spécifique un produit de transcription (ARN) d'un desdits gènes choisis parmi ladite liste de dix-sept gènes de l'invention, ou un produit de traduction d'un desdits gènes choisis parmi ladite liste de dix-sept gènes de l'invention (protéine, ou forme post-traductionnelle de cette protéine, telle que fragment spécifique de cette protéine).

L'invention peut être mise en oeuvre via des réactifs qui détectent de manière spécifique chacun des produits de transcription (ARN) desdits gènes choisis parmi ladite liste de dix-sept gènes de l'invention, ou chacun des produits de traduction desdits gènes choisis parmi ladite liste de dix-sept gènes de l'invention (protéine, ou forme post-traductionnelle de cette protéine, telle que fragment spécifique de cette protéine).

Avantageusement, un ensemble de tels réactifs est formé, qui détecte chacun desdits produits de transcription desdits gènes choisis et/ou qui détecte chacun desdits produits de traduction desdits gènes choisis parmi ladite liste de dix-sept gènes de l'invention, c'est-à-dire un ensemble de réactifs qui détecte de manière spécifique au moins un produit d'expression pour chacun de ces gènes.

De préférence, lesdits réactifs non seulement détectent spécifiquement un produit de transcription ou traduction, mais permettent également de le quantifier.

L'invention est notamment relative à un **article manufacturé** comprenant lesdits réactifs comme produit de combinaison (ou sous forme combinée, ou en préparation combinée), notamment pour leur utilisation simultanée, séparée ou étalée dans le temps. Cet article manufacturé peut par exemple se présenter sous la forme d'un ensemble de réactifs, ou d'un kit.

Bien entendu les caractéristiques de combinaisons de gènes choisies décrites ci-dessus, ainsi que celles illustrées ci-dessous, s'appliquent aux réactifs de l'invention *mutatis mutandis.*

Lesdits réactifs peuvent par exemple s'hybrider de manière spécifique à l'ARN desdits gènes choisis et/ou à l'ADNc correspondant à ces ARN (en conditions au moins stringentes d'hybridation), ou se lier de manière spécifique aux protéines codées par lesdits gènes choisis (ou à des fragments spécifiques de ces protéines), par exemple selon une réaction de type antigène-anticorps.

Des conditions au moins stringentes d'hybridation sont connues de la personne du métier. Il peut par exemple s'agir des conditions suivantes :

- pour une hybridation sur filtre : dans 5xSSC, 2% dodécyl sulfate de sodium (SDS), 100 microgrammes/mL d'ADN simple-brin à 55-65°C pendant 8 heures, et lavage dans 0,2xSSC et 0,2% SDS à 60-65°C pendant trente minutes ;
- pour une hybridation par PCR : les conditions de PCR indiquées en exemple 1 ci-dessous.

Lesdits réactifs de l'invention peuvent notamment être :

- des acides nucléiques (ADN, ARN, ARNm, ADNc), y compris des aptamères oligonucléotidiques, optionnellement marqués pour permettre leur détection, notamment avec des marqueurs fluorescents bien connus de la personne du métier, ou

- des ligands de protéines, tels que des protéines, polypeptides ou peptides, par exemple des aptamères, et/ou des anticorps ou fragments d'anticorps.

Les acides nucléiques de l'invention peuvent par exemple être des amorces et/ou des sondes (*cf.* SEQ ID NO : 1 à 34 dans le tableau 32 ci-dessous), notamment des paires d'amorces (*cf.* les paires d'amorces indiquées dans le tableau 32 ci-dessous). Pour chacun desdits gènes choisis parmi ladite liste de dix-sept gènes de l'invention, la personne du métier peut construire une paire d'amorces et/ou une sonde qui s'hybrident spécifiquement à ce gène. Un article manufacturé de l'invention peut donc comprendre le nombre d'amorces et/ou sondes nécessaire à la détection des ARN ou ADNc de chacun desdits gènes choisis.

La séquence des acides nucléiques de l'invention peut par exemple être constituée de 9 à 40 nucléotides, plus particulièrement de 10 à 30 nucléotides, plus particulièrement de 14 à 29 nucléotides, plus particulièrement de 19 à 24 nucléotides.

Les séquences des amorces d'une même paire peuvent par exemple être les séquences d'un fragment de la séquence d'un desdits gènes choisis et d'un fragment de sa séquence complémentaire (*cf.* tableau 1 indiquant les numéros d'accession de séquences de ces gènes). L'une et/ou l'autre de ces deux séquences d'amorces peuvent ne pas être strictement identiques à la séquence d'un fragment du gène ou de sa séquence complémentaire ; l'une et/ou l'autre de ces deux séquences d'amorces peuvent :

- en dériver par une ou plusieurs substitutions et/ou additions et/ou délétions nucléotidiques, plus particulièrement par une ou plusieurs substitutions nucléotidiques, et/ou présenter une identité de séquence d'au moins 80%, ou d'au moins 85%, ou d'au moins 90%, ou d'au moins 95% avec la séquence de ce fragment ou de sa séquence complémentaire (identité calculée sur la plus longue des deux séquences alignées - alignement optimal),
- dans la mesure où la paire d'amorces en résultant a conservé la capacité à s'hybrider spécifiquement à un desdits gènes choisis.

Une paire d'amorces de l'invention présente avantageusement un delta Tm de 1°C environ ou moins. Selon un mode de réalisation de l'invention, une paire d'amorces de l'invention cible un amplicon de 70 à 120 pb environ (c'est-à-dire que l'amorce sens et l'amorce antisens s'hybrident à des positions telles sur l'acide nucléique cible, que l'amplicon produit par élongation de ces amorces hybridées a une longueur de 70 à 120 pb environ).

Des exemples de telles amorces et paires d'amorces sont présentées dans le tableau 32 ci-dessous (SEQ ID NO: 1 à 34, formant 17 paires d'amorces).

La séquence d'une sonde de l'invention, peut par exemple être :

- la séquence d'un fragment de la séquence d'un desdits gènes choisis (*cf.* tableau 1 indiquant les numéros d'accession de séquences de ces gènes), ledit fragment s'hybridant de manière spécifique à la séquence de ce gène ;
- une séquence :

  ◦ qui dérive de la séquence d'un tel fragment par une ou plusieurs substitutions et/ou additions et/ou délétions nucléotidiques, plus particulièrement par une ou plusieurs substitutions nucléotidiques, et/ou une séquence qui présente une identité de séquence d'au moins 80%, ou d'au moins 85%, ou d'au moins 90%, ou d'au moins 95% avec la séquence de ce fragment ou de sa séquence complémentaire (identité calculée sur la plus longue des deux séquences alignées -alignement optimal-), mais
  ◦ qui a conservé la capacité à s'hybrider spécifiquement à un desdits gènes choisis ;

et/ou
- une séquence complémentaire de telles séquences.

Une sonde de l'invention peut notamment être une sonde pour amplification en temps réel, destinée à être utilisée avec une paire d'amorces de l'invention. Alternativement, la détection en PCR en temps réel peut utiliser des molécules dites intercalentes (par exemple ; SYB green) qui ont la propriété de s'intercaler dans les structures doubles brins.

Les ligands de l'invention qui se lient de manière spécifique aux protéines codées par les gènes choisis parmi ladite liste de dix-sept gènes de l'invention (ou à des fragments spécifiques de ces protéines) peuvent être par exemple des protéines, polypeptides ou peptides, par exemple des aptamères, ou des anticorps ou fragments d'anticorps.

Pour chacun desdits gènes choisis, la personne du métier peut produire un tel ligand.

Des anticorps peuvent par exemple être produits par immunisation d'un mammifère non-humain (tel qu'un lapin) par une protéine codée par ledit gène choisi, ou par un fragment antigénique d'une telle protéine, éventuellement associée ou couplée à un adjuvant d'immunisation (tel qu'un adjuvant de Freund ou tel que KLH -*keyhole limpet hemo-*

*cyanin*-), par exemple par injection intrapéritonéale ou sous-cutanée, et par collecte des anticorps ainsi obtenus dans le sérum dudit mammifère.

Des anticorps monoclonaux peuvent être produits selon une technique d'hybridation lymphocytaire (hybridomes) telle que la technique de Köhler and Milstein 1975 (*cf.* également US 4 376 110), la technique d'hybridomes à cellules B humaines (Kosbor *et al.* 1983; Cole *et al.* 1983), ou la technique d'immortalisation des lymphocytes à l'aide du virus d'Epstein-Barr -EBV- (Cole *et al.* 1985). De tels anticorps peuvent par exemple être des IgG, IgM, IgE, IgA, IgD ou toute sous-classe de ces immunoglobulines.

Des anticorps modifiés par génie génétique peuvent être produits, tels que des anticorps recombinant, chimères, humanisés par greffage d'un ou plusieurs CDR -*Complementary Determining Region*-.

Les anticorps mis en oeuvre dans l'invention peuvent être des fragments d'anticorps ou des dérivés artificiels de tels fragments, dans la mesure où ces fragments ou dérivés présentent ladite propriété de liaison spécifique. De tels fragments peuvent par exemple être des fragments Fab, F(ab')2, Fv, Fab/c, scFv (*single chain Fragment variable*).

Des exemples d'anticorps sont donnés dans le tableau 29 suivant.

**Tableau 29 : exemples d'anticorps spécifiques**

| Gène codant | Anticorps | Exemple de fournisseur | Référence catalogue du produit |
|---|---|---|---|
| MBL2 | Anticorps polyclonal anti-MBL2 humain (IgG de lapin) | Sigma-Aldrich | HPA 002027 |
| G1P2 | Anticorps polyclonal anti-G1P2 humain (IgG de chèvre) | R&D Systems | AF4845 |
| MDK | Anticorps polyclonal anti-MDK humain (IgG de lapin) | Calbiochem | NE 1044 |
| LGALS3BP | Anticorps polyclonal anti-LGALS3BP humain (IgG de lapin) | Sigma-Aldrich | AV54779 |
| CXCL10 | Anticorps polyclonal anti-CXCL10/IP-10 humain (IgG de chèvre) | R&D Systems | AB-266-PB |
| FGF7 | Anticorps monoclonal anti-FGF7 produit chez la souris (clone 11F1, IgG2b) | Novus Biologicals | NB 110-74673 |
| IL8 | Anticorps monoclonal anti-IL8 humaine produit chez la souris (clone 6G4) | Sigma-Aldrich | WH0003576M5 |
| TGFB2 | Anticorps monoclonal anti-TGFB2 huamin produit chez la souris (clone Zg-12, IgG2b) | Santa Cruz Biotechnology | sc-80347 |
| CCL21 | Anticorps polyclonal anti- CCL21 humain (IgG de souris) | Novus Biologicals | H0006366-BO1P |
| CXCL6 | Anticorps monoclonal anti-CXCL6/GCP2 humaine (clone 60910), (IgG1 de souris) | R&D Systems | MAB333 |
| MMP2 | Anticorps anti-MMP2 [EP1329Y] | Abcam | ab51127 |
| CXCL11 | Anticorps monoclonal (clone 87328) anti- CXCL11/I-TAC humaine (IgG2A de souris) | R&D Systems | MAB672 |
| AFP | Anticorps monoclonal anti-AFP produit chez la souris (clone 39, IgG1) | Santa Cruz Biotechnology | sc-130302 |
| VEGFD | Anticorps monoclonal anti-VEGFD humaine produit chez la souris (clone MM0007-7E79, IgG2) | Novus Biologicals | NB 110-60973 |
| CRP | Anticorps monoclonal anti-CRP humaine produit chez la souris (clone 3H109, IgG1) | Santa Cruz Biotechnology | sc-70883 |
| CXCL9 | Anticorps monoclonal anti- CXCL9 humaine produit chez la souris (clone 49106, IgG1) | R&D Systems | MAB392 |

D'autres exemples de moyens de dosage des niveaux de transcription de gènes choisis sont par ailleurs présentés dans le tableau 44 ci-dessous (kits d'immuno-essai).

Lesdits réactifs peuvent en outre comprendre un marqueur pour leur détection (par exemple, un fluorophore).

Lesdits réactifs peuvent être sous la forme de composition(s), de composition(s) pharmaceutique(s), par exemple dans un(des) tube(s) ou dans un(des) puit(s) d'une plaque d'amplification d'acide nucléique

Lesdits réactifs peuvent être en mélange, ou sous formes distinctes ou physiquement séparées l'une de l'autre.

Lesdits réactifs peuvent être fixés à un support solide, par exemple un support en polymère, en plastique, notamment en polystyrène, en verre ou en silicium.

Lesdits réactifs peuvent être attachés directement ou indirectement audit support solide, par exemple *via* un agent de liaison ou de capture qui est attaché au support solide. Cet agent de liaison ou de capture peut comprendre une partie fixée audit support solide et une partie qui comprend un ligand qui se lie de manière spécifique à un desdits gènes choisis. Un tel ligand peut par exemple être un anticorps, un anticorps monoclonal, notamment un anticorps humain tel qu'une IgG, IgM ou IgA, ou un fragment d'un tel anticorps ayant conservé la spécificité de liaison.

Ledit support solide peut par exemple être une plaque en plastique, notamment en polystyrène, comprenant plusieurs puits d'analyse, telle qu'une plaque de titrage ou microtitrage protéique, par exemple, une plaque ELISA.

Ledit support solide peut encore être des microbilles magnétiques ou non, pour micro-titrages, par exemple selon la technique décrite par Luminex.

Ledit support solide peut par exemple être une puce à acide nucléique, à protéine ou à peptide, par exemple une puce en plastique, verre ou silicium.

Lesdits réactifs peuvent ne pas être fixés à un support solide et peuvent par exemple être contenus dans une solution, telle qu'un tampon, par exemple pour leur conservation jusqu'à utilisation. Plus particulièrement, les réactifs peuvent être des acides nucléiques non liés à un support solide, dont la séquence nucléotidique est adaptée à l'amplification spécifique (cas d'amorces ou de paires d'amorces) et/ou à l'hybridation spécifique (cas de sondes) du produit de transcription (ARN) d'un desdits gènes choisis parmi ladite liste de dix-sept gènes de l'invention.

En sus de réactifs qui détectent les produits de transcription ou de traduction de gènes de mammifères, plus particulièrement de gènes humains, et notamment de gènes choisis parmi ladite liste de dix-sept gènes de l'invention, un article manufacturé conforme à l'invention peut optionnellement comprendre d'autres réactifs, par exemple des réactifs permettent de doser ou déterminer un ou plusieurs facteurs virologiques et/ou un ou plusieurs facteurs cliniques.

Par exemple, un article manufacturé conforme à l'invention peut comprendre des réactifs qui détectent de manière spécifique un ou des virus de l'hépatite, et/ou son ou leur génotype.

Selon un mode de réalisation, l'invention est relative à un article manufacturé comprenant des réactifs en préparation combinée pour leur utilisation simultanée, séparée ou étalée dans le temps, lesdits réactifs étant constitués :

- de réactifs qui détectent de manière spécifique (de préférence, qui détectent de manière spécifique et permettent de quantifier) chacun des produits de transcription ou de traduction de 2 à 9 gènes de mammifères, plus particulièrement de 2 à 9 gènes humains, (par exemple, en s'hybridant de manière spécifique à l'ARN de ces gènes et/ou à l'ADNc obtenu par transcription inverse de ces ARN, ou en se liant de manière spécifique aux protéines codées par ces gènes), lesdits 2 à 9 gènes de mammifères, ou, le cas échéant, lesdits 2 à 9 gènes humains, comprenant lesdits gènes choisis parmi ladite liste de dix-sept gènes de l'invention, et

- optionnellement, de réactifs qui permettent de détecter, doser ou déterminer un ou plusieurs facteur(s) virologique(s) tel(s) que défini(s) dans la description de l'invention ; et/ou un ou plusieurs facteur(s) biologique(s) autre(s) tel(s) que défini(s) dans la description de l'invention ;

- optionnellement, de réactifs qui détectent de manière spécifique (de préférence qui détectent de manière spécifique et permettent de quantifier) un virus de l'hépatite et/ou le génotype d'un virus de l'hépatite.

Dans cet article manufacturé, le nombre de gènes de mammifères, plus particulièrement de gènes humains dont les produits de transcription ou de traduction peuvent être détectés peut être de 2 à 35, plus particulièrement de 2 à 34, plus particulièrement de 2 à 33, plus particulièrement de 2 à 28, plus particulièrement de 2 à 26, plus particulièrement de 2 à 25, plus particulièrement de 2 à 24, plus particulièrement de 2 à 23, notamment de 2 à 22, plus particulièrement de 2 à 20, plus particulièrement de 2 à 19, plus particulièrement de 2 à 10, plus particulièrement de 2 à 9, plus particulièrement de 2 à 8, plus particulièrement de 2 à 7, plus particulièrement de 2 à 6 (par exemple de 2, 3, 4, 5 ou 6), plus particulièrement de 2 à 5 (par exemple de 2, 3, 4 ou 5).

Les gènes de mammifères, plus particulièrement les gènes humains, dont les produits de transcription ou de traduction peuvent être détectés par les réactifs contenus dans l'article manufacturé de l'invention comprennent lesdits gènes choisis parmi ladite liste de dix-sept gènes de l'invention, et optionnellement d'autres gènes, qui ne sont pas des gènes choisis parmi ladite liste de dix-sept gènes de l'invention, mais dont le produit d'expression, plus particulièrement, de traduction peut être d'intérêt, tels que les gènes ici listés en tant que « autres facteurs biologiques » (par exemple, le gène codant la gamma glutamyl transpeptidase ou GGT et/ou le gène codant la phosphatase alcaline ou PAL).

Dans l'article manufacturé de l'invention, le nombre de réactifs qui détectent de manière spécifique le produit d'expression de gènes de mammifère (plus particulièrement de gènes humains) qui ne sont pas des gènes choisis parmi ladite liste de dix-sept gènes de l'invention (par exemple, un réactif détectant de manière spécifique GGT et un réactif détectant de manière spécifique PAL), est de préférence au maximum de 5, plus particulièrement de 4 ou moins, plus particulièrement de 3 ou moins, plus particulièrement de 2 ou moins, plus particulièrement de 2 ou 1 ou 0.

Ledit article manufacturé peut donc par exemple être :

- un ou des tubes,
- un kit, notamment un kit comprenant un ou des tubes,
- un support solide, par exemple, en plastique, polystyrène, verre, silicium ou polymère, ou comprenant un matériau magnétique tel que de l'oxyde de fer, tel que :

  ◦ une plaque en plastique comprenant plusieurs puits d'analyse telle que :

    ▪ une plaque d'amplification d'acide nucléique comportant des puits destinés à recevoir un échantillon biologique et un mélange réactionnel d'amplification d'acide nucléique,
    ▪ une plaque de titrage ou microtitrage protéique, plus particulièrement une plaque ELISA,

  ◦ des microbilles magnétiques (par exemple, des microbilles faites à partir d'oxyde de fer, et recouvertes d'un polymère auquel les protéines ou polypeptides peuvent adhérer ou être attachées par couplage chimique) ;
  ◦ une puce à acide nucléique, à protéine, à polypeptide ou à peptide.

Optionnellement, l'article manufacturé de l'invention comprend en outre des instructions (par exemple, une feuille d'instructions) pour doser le niveau d'expression desdits gènes choisis sur un échantillon biologique collecté ou obtenu dudit sujet, plus particulièrement pour mettre en oeuvre une méthode de l'invention.

Ledit article manufacturé peut en outre comprendre un ou plusieurs des éléments suivants :

- un instrument pour le prélèvement dudit échantillon, notamment :

  ◦ une aiguille et/ou une seringue, plus particulièrement une aiguille et/ou une seringue pour le prélèvement d'un liquide intracorporel, tel que du sang, et/ou
  ◦ une aiguille adaptés à la cyto-ponction hépatique, par exemple une aiguille de diamètre 18 à 22G), et/ou
  ◦ une aiguille et/ou un cathéter et/ou un pistolet à biopsie adaptés à la PBH ;

- un produit programme d'ordinateur ou logiciel, notamment un produit programme d'ordinateur ou logiciel d'analyse statistique, par exemple un produit programme d'ordinateur de l'invention tel que ci-dessous décrit ;
- des réactifs d'extraction d'ARN ;
- une transcriptase inverse ;
- une polymérase, par exemple une Taq polymérase ;
- des nucléotides (dNTP).

L'invention est notamment relative audit article manufacturé, ou auxdits réactifs, pour leur utilisation dans une méthode pour pronostiquer si un sujet infecté par un ou des VHC a une forte probabilité de répondre à un traitement anti-VHC qui comprendra l'administration d'interféron et de ribavirine (ou de leurs pro-drogues), ou si, au contraire, ce sujet a une forte probabilité de ne pas répondre à ce traitement anti-VHC, plus particulièrement audit article manufacturé, ou auxdits réactifs, pour leur utilisation dans une méthode pronostique de l'invention.

Cette utilisation peut notamment comprendre :

- le prélèvement d'un échantillon biologique dudit sujet, notamment par insertion d'une aiguille ou cathéter dans le corps dudit sujet, et
- la mise en oeuvre desdits réactifs dans ladite méthode sur cet échantillon biologique, ou sur un échantillon comprenant des acides nucléiques et/ou protéines et/ou polypeptides et/ou peptides extraits ou purifiés dudit échantillon biologique, ou sur un échantillon comprenant des ADNc susceptibles d'avoir été obtenus par transcription inverse desdits acides nucléiques.

Cette utilisation peut par exemple comprendre :

- le prélèvement d'un échantillon biologique dudit sujet, optionnellement transformé par :

  ◦ extraction ou purification des ARN dudit échantillon prélevé et, optionnellement par transcription inverse des ARN extraits, ou par

  ◦ extraction ou purification de ses protéines dudit échantillon prélevé, et

- la mise en oeuvre desdits réactifs de l'invention sur cet échantillon biologique optionnellement transformé.

Ledit prélèvement d'échantillon biologique peut être fait par insertion d'un instrument de prélèvement, notamment par insertion d'une aiguille ou d'un cathéter, dans le corps dudit sujet.

L'insertion de l'instrument de prélèvement est notamment faite pour prélever du fluide intracorporel dudit sujet (tel que par exemple du sang) et/ou une portion de tissu hépatique dudit sujet (par exemple par PBH) et/ou des cellules hépatiques dudit sujet (par exemple par cyto-ponction hépatique).

Cet instrument peut donc par exemple être inséré :

- dans une veine, une artère ou un vaisseau sanguin dudit sujet pour prélever du sang dudit sujet ; et/ou
- dans le foie dudit sujet, pour prélever un échantillon de parenchyme hépatique, c'est-à-dire pour faire une ponction biopsie hépatique (PBH), par exemple par voie transjugulaire ou par voie transpariétale ; et/ou
- à travers la peau jusqu'au foie dudit sujet, de sorte à faire une cyto-ponction hépatique. L'invention est notamment relative audit article manufacturé, ou auxdits réactifs, pour leur utilisation dans une méthode pour le traitement d'une hépatopathie qui comporte une atteinte tissulaire du foie, plus particulièrement une fibrose hépatique, plus particulièrement une méthode de thérapie anti-VHC.

Cette utilisation peut notamment comprendre la mise en oeuvre desdits réactifs dans une méthode de l'invention pour pronostiquer si un sujet infecté par un ou des VHC a une forte probabilité de répondre à un traitement anti-VHC qui comprendra l'administration d'interféron et de ribavirine, ou si, au contraire, ce sujet a une forte probabilité de ne pas répondre à ce traitement anti-VHC, plus particulièrement dans une méthode de thérapie anti-VHC qui comprend administration d'interféron et administration de ribavirine (ou de leurs pro-drogues), plus particulièrement dans une méthode de thérapie anti-VHC qui comprend, à titre de traitement de première intention, administration d'interféron et administration de ribavirine (ou de leurs pro-drogues).

Si ledit sujet est pronostiqué non-répondeur, le praticien peut choisir de ne pas lui administrer un traitement qui comprend (plus particulièrement qui est essentiellement constitué de) administration d'interféron et administration de ribavirine (ou de leurs pro-drogues), plus particulièrement de ne pas lui administrer un tel traitement à titre de traitement de première intention. Dans une telle situation, le praticien peut par exemple choisir d'administrer un traitement anti-VHC qui ne comprend pas (ou qui n'est pas essentiellement constitué de) administration d'interféron et administration de ribavirine (ou de leurs pro-drogues), plus particulièrement de lui administrer un tel traitement à titre de traitement de première intention. Le praticien peut alternativement choisir de ne pas lui administrer de traitement anti-VHC, à tout le moins en première intention. Si ledit sujet est pronostiqué répondeur, le praticien peut choisir d'administrer un traitement anti-VHC, notamment un traitement qui comprend (plus particulièrement qui est essentiellement constitué de) administration d'interféron et administration de ribavirine (ou de leurs pro-drogues), plus particulièrement d'administrer en première intention un traitement qui comprend (plus particulièrement qui est essentiellement constitué de) administration d'interféron et administration de ribavirine (ou de leurs pro-drogues).

Cette utilisation peut par exemple comprendre :

- la mise en oeuvre desdits réactifs de l'invention sur échantillon biologique qui a été prélevé dudit sujet, et qui optionnellement a été transformé, par exemple :

  ◦ par extraction et/ou purification des ARN dudit échantillon prélevé et, optionnellement par transcription inverse des ARN extraits, ou

  ◦ par extraction et/ou purification des protéines et/ou polypeptides et/ou peptides dudit échantillon prélevé,

pour pronostiquer si un sujet infecté par un ou des VHC a une forte probabilité de répondre à un traitement anti-VHC qui comprendra l'administration d'interféron et de ribavirine, ou si, au contraire, ce sujet a une forte probabilité de ne pas répondre à ce traitement anti-VHC,

- optionnellement, déterminer le génotype de VHC dont est infecté ledit patient et/ou déterminer son score de fibrose hépatique (plus particulièrement, déterminer si ce score est un score d'au moins F2 selon le système Metavir).

Si ledit sujet est pronostiqué non-répondeur, le praticien peut choisir de ne pas lui administrer un traitement qui comprend (plus particulièrement qui est essentiellement constitué de) administration d'interféron et administration de ribavirine (ou de leurs pro-drogues), plus particulièrement de ne pas lui administrer un tel traitement à titre de traitement de première intention. Dans une telle situation, le praticien peut par exemple choisir d'administrer un traitement anti-VHC qui ne comprend pas (ou qui n'est pas essentiellement constitué de) administration d'interféron et administration de ribavirine (ou de leurs pro-drogues), plus particulièrement de lui administrer un tel traitement à titre de traitement de première intention. Le praticien peut alternativement choisir de ne pas lui administrer de traitement anti-VHC, à tout le moins en première intention. Si ledit sujet est pronostiqué répondeur, le praticien peut choisir d'administrer un traitement anti-VHC, notamment un traitement qui comprend (plus particulièrement qui est essentiellement constitué de) administration d'interféron et administration de ribavirine (ou de leurs pro-drogues), plus particulièrement d'administrer en première intention un traitement qui comprend (plus particulièrement qui est essentiellement constitué de) administration d'interféron et administration de ribavirine (ou de leurs pro-drogues).

Ledit traitement peut par exemple être un traitement anti-VHC comme ci-dessus décrit et ci-dessous illustré.

L'invention est également relative à un médicament ou association médicamenteuse destiné au traitement d'une hépatopathie comportant une atteinte tissulaire du foie, plus particulièrement une fibrose hépatique (tel que interféron standard ou interféron pégylé, en monothérapie, ou en plurithérapie associant un ou plusieurs autres principes actifs, notamment de la ribavirine), notamment un traitement anti-VHC, pour son utilisation dans la méthode de traitement de l'invention.

L'invention peut être mise en oeuvre par un **produit programme d'ordinateur**, destiné à être stocké dans une mémoire d'une unité de traitement, ou sur un support mémoire amovible destiné à coopérer avec un lecteur de ladite unité de traitement. Le produit programme d'ordinateur de l'invention comprend des instructions pour la mise en oeuvre d'une méthode de l'invention, notamment pour la mise en oeuvre d'une analyse statistique adaptée à la mise en oeuvre d'une méthode de l'invention (notamment adaptée à l'analyse statistique multivariée des mesures, et plus particulièrement des niveaux d'expression desdits gènes choisis) et/ou pour la construction d'un modèle de classification multivariée adapté à la mise en oeuvre d'une méthode de l'invention.

L'invention peut également être mise en oeuvre via une **installation informatique**, un dispositif informatique, ou ordinateur, comprenant une unité de traitement dans la mémoire de laquelle sont stockés ou enregistrés :

- un produit programme d'ordinateur de l'invention, et, optionnellement,
- des dosages, ou des valeurs de dosage, des niveaux d'expression (transcription et/ou traduction) desdits gènes choisis.

Le terme "comprenant", avec lequel "incluant" ou "contenant" est synonyme, est un terme ouvert, et n'exclut pas la présence d'un ou plusieurs élément(s), ingrédient(s) ou étape(s) de méthode additionnel(s) qui ne serait(seraient) pas explicitement indiqué(s), tandis que le terme "consistant" ou "constitué" est un terme fermé, qui exclut la présence de tout autre élément additionnel, étape, ou ingrédient qui ne serait pas explicitement exposé. Le terme "consistant essentiellement" ou " essentiellement constitué" est un terme partiellement ouvert, qui n'exclut pas la présence d'un ou plusieurs élément(s), ingrédient(s) ou étape(s) additionnel(s), dans la mesure où cet(ces) élément(s), ingrédient(s) ou étape(s) additionnel(s) n'affecte(nt) pas matériellement les propriétés de base de l'invention.

Par conséquent, le terme "comprenant" (ou "comprend(comprennent)") inclut les termes "consistant", "constitué", aussi bien que les termes "consistant essentiellement" et " essentiellement constitué".

Dans le but de faciliter la lecture de l'invention, la description a été séparée en divers paragraphes, sections et modes de réalisation. Il ne doit pas être considéré que ces séparations déconnectent la substance d'un paragraphe, section ou mode de réalisation, de celle d'un autre paragraphe, section ou mode réalisation. Au contraire, la description englobe toutes les combinaisons possibles des différents paragraphes, sections, phrases et modes de réalisations qu'elle contient.

Les exemples qui suivent sont donnés à titre purement illustratif. Ils ne limitent en aucune façon l'invention.

## EXEMPLES

### EXEMPLE 1 : CONSTRUCTION DE MODELES DE CLASSIFICATION

#### 1. Populations et patients, dosage du niveau d'expression de gènes, détermination de la réponse au traitement :

L'étude réalisée a été approuvée par le Comité Ethique local, conformément à la Déclaration d'Helsinki, et tous les patients ont donné leur consentement éclairé par écrit.

#### Présentation des patients

Les patients sont des patients adultes infectés par le virus de l'hépatite C (VHC), suivis à l'Hôpital Beaujon (Clichy, France).

Le diagnostic clinique d'infection par le virus de l'hépatite C des patients sélectionnés a été établi sur la base de la détection d'anticorps dirigés contre des protéines du VHC et de la détection d'ARN circulant du VHC.

La sérologie du VHC de dépistage a été réalisée par le test Abbott de 3ème génération (AxSYM™ HCV Version 3.0 (Abbott) Technique MEIA ; index > 1 = positif ; index < 1 = négatif) et le test de quantification de l'ARN de VHC VERSANT® HCV-RNA 3.0 (bDNA) ASSAY de la société Siemens Healthcare Diagnostics (limite de quantification = 615 - 7 690 000 UI/mL).

Afin d'établir une cohorte homogène et parfaitement représentative de la pathologie exemplifiée, les patients susceptibles de présenter des affections chroniques hépatiques d'origines autres que le virus de l'hépatite C (telles qu'une affection chronique hépatique due à une infection par le virus de l'hépatite B) ont été exclus de l'étude. D'autres critères d'exclusion ont en outre été appliqués, à savoir consommation d'alcool excessive, hémochromatose, hépatite auto-immune, maladie de Wilson, déficience en $\alpha$-1 antitrypsine, angiocholite sclérosante primitive, cirrhose biliaire primitive, la prise antérieure d'un traitement anti-VHC.

Cent quarante patients ont ainsi été sélectionnés.

Le tableau 30 ci-dessous présente les données cliniques, biologiques et virologiques des patients ainsi sélectionnés. Ces données ont été collectées avant que le patient ne reçoive de traitement antiviral, en l'occurrence lors de la ponction biopsique hépatique (PBH). Dans le tableau 30 ci-dessous :

UI = Unité Internationale

Patients NR = patients non répondeurs au traitement ;

Patients R = patients répondeurs au traitement ;

Patients RR= patients répondeurs-rechuteurs ;

*cf.* ci-dessous pour la définition de ces trois sous-populations ou cohortes.

**Tableau 30 : données cliniques, biologiques et virologiques**

| Données cliniques, biologiques et virologiques | Patients | Patients NR | Patients R | Patients RR |
|---|---|---|---|---|
| **n** | 140 | 51 | 68 | 21 |
| **Sexe :** mâle (%) / femelle (%) | 89 (64) / 51 (36) | 31 (61) / 20 (39) | 43 (63) / 25 (37) | 15 (71) / 6 (29) |
| **Age** [moyenne $\pm$ déviation standard (gamme)] | 45,8 $\pm$ 8,5 (27-72) | 47,3 $\pm$ 8,6 (33-72) | 44,7 $\pm$ 9,0 (27-65) | 44,4 $\pm$ 4,9 (34-66) |
| **Source d'infection** [n(%)] | | | | |
| transfusion sanguine | 30(21) | 11(22) | 16(23) | 3(14) |
| administration intraveineuse d'une drogue | 42(30) | 17(33) | 21(31) | 4(19) |
| inconnue | 68(49) | 23(45) | 31(46) | 14(67) |
| **Alanine aminotransférase** (ALT) UI/L [moyenne $\pm$ déviation standard (gamme)] | 106 $\pm$ 73 (18-459) | 112 $\pm$ 81 (30-354) | 102 $\pm$ 74 (20-459) | 100 $\pm$ 36 (18 176) |
| **Génotypes VHC** [n(%)] | | | | |
| 1 | 76 (54,3) | 40 (78,4) | 28 (41,2) | 8 (38,1) |
| 2 | 13 (9,3) | | 10 (14,7) | 3 (14,3) |
| 3 | 19 (13,6) | 3 (5,9) | 12 (17,6) | 4 (19,0) |
| 4 | 31 (22,1) | 8 (15,7) | 17 (25,0) | 6 (28,6) |
| 5 | 1 (0,7) | 0 | 1 (1,5) | 0 |
| **Score de fibrose** (score F Metavir) [n(%)] | | | | |
| 0 | 1 (0,7) | 0 | 1 (1,5) | 0 |
| 1 | 45 (32,1) | 15 (29,5) | 26 (38,2) | 4 (19,0) |
| 2 | 53 (37,9) | 18 (35,3) | 29 (42,7) | 6 (28,6) |
| 3 | 18 (12,9) | 9 (17,6) | 3 (4,4) | 6 (28,6) |
| 4 | 22 (15,7) | 9 (17,6) | 9 (13,2) | 4 (19,0) |
| inconnu | 1 (0,7) | 0 | 0 | 1 (4,8) |

**Prélèvements** des échantillons :

Une biopsie ponction hépatique (PBH) a été réalisée sur chaque patient, avant qu'il ne reçoive le traitement antiviral. Les PBH ont été réalisées en respectant les bonnes pratiques cliniques. Les biopsies ont été immédiatement stockées à -80°C en vue de l'extraction des ARN totaux, et traitées avec de la paraffine pour des études histologiques. Un échantillon de sérum a été prélevé sur chacun des patients inclus dans l'étude dans un délai de +/- 6 mois par rapport à la date de la biopsie, mais toujours avant que le patient n'ait reçu le traitement antiviral.

**Traitement des échantillons de biopsies hépatiques (pour le dosage des ARN) :**

Les niveaux d'expression des gènes (en l'occurrence, niveau de transcription en ARN) ont été dosés sur chacune des 140 biopsies (1 biopsie par patient).
Les biopsies hépatiques ont été broyées dans de l'azote en utilisant un pilon et un mortier en céramique (broyage 100% manuel).
La poudre a été récupérée à l'aide d'un scalpel (Swann Morton 22, Référence 0208).

a) Extraction des ARN

La poudre ainsi obtenue a été solubilisée dans 1 mL de RNAble® Réf. GEXEXT00, Laboratoires Eurobio, France, auquel ont été ajoutés 100 µL de chloroforme.
Le mélange obtenu a été placé dans la glace ou à 4°C pendant 5 minutes puis a été centrifugé à 13 000 g pendant 15 minutes.
La phase supérieure aqueuse contenant les ARN a été récupérée dans un tube neuf et 1 volume d'isopropanol y a été ajouté.
Le tube a été agité par retournement et a été placé à 4°C toute la nuit, puis a été centrifugé à 13 000 g pendant 15 minutes. Le surnageant a été éliminé, et le culot contenant les ARN a été repris dans un volume d'éthanol à 70% (préparé extemporanément) et à nouveau centrifugé.
Le culot de précipité d'ARN obtenu a été séché à l'air libre pendant environ 1 heure, puis dissout dans 15 µL d'eau puis stocké à -80°C.

b) Dosage des ARN

L'évaluation de la concentration des ARN extraits a été réalisée par la mesure de la densité optique par un spectromètre (Nanodrop), et a été vérifiée après un cycle de congélation/décongélation.
Les ARN extraits ont ensuite été dilués pour obtenir une solution à 50 ng/µL.
Les contrôles qualités de l'ARN ont été réalisés par PCR temps réel (*cf.* ci-dessous), par ciblage d'un gène contrôle (dit endogène), à expression ubiquitaire, pour vérifier que l'ARN n'a pas été dégradé (en l'occurrence, ciblage de RPLPO).

Etape de transcription inverse *(Reverse Transcription* ou RT) :

La transcription inverse a été réalisée sur 200 ng d'ARN dans un mélange réactionnel réalisé dans un volume de 20 µL comprenant les réactifs suivants :

**Tableau 31** :

| Réactif et référence produit | Solution de départ | Volume |
|---|---|---|
| Transcriptase inverse SUPERSCRIPT II RNase H, Invitrogen, réf: 18064014 | 200 U/µL | 0,5 µL |
| Tampon SUPER SCRIPT 5X Invitrogen, réf : 18064014 | - | 4,0 µL |
| RNAsin Promega, réf: N2111 | 40 U/µL | 0,5 µL |
| DTT | 100 mM | 2,0 µL |
| Les 4 dNTP GE Healthcare, réf: 28406552 | 10 mM | 1 µL |

(suite)

| Réactif et référence produit | Solution de départ | Volume |
|---|---|---|
| Amorces Pd(N) RANDOMS HEXAMERS 50 (A260) units, 51 Perbio, réf : MB216601 | 0,5 μg/μL | 6,0 μL |
| ARN | 50 ng/μL | 4,0 μL |
| H$_2$0 | | QSP 20 μL |

Les réactions de transcription inverse ont été effectuées aux températures suivantes :

- à 20°C pendant 10 minutes, puis

- à 42°C pendant 30 minutes, et

- à 99°C pendant 5 minutes.

A ce stade, les mélanges réactionnels ont été congelés, ou aliquotés, ou directement utilisés pour une amplification par PCR temps réel.

Etape de PCR en temps réel quantitative (PCRq) :

L'amplification a été réalisée en utilisant un light Cycler® 480 (Roche Diagnostics, Mannheim, Allemagne). Les résultats sont générés par le logiciel light Cycler® Software 4.05/4.1.

La technologie Light Cycler® permet le suivi en continu de l'apparition des produits d'amplification grâce à l'émission d'une quantité de fluorescence proportionnelle à la quantité de produit amplifié, elle-même dépendante de la quantité de cibles initialement présentes dans l'échantillon à analyser. La quantification (en valeurs relatives) de l'expression génique est réalisée par la méthode connue sous le nom de $2^{-\Delta Ct}$ ($2^{-\Delta Ct} = 2^{-(Ctcible - Ct\ référence)}$ ; *cf.* Livak et Schmittgen 2001 ; Schmitten et Livak 2008), exploitant les valeurs de « Cycle Threshold » ou Ct déterminées par l'appareil de PCR quantitative en temps réel. Plus la valeur de Ct est faible, plus la quantité initiale d'ARN transcrit est élevée.

Les mélanges réactionnels et le protocole utilisés sont décrits dans la notice de la trousse LIGHT CYCLER® 480 SYBR GREEN I MASTER MIX (Roche Diagnostics, Mannheim, Allemagne ; US 4,683,202 ; US 4,683,195 ; US 4,965,188 ; US 6,569,627). Après l'étape de transcription inverse, les mélanges réactionnels (cDNAs) ont été dilués au 1/40ème (pour la vérification de la qualité) ou au 1/100ème (pour les gènes cibles) avant leur utilisation en PCRq.

Pour chaque gène, les PCRq ont été réalisées dans un volume réactionnel de 10 μL sur une plaque à 384 puits :

- 5 μL de la réaction de transcription inverse diluée au 1/40ème (ou au 1/100ème) ;
- 4,8 μL de mélange réactionnel de la trousse light Cycler® 480 SYBR Green I Master mix
- 0,1 μL d'une solution à 50 μM pour chacune des deux amorces, soit un volume final de 0,5 μM pour chaque amorce.

Les mélanges réactionnels sont généralement préparés pour des plaques de 384 puits. Les amorces suivantes ont été utilisées :

**Tableau 32 : exemples d'amorces**

| Symbole | Amorce sens | SEQ ID NO : | Amorce antisens | SEQ ID NO : |
|---|---|---|---|---|
| MBL2 | GGCACGTATCAAAAAGTGGCTG | 1 | ATTTCACCATTGGTCAGGAAGAACT | 2 |
| G1P2 | GAGGCAGCGAACTCATCTTTGCCA | 3 | CCGCCAGCATCTTCACCGTCA | 4 |
| MDK | GGGCAGCGAGATGCAGCAC | 5 | CCACTCAGCGCACTCGCTCC | 6 |
| LGALS3BP | TGACCCCTCCGAGGCTCTTC | 7 | ATGTCACCATCGTTCACGCCTT | 8 |
| CXCL10 | CTGACTCTAAGTGGCATTCAAGGAG | 9 | GGTTGATTACTAATGCTGATGCAGG | 10 |
| FGF7 | CACAGTGGTACCTGAGGATCGATAA | 11 | GCCACTGTCCTGATTTCCATGAT | 12 |
| IL8 | CACCGGAAGGAACCATCTCACTGT | 13 | TCCTTGGCAAAACTGCACCTTCA | 14 |
| TGFB2 | AGAGTGCCTGAACAACGGATT | 15 | CCATTCGCCTTCTGCTCTT | 16 |
| CCL21 | CTCCATCCCAGCTATCCTGTTCTT | 17 | TCTGCACATAGCTCTGCCTGAGA | 18 |
| CXCL6 | GTTTACGCGTTACGCTGAGAGTAAA | 19 | CGTTCTTCAGGGAGGCTACCA | 20 |
| MMP2 | ACTGCGGTTTTCTCGAATCCA | 21 | GGTATCCATCGCCATGCTCC | 22 |
| SFN | CGACAAGAAGCGCATCATTGAC | 23 | CTGTTGGCGATCTCGTAGTGGA | 24 |
| CXCL11 | GTGTGCTACAGTTGTTCAAGGCTT | 25 | CTCAATATCTGCCACTTTCACTGCT | 26 |
| AFP | ACCCGAACTTTCCAAGCCATAACT | 27 | CCACATCCAGGACTAGTTTCTGGATT | 28 |
| VEGFD | CCTCGTACATTTCCAAACAGCTCT | 29 | TGGCAAGCACTTACAACCTGTATG | 30 |
| CRP | GACGTGACCATGGAGAAGCTGTT | 31 | AAGCCTTCCTCGACATGTCTGTCT | 32 |
| CXCL9 | ATCCACCTACAATCCTTGAAAGAC | 33 | TCCATTCTTCAGTGTAGCAATGATTT | 34 |
| | | | | |
| RPLP0 | GGCGACCTGGAAGTCCAACT | 35 | CCATCAGCACCACAGCCTTC | 36 |

Les PCRq ont été réalisées suivant les conditions de température suivantes :

- une étape d'initiation de la dénaturation à 95°C pendant 10 minutes ;
- 50 cycles de :

  - dénaturation à 95°C pendant 15 secondes ;
  - hybridation / élongation à 65°C pendant 30 secondes.

Chaque échantillon cible est amplifié en duplicate. Afin de s'affranchir des variations des quantités initiales d'ARN total d'un échantillon à l'autre, on réalise en parallèle sur le même échantillon l'amplification en duplicate des ARN d'un gène utilisé comme contrôle endogène, tel qu'un gène impliqué dans des cascades métabolique cellulaires, par exemple RPLP0 (aussi connu sous le nom 36B4 ; GENBANK accession NM_001002) ou TBP (GENBANK accession NM_003194). En l'occurrence, c'est le gène RPLP0 qui a été ici utilisé comme contrôle endogène. La qualité de l'extraction d'ARN faite à partir des 140 biopsies a été évaluée sur la base de la valeur de Ct du gène de référence RPLPO. La classification a été réalisée de la façon suivante :

- Ct RPLPO inférieur à 22 : qualité ARN très bonne ;
- Ct RPLPO de 22 à 24 : qualité ARN bonne ;
- Ct RPLPO supérieur à 24 et inférieur à 26 : qualité ARN moyenne ;
- Ct RPLPO supérieur ou égal à 26 : qualité ARN mauvaise.

Afin d'augmenter la fiabilité des analyses bio-stastistiques, seules les données provenant d'extraction d'ARN de très bonne et bonne qualité (Ct RPLPO < 24) ont été retenues, 128 biopsies [91,4% des 140 échantillons] dont 107 présentant un statut de répondeur ou non répondeur strict ; *cf.* tableau 33 ci-dessous. La quantité de transcrits d'un gène cible a été déduite du Ct (« *Cycle threshold* ») qui correspond au nombre de cycles de PCR nécessaire pour obtenir un signal de fluorescence significatif. Les échantillons cibles ont été normalisés sur la base de leur contenu en RPLPO (ou, le cas échéant, en TBP), conformément à la méthode du $2^{-\Delta Ct}$.

Cette valeur de dosage normalisée est ici de manière générale notée « BMQ » (pour biomarqueur). Les valeurs BMQ obtenues pour chacun des 128 patients sont présentées dans les tableaux 34 à 36 ci-dessous.

**Traitement des échantillons de sérum (pour le dosage des protéines sériques) :**

Les dosages protéiques ont été réalisés à l'aide des kits indiqués dans le tableau 44 ci-dessous, en suivant les recommandations du fabricant.

**Tableau 33 : données cliniques, biologiques et virologiques**

| Données cliniques, biologiques et virologiques | Patients | Patients NR | Patients R | Patients RR |
|---|---|---|---|---|
| **n** | 128 | 44 | 63 | 21 |
| **Sexe :** mâle (%) / femelle (%) | 82 (64) / 46 (36) | 25 (57) / 19 (43) | 42 (67) / (21 (33) | 15 (71) / 6 (29) |
| **Age** [moyenne $\pm$ déviation standard (gamme)] | 47,0 $\pm$ 8,7 (27-73) | 46,1 $\pm$ 8,9 (27-66) | 48,1 $\pm$ 8,9 (35-73) | 47,4 $\pm$ 7,8 (35-67) |
| **Source d'infection** [n(%)] | | | | |
| transfusion sanguine | 28 (22) | 10 (23) | 15 (24) | 3 (14) |
| administration intraveineuse d'une drogue | 37 (29) | 13 (30) | 20 (32) | 4 (19) |
| inconnue | 63 (49) | 21 (48) | 28 (44) | 14 (67) |
| **Alanine aminotransférase** (ALT) UI/L [moyenne $\pm$ déviation standard (gamme)] | 112 $\pm$ 82 (18-459) | 114 $\pm$ 80 (30-354) | 119 $\pm$ 92 (30-459) | 88 $\pm$ 37 (18-176) |
| **Génotypes VHC** [n(%)] | | | | |
| 1 | 70 (55) | 37 (84) | 25 (40) | 8 (38) |
| 2 | 12 (9) | 0 (0) | 9 (14) | 3 (14) |
| 3 | 19 (15) | 3 (7) | 12 (19) | 4 (19) |
| 4 | 26 (20) | 4 (9) | 16 (25) | 6 (29) |
| 5 | 1 (1) | 0 (0) | 1 (2) | 0 (0) |

(suite)

| Données cliniques, biologiques et virologiques | Patients | Patients NR | Patients R | Patients RR |
|---|---|---|---|---|
| **Score de fibrose** (score F Metavir) [n(%)] | | | | |
| 0 | 0 (0) | 0 (0) | 0 (0) | 0 (0) |
| 1 | 41 (32) | 14 (32) | 23 (37) | 4 (19) |
| 2 | 49 (38) | 15 (34) | 28 (44) | 6 (29) |
| 3 | 17 (13) | 8 (18) | 3 (5) | 6 (29) |
| 4 | 20 (16) | 7 (16) | 9 (14) | 4 (19) |
| inconnu | 1 (1) | 0 (0) | 0 (0) | 1 (5) |

**Tableau 34 : valeurs BMQ des patients pour les gènes MBL2, G1P2, MDK, LGALS3BP et CXCL10 (Ct normalisé conformément à la méthode du $2^{-\Delta Ct}$)**

| Patient | Statut (NR, R ou RR) | MBL2 | G1P2 | MDK | LGALS3BP | CXCL10 |
|---|---|---|---|---|---|---|
| 59 | R | 0,993 | 0,877 | 0,090 | 0,045 | 0,01 |
| 62 | R | 5,333 | 0,943 | 0,043 | 0,113 | 0,10 |
| 73 | R | 1,440 | 1,064 | 0,055 | 0,060 | 0,23 |
| 125 | R | 1,419 | 1,257 | 0,058 | 0,012 | 0,02 |
| 306 | R | 5,134 | 3,568 | 0,125 | 0,273 | 0,12 |
| 344 | R | 1,047 | 0,646 | 0,045 | 0,037 | 0,06 |
| 346 | R | 4,004 | 0,601 | 0,032 | 0,133 | 0,02 |
| 504 | R | 1,796 | 0,563 | 0,028 | 0,044 | 0,06 |
| 513 | R | 1,510 | 0,280 | 0,034 | 0,035 | 0,04 |
| 528 | R | 2,694 | 1,261 | 0,188 | 0,082 | 0,07 |
| 530 | R | 4,127 | 0,144 | 0,023 | 0,052 | 0,02 |
| 546 | R | 4,317 | 0,236 | 0,039 | 0,056 | 0,02 |
| 569 | R | 2,166 | 0,092 | 0,008 | 0,047 | 0,02 |
| 570 | R | 4,611 | 0,118 | 0,046 | 0,028 | 0,04 |
| 575 | R | 4,563 | 15,348 | 0,525 | 0,360 | 0,44 |
| 577 | R | 6,255 | 0,224 | 0,024 | 0,093 | 0,02 |
| 583 | R | 1,129 | 1,297 | 0,056 | 0,146 | 0,12 |
| 601 | R | 3,758 | 1,053 | 0,125 | 0,069 | 0,03 |
| 613 | R | 1,809 | 0,108 | 0,017 | 0,035 | 0,03 |
| 614 | R | 2,114 | 0,135 | 0,036 | 0,057 | 0,10 |
| 639 | R | 3,306 | 0,109 | 0,031 | 0,021 | 0,03 |
| 45 | R | 7,835 | 0,959 | 0,037 | 0,063 | 0,13 |
| 50 | R | 0,622 | 1,586 | 0,132 | 0,087 | 0,02 |
| 55 | R | 1,828 | 1,275 | 0,054 | 0,046 | 0,03 |
| 63 | R | 1,338 | 12,295 | 0,106 | 0,115 | 0,25 |
| 65 | R | 2,297 | 1,613 | 0,105 | 0,048 | 0,06 |
| 66 | R | 2,780 | 0,308 | 0,054 | 0,028 | 0,05 |

(suite)

| Patient | Statut (NR, R ou RR) | MBL2 | G1P2 | MDK | LGALS3BP | CXCL10 |
|---|---|---|---|---|---|---|
| 71 | R | 0,588 | 0,564 | 0,021 | 0,014 | 0,01 |
| 59 | R | 0,993 | 0,877 | 0,090 | 0,045 | 0,01 |
| 72 | R | 1,532 | 0,176 | 0,001 | 0,015 | 0,01 |
| 76 | R | 3,053 | 0,653 | 0,045 | 0,064 | 0,27 |
| 86 | R | 1,636 | 14,123 | 0,060 | 0,103 | 0,40 |
| 88 | R | 1,516 | 2,042 | 0,024 | 0,034 | 0,08 |
| 90 | R | 2,021 | 1,912 | 0,115 | 0,048 | 0,08 |
| 91 | R | 0,889 | 0,664 | 0,051 | 0,023 | 0,11 |
| 92 | R | 0,618 | 0,049 | 0,001 | 0,008 | 0,03 |
| 222 | R | 1,165 | 5,187 | 0,063 | 0,021 | 0,15 |
| 227 | R | 0,933 | 0,119 | 0,002 | 0,014 | 0,02 |
| 366 | R | 2,901 | 0,159 | 0,012 | 0,015 | 0,05 |
| 501 | R | 1,223 | 1,424 | 0,046 | 0,032 | 0,13 |
| 502 | R | 1,189 | 0,465 | 0,056 | 0,022 | 0,12 |
| 503 | R | 1,320 | 0,755 | 0,014 | 0,024 | 0,01 |
| 506 | R | 1,121 | 0,678 | 0,057 | 0,016 | 0,15 |
| 508 | R | 1,784 | 0,143 | 0,032 | 0,028 | 0,12 |
| 523 | R | 1,113 | 0,134 | 0,045 | 0,028 | 0,08 |
| 529 | R | 0,724 | 1,210 | 0,084 | 0,031 | 0,00 |
| 532 | R | 0,790 | 0,245 | 0,207 | 0,029 | 0,02 |
| 535 | R | 0,676 | 15,348 | 0,057 | 0,053 | 0,12 |
| 536 | R | 1,717 | 0,313 | 0,010 | 0,015 | 0,03 |
| 537 | R | 0,719 | 0,080 | 0,014 | 0,012 | 0,01 |
| 538 | R | 1,193 | 5,152 | 0,108 | 0,068 | 0,04 |
| 556 | R | 1,161 | 6,869 | 0,011 | 0,022 | 0,04 |
| 560 | R | 1,395 | 3,249 | 0,028 | 0,038 | 0,15 |
| 565 | R | 2,136 | 4,857 | 0,049 | 0,022 | 0,26 |
| 567 | R | 4,532 | 2,497 | 0,038 | 0,068 | 0,11 |
| 568 | R | 0,990 | 0,055 | 0,005 | 0,007 | 0,01 |
| 571 | R | 2,196 | 0,609 | 0,050 | 0,057 | 0,59 |
| 572 | R | 1,376 | 2,129 | 0,009 | 0,020 | 0,02 |
| 581 | R | 1,098 | 1,210 | 0,034 | 0,036 | 0,03 |
| 585 | R | 1,636 | 3,411 | 0,063 | 0,121 | 0,19 |
| 598 | R | 0,923 | 0,200 | 0,009 | 0,009 | 0,06 |
| 604 | R | 1,717 | 1,061 | 0,157 | 0,124 | 0,03 |
| 605 | R | 1,939 | 5,315 | 0,173 | 0,048 | 0,04 |
| 629 | R | 2,189 | 0,074 | 0,012 | 0,059 | 0,01 |

(suite)

| Patient | Statut (NR, R ou RR) | MBL2 | G1P2 | MDK | LGALS3BP | CXCL10 |
|---------|---------------------|------|------|-----|----------|--------|
| 308 | NR | 1,185 | 6,658 | 0,446 | 0,251 | 0,07 |
| 521 | NR | 0,004 | 4,362 | 1,676 | 0,576 | 0,49 |
| 526 | NR | 3,138 | 5,260 | 0,189 | 0,130 | 0,17 |
| 549 | NR | 5,352 | 2,297 | 0,156 | 0,142 | 0,15 |
| 574 | NR | 4,228 | 4,959 | 0,191 | 0,241 | 1,04 |
| 618 | NR | 1,490 | 4,757 | 0,152 | 0,221 | 0,20 |
| 619 | NR | 1,873 | 1,542 | 0,075 | 0,151 | 0,15 |
| 636 | NR | 0,000 | 18,765 | 0,712 | 0,459 | 0,87 |
| 646 | NR | 0,068 | 35,383 | 0,314 | 0,529 | 0,16 |
| 657 | NR | 5,242 | 2,949 | 0,782 | 0,075 | 0,17 |
| 658 | NR | 2,395 | 0,322 | 0,018 | 0,019 | 0,07 |
| 664 | NR | 5,637 | 2,704 | 0,046 | 0,063 | 0,08 |
| 6 | NR | 0,115 | 0,685 | 0,688 | 0,044 | 0,02 |
| 46 | NR | 3,010 | 8,969 | 0,308 | 0,164 | 0,21 |
| 58 | NR | 2,462 | 13,881 | 0,631 | 0,149 | 0,07 |
| 75 | NR | 5,483 | 8,664 | 1,248 | 0,265 | 0,55 |
| 80 | NR | 1,834 | 0,963 | 0,030 | 0,032 | 0,15 |
| 83 | NR | 0,013 | 6,169 | 0,476 | 0,164 | 0,18 |
| 145 | NR | 5,278 | 7,490 | 0,255 | 0,193 | 0,02 |
| 167 | NR | 0,532 | 5,046 | 0,521 | 0,099 | 0,08 |
| 509 | NR | 0,016 | 6,126 | 0,172 | 0,045 | 0,22 |
| 516 | NR | 3,470 | 3,160 | 0,043 | 0,065 | 0,11 |
| 524 | NR | 1,400 | 0,286 | 0,028 | 0,042 | 0,08 |
| 527 | NR | 1,223 | 9,318 | 0,276 | 0,457 | 0,07 |
| 534 | NR | 3,249 | 2,129 | 0,140 | 0,048 | 0,11 |
| 582 | NR | 1,729 | 4,675 | 0,034 | 0,037 | 0,25 |
| 596 | NR | 0,809 | 0,914 | 0,069 | 0,059 | 0,33 |
| 602 | NR | 0,001 | 0,091 | 0,012 | 0,019 | 0,04 |
| 645 | NR | 0,000 | 0,069 | 0,032 | 0,017 | 0,00 |
| 647 | NR | 2,819 | 20,393 | 0,053 | 0,097 | 0,12 |
| 649 | NR | 1,157 | 3,918 | 0,053 | 0,051 | 0,51 |
| 650 | NR | 2,107 | 12,381 | 0,233 | 0,241 | 0,27 |
| 651 | NR | 1,765 | 4,773 | 0,253 | 0,146 | 0,05 |
| 659 | NR | 1,866 | 0,690 | 0,024 | 0,016 | 0,05 |
| 660 | NR | 0,782 | 6,612 | 0,037 | 0,074 | 0,10 |
| 662 | NR | 0,008 | 5,483 | 0,047 | 0,049 | 0,38 |
| 665 | NR | 1,306 | 0,576 | 0,026 | 0,042 | 0,04 |

(suite)

| Patient | Statut (NR, R ou RR) | MBL2 | G1P2 | MDK | LGALS3BP | CXCL10 |
|---------|---------------------|------|------|-----|----------|--------|
| 666 | NR | 3,506 | 5,796 | 0,221 | 0,095 | 0,05 |
| 563 | NR | 1,532 | 0,235 | 0,011 | 0,038 | 0,07 |
| 573 | NR | 4,500 | 6,892 | 0,047 | 0,134 | 0,40 |
| 599 | NR | 0,486 | 1,248 | 0,010 | 0,013 | 0,04 |
| 641 | NR | 2,928 | 2,454 | 0,028 | 0,035 | 0,02 |
| 49 | RR | 1,993 | 1,905 | 0,076 | 0,128 | 0,17 |
| 505 | RR | 0,824 | 4,084 | 0,037 | 0,082 | 0,01 |
| 514 | RR | 5,098 | 0,525 | 0,021 | 0,082 | 0,04 |
| 579 | RR | 1,091 | 0,147 | 0,006 | 0,019 | 0,04 |
| 643 | RR | 3,193 | 0,390 | 0,054 | 0,058 | 0,05 |
| 56 | RR | 2,505 | 3,249 | 0,349 | 0,062 | 0,13 |
| 60 | RR | 0,771 | 7,945 | 0,192 | 0,059 | 0,08 |
| 87 | RR | 3,063 | 11,432 | 0,077 | 0,104 | 0,42 |
| 531 | RR | 0,002 | 0,103 | 0,034 | 0,006 | 0,01 |
| 533 | RR | 2,042 | 1,145 | 0,041 | 0,046 | 0,05 |
| 543 | RR | 1,306 | 1,548 | 0,075 | 0,112 | 0,26 |
| 554 | RR | 0,002 | 0,148 | 0,014 | 0,029 | 0,05 |
| 557 | RR | 1,094 | 3,317 | 0,001 | 0,057 | 0,17 |
| 558 | RR | 0,862 | 0,289 | 0,006 | 0,015 | 0,03 |
| 559 | RR | 0,933 | 0,871 | 0,007 | 0,028 | 0,06 |
| 562 | RR | 0,002 | 3,668 | 0,075 | 0,112 | 0,18 |
| 576 | RR | 1,464 | 5,187 | 0,069 | 0,136 | 0,25 |
| 588 | RR | 2,704 | 0,053 | 0,008 | 0,127 | 0,00 |
| 589 | RR | 2,809 | 7,387 | 0,054 | 0,094 | 0,29 |

**Tableau 35 : valeurs BMQ des patients pour les gènes FGF7, IL8, TGFB2, CCL21, CXCL6, MMP2, SFN (Ct normalisé conformément à la méthode du $2^{-\Delta Ct}$)**

| Patient | Statut (NR, R ou RR) | FGF7 | IL8 | TGFB2 | CCL21 | CXCL6 | MMP2 | SFN |
|---------|---------------------|------|-----|-------|-------|-------|------|-----|
| 59 | R | 0,069 | 0,329 | 1,735 | 0,006 | 0,000 | 0,318 | 1,505 |
| 62 | R | 0,000 | 0,058 | 1,803 | 0,017 | 0,080 | 0,648 | 3,494 |
| 73 | R | 0,133 | 0,191 | 0,000 | 0,004 | 0,027 | 0,078 | 0,939 |
| 125 | R | 0,000 | 0,000 | 0,635 | 0,005 | 0,000 | 0,072 | 0,399 |
| 306 | R | 0,000 | 0,372 | 0,000 | 0,018 | 0,186 | 0,722 | 0,000 |
| 344 | R | 0,231 | 0,923 | 0,401 | 0,067 | 0,330 | 0,476 | 6,275 |
| 346 | R | 0,000 | 0,182 | 1,889 | 0,004 | 0,029 | 0,357 | 0,712 |
| 504 | R | 0,000 | 2,289 | 0,000 | 0,012 | 0,142 | 0,193 | 4,228 |

(suite)

| Patient | Statut (NR, R ou RR) | FGF7 | IL8 | TGFB2 | CCL21 | CXCL6 | MMP2 | SFN |
|---|---|---|---|---|---|---|---|---|
| 513 | R | 0,936 | 0,538 | 3,694 | 0,006 | 0,000 | 0,347 | 3,021 |
| 528 | R | 0,555 | 4,547 | 3,824 | 0,022 | 1,189 | 1,414 | 2,282 |
| 530 | R | 0,080 | 0,000 | 2,514 | 0,009 | 0,000 | 0,690 | 1,469 |
| 546 | R | 0,165 | 0,000 | 1,177 | 0,012 | 0,062 | 2,742 | 2,809 |
| 569 | R | 0,000 | 0,559 | 0,306 | 0,003 | 0,000 | 0,343 | 0,655 |
| 570 | R | 0,075 | 0,371 | 4,823 | 0,006 | 0,000 | 0,318 | 1,288 |
| 575 | R | 0,000 | 0,892 | 2,969 | 0,036 | 0,226 | 0,609 | 0,449 |
| 577 | R | 0,011 | 0,262 | 1,641 | 0,007 | 0,084 | 0,166 | 0,000 |
| 583 | R | 0,121 | 0,230 | 1,548 | 0,015 | 0,367 | 1,057 | 1,283 |
| 601 | R | 0,205 | 0,459 | 4,874 | 0,005 | 0,077 | 0,260 | 0,883 |
| 613 | R | 0,094 | 0,111 | 2,063 | 0,007 | 0,000 | 0,287 | 15,085 |
| 614 | R | 0,282 | 0,218 | 2,362 | 0,011 | 0,078 | 0,563 | 2,204 |
| 639 | R | 0,151 | 0,399 | 0,000 | 0,005 | 0,000 | 0,365 | 9,126 |
| 45 | R | 0,101 | 1,641 | 1,647 | 0,033 | 0,092 | 0,302 | 0,000 |
| 50 | R | 0,102 | 0,444 | 0,529 | 0,013 | 0,027 | 0,274 | 0,213 |
| 55 | R | 0,060 | 1,395 | 0,000 | 0,006 | 0,053 | 0,087 | 0,365 |
| 63 | R | 0,177 | 0,199 | 0,412 | 0,025 | 0,000 | 0,115 | 0,766 |
| 65 | R | 0,098 | 0,168 | 0,232 | 0,012 | 0,013 | 0,113 | 0,106 |
| 66 | R | 0,120 | 0,420 | 0,793 | 0,013 | 0,119 | 0,342 | 0,465 |
| 72 | R | 0,094 | 0,064 | 1,017 | 0,005 | 0,052 | 0,091 | 0,396 |
| 76 | R | 0,210 | 1,257 | 1,125 | 0,012 | 0,051 | 0,221 | 0,742 |
| 86 | R | 0,266 | 0,451 | 1,193 | 0,023 | 0,102 | 0,211 | 0,507 |
| 88 | R | 0,277 | 0,297 | 1,032 | 0,010 | 0,072 | 0,129 | 0,440 |
| 90 | R | 0,088 | 0,089 | 0,518 | 0,012 | 0,045 | 0,170 | 0,119 |
| 91 | R | 0,154 | 0,040 | 2,181 | 0,008 | 0,096 | 0,146 | 0,000 |
| 92 | R | 0,000 | 0,087 | 0,278 | 0,005 | 0,027 | 0,029 | 0,136 |
| 222 | R | 0,083 | 0,330 | 0,844 | 0,016 | 0,023 | 0,082 | 0,497 |
| 227 | R | 0,060 | 0,260 | 0,000 | 0,003 | 0,000 | 0,110 | 0,254 |
| 366 | R | 0,039 | 0,179 | 0,995 | 0,009 | 0,020 | 0,269 | 0,081 |
| 501 | R | 0,112 | 0,601 | 0,141 | 0,016 | 0,070 | 0,175 | 0,278 |
| 502 | R | 0,108 | 2,676 | 1,165 | 0,009 | 0,557 | 0,475 | 5,152 |
| 503 | R | 0,000 | 0,109 | 0,295 | 0,004 | 0,011 | 0,081 | 1,257 |
| 506 | R | 0,241 | 10,483 | 1,210 | 0,019 | 0,343 | 0,578 | 1,564 |
| 508 | R | 0,097 | 0,446 | 0,593 | 0,013 | 0,000 | 0,161 | 0,271 |
| 523 | R | 0,382 | 2,329 | 1,537 | 0,018 | 0,176 | 0,251 | 0,536 |
| 529 | R | 0,119 | 0,354 | 0,277 | 0,012 | 0,053 | 0,248 | 0,452 |

(suite)

| Patient | Statut (NR, R ou RR) | FGF7 | IL8 | TGFB2 | CCL21 | CXCL6 | MMP2 | SFN |
|---|---|---|---|---|---|---|---|---|
| 532 | R | 0,050 | 0,387 | 0,796 | 0,010 | 0,032 | 0,106 | 1,454 |
| 535 | R | 0,041 | 0,328 | 0,399 | 0,025 | 0,026 | 0,177 | 0,507 |
| 536 | R | 0,037 | 0,119 | 0,705 | 0,010 | 0,180 | 0,123 | 0,148 |
| 537 | R | 0,215 | 0,058 | 0,272 | 0,007 | 0,027 | 0,177 | 1,218 |
| 538 | R | 0,000 | 0,041 | 0,337 | 0,014 | 0,000 | 0,111 | 0,139 |
| 556 | R | 0,033 | 0,063 | 0,540 | 0,008 | 0,000 | 0,124 | 0,168 |
| 560 | R | 0,021 | 0,530 | 0,717 | 0,008 | 0,082 | 0,188 | 0,911 |
| 565 | R | 0,000 | 1,079 | 0,441 | 0,006 | 0,000 | 0,247 | 0,172 |
| 567 | R | 0,076 | 1,297 | 3,227 | 0,006 | 0,356 | 0,578 | 0,295 |
| 568 | R | 0,011 | 0,127 | 0,312 | 0,002 | 0,000 | 0,048 | 0,046 |
| 571 | R | 0,000 | 0,804 | 1,028 | 0,026 | 0,036 | 0,291 | 1,338 |
| 572 | R | 0,008 | 0,064 | 1,110 | 0,005 | 0,005 | 0,068 | 0,078 |
| 581 | R | 0,173 | 2,549 | 4,084 | 0,006 | 0,129 | 0,642 | 1,145 |
| 585 | R | 0,032 | 0,818 | 1,248 | 0,017 | 0,112 | 0,370 | 0,306 |
| 598 | R | 0,118 | 0,660 | 0,940 | 0,008 | 0,033 | 0,093 | 0,128 |
| 604 | R | 0,051 | 0,084 | 0,829 | 0,011 | 0,082 | 0,239 | 0,263 |
| 605 | R | 0,023 | 0,178 | 0,688 | 0,009 | 0,000 | 0,082 | 0,398 |
| 629 | R | 0,071 | 0,152 | 0,927 | 0,003 | 0,000 | 0,293 | 1,366 |
| 308 | NR | 0,000 | 0,853 | 1,569 | 0,022 | 0,115 | 0,415 | 1,357 |
| 521 | NR | 1,231 | 3,732 | 12,168 | 0,052 | 0,170 | 2,732 | 0,000 |
| 526 | NR | 0,075 | 2,370 | 1,279 | 0,028 | 0,169 | 0,362 | 1,137 |
| 549 | NR | 0,000 | 0,979 | 0,616 | 0,023 | 0,080 | 1,828 | 7,835 |
| 574 | NR | 0,000 | 5,756 | 3,824 | 0,031 | 0,139 | 0,710 | 0,000 |
| 618 | NR | 0,104 | 1,035 | 1,329 | 0,025 | 0,294 | 0,607 | 1,419 |
| 619 | NR | 0,000 | 0,301 | 5,081 | 0,011 | 0,064 | 0,225 | 3,519 |
| 636 | NR | 0,109 | 0,270 | 2,144 | 0,048 | 0,081 | 0,774 | 0,880 |
| 646 | NR | 0,418 | 0,518 | 1,729 | 0,018 | 0,087 | 0,547 | 1,952 |
| 657 | NR | 1,741 | 4,840 | 0,000 | 0,013 | 2,412 | 1,075 | 1,444 |
| 658 | NR | 0,559 | 0,557 | 0,000 | 0,009 | 0,000 | 0,195 | 0,000 |
| 664 | NR | 0,536 | 0,953 | 0,856 | 0,029 | 0,139 | 1,098 | 0,719 |
| 6 | NR | 0,000 | 2,648 | 5,296 | 0,004 | 0,000 | 1,505 | 0,345 |
| 46 | NR | 0,394 | 0,911 | 0,269 | 0,021 | 0,115 | 0,478 | 0,366 |
| 58 | NR | 0,136 | 0,616 | 0,613 | 0,016 | 0,152 | 0,266 | 1,753 |
| 75 | NR | 0,125 | 0,908 | 1,404 | 0,033 | 0,082 | 0,755 | 2,274 |
| 80 | NR | 0,270 | 0,745 | 1,032 | 0,018 | 0,096 | 0,124 | 1,490 |
| 83 | NR | 0,322 | 0,231 | 1,173 | 0,073 | 0,154 | 0,325 | 0,730 |

(suite)

| Patient | Statut (NR, R ou RR) | FGF7 | IL8 | TGFB2 | CCL21 | CXCL6 | MMP2 | SFN |
|---|---|---|---|---|---|---|---|---|
| 145 | NR | 0,333 | 0,755 | 1,905 | 0,023 | 0,196 | 0,895 | 0,323 |
| 167 | NR | 0,037 | 0,923 | 3,215 | 0,018 | 0,112 | 0,228 | 0,000 |
| 509 | NR | 0,131 | 1,157 | 1,235 | 0,025 | 0,218 | 0,378 | 0,236 |
| 516 | NR | 0,069 | 0,844 | 0,895 | 0,017 | 0,049 | 0,323 | 0,145 |
| 524 | NR | 0,296 | 0,949 | 0,927 | 0,009 | 0,025 | 0,262 | 0,129 |
| 527 | NR | 0,172 | 0,657 | 1,619 | 0,045 | 0,060 | 0,529 | 0,367 |
| 534 | NR | 0,065 | 1,828 | 0,237 | 0,017 | 0,194 | 0,437 | 0,584 |
| 582 | NR | 0,094 | 2,949 | 4,014 | 0,012 | 0,409 | 0,732 | 0,277 |
| 596 | NR | 0,018 | 0,883 | 2,049 | 0,017 | 0,080 | 0,384 | 0,078 |
| 602 | NR | 0,019 | 0,936 | 1,021 | 0,010 | 0,059 | 0,115 | 0,241 |
| 645 | NR | 2,479 | 0,000 | 0,078 | 0,004 | 0,000 | 2,085 | 0,000 |
| 647 | NR | 0,299 | 0,192 | 0,927 | 0,028 | 0,110 | 0,356 | 0,384 |
| 649 | NR | 0,409 | 2,742 | 1,959 | 0,034 | 0,167 | 0,311 | 0,563 |
| 650 | NR | 0,216 | 5,816 | 1,042 | 0,022 | 0,446 | 0,398 | 1,177 |
| 651 | NR | 0,000 | 0,940 | 0,868 | 0,009 | 0,150 | 0,115 | 1,173 |
| 659 | NR | 0,212 | 4,423 | 0,838 | 0,006 | 0,237 | 0,191 | 0,300 |
| 660 | NR | 0,229 | 1,113 | 1,602 | 0,021 | 0,308 | 0,224 | 0,000 |
| 662 | NR | 0,161 | 0,046 | 0,244 | 0,022 | 0,051 | 0,233 | 0,099 |
| 665 | NR | 0,126 | 0,949 | 0,821 | 0,014 | 0,061 | 0,177 | 0,835 |
| 666 | NR | 0,000 | 2,799 | 0,832 | 0,028 | 0,000 | 0,222 | 0,226 |
| 642 | NR | 2,099 | 13,408 | 2,158 | 0,046 | 1,682 | 1,699 | 1,459 |
| 67 | NR | 0,084 | 0,104 | 0,380 | 0,006 | 0,036 | 0,191 | 0,184 |
| 563 | NR | 0,000 | 0,330 | 0,871 | 0,005 | 0,06 | 0,21 | 1,11 |
| 573 | NR | 0,038 | 0,323 | 1,306 | 0,028 | 0,06 | 0,41 | 0,85 |
| 599 | NR | 0,000 | 0,262 | 0,536 | 0,005 | 0,01 | 0,03 | 0,00 |
| 641 | NR | 0,478 | 0,657 | 0,362 | 0,012 | 0,15 | 0,29 | 0,21 |
| 49 | RR | 0,054 | 0,284 | 1,032 | 0,018 | 0,00 | 0,19 | 2,91 |
| 505 | RR | 0,000 | 0,410 | 1,098 | 0,006 | 0,00 | 0,12 | 0,00 |
| 514 | RR | 0,000 | 1,053 | 1,376 | 0,011 | 0,07 | 0,40 | 1,20 |
| 579 | RR | 0,622 | 0,483 | 2,685 | 0,004 | 0,07 | 0,23 | 2,97 |
| 643 | RR | 1,959 | 15,835 | 4,627 | 0,014 | 1,20 | 1,78 | 1,18 |
| 56 | RR | 0,000 | 0,363 | 0,943 | 0,011 | 0,00 | 0,15 | 0,00 |
| 60 | RR | 0,000 | 0,145 | 0,272 | 0,015 | 0,00 | 0,12 | 0,45 |
| 87 | RR | 0,099 | 1,608 | 2,242 | 0,021 | 0,12 | 0,36 | 0,28 |
| 531 | RR | 0,904 | 0,053 | 8,340 | 0,002 | 0,00 | 2,79 | 0,12 |
| 533 | RR | 0,116 | 3,084 | 1,840 | 0,008 | 0,19 | 0,33 | 0,18 |

(suite)

| Patient | Statut (NR, R ou RR) | FGF7 | IL8 | TGFB2 | CCL21 | CXCL6 | MMP2 | SFN |
|---|---|---|---|---|---|---|---|---|
| 543 | RR | 0,611 | 0,597 | 1,193 | 0,040 | 0,06 | 0,66 | 1,04 |
| 554 | RR | 0,000 | 1,072 | 1,439 | 0,005 | 0,12 | 0,26 | 0,95 |
| 557 | RR | 0,074 | 0,700 | 0,898 | 0,007 | 0,06 | 0,22 | 0,32 |
| 558 | RR | 0,094 | 0,983 | 1,343 | 0,002 | 0,10 | 0,28 | 0,20 |
| 559 | RR | 0,005 | 0,536 | 1,173 | 0,005 | 0,07 | 0,15 | 0,20 |
| 562 | RR | 0,104 | 1,380 | 2,445 | 0,007 | 0,14 | 0,69 | 0,51 |
| 576 | RR | 0,065 | 1,064 | 1,500 | 0,010 | 0,13 | 0,55 | 0,75 |
| 588 | RR | 0,114 | 0,255 | 1,424 | 0,002 | 0,04 | 0,29 | 0,18 |
| 589 | RR | 0,082 | 0,315 | 2,204 | 0,012 | 0,03 | 0,41 | 1,63 |
| 591 | RR | 0,204 | 0,113 | 1,244 | 0,013 | 0,05 | 0,47 | 0,17 |
| 592 | RR | 0,294 | 7,781 | 6,566 | 0,020 | 1,09 | 1,60 | 0,53 |

**Tableau 36 : valeurs BMQ des patients pour les gènes CXCL11, AFP, VEGFD, CRP, CXCL9 (Ct normalisé conformément à la méthode du $2^{-\Delta Ct}$)**

| Patient | Statut (NR, R ou RR) | CXCL11 | AFP | VEGFD | CRP | CXCL9 |
|---|---|---|---|---|---|---|
| 59 | R | 0,111 | 0,026 | 0,000 | 1,390 | 0,166 |
| 62 | R | 0,454 | 0,024 | 1,705 | 0,710 | 1,526 |
| 73 | R | 0,518 | 0,005 | 0,867 | 0,707 | 0,264 |
| 125 | R | 0,095 | 0,030 | 0,000 | 0,197 | 0,092 |
| 306 | R | 1,753 | 0,045 | 1,017 | 1,834 | 1,464 |
| 344 | R | 0,186 | 0,053 | 2,141 | 3,719 | 1,386 |
| 346 | R | 0,123 | 0,007 | 0,962 | 337,775 | 0,225 |
| 504 | R | 0,132 | 0,023 | 0,000 | 7,387 | 0,349 |
| 513 | R | 0,062 | 0,031 | 0,705 | 0,519 | 0,129 |
| 528 | R | 0,291 | 0,004 | 2,099 | 40,085 | 0,613 |
| 530 | R | 0,121 | 0,161 | 0,376 | 14,774 | 0,174 |
| 546 | R | 0,167 | 0,049 | 0,000 | 0,269 | 0,148 |
| 569 | R | 0,140 | 0,048 | 0,261 | 9,190 | 0,246 |
| 570 | R | 0,253 | 0,113 | 0,107 | 1,945 | 0,572 |
| 575 | R | 1,329 | 0,050 | 0,437 | 0,809 | 0,969 |
| 577 | R | 0,102 | 0,058 | 0,747 | 0,737 | 0,236 |
| 583 | R | 0,824 | 0,038 | 0,162 | 1,866 | 0,620 |
| 601 | R | 0,208 | 0,008 | 0,420 | 1,227 | 0,129 |
| 613 | R | 0,158 | 0,014 | 0,251 | 1,670 | 1,091 |
| 614 | R | 0,459 | 0,019 | 0,207 | 3,745 | 0,804 |
| 639 | R | 0,095 | 0,108 | 0,760 | 0,940 | 0,168 |

(suite)

| Patient | Statut (NR, R ou RR) | CXCL11 | AFP | VEGFD | CRP | CXCL9 |
|---|---|---|---|---|---|---|
| 45 | R | 0,597 | 0,032 | 0,182 | 1,575 | 0,793 |
| 50 | R | 0,043 | 0,010 | 0,147 | 0,480 | 0,174 |
| 55 | R | 0,168 | 0,033 | 0,207 | 0,747 | 0,076 |
| 63 | R | 0,376 | 0,036 | 0,419 | 1,765 | 0,235 |
| 65 | R | 0,219 | 0,024 | 0,299 | 0,874 | 0,275 |
| 66 | R | 0,192 | 0,013 | 0,255 | 2,420 | 0,386 |
| 72 | R | 0,024 | 0,005 | 0,118 | 2,321 | 0,107 |
| 76 | R | 0,399 | 0,034 | 0,221 | 1,548 | 0,844 |
| 86 | R | 0,329 | 0,025 | 0,077 | 2,107 | 0,316 |
| 88 | R | 0,127 | 0,047 | 0,239 | 0,207 | 0,183 |
| 90 | R | 0,233 | 0,014 | 0,272 | 0,633 | 0,493 |
| 91 | R | 0,285 | 0,021 | 0,197 | 0,605 | 0,835 |
| 92 | R | 0,091 | 0,011 | 0,015 | 1,747 | 0,283 |
| 222 | R | 0,300 | 0,067 | 0,116 | 0,946 | 0,185 |
| 227 | R | 0,082 | 0,042 | 0,046 | 0,693 | 0,085 |
| 366 | R | 0,108 | 0,040 | 0,680 | 2,918 | 0,430 |
| 501 | R | 0,538 | 0,047 | 0,090 | 3,864 | 0,320 |
| 502 | R | 0,193 | 0,014 | 0,135 | 5,836 | 0,702 |
| 503 | R | 0,059 | 0,027 | 0,126 | 2,848 | 0,091 |
| 506 | R | 0,714 | 0,014 | 0,835 | 0,566 | 1,619 |
| 508 | R | 0,620 | 0,049 | 0,130 | 8,311 | 0,838 |
| 523 | R | 0,312 | 0,018 | 0,086 | 0,159 | 1,270 |
| 529 | R | 0,262 | 0,025 | 0,083 | 0,859 | 0,523 |
| 532 | R | 0,074 | 0,035 | 0,148 | 0,099 | 0,330 |
| 535 | R | 0,191 | 0,006 | 0,229 | 0,850 | 0,050 |
| 536 | R | 0,129 | 0,006 | 0,100 | 17,509 | 0,344 |
| 537 | R | 0,053 | 0,019 | 0,122 | 1,429 | 0,200 |
| 538 | R | 0,134 | 0,006 | 0,178 | 0,189 | 0,071 |
| 556 | R | 0,088 | 0,021 | 0,000 | 0,880 | 0,058 |
| 560 | R | 0,611 | 0,012 | 0,082 | 0,326 | 0,946 |
| 565 | R | 0,536 | 0,049 | 0,337 | 3,918 | 0,156 |
| 567 | R | 0,463 | 0,005 | 0,273 | 2,056 | 0,429 |
| 568 | R | 0,027 | 0,002 | 0,068 | 3,238 | 0,107 |
| 571 | R | 2,370 | 0,088 | 0,624 | 1,414 | 2,603 |
| 572 | R | 0,092 | 0,018 | 0,053 | 4,908 | 0,087 |
| 581 | R | 0,100 | 0,007 | 0,355 | 2,014 | 0,132 |
| 585 | R | 0,976 | 0,012 | 0,334 | 0,874 | 1,185 |

(suite)

| Patient | Statut (NR, R ou RR) | CXCL11 | AFP | VEGFD | CRP | CXCL9 |
|---|---|---|---|---|---|---|
| 598 | R | 0,235 | 0,018 | 0,126 | 1,064 | 0,249 |
| 604 | R | 0,451 | 0,043 | 0,151 | 0,622 | 0,238 |
| 605 | R | 0,156 | 0,026 | 0,114 | 0,838 | 0,089 |
| 629 | R | 0,064 | 0,013 | 0,618 | 3,824 | 0,108 |
| 308 | NR | 4,942 | 0,100 | 0,293 | 1,636 | 0,096 |
| 521 | NR | 1,459 | 0,117 | 0,601 | 0,336 | 0,712 |
| 526 | NR | 0,597 | 0,012 | 0,294 | 2,129 | 1,352 |
| 549 | NR | 0,838 | 0,062 | 0,350 | 0,809 | 1,248 |
| 574 | NR | 4,516 | 0,095 | 2,136 | 1,993 | 0,584 |
| 618 | NR | 0,503 | 0,025 | 0,580 | 1,157 | 0,236 |
| 619 | NR | 0,516 | 0,000 | 0,804 | 4,976 | 0,459 |
| 636 | NR | 2,042 | 0,128 | 0,236 | 0,312 | 0,432 |
| 646 | NR | 1,072 | 0,014 | 0,000 | 1,039 | 0,470 |
| 657 | NR | 0,405 | 0,041 | 0,518 | 0,367 | 0,149 |
| 658 | NR | 0,081 | 0,044 | 0,000 | 15,725 | 0,304 |
| 664 | NR | 0,114 | 0,110 | 0,976 | 38,452 | 0,284 |
| 6 | NR | 0,018 | 0,002 | 0,125 | 0,010 | 0,018 |
| 46 | NR | 0,180 | 0,007 | 0,000 | 0,758 | 0,073 |
| 58 | NR | 0,263 | 0,050 | 0,289 | 0,194 | 0,070 |
| 75 | NR | 1,087 | 0,068 | 1,227 | 0,112 | 0,500 |
| 80 | NR | 0,410 | 0,041 | 0,209 | 3,411 | 0,283 |
| 83 | NR | 1,053 | 0,043 | 0,365 | 0,635 | 0,476 |
| 145 | NR | 0,204 | 0,047 | 0,149 | 1,636 | 0,307 |
| 167 | NR | 0,155 | 0,012 | 0,184 | 3,811 | 0,153 |
| 509 | NR | 0,983 | 0,008 | 0,286 | 0,263 | 1,102 |
| 516 | NR | 0,382 | 0,063 | 0,435 | 12,252 | 0,660 |
| 524 | NR | 0,258 | 0,018 | 0,000 | 2,612 | 0,642 |
| 527 | NR | 0,198 | 0,031 | 0,460 | 1,315 | 0,179 |
| 534 | NR | 1,347 | 0,069 | 0,115 | 3,494 | 0,369 |
| 582 | NR | 1,007 | 0,031 | 0,232 | 2,099 | 0,351 |
| 596 | NR | 0,564 | 0,005 | 0,267 | 0,635 | 1,240 |
| 602 | NR | 0,124 | 0,024 | 0,048 | 1,177 | 0,328 |
| 645 | NR | 0,013 | 0,000 | 3,458 | 0,017 | 0,006 |
| 647 | NR | 0,402 | 0,018 | 0,480 | 1,495 | 0,212 |
| 649 | NR | 0,376 | 0,049 | 0,423 | 1,479 | 0,603 |
| 650 | NR | 0,321 | 0,052 | 1,227 | 0,351 | 0,241 |
| 651 | NR | 0,257 | 0,035 | 0,279 | 0,809 | 0,133 |

(suite)

| Patient | Statut (NR, R ou RR) | CXCL11 | AFP | VEGFD | CRP | CXCL9 |
|---------|----------------------|--------|-----|-------|-----|-------|
| 659 | NR | 0,092 | 0,067 | 0,090 | 0,576 | 0,286 |
| 660 | NR | 0,098 | 0,035 | 0,127 | 0,717 | 0,207 |
| 662 | NR | 0,230 | 0,040 | 0,111 | 0,315 | 0,237 |
| 665 | NR | 0,070 | 0,009 | 0,710 | 1,847 | 0,280 |
| 666 | NR | 0,158 | 0,037 | 0,415 | 0,543 | 0,186 |
| 642 | NR | 2,488 | 0,049 | 0,117 | 0,768 | 2,780 |
| 67 | NR | 0,044 | 0,021 | 0,078 | 3,972 | 0,278 |
| 563 | NR | 0,233 | 0,007 | 0,000 | 1,597 | 1,444 |
| 573 | NR | 0,740 | 0,064 | 0,339 | 0,355 | 0,319 |
| 599 | NR | 0,200 | 0,007 | 0,164 | 0,763 | 0,104 |
| 641 | NR | 0,134 | 0,010 | 0,163 | 0,607 | 0,204 |
| 49 | RR | 0,382 | 0,111 | 1,007 | 0,570 | 1,343 |
| 505 | RR | 0,029 | 0,054 | 0,384 | 0,234 | 0,045 |
| 514 | RR | 0,017 | 0,089 | 0,000 | 23,344 | 0,476 |
| 579 | RR | 0,193 | 0,024 | 0,441 | 5,856 | 0,314 |
| 643 | RR | 0,222 | 0,011 | 4,500 | 6,751 | 0,626 |
| 56 | RR | 0,372 | 0,027 | 0,000 | 0,428 | 0,388 |
| 60 | RR | 0,272 | 0,021 | 0,361 | 0,187 | 0,078 |
| 87 | RR | 0,361 | 0,020 | 0,135 | 0,338 | 0,171 |
| 531 | RR | 0,007 | 0,001 | 0,576 | 0,064 | 0,009 |
| 533 | RR | 0,274 | 0,015 | 0,061 | 0,107 | 0,346 |
| 543 | RR | 0,712 | 0,025 | 0,468 | 1,227 | 2,014 |
| 554 | RR | 0,168 | 0,031 | 0,274 | 4,993 | 0,578 |
| 557 | RR | 0,384 | 0,010 | 0,133 | 0,722 | 0,208 |
| 558 | RR | 0,067 | 0,023 | 0,235 | 0,208 | 0,111 |
| 559 | RR | 0,230 | 0,009 | 0,209 | 1,094 | 0,425 |
| 562 | RR | 0,306 | 0,035 | 0,266 | 0,818 | 0,245 |
| 576 | RR | 1,419 | 0,041 | 0,216 | 2,211 | 1,366 |
| 588 | RR | 0,024 | 0,020 | 0,351 | 1,676 | 0,034 |
| 589 | RR | 0,399 | 0,050 | 0,287 | 0,034 | 0,197 |
| 591 | RR | 0,425 | 0,016 | 0,112 | 0,186 | 0,674 |
| 592 | RR | 0,930 | 0,034 | 0,299 | 0,908 | 0,976 |

**Tableau 44 : kits pour les dosages protéiques**

| Marqueurs | IL8 | LGALS3BP | MDK | CXCL10 / IP-10 | CCL21 |
|---|---|---|---|---|---|
| Kit EIA | QUANTIKINE HUMAN CXCL8/IL-8 IMMUNOASSAY | 90K/Mac-2 BP ELISA | HUMAN MIDKINE ELISA | QUANTIKINE HUMAN CXCL10/IP10 IMMUNOASSAY | HUMAN CCL21/6Ckine IMMUNOASSAY |
| Fournisseur | R&D Systems | Abnova | Abnova | R&D Systems | R&D Systems |
| Référence | D8000C | KA0140 | KA0028 V. 02 | DIP100 | D6C00 |
| Type d'ELISA | Sandwich | Sandwich | Sandwich | Sandwich | Sandwich |
| Types d'échantillons | Sérum, plasma, milieu de culture cellulaire | sérum, surnageant de culture cellulaire | Sérum, plasma, tissu, milieu de culture cellulaire | Sérum, plasma, salive, milieu de culture cellulaire | sérum, plasma |
| prise d'essai | 50μL | 20μL | 25μL | 75μL | 100μL |
| phase solide | mAc anti-IL8 | mAc anti-LGALS3BP | pAc anti MDK | mAc anti-IP10 | mAc anti-CCL21 |
| conjugué | pAc-HRP anti-IL8 | mAc-HRP anti-LGALS3BP | pAc-biotin anti-MDK | pAc-HRP anti-IP10 | pAc-HRP anti-CCL21 |
| sensibilité | 3,5 pg/mL | 0,92 ng/mL | 0,33 ng/mL | 1,67 pg/mL | 9,9 pg/mL |
| gamme de détection | 31,2-2000 pg/mL | 12,5 à 200 ng/mL | 2 - 10 ng/mL | 7,8-500 pg/mL | 91-371pg/mL |
| spécificité | IL8 humaine et recombinante, pas de réaction croisée avec ANG, AR, CNTF, b-ECGF, EGF, Epo, FGF acide, FGF basique, FGF-4, FGF-5, FGF-6, GCSF, GM-CSF, GROa, GROb, GROg, sgp130, HBEGF, HGF, I-309, IFN-g, IGF-I, IGF-II, IL-1a, IL-1b, IL-1ra, IL-1 sRI | LGALS3BP humaine | MIDKINE humaine | IP10 native et recombinante, pas de réaction croisée avec BLC/BCA-1, ENA-78, GCP-2, GROa, GROg, IFN-g, IL-8, IL-8 (endothelial cell-derived), I-TAC, MIG, NAP-2, SDF-1a, SDF-1b humain recombinant, BLC/BCA-1, CRG-2 (IP-10), GCP-2, KC, MIG, SDF-1a souris recombinant et Il-8 porc recombinante | CCL21 humaine |
| pAc = anticorps polyclonal  mAc = anticorps monoclonal | | | | | |

**Administration du traitement anti-viral et analyse de la réponse du patient :**

Après PBH et prélèvement de sérum, chaque patient a reçu un traitement antiviral qui est actuellement considéré comme le traitement standard de l'hépatite C, à savoir un traitement basé sur l'association de deux agents antiviraux, en l'occurrence alpha interféron et ribavirine.

Dans le cadre de l'essai ici décrit, tous les patients ont reçu le traitement suivant :

- soit :

    ◦ de l'interféron alpha-2b pégylé (PEG-INTRON®; Schering Plough Corporation ; Kenilworth, NJ ; U.S.A.) à une dose de 1,5 g/kg/semaine, et
    ◦ de la ribavirine (REBETOL® ; Schering Plough Corporation ; Kenilworth, NJ ; U.S.A.) à une dose de :

        ▪ 800 à 1200 mg/kg/jour pour ceux des patients qui étaient infectés par au moins un génotype 1 et/ou 4 et/ou 5 de VHC, ou à une dose de
        ▪ 800 mg/kg/jour pour ceux des patients qui étaient infectés par au moins un génotype 2 et/ou 3 de VHC,

- soit :

    ◦ de l'interféron alpha-2a pégylé (PEGASYS® ; Roche Corporation ; F. Hoffmann-La Roche Ltd. ; Basel, Suisse) à une dose de 180 g/kg/semaine, et

    ◦ de la ribavirine (COPEGUS® ; Roche Corporation ; F. Hoffmann-La Roche Ltd. ; Basel, Suisse) à une dose de 1000 à 1200 mg/kg/jour.

Le traitement a été administré pendant 24 semaines pour ceux des patients qui étaient infectés par au moins un génotype 2 et/ou 3 de VHC, et pendant 48 semaines pour ceux des patients qui étaient infectés par au moins un génotype 1 et/ou 4 et/ou 5 de VHC.

La charge virale a été mesurée en semaine 24, à la fin du traitement et 6 mois après la fin du traitement, par quantification des ARN de VHC présents dans le sérum de chaque patient, à l'aide du test de quantification de l'ARN de VHC VERSANT® HCV RNA 3.0 (bDNA) ASSAY de la société Siemens Healthcare Diagnostics (limite de quantification = 615 - 7 690 000 UI/mL).

Chaque patient a été classé en fonction de sa réponse au traitement telle que mesurée par le test de mesure de la charge virale sérique en VHC.

Un patient a été considéré être :

- un patient **répondeur au traitement (patient classé R)**, lorsque la charge virale de VHC est devenue indétectable dans le sang du patient à l'issue du traitement et qu'elle est restée indétectable 6 mois après l'arrêt du traitement ;
- un patient **non répondeur au traitement (patient classé NR)**, lorsque la charge virale de VHC est restée détectable dans le sang du patient à l'issue du traitement ;
- un patient **répondeur-rechuteur (patient classé RR)**, lorsque la charge virale de VHC est devenue indétectable dans le sang du patient à l'issue du traitement, mais qu'elle est redevenue détectable 6 mois après l'arrêt du traitement.

La charge virale de VHC a été considérée indétectable dans le sang du patient, lorsque le dosage des ARN de VHC dans le sérum du patient a conduit à une valeur inférieure à 12 Unités Internationales (UI) par mL de sérum, telle que mesurée à l'aide du kit VERSANT® HCV RNA 3.0 (bDNA) ASSAY de la société Siemens Healthcare Diagnostics ci-dessus indiqué.

Trois sous-populations, ou cohortes, ont ainsi été formées (sous-population des patients R, sous-population des patients NR et sous-population des patients RR).

**2. Comparaison des valeurs de dosage des sous-populations NR et R pour établir un modèle de classification multivariée**

Les valeurs de dosage obtenues au §1. ci-dessus pour les sous-populations « répondeurs » (R) et « non répondeurs » (NR) ont été comparées entre elles pour construire un modèle de classification multivariée qui, à partir de la combinaison de ces valeurs, classe le patient test parmi les patients qui ont une forte probabilité de répondre au traitement anti-VHC (classe de R) ou parmi la classe des patients qui ont une forte probabilité de ne pas répondre au traitement anti-VHC (classe des NR).

Les valeurs de dosage obtenues au §1. ci-dessus pour la sous-population « répondeurs-rechuteurs » (RR) ont également été comparées aux valeurs de dosage obtenues pour les sous-populations R et NR. Il a été observé que la sous-population RR ségrége très fortement avec celle des R : les patients RR sont majoritairement classés comme des R.

Un modèle de classification peut par exemple être obtenu en suivant une méthode d'analyse statistique multivariée, ou une méthode d'analyse mathématique multivariée.

Modèles mROC :

Une méthode d'analyse mathématique multivariée appropriée est la méthode mROC (méthode *Receiver Operating Characteristic* multivariée).

En utilisant les valeurs de dosage obtenues au §1. ci-dessus pour les sous-populations R et NR, des modèles mROC ont été construits comme décrit dans Kramar *et al.* 1999 et Kramar *et al.* 2001. A cet effet, le logiciel mROC version 1.0, disponible commercialement auprès des concepteurs (Andrew Kramar, Antoine Fortune, David Farragi, Benjamin Reiser) a été utilisé.

Andrew Kramar et Antoine Fortune peuvent être contactés auprès de, ou via, l'Unité de Biostatistique du Centre Régional de Lutte contre le Cancer (CRLC) Val d'Aurelle - Paul Lamarque (208, rue des Apothicaires ; Parc Euromédecine ; 34298 Montpellier Cedex 5 ; France).

David Faraggi et Benjamin Reiser peuvent être contactés auprès du, ou via le, Département de Statistique de l'Université de Haifa (Mount Carmel ; Haifa 31905 ; Israël).

A partir des données de dosage entrées, la méthode mROC génère une règle décisionnelle sous la forme d'une fonction linéaire [Z = f(BMQ$_1$, BMQ$_2$, BMQ$_3$,... )] du type Z = $\alpha$.BMQ$_1$ + $\beta$.BMQ$_2$ + $\gamma$.BMQ$_3$...,

où BMQ$_1$, BMQ$_2$, BMQ$_3$... sont les valeurs de dosage des niveaux d'expression de chacun des gènes choisis, et

l'utilisateur identifie la valeur de référence ou seuil ($\delta$) qui confère les meilleures performances à cette combinaison.

Cette fonction et ce seuil constituent un modèle de classification multivariée.

La fonction f calculée par la méthode mROC a ensuite été appliquée aux valeurs de dosage du niveau d'expression des gènes BMQ$_1$, BMQ$_2$, BMQ$_3$... mesurées pour un sujet test p. La valeur Z calculée pour un sujet test p a ensuite été comparée au seuil $\delta$.

Par exemple, lorsque la valeur moyenne de la combinaison des niveaux d'expression desdits gènes choisis dans la cohorte des individus « R » est inférieure à celle de la cohorte des individus « NR » :

- si $Z \geq \delta$, le test est positif : il est déclaré que le sujet p est un patient NR (le sujet est pronostiqué non-répondeur au traitement) ; et
- si $Z < \delta$, le test est négatif : il est déclaré que le sujet p est un patient R (le sujet est pronostiqué répondeur au traitement).

Inversement, lorsque la valeur moyenne de la combinaison des niveaux d'expression desdits gènes choisis dans la cohorte des individus « R » est supérieure à celle de la cohorte des individus « NR » :

- si $Z \geq \delta$, le test est négatif : il est déclaré que le sujet p est un patient R (le sujet est pronostiqué répondeur au traitement) ; et
- si $Z < \delta$, le test est positif : il est déclaré que le sujet p est un patient NR (le sujet est pronostiqué non-répondeur au traitement).

Modèles WKNN :

Une méthode d'analyse statistique multivariée appropriée est la méthode WKNN (*Weighed k Nearest Neighbors*).

En utilisant les valeurs de dosage obtenues au §1. ci-dessus pour les sous-populations R et NR, des modèles WKNN ont été construits comme décrit dans Hechenbichler et Schliep 2004.

Schématiquement, une méthode WKNN attribue chaque nouveau cas (*y,x*) à la classe *l* de poids maximal dans un voisinage à *k* voisins selon la formule :

$$l = \max{}_r\left( \sum_{i=1}^{k} K\big(D\big(x, x_{(i)}\big)\big) I\big(y_{(i)} = r\big)\right)$$

où *r* représente l'indice des classes cliniques d'intérêt (en l'occurrence, sous-population R ou sous-population NR), et est égal à 0 ou 1.

Pour construire les modèles WKNN, le logiciel R (librairie WKNN), librement disponible sur http://www.r-project.org/, a été utilisé. Les paramètres de réglage suivants ont été utilisés :

- Kernel (K) : bipondérée (« biweight ») ;

- Distance (D) : minkowski de paramètre 2 ;
- Nombre de voisins (k) : 3 ;
  ou
- Kernel (K) : tripondéré (« triweight ») ;
- Distance (D) : minkowski de paramètre 1 ;
- Nombre de voisins (k) : 4 ;
  ou
  ou- Kernel (K) : bipondérée (« biweight ») ;
- Distance (D) : minkowski de paramètre 2 ;
- Nombre de voisins (k) : 3.

Les modèles WKNN ainsi construits ont ensuite été utilisés pour déterminer le statut R ou NR des sujets, en entrant les valeurs de dosage de ces sujets dans les modèles WKNN ainsi construits.

Les valeurs de dosage des niveaux d'expression des gènes choisis d'un sujet test *p* ont été comparées à celles de ces voisins (*k*). Le modèle WKNN calcule le poids devant être attribué à la classe « sous-population R » et celui devant être attribué à la classe « sous-population NR » pour ce sujet p. Le sujet p est alors classé par le modèle WKNN dans la classe majoritaire, par exemple, dans la classe « sous-population NR » si les poids des classes « sous-population R » et « sous-population NR » calculés par la méthode WKNN étaient de 0,3 et 0,7, respectivement.

L'erreur LOOCV (« Leave-One-Out-Cross-Validation ») est telle que définie dans Hastie, Tibishirani et Friedman, 2009.

Modèles à forêt aléatoire (*Random Forest* ou RF) :

En utilisant les valeurs de dosage obtenues au §1. ci-dessus pour les sous-populations R et NR, des modèles à forêt aléatoires (*Random Forest* ou RF) ont été construits comme décrit dans Breiman 2001, Liaw et Wiener 2002.

A cet effet, le logiciel R, librement disponible sur http://www.r-project.org/, a été utilisé. Les paramètres de réglage suivants ont été utilisés :

- NumberOfTrees = 500 ;
- NumberOfDescriptors = sqrt(D).

Les données numériques listées dans le fichier de sortie de R permettent d'évaluer les signatures avec le calcul des paramètres suivants : calcul des valeurs de Vrai Positif (VP), Faux Positif (FP), Vrai Négatif (VN) et Faux Négatif (FN), *cf.* ci-dessous.

Les données extraites du fichier sortie des modèles RF ainsi construits ont présenté la forme suivante :
"*OOB estimate oferror rate: taux d'erreur global*
*Confusion matrix:*

|  | NR | R | Class. error |
|---|---|---|---|
| NR | VP | FN | taux d'erreur declassificationdes NR |
| R | FP | VN | taux d'erreur declassificationdes R |

*ROC score (out-of-bag data): score ROC pour echantillons predits*"

OOB est l'acronyme de *Out-Of-Bag*, et représente une évaluation de l'erreur.

Ces données sorties indiquent directement les valeurs des paramètres VP (nombre de patients NR qui ont été classés NR), FP (nombre de patients R qui ont été classés NR), VN (nombre de patients R qui ont été classés R) et FN (nombre de patients NR qui ont été classés R).

Les formules ci-dessous permettent de calculer les valeurs de sensibilité (Se), de spécificité (Spe), de Valeur Prédictive Positive (VPP), et de Valeur Prédictive Négative (VPN) :

$$Se = VP/(VP + FN) ;$$

$$Sp = VN/(VN + FP) ;$$

$$VPP = VP / (VP + FP) ;$$

$$VPN = VN / (VN + FN).$$

Les données sorties indiquent également de manière directe le taux d'erreur et le score ROC du modèle construit.

Les modèles RF ainsi construits ont ensuite été utilisés pour déterminer le score de fibrose hépatique de sujets tests. Les valeurs de dosage des niveaux d'expression des gènes de ces sujets tests ont été entrées dans un modèle RF, lequel génère des données sorties comme ci-dessus présentées, et classe le sujet testé dans la classe « sous-population R » ou « sous-population NR ».

L'erreur LOOCV est telle que définie dans Hastie, Tibishirani et Friedman, 2009.

Modèles à réseaux de neurones :

Une autre méthode d'analyse statistique multivariée appropriée est une méthode à réseaux de neurones. Schématiquement, un réseau de neurones comprend un graphe pondéré orienté dont les noeuds symbolisent les neurones. Le réseau est construit à partir des valeurs de dosage des sous-populations (en l'occurrence, R versus NR), et est ensuite utilisé pour déterminer à quelle classe (en l'occurrence, R ou NR) appartient un nouvel élément (en l'occurrence, un patient test p).

En utilisant les valeurs de dosage obtenues au §1. ci-dessus pour les sous-populations R et NR, des modèles à réseaux de neurones ont été construits comme décrit dans Intrator et Intrator 1993, Riedmiller et Braun 1993, Riedmiller 1994, Anastasiadis *et. al.* 2005 ; *cf.* http://cran.r-project.org/web/packages/neuralnet/index.html.

A cet effet, le logiciel R librement disponible sur http://www.r-project.org/, a été utilisé (version 1.3 de Neuralnet, écrit par Stefan Fritsch et Frauke Guenther, suivant le travail de Marc Suling).

Les options de calcul suivantes ont été utilisées :

« *NumberOfHiddenNodes = 1 et 2*
*WeightDecayfactor = 0.001*
*CrossValidate = True*
*Cross ValidationFolds = 5*
*MaxNumberIterations = 2000*
*MaxNumberWeights= 2000* ».

Pour chacune des combinaisons, la matrice de confusion est extraite sous une forme du type suivant :

« *Cross-validation results (5-fold):*

| Nodes | | Decay | ROC | Score Best |
|---|---|---|---|---|
| 1 | 1 | | | |
| 2 | 2 | | | *** |

*Contingency Table (best CV model):*

| | Predicted | |
|---|---|---|
| Actual | R | NR |
| R | VN | FP |
| NR | FN | VP |

Dans cet exemple, on observe que le meilleur modèle est le modèle 2, signalé par « *** » dans la colonne « ScoreBest ».

Ces données sorties indiquent directement les valeurs des paramètres VP (nombre de patients NR qui ont été classés NR), FP (nombre de patients R qui ont été classés NR), VN (nombre de patients R qui ont été classés R) et FN (nombre de patients NR qui ont été classés R).

Les paramètres suivants sont évalués : la sensibilité (Se), la spécificité (Spe), la Valeur Prédictive Positive (VPP) et la Valeur Prédictive Négative (VPN) (*cf.* formules de Se, Spe, VPP et VPN ci-dessus).

Le score ROC est extrait directement du fichier de sortie sur la ligne identifiée par « *** » qui correspond au meilleur modèle. Le taux d'erreur est calculé par la formule suivante :

$$\text{Class\_err} = (FP + FN) / (FP + VP + FN + VN).$$

Les modèles à réseau de neurones ainsi construits ont ensuite été utilisés pour déterminer si un sujet test a une forte probabilité de répondre, ou au contraire de ne pas répondre, au traitement anti-VHC. Les valeurs de dosage des niveaux d'expression des gènes de ces sujets tests ont été entrées dans un modèle à réseau de neurones, lequel génère des données sorties comme ci-dessus présentées, et classe le sujet testé dans la classe « sous-population R » ou « sous-population NR ».

### 3. Exemples de modèles de classification obtenus :

Les inventeurs ont ainsi identifié des gènes dont les niveaux d'expression constituent des biomarqueurs qui, lorsqu'ils sont pris en combinaison, sont pertinents pour déterminer le statut « répondeur » (R) ou « non répondeur » (NR) d'un sujet.

Ces gènes sont les dix-sept gènes suivants : MBL2, LGALS3BP, IL8, G1P2, CXCL10, CCL21, AFP, CRP, CXCL11, CXCL6, CXCL9, FGF7, MDK, MMP2, SFN, TGFB2, VEGFD.

De manière particulièrement avantageuse, il peut être observé que ces dix-sept gènes sont tous des gènes codant des protéines non-membranaires, c'est-à-dire des gènes qui codent pour une protéine dont la localisation est intracellulaire et/ou extracellulaire, et qui est donc susceptible d'être détectée dans un fluide biologique du sujet, tel que le sang, le sérum ou le plasma.

Les inventeurs ont en outre identifié que les combinaisons les plus pertinentes comprennent :

- au moins un gène parmi MBL2, LGALS3BP, IL8 ; et
- au moins un gène parmi G1P2, CCL21, CXCL10 ; et
- optionnellement, au moins un gène parmi AFP, CRP, CXCL11, CXCL6, CXCL9, FGF7, MDK, MMP2, SFN, TGFB2, VEGFD.

A titre illustratif, des exemples de combinaisons de biomarqueurs appropriées comprennent notamment les combinaisons de deux, trois ou cinq biomarqueurs (combinaisons des niveaux d'expression de deux, trois ou cinq gènes) présentées dans les tableaux 2, 7 et 12 ci-dessus, en partie descriptive.

Des exemples de modèles de classification qui peuvent être mis en oeuvre avec ces combinaisons de biomarqueurs sont présentés dans :

- les tableaux 3 et 4 à 6 ci-dessus (combinaison des niveaux de traduction de deux gènes choisis conformément à l'invention),
- les tableaux 8 et 9 à 11 ci-dessus (combinaison des niveaux de traduction de trois gènes choisis conformément à l'invention),
- les tableaux 13 et 14 à 16 ci-dessus (combinaison des niveaux de transcrits ARN de gènes choisis conformément à l'invention),
- les tableaux 17 et 18 à 20 ci-dessus (combinaison des niveaux de traduction de gènes choisis conformément à l'invention),
- les tableaux 21 et 22 à 24 ci-dessus (combinaison des niveaux de transcrits ARN de gènes choisis conformément à l'invention, combinée en outre à d'autres facteurs),
- les tableaux 25 et 26 à 28 ci-dessus (combinaison des niveaux de traduction de gènes choisis conformément à l'invention, combinée en outre à d'autres facteurs) ;

*cf.* également les exemples ci-dessous.

Les combinaisons prédictives de l'invention sont des combinaisons des niveaux d'expression de gènes, choisies comme ci-dessus indiqué.

Il peut néanmoins être choisi de faire intervenir dans ces combinaisons un ou plusieurs facteurs autres que les niveaux d'expression de ces gènes, pour combiner cet ou ces autres facteurs et les niveaux d'expression des gènes choisis en une règle décisionnelle.

Cet ou ces autres facteurs sont préférablement choisis de sorte à construire un modèle de classification dont le pouvoir prédictif est encore amélioré par rapport au modèle qui ne comprendrait pas cet ou ces autres facteurs.

Cet ou ces autres facteurs peuvent par exemple être des facteurs cliniques, biologiques, virologiques, par exemple :

- un ou plusieurs facteurs cliniques, tels que sexe (féminin F ou masculin M), âge à la date du prélèvement (par exemple, âge à la date de la PBH, âge à la date de la cytoponction hépatique, âge à la date du prélèvement de sang, de sérum, de plasma ou d'urine), âge à la date de la contamination, âge à la date de début du traitement, indice de masse corporelle (IMC), indice de sensibilité à l'insuline (HOMA), diabète, consommation d'alcool, degré de stéatose, mode de contamination, activité Metavir, score de fibrose hépatique mesuré selon le système Metavir (score F Metavir) ou selon le système Ishak, et/ou

- un ou plusieurs facteurs biologiques autres que les niveaux d'expression desdits gènes choisis, tels que concentration en haptoglobine (Hapto), concentration en apolipoprotéine A1 (ApoA1), teneur en bilirubine totale (BLT), concentration en gamma glutamyl transpeptidase (GGT), concentration en aspartate aminotransférase (AST), concentration en alanine aminotransférase (ALT), teneur en plaquettes (PLQ), taux de prothrombine (TP), teneur en cholestérol HDL (Chol-HDL), teneur en cholestérol total, concentration en ferritine (Ferritine), teneur glycémique (glycémie), concentration en peptide C, teneur en insuline (insulinémie), concentration en triglycérides (TG), teneur en albumine, coefficient de saturation en fer de la transferrine (CS), concentration en phosphatase alcaline (PAL) ; et/ou

- un ou plusieurs facteurs virologiques, tels que génotype viral, durée de l'infection, charge virale avant traitement (CVavantTTT), charge virale mesurée chez le patient à la date du début du traitement (charge virale à J0), charge virale mesurée chez le patient à la date du prélèvement (charge virale à PBH, charge virale à la date de la cytoponction hépatique, charge virale à la date du prélèvement de sang, de sérum, de plasma ou d'urine).

**EXEMPLE 2 : ARN de ponction biopsique hépatique (PBH) / applications à des patients tests**

**2a) exemple d'application de la combinaison des niveaux d'expression (ARN) des gènes MBL2, G1P2, LGALS3BP, TGFB2, CRP (combinaison n°1 dans le tableau 12 ci-dessus) :**

Par la méthode WKNN (*cf.* exemple 1 ci-dessus), l'erreur LOOCV associée à la combinaison des niveaux de transcription (ARN) des gènes MBL2, G1P2, LGALS3BP, TGFB2, CRP (combinaison n°1 dans le tableau 12 ci-dessus) est de 12 (*cf.* tableau 13 ci-dessus).

Les meilleures performances de cette combinaison selon la méthode WKNN (calculée sur la population des répondeurs (R) et non-répondeurs (NR) de l'exemple 1 (n = 107 patients ; *cf.* tableau 33 ci-dessus)) sont les suivantes : sensibilité (Se) = 82% ; spécificité (Sp) = 92% (*cf.* tableau 13 ci-dessus).

Les paramètres de réglage utilisés pour la méthode WKNN ont été les suivants :

Kernel (K) : bipondérée (« biweight »)
Distance (D) : minkowski de paramètre 2
Nombre de voisins (k) : 3

Selon ce modèle, 71% des répondeurs-rechuteurs (RR) sont classés comme des répondeurs (R) et 29% comme des non-répondeurs (NR).

Un exemple de prédiction sur 20 sujets (patients humains) est donné dans le tableau 37 ci-dessous, qui présente les valeurs de dosage des niveaux d'expression des gènes choisis (valeurs BMQ obtenues par la méthode du $2^{-\Delta Ct}$ ; *cf.* exemple 1 ci-dessus).

Un ou plusieurs facteurs cliniques, biologiques et virologiques peuvent être combinés aux cinq biomarqueurs indiqués ci-dessus (niveaux d'expression de cinq gènes), et conduire à une règle décisionnelle dont le pouvoir prédictif est encore supérieur à celui de la règle ci-dessus présentée.

Les tableaux 38 à 40 suivants présentent des exemples de tels facteurs cliniques, biologiques et virologiques, ainsi que leurs valeurs pour les sujets tests du tableau 37.

ND = non déterminé

**Tableau 37 : exemple d'application d'un modèle de classification basé sur la combinaison des niveaux d'expression des gènes MBL2, G1P2, LGALS3BP, TGFB2, CRP (combinaison n°1 du tableau 12 ci-dessus)**

| n° du sujet test | modèle WKNN (kernel = bipondérée ; distance = minkowski de paramètre 2 ; k = 3) | | | | | | Statut R ou NR, tel que déterminé après traitement |
|---|---|---|---|---|---|---|---|
| | MBL2 | G1P2 | LGALS3BP | TGFB2 | CRP | Prédiction WKNN | |
| 59 | 0,99 | 0,88 | 0,04 | 1,74 | 1,39 | R | R |
| 65 | 2,30 | 1,61 | 0,05 | 0,23 | 0,87 | R | R |
| 75 | 5,48 | 8,66 | 0,27 | 1,40 | 0,11 | NR | NR |
| 83 | 0,01 | 6,17 | 0,16 | 1,17 | 0,64 | NR | NR |
| 90 | 2,02 | 1,91 | 0,05 | 0,52 | 0,63 | R | R |
| 91 | 0,89 | 0,66 | 0,02 | 2,18 | 0,60 | R | R |
| 92 | 0,62 | 0,05 | 0,01 | 0,28 | 1,75 | R | R |
| 125 | 1,42 | 1,26 | 0,01 | 0,64 | 0,20 | R | R |
| 167 | 0,53 | 5,05 | 0,10 | 3,22 | 3,81 | NR | NR |
| 308 | 1,19 | 6,66 | 0,25 | 1,57 | 1,64 | NR | NR |
| 346 | 4,00 | 0,60 | 0,13 | 1,89 | 337,77 | R | R |
| 366 | 2,90 | 0,16 | 0,01 | 1,00 | 2,92 | R | R |
| 501 | 1,22 | 1,42 | 0,03 | 0,14 | 3,86 | R | R |
| 503 | 1,32 | 0,76 | 0,02 | 0,30 | 2,85 | R | R |
| 509 | 0,02 | 6,13 | 0,04 | 1,24 | 0,26 | NR | NR |
| 521 | 0,00 | 4,36 | 0,58 | 12,17 | 0,34 | NR | NR |
| 526 | 3,14 | 5,26 | 0,13 | 1,28 | 2,13 | NR | NR |
| 527 | 1,22 | 9,32 | 0,46 | 1,62 | 1,31 | NR | NR |
| 573 | 4,50 | 6,89 | 0,13 | 1,31 | 0,35 | NR | NR |
| 574 | 4,23 | 4,96 | 0,24 | 3,82 | 1,99 | NR | NR |

**Tableau 38 (données cliniques) :**

| n° sujet | sexe | âge à PBH | IMC (kg/m2) | indice de sensibilité à l'insuline (HOMA) | diabète | consommation d'alcool (g/jour) | degré de stéatose | mode de contamination | activité Metavir | Score Fibrose Ishak | Score Fibrose Metavir | Age au début du traitement (années) |
|---|---|---|---|---|---|---|---|---|---|---|---|---|
| 59 | F | 50,9 | 23,7 | 1,5 | Non | 0 | 0 | Transfusion | 1 | 2 | 1 | 51,3 |
| 65 | M | 35,6 | 29,6 | 3,9 | Non | 0 | 0 | Zone endémique | 1 | 2 | 1 | 35,7 |
| 75 | F | 53,0 | 27,9 | ND | Non | 0 | 0 | ND | 2 | 2 | 2 | 53,3 |
| 83 | F | 51,4 | 25,7 | ND | Non | ND | 2 | ND | 1 | 2 | 1 | 52,0 |
| 90 | F | 58,7 | 25,2 | ND | Non | 0 | 2 | Transfusion | 1 | 3 | 2 | 59,0 |
| 91 | F | 47,8 | 21,4 | ND | Non | 0 | 0 | ND | 1 | 2 | 1 | 48,0 |
| 92 | M | 35,1 | 28,7 | ND | Non | 0 | 0 | Toxicomanie | 1 | 3 | 2 | 35,8 |
| 125 | M | 27,4 | 22,6 | 1,4 | Non | 0 | 0 | ND | 1 | 1 | 1 | 27,6 |
| 167 | F | 48,6 | 37,8 | 2,3 | Non | ND | 0 | Transfusion | 2 | 2 | 1 | 48,8 |
| 308 | M | 34,9 | 27,7 | ND | Non | 0 | 0 | Zone endémique | 1 | 1 | 1 | 35,4 |
| 346 | F | 50,4 | 17,8 | ND | Non | 30 | 0 | ND | 1 | 3 | 2 | 50,6 |
| 366 | M | 42,8 | 28,6 | ND | Non | 10 | 1 | Toxicomanie | 1 | 3 | 2 | 43,6 |
| 501 | M | 47,7 | 33,1 | 4,8 | Non | 0 | 2 | Zone endémique | 1 | 4 | 2 | 47,8 |
| 503 | F | 55,0 | 21,6 | 1,0 | Non | 0 | 0 | Transfusion | 0 | 4 | 2 | 55,1 |
| 509 | F | 48,2 | 20,8 | 1,4 | Non | ND | 0 | Transfusion | 1 | 4 | 3 | 48,4 |
| 521 | M | 58,4 | 23,8 | ND | Non | 30 | 0 | Transfusion | 1 | 4 | 3 | 58,9 |
| 526 | F | 73,0 | 24,8 | ND | Non | 0 | 1 | Transfusion | 2 | 6 | 4 | 73,2 |
| 527 | M | 47,4 | 37,3 | ND | Non | 20 | 1 | Toxicomanie | 1 | 5 | 3 | 48,2 |
| 573 | M | 46,6 | 25,5 | ND | Non | 0 | 2 | Toxicomanie | 2 | 2 | 1 | 47,7 |
| 574 | M | 58,3 | 23,6 | ND | Non | 0 | 1 | Nosocomiale | 1 | 3 | 2 | 60,0 |

**Tableau 39 (données virologiques) :**

| n° patient | génotype Viral | durée de l'infection (années) | charge virale à PBH (copies/mL .$10^3$) | charge virale au début du traitement (copies/mL .$10^3$) |
|---|---|---|---|---|
| 59 | 5 | 26,8 | 524 | 1120 |
| 65 | 4 | ND | 1135 | 450 |
| 75 | 1 | ND | 3276 | 2347 |
| 83 | 1 | ND | 1579 | 3928 |
| 90 | 1 | ND | 515 | 515 |
| 91 | 1 | ND | 3902 | 3902 |
| 92 | 1 | 16,1 | 3,2 | 3,2 |
| 125 | 4 | ND | 695 | 695 |
| 167 | 4 | ND | 12616 | 12616 |
| 308 | 4 | ND | 423 | 423 |
| 346 | 3 | ND | 6 | 6 |
| 366 | 4 | 25,0 | 4700 | 7573 |
| 501 | 4 | ND | 750 | 750 |
| 503 | 1 | 24,0 | 566 | 843 |
| 509 | 1 | 49,1 | 8779 | 8779 |
| 521 | 1 | 34,8 | 14654 | 14432 |
| 526 | 1 | 38,4 | 778 | 778 |
| 527 | 1 | 20,4 | ND | 3457 |
| 573 | 1 | 25,9 | ND | 8419 |
| 574 | 1 | 47,0 | ND | 13034 |

**Tableau 40 (données biologiques) :**

| n° sujet | A2M (g/L) | Hapto (g/L) | Apo A1 (g/L) | BLT (µmol/L) | GGT (U/L) | AST (U/L) | ALT (U/L) | PLQ (x 10³/mm³) | TP (%) | Chol-HDL (mmol/L) |
|---|---|---|---|---|---|---|---|---|---|---|
| 59 | 2,3 | 0,97 | 2,04 | 20 | 18 | 49 | 80 | 239 | 100 | 2,13 |
| 65 | 4,07 | 1,15 | 1,17 | 8 | 46 | 61 | 152 | 210 | 92 | 0,72 |
| 75 | ND | ND | ND | 12 | 135 | 64 | 50 | 233 | 100 | 2,15 |
| 83 | ND | ND | ND | 13 | 82 | 78 | 95 | 232 | 101 | ND |
| 90 | ND | ND | ND | 11 | 21 | 61 | 119 | 359 | 100 | ND |
| 91 | ND | ND | ND | 9 | 39 | 64 | 79 | 353 | 99 | ND |
| 92 | ND | ND | ND | 12 | 92 | 29 | 128 | 355 | 106 | 0,9 |
| 125 | 1,15 | 0,64 | 1,45 | 16 | 47 | 23 | 47 | 229 | 99 | 1,16 |
| 167 | 2,9 | 0,54 | 1,64 | 15 | 378 | 163 | 144 | 183 | 78 | 1,28 |
| 308 | ND | ND | ND | 16 | 246 | 30 | 36 | 214 | 100 | ND |
| 346 | ND | ND | ND | 12 | 23 | 27 | 41 | 217 | 95 | 1,32 |
| 366 | 1,31 | 0,37 | 1,06 | 15 | 29 | 40 | 105 | 226 | 98 | ND |
| 501 | 3,29 | 1,02 | 1,18 | 13 | 49 | 82 | 185 | 195 | 100 | 0,98 |
| 503 | 4,05 | 0,48 | 1,62 | 9 | 16 | 81 | 100 | 200 | 81 | 1,38 |
| 509 | 4,24 | 0,39 | 1,89 | 13 | 43 | 154 | 243 | 121 | 86 | 1,88 |
| 521 | ND | ND | ND | 11 | 127 | 83 | 166 | 189 | 100 | 1,62 |
| 526 | ND | ND | ND | 17 | 172 | 128 | 210 | 187 | 77 | ND |
| 527 | ND | ND | ND | 16 | 127 | 35 | 80 | 182 | 100 | ND |
| 573 | ND | ND | ND | 21 | 249 | 61 | 88 | 157 | 100 | ND |
| 574 | ND | ND | ND | 13 | 67 | 62 | 87 | 215 | 100 | ND |

**Suite et fin du Tableau 40 (données biologiques) :**

| n° sujet | ferritine (µg/L) | glycémie (mmol/L) | peptide C (ng/mL) | insuline (µUI/mL) | TG (mmol/L) | albumine (g/L) | CS (%) | cholestérol total (mmol/L) | PAL (U/L) |
|---|---|---|---|---|---|---|---|---|---|
| 59 | 30 | 5 | 2,18 | 6,65 | 0,71 | 46 | 27 | 5,46 | 77 |
| 65 | 147 | 5,2 | 3,31 | 17,07 | 1,09 | 46 | 43 | 3,82 | 49 |
| 75 | 271 | 5,4 | ND | ND | 0,69 | 44 | 40 | 4,72 | 149 |
| 83 | 71 | 5,6 | ND | ND | 0,76 | 44 | 37 | 3,68 | 97 |
| 90 | 137 | 4,2 | ND | ND | 0,88 | 48 | 42 | 5,4 | 52 |
| 91 | 206 | 4,7 | ND | ND | 1 | 43 | 36 | 4,5 | 72 |
| 92 | 133 | 5,4 | ND | ND | 0,81 | 48 | 22 | 4,26 | 68 |
| 125 | 97 | 5,2 | 1,67 | 5,91 | 0,57 | 48 | 33 | 4,14 | 43 |
| 167 | 702 | 5,22 | 2,3 | 9,82 | 0,62 | 43 | 70 | 4,58 | 114 |
| 308 | 455 | 5,1 | ND | ND | 1,13 | 48 | 46 | 4,81 | 73 |
| 346 | ND | 5,28 | ND | ND | 0,51 | 39 | 4 | 5,47 | 43 |
| 366 | 166 | 5,4 | ND | ND | 1,21 | 47 | 36 | 5,81 | 54 |
| 501 | 583 | 5,5 | 3,17 | 19,7 | 0,75 | 48 | 26 | 3,84 | 60 |
| 503 | 140 | 4,8 | 1,39 | 4,55 | 0,78 | ND | 46 | 5,43 | 53 |
| 509 | 179 | 4,3 | 2,8 | 7,5 | 0,56 | 48 | 32 | 4,02 | 56 |
| 521 | 514 | 6,2 | ND | ND | 1,76 | 47 | 32 | 4,62 | 62 |
| 526 | 320 | 4,4 | ND | ND | 0,7 | 41 | 35 | 4,33 | 108 |
| 527 | 148 | 5,7 | ND | ND | 0,68 | 48 | 16 | 3,67 | 67 |
| 573 | 296 | 5,5 | ND | ND | 0,96 | 47 | 43 | 4,97 | 40 |
| 574 | 337 | ND | ND | ND | | 47 | 27 | ND | 67 |

**2b) exemple d'application de la combinaison des niveaux d'expression (ARN) des gènes MDK, LGALS3BP, CXCL10, IL8, CCL21 (combinaison n°24 dans le tableau 12 ci-dessus) :**

L'AUC relative à la combinaison des niveaux d'expression des gènes MDK, LGALS3BP, CXCL10, IL8, CCL21 (combinaison n°24 dans le tableau 12 ci-dessus) calculée sur la population des répondeurs (R) et non-répondeurs (NR) de l'exemple 1 (n = 107 patients ; *cf.* tableau 33 ci-dessus) est de 0,771 (*cf.* tableau 16 ci-dessus).

Par la méthode mROC (*cf.* exemple 1), le seuil maximisant l'indice de Youden ($\delta$) pour cette combinaison est de -2,309 (*cf.* tableau 14 ci-dessus).

Pour le choix de ce seuil, les performances de la combinaison sont les suivantes : Sensibilité (Se) = 73%; spécificité (Spe) = 74% (*cf.* tableau 13 ci-dessus).

Un exemple de règle décisionnelle est la règle suivante :

$$Z = 0,359 \text{ x } CCL21^t + 0,028 \text{ x } CXCL10^t + 0,055 \text{ x } IL8 + 0,107 \text{ x } LGALS3BP^t + 0,22 \text{ x } MDK^t$$

(fonction Z24ARN ; *cf.* tableau 14 ci-dessus), où :

- MDK, LGALS3BP, CXCL10, IL8, CCL21 sont les valeurs de dosage des niveaux d'expression des gènes indiqués (valeurs obtenues par la méthode du $2^{-\Delta Ct}$ ; *cf.* exemple 1 ci-dessus), et où
- l'exposant t (ici porté par CCL21, CXCL10, LGALS3BP et MDK) indique que la valeur à appliquer dans la règle décisionnelle est la transformée Box-Cox (Box et Cox, 1964) de la valeur de dosage (BMQ) du niveau d'expression du gène considéré, afin de la normaliser selon la formule suivante :

$$BMQ^t = (BMQ^\lambda - 1)/\lambda.$$

Si $Z \geq -2,309$ : le test diagnostic est positif (prédiction mROC = 1), le sujet est déclaré 'NR' (sujet pronostiqué non répondeur au traitement).

Si $Z < -2,309$ : le test est négatif (prédiction mROC = 0), le sujet est déclaré 'R' (sujet pronostiqué répondeur au traitement).

Selon ce modèle, 62% des répondeurs-rechuteurs (RR) sont classés parmi les répondeurs (R) et 38% sont classés parmi les non-répondeurs (NR).

Un exemple de prédiction sur 20 sujets (patients humains) est donné dans le tableau 41 ci-dessous, qui présente les valeurs de dosage des niveaux d'expression des gènes choisis (valeurs BMQ obtenues par la méthode du $2^{-\Delta Ct}$ ; *cf.* exemple 1 ci-dessus).

Un ou plusieurs facteurs cliniques, biologiques et virologiques peuvent être combinés aux cinq marqueurs indiqués ci-dessus (niveaux d'expression de cinq gènes), et conduire à une règle décisionnelle dont le pouvoir prédictif est encore supérieur à celui de la règle ci-dessus présentée.

Les tableaux 38 à 40 ci-dessus présentent des exemples de tels facteurs cliniques, biologiques et virologiques, ainsi que leurs valeurs pour les patients tests du tableau 41.

**Tableau 41 : exemple d'application d'un modèle de classification basé sur la combinaison des niveaux d'expression des gènes MDK, LGALS3BP, CXCL10, IL8, CCL21 (combinaison n°24 du tableau 12 ci-dessus)**

| n° du sujet test | modèle mROC (fonction Z24ARN ; $\delta$ = -2,309) | | | | | | | Statut R ou NR, tel que déterminé après traitement |
|---|---|---|---|---|---|---|---|---|
| | MDK | LGALS3BP | CXCL10 | IL8 | CCL21 | Z | Prédiction mROC | |
| 59 | 0,090 | 0,045 | 0,010 | 0,329 | 0,006 | -2,625 | R | R |
| 65 | 0,105 | 0,048 | 0,060 | 0,168 | 0,012 | -2,370 | R | R |
| 75 | 1,248 | 0,265 | 0,550 | 0,908 | 0,033 | -1,246 | NR | NR |
| 83 | 0,476 | 0,164 | 0,180 | 0,231 | 0,073 | -1,303 | NR | NR |
| 90 | 0,115 | 0,048 | 0,080 | 0,089 | 0,012 | -2,359 | R | R |
| 91 | 0,051 | 0,023 | 0,110 | 0,040 | 0,008 | -2,717 | R | R |
| 92 | 0,001 | 0,008 | 0,030 | 0,087 | 0,005 | -3,496 | R | R |
| 125 | 0,058 | 0,012 | 0,020 | 0,000 | 0,005 | -2,962 | R | R |
| 167 | 0,521 | 0,099 | 0,080 | 0,923 | 0,018 | -1,801 | NR | NR |
| 308 | 0,446 | 0,251 | 0,070 | 0,853 | 0,022 | -1,650 | NR | NR |

(suite)

| n° du sujet test | modèle mROC (fonction Z24ARN ; $\delta$ = -2,309) | | | | | | | Statut R ou NR, tel que déterminé après traitement |
|---|---|---|---|---|---|---|---|---|
| | MDK | LGALS3BP | CXCL10 | IL8 | CCL21 | Z | Prédiction mROC | |
| 346 | 0,032 | 0,133 | 0,020 | 0,182 | 0,004 | -2,800 | R | R |
| 366 | 0,012 | 0,015 | 0,050 | 0,179 | 0,009 | -2,927 | R | R |
| 501 | 0,046 | 0,032 | 0,130 | 0,601 | 0,016 | -2,411 | R | R |
| 503 | 0,014 | 0,024 | 0,010 | 0,109 | 0,004 | -3,144 | R | R |
| 509 | 0,172 | 0,045 | 0,220 | 1,157 | 0,025 | -1,964 | NR | NR |
| 521 | 1,676 | 0,576 | 0,490 | 3,732 | 0,052 | -0,784 | NR | NR |
| 526 | 0,189 | 0,130 | 0,170 | 2,370 | 0,028 | -1,716 | NR | NR |
| 527 | 0,276 | 0,457 | 0,070 | 0,657 | 0,045 | -1,453 | NR | NR |
| 573 | 0,047 | 0,134 | 0,400 | 0,323 | 0,028 | -2,037 | NR | NR |
| 574 | 0,191 | 0,241 | 1,040 | 5,756 | 0,031 | -1,378 | NR | NR |

**2c) combinaison des niveaux d'expression (ARN) des gènes MDK, LGALS3BP, CXCL10, IL8, CCL21** (combinaison n°24 dans le tableau n°12 ci-dessus), **combinée en outre à des facteurs cliniques et/ou à d'autres facteurs biologiques et/ou à des facteurs virologiques** :

Un ou plusieurs facteurs cliniques et/ou un ou plusieurs facteurs biologiques et/ou un ou plusieurs facteurs virologiques peuvent être combinés aux niveaux d'expression de gènes sélectionnés conformément à l'invention (en l'occurrence, niveaux de transcription ARN mesurés dans un échantillon de PBH), et ainsi conduire à une règle décisionnelle dont le pouvoir prédictif est encore supérieur à celui de la seule combinaison desdits niveaux d'expression.

Par exemple, la combinaison :

- des niveaux d'expression (ARN) des gènes MDK, LGALS3BP, CXCL10, IL8, CCL21 (combinaison n°24 dans le tableau 12 ci-dessus ; *cf.* exemple 2b ci-dessus), et
- de la valeur d'un autre facteur biologique, à savoir la concentration en phosphatase alcaline (PAL), et
- de la valeur d'un facteur virologique, à savoir la charge virale avant le début du traitement (CVavantTTT),

conduit à une règle décisionnelle dont l'aire sous la courbe ROC (AUC), calculée sur l'ensemble des patients de la population d'étude de l'exemple 1 pour lesquels les données de PAL et de CVavantTTT étaient disponibles (n = 97 patients), est de 0,827 (*cf.* tableau 24 ci-dessus), alors qu'elle est de 0,771 (*cf.* tableau 16 ci-dessus), lorsque la combinaison des niveaux d'expression des gènes MDK, LGALS3BP, CXCL10, IL8, CCL21 est utilisée seule, sans être combinée à ce facteur biologique et à ce facteur virologique.

Par la méthode mROC (*cf.* exemple 1), le seuil maximisant l'indice de Youden pour cette combinaison est de 5,454 (*cf.* tableau 22 ci-dessus).

Pour le choix de ce seuil, les performances de la combinaison sont les suivantes :

Sensibilité (Se) = 81% ; spécificité (Spe) = 71% (*cf.* tableau 21 ci-dessus).

Un exemple de règle décisionnelle est la règle suivante :

$$Z = -0,051 \times CXCL10^t + 0,032 \times IL8 + 0,357 \times CCL21^t + 0,189 \times MDK^t + 0,182 \times$$

$$LGALS3BP^t + 0,052 \times CVavantTTT^t + 2,644 \times PAL^t$$

(fonction Z24ARNsupp ; *cf.* tableau 22 ci-dessus), où :

- MDK, LGALS3BP, CXCL10, IL8, CCL21 sont les valeurs de dosage des niveaux d'expression des gènes indiqués (valeurs obtenues par la méthode du $2^{-\Delta Ct}$ ; *cf.* exemple 1 ci-dessus),
- CVavantTTT et PAL sont les valeurs du facteur virologique et du facteur biologique ci-dessus indiqués (charge virale CVavantTTT en copies/mL.$10^3$, et concentration PAL en UI/mL), et où

- l'exposant t (ici porté par CXCL10, CCL21, LGALS3BP, MDK, CVavantTTT et PAL) indique que la valeur à appliquer dans la règle décisionnelle (fonction Z24ARNsupp) est la transformée Box-Cox (Box et Cox, 1964) de la valeur de dosage du biomarqueur BMQ considéré, afin de la normaliser selon la formule suivante :

$$BMQ_t = (BMQ^\lambda - 1)/\lambda.$$

Dans l'exemple de règle décisionnelle ci-dessus indiquée, les paramètres $\lambda$ sont 0,04 pour CXCL10 ; 0,02 pour CCL21 ; -0,07 pour LGALS3BP ; 0,15 pour MDK ; 0,2 pour CVavantTTT ; et -0,26 pour PAL (*cf.* tableau 13 ci-dessus).

Si $Z \geq 5,454$ : le test diagnostic est positif (prédiction mROC = 1), le sujet est déclaré 'NR'. Si $Z < 5,454$ : le test est négatif (prédiction mROC = 0), le sujet est déclaré 'R'.

Selon ce modèle, 56% des répondeurs-rechuteurs (RR) sont classés parmi les répondeurs (R) et 44% sont classés parmi les non-répondeurs (NR).

**EXEMPLE 3 : Protéines sériques (combinaison des niveaux d'expression de 5 gènes)**

Les niveaux d'expression des protéines CXCL10, LGALS3BP, IL8, CCL21 et MDK (combinaison n°24 dans le tableau 12 ci-dessus) ont été mesurés dans le sérum de 167 patients. Les dosages protéiques ont été réalisés comme décrit en exemple 1 et tableau 44 ci-dessus.

Ce groupe de 167 patients a été constitué comme suit :

- 67 patients, pour lesquels un prélèvement sérique était disponible, ont été sélectionnés selon les mêmes critères d'inclusion et d'exclusion que les 140 patients décrits dans le tableau 30 ci-dessus, et ont été ajoutés à ce groupe de 140 patients ;
- parmi les 207 patients ainsi regroupés, ont été sélectionnés ceux des patients répondeurs au traitement (statut R) ou non-répondeurs au traitement (statut NR) pour lesquels toutes les données cliniques, biologiques et virologiques étaient disponibles,

ce qui a conduit à un groupe de 167 patients (*cf.* tableau 45 ci-dessus).

Parmi ces 167 patients :

- 90 d'entre eux se sont avérés être répondeurs au traitement (statut R) ;
- 77 d'entre eux se sont avérés être non répondeurs au traitement (statut NR).

**Tableau 45 : données cliniques, biologiques et virologiques**

| Données cliniques, biologiques et virologiques | Patients | Patients NR | Patients R |
|---|---|---|---|
| **n** | 167 | 77 | 90 |
| **Sexe :** mâle (%) / femelle (%) | 107 (64) / 60 (36) | 51 (66) / 26 (34) | 56 (62) / 34 (38) |
| **Age** [moyenne $\pm$ déviation standard (gamme)] | 48,1 $\pm$ 9,5 (27 - 73) | 48,7 $\pm$ 8,9 (32 - 73) | 47,5 $\pm$ 10 (27 - 71) |
| **Source d'infection** [n(%)] | | | |
| transfusion sanguine | 27 (16,2) | 9 (11,7) | 18 (20) |
| administration intraveineuse d'une drogue | 58 (34,7) | 30 (38,4) | 28 (31,1) |
| inconnue | 82 (49,1) | 38 (49,9) | 44 (48,9) |
| **Alanine aminotransférase** (ALT) UI/L [moyenne $\pm$ déviation standard (gamme)] | 124 $\pm$ 92 (20 - 520) | 120 $\pm$ 78 (20 -397) | 127 $\pm$ 103 (20 - 520) |
| **Génotypes VHC** [n(%)] | | | |
| 1 | 98 (58,7) | 63 (81,8) | 35 (38,9) |
| 2 | 13 (7,8) | 0(0) | 13(14,4) |
| 3 | 21 (12,6) | 2 (2,6) | 19(21,1) |

(suite)

| Données cliniques, biologiques et virologiques | | Patients | Patients NR | Patients R |
|---|---|---|---|---|
| | 4 | 34 (20,3) | 12 (15,6) | 22(24,4) |
| | 5 | 1 (0,6) | 0(0) | 1(1,1) |
| **Score de fibrose** (score F Metavir) [n(%)] | | | | |
| | 0 | 2 (1,2) | 1 (1,29) | 1 (1,1) |
| | 1 | 50 (29,94) | 14 (18,18) | 36 (40) |
| | 2 | 58 (34,73) | 25 (32,47) | 33 (36,7) |
| | 3 | 26 (15,57) | 15 (19,48) | 11 (12,2) |
| | 4 | 29 (17,37) | 20 (25,97) | 9 (10) |
| | inconnu | 2(1,2) | 2 (2,6) | 0(0) |

La distribution des concentrations sériques des protéines CXCL10, LGALS3BP, IL8, CCL21 et MDK selon le statut R ou NR du patient est présentée en Figures 1A et 1B.

**3a) exemple de modèle de classification multivariée à partir de la combinaison des niveaux d'expression sérique des protéines CXCL10, LGALS3BP, IL8, CCL21 et MDK (combinaison n°24 dans le tableau 12 ci-dessus) :**

L'AUC relative à la combinaison des niveaux d'expression des gènes CXCL10, LGALS3BP, IL8, CCL21 et MDK (combinaison n°24 dans le tableau 12 ci-dessus) calculée sur la population d'étude complète de l'exemple 3 (n = 167 patients) est de 0,838 (*cf.* tableau 20 ci-dessus).

Par la méthode mROC (*cf.* exemple 1), le seuil maximisant l'indice de Youden ($\delta$) pour cette combinaison est de 2,231 (*cf.* tableau 18 ci-dessus).

Pour le choix de ce seuil, les performances de la combinaison sont les suivantes :
Sensibilité (Se) = 82% ; spécificité (Spe) = 74% (*cf.* tableau 17 ci-dessus).

Un exemple de règle décisionnelle est la règle suivante :

$$Z = 0{,}025 * CXCL10^t + 0{,}071 * IL8^t + 0{,}465 * LGALS3BP^t - 0{,}001 * CCL21^t - 0{,}341 * MDK$$

(fonction Z24PROT ; *cf.* tableau 18 ci-dessus), où CXCL10, LGALS3BP, IL8, CCL21 et MDK sont les valeurs de dosage des biomarqueurs BMQ, c'est-à-dire les valeurs de dosage des niveaux d'expression des gènes indiqués (en l'occurrence, concentration sérique des protéines), et
où l'exposant t (ici porté par CXCL10, LGALS3BP, IL8 et CCL21) indique que la valeur à appliquer dans la règle décisionnelle est la transformée Box-Cox (Box et Cox, 1964) de la valeur de dosage du niveau d'expression du gène considéré, afin de la normaliser selon la formule suivante :

$$BMQ^t = (BMQ^\lambda - 1)/\lambda.$$

Dans l'exemple de règle décisionnelle ci-dessus indiquée, les paramètres $\lambda$ sont 0,41 pour CXCL10, de 0,33 pour LGALS3BP, de 0,23 pour IL8 et de -0,01 pour CCL21 (*cf.* tableau 19 ci-dessus).

Si $Z \geq 2{,}231$ : le test diagnostic est positif (prédiction mROC = 1), le sujet est déclaré 'NR' (sujet pronostiqué non répondeur au traitement),
Si $Z < 2{,}231$ : le test est négatif (prédiction mROC = 0), le sujet est déclaré 'R' (sujet pronostiqué répondeur au traitement).

Un exemple de prédiction sur 20 sujets (patients humains) est donné dans le tableau 42 ci-dessous, qui présente les valeurs de dosage (BMQ) des niveaux d'expression sérique des gènes choisis.

Un ou plusieurs facteurs cliniques et/ou un ou plusieurs facteurs biologiques et/ou un ou plusieurs facteurs virologiques peuvent être combinés aux niveaux d'expression sérique de protéines sélectionnées conformément à l'invention, et conduire à une règle décisionnelle dont le pouvoir prédictif peut être encore supérieur à celui de la règle ci-dessus présentée (*cf.* exemple 1 ; *cf.* exemple 3b ci-dessous).

**Tableau 42 : exemple d'application d'un modèle de classification basé sur la combinaison des niveaux d'expression sérique des gènes CXCL10, LGALS3BP, IL8, CCL21 et MDK (combinaison n°24 du tableau 12 ci-dessus)**

| n° du sujet test | Modèle mROC (fonction Z24PROT ; $\delta$ = 2,231) | | | | | | | Statut R ou NR, tel que déterminé après traitement |
|---|---|---|---|---|---|---|---|---|
| | MDK (ng/mL) | IL8 (pg/mL) | LGALS3BP (µg/mL) | CXCL10 (ng/mL) | CCL21 (pg/mL) | Score Z | Prédiction mROC | |
| 6 | 0,096 | 8,6 | 16,84 | 573,0 | 720,6 | 3,090 | NR | NR |
| 45 | 0,080 | 13,8 | 2,09 | 268,3 | 581,7 | 1,154 | R | R |
| 58 | 1,351 | 9,5 | 14,77 | 437,7 | 585,1 | 2,437 | NR | NR |
| 59 | 0,188 | 14,9 | 5,15 | 137,9 | 346,0 | 1,605 | R | R |
| 62 | 0,665 | 7,1 | 8,87 | 422,0 | 507,2 | 2,097 | R | R |
| 65 | 0,325 | 14,8 | 5,24 | 711,2 | 368,8 | 2,014 | R | R |
| 66 | 0,398 | 23,3 | 3,07 | 560,7 | 305,3 | 1,575 | R | R |
| 73 | 0,217 | 13,5 | 4,15 | 330,1 | 391,5 | 1,613 | R | R |
| 75 | 1,018 | 31,0 | 23,76 | 1036,3 | 372,8 | 3,608 | NR | NR |
| 80 | 0,137 | 10,3 | 11,73 | 1068,4 | 283,7 | 2,936 | NR | NR |
| 83 | 0,184 | 17,4 | 13,11 | 1036,3 | 384,1 | 3,093 | NR | NR |
| 86 | 1,110 | 8,4 | 8,99 | 174,8 | 373,2 | 1,756 | R | R |
| 90 | 0,399 | 1,9 | 6,6 | 393,1 | 285,0 | 1,771 | R | R |
| 91 | 0,956 | 20,6 | 3,04 | 399,2 | 443,0 | 1,252 | R | R |
| 92 | 0,145 | 7,5 | 1,88 | 168,9 | 387,3 | 0,893 | R | R |
| 145 | 0,313 | 9,8 | 11,61 | 203,4 | 578,8 | 2,333 | NR | NR |
| 167 | 0,245 | 13,7 | 10,73 | 708,4 | 498,2 | 2,677 | NR | NR |
| 308 | 0,489 | 14,2 | 10,63 | 304,7 | 306,4 | 2,327 | NR | NR |
| 509 | 0,145 | 41,8 | 15,45 | 911,4 | 665,8 | 3,368 | NR | NR |
| 512 | 0,392 | 25,1 | 6,82 | 911,4 | 548,1 | 2,381 | NR | NR |

**3b) combinaison des niveaux d'expression dans le sérum des gènes CXCL10, IL8, LGALS3BP, CCL21 et MDK (combinaison n°24 dans le tableau 12 ci-dessus), combinée en outre à un facteur clinique et à des facteurs biologiques** :

Un ou plusieurs facteurs cliniques et/ou un ou plusieurs facteurs biologiques (autres que le niveau d'expression de gènes choisi conformément à l'invention) et/ou un ou plusieurs facteurs virologiques peuvent être combinés aux niveaux d'expression sériques de gènes sélectionnés conformément à l'invention (protéines sériques), et ainsi conduire à une règle décisionnelle dont le pouvoir prédictif est encore supérieur à celui de la seule combinaison desdits niveaux d'expression sérique.

Par exemple, la combinaison :

- des niveaux sériques de traduction des gènes CXCL10, IL8, LGALS3BP, CCL21 et MDK (*cf.* exemple 3a ci-dessous ; combinaison n°24 dans le tableau 12 ci-dessus), et
- des valeurs des (autres) facteurs biologiques suivants :

  ◦ concentration en gamma glutamyl transpeptidase (GGT),
  o concentration en phosphatase alcaline (PAL) ; et

- de la valeur du facteur virologique suivant :

  ○ charge virale avant traitement (CVavantTTT),

conduit à une règle décisionnelle dont l'aire sous la courbe ROC (AUC), calculée sur la population d'étude complète de l'exemple 3 (n = 167 patients), est de 0,872 (*cf.* tableau 28 ci-dessus), alors qu'elle est de 0,838 (*cf.* tableau 20 ci-dessus), lorsque la combinaison des niveaux d'expression des gènes MDK, LGALS3BP, CXCL10, CCL21 et IL8 est utilisée seule, sans être combinée aux (autres) facteurs biologiques et/ou au facteur virologique ci-dessus indiqués.

Par la méthode mROC (*cf.* exemple 1), le seuil maximisant l'indice de Youden pour cette combinaison est de 4,516 (*cf.* tableau 26 ci-dessus).

Pour le choix de ce seuil, les performances de la combinaison sont les suivantes :
Sensibilité (Se) = 82% ; spécificité (Spe) = 77% (*cf.* tableau 25 ci-dessus).

Un exemple de règle décisionnelle est la règle suivante :

$$Z = -0{,}353 \times MDK + 0{,}059 \times IL8^t + 0{,}456 \times LGALS3BP^t + 0{,}010 \times CXCL10^t - 0{,}118 \times CCL21^t + 0{,}058 \times CVavantTTT^t + 0{,}227 \times GGG^t + 0{,}408 \times PAL^t$$

(fonction Z24PROTsupp ; *cf.* tableau 26 ci-dessus), où :

- MDK, LGALS3BP, CXCL10, CCL21 et IL8 sont les valeurs de dosage BMQ des biomarqueurs, c'est-à-dire les valeurs de dosage des niveaux d'expression des gènes indiqués (en l'occurrence, concentration sérique des protéines),
- CVavantTTT, GGT et PAL sont les valeurs du facteur virologique et des facteurs biologiques ci-dessus indiqués, et
- l'exposant t (ici porté par IL8, CXCL10, LGALS3BP, CCL21, CVavantTTT, GGT et PAL) indique que la valeur à appliquer dans la règle décisionnelle est la transformée Box-Cox (Box et Cox, 1964) de la valeur du biomarqueur considéré, afin de la normaliser selon la formule suivante :

$$BMQ^t = (BMQ^\lambda - 1)/\lambda.$$

Dans l'exemple de règle décisionnelle ci-dessus indiquée, les paramètres $\lambda$ sont de 0,23 pour IL8, de 0,41 pour CXCL10, de 0,33 pour LGALS3BP, de -0,01 pour CCL21, de 0,20 pour CVavantTTT, de -0,01 pour GGT et de -0,11 pour PAL (*cf.* tableau 27 ci-dessus).

Si $Z \geq 4{,}516$, le test diagnostic est positif (prédiction mROC = 1), le sujet est déclaré 'NR',
Si $Z < 4{,}516$, le test est négatif (prédiction mROC = 0), le sujet est déclaré 'R'.

Un exemple de prédiction sur 20 sujets (patients humains) est donné dans le tableau 43 ci-dessous, qui présente les valeurs de dosage (BMQ) des biomarqueurs choisis (niveaux d'expression sérique de cinq gènes choisis conformément à l'invention, et valeur du facteur virologique CVavantTTT, et valeurs des facteurs biologiques GGT et PAL). Il s'agit des mêmes 20 patients que ceux de l'exemple 3a ci-dessus.

**Tableau 43 : exemple d'application d'un modèle de classification basé sur la combinaison des niveaux d'expression sérique des gènes CXCL10, LGALS3BP, IL8, CCL21 et MDK (combinaison n°24 du tableau 12 ci-dessus), combinée en outre à d'autres facteurs**

| n° du sujet test | Modèle mROC (fonction Z24PROTsupp ; $\delta$ = 4,516) | | | | | | | | | | **Statut R ou NR,** tel que déterminé après traitement |
| --- | --- | --- | --- | --- | --- | --- | --- | --- | --- | --- | --- |
| | **MDK (ng/mL)** | **IL8 (pg/mL)** | **LGALS3BP (µg/ML )** | **CXCL10 (ng/mL)** | **CCL21 (pg/mL )** | **CVavantTTT** | **GGT** | **PAL** | **Score Z** | **Prédiction mROC** | |
| 6 | 0,096 | 8,6 | 16,84 | 573,0 | 720,6 | 10450 | 78 | 40 | 5,571 | NR | NR |
| 45 | 0,080 | 13,8 | 2,09 | 268,3 | 581,7 | 3645 | 34 | 63 | 3,404 | R | R |
| 58 | 1,351 | 9,5 | 14,77 | 437,7 | 585,1 | 5079 | 162 | 51 | 4,954 | NR | NR |
| 59 | 0,188 | 14,9 | 5,15 | 137,9 | 346,0 | 1120 | 18 | 77 | 3,581 | R | R |
| 62 | 0,665 | 7,1 | 8,87 | 422,0 | 507,2 | 185 | 46 | 52 | 3,618 | R | R |
| 65 | 0,325 | 14,8 | 5,24 | 711,2 | 368,8 | 450 | 46 | 49 | 3,608 | R | R |
| 66 | 0,398 | 23,3 | 3,07 | 560,7 | 305,3 | 8132 | 65 | 83 | 4,220 | R | R |
| 73 | 0,217 | 13,5 | 4,15 | 330,1 | 391,5 | 7611 | 31 | 61 | 4,074 | R | R |
| 75 | 1,018 | 31,0 | 23,76 | 1036,3 | 372,8 | 2347 | 135 | 149 | 5,952 | NR | NR |
| 80 | 0,137 | 10,3 | 11,73 | 1068,4 | 283,7 | 12932 | 132 | 100 | 5,811 | NR | NR |
| 83 | 0,184 | 17,4 | 13,11 | 1036,3 | 384,1 | 3928 | 82 | 97 | 5,410 | NR | NR |
| 86 | 1,110 | 8,4 | 8,99 | 174,8 | 373,2 | 57 | 287 | 77 | 3,759 | R | R |
| 90 | 0,399 | 1,9 | 6,6 | 393,1 | 285,0 | 515 | 21 | 52 | 3,414 | R | R |
| 91 | 0,956 | 20,6 | 3,04 | 399,2 | 443,0 | 3902 | 39 | 72 | 3,529 | R | R |
| 92 | 0,145 | 7,5 | 1,88 | 168,9 | 387,3 | 3,2 | 92 | 68 | 2,371 | R | R |
| 145 | 0,313 | 9,8 | 11,61 | 203,4 | 578,8 | 18304 | 133 | 48 | 5,396 | NR | NR |
| 167 | 0,245 | 13,7 | 10,73 | 708,4 | 498,2 | 12616 | 378 | 114 | 5,833 | NR | NR |
| 308 | 0,489 | 14,2 | 10,63 | 304,7 | 306,4 | 423 | 246 | 73 | 4,542 | NR | NR |
| 509 | 0,145 | 41,8 | 15,45 | 911,4 | 665,8 | 8779 | 43 | 56 | 5,615 | NR | NR |
| 512 | 0,392 | 25,1 | 6,82 | 911,4 | 548,1 | 12460 | 323 | 62 | 5,268 | NR | NR |

**EXEMPLE 4 : Protéines sériques (combinaison des niveaux d'expression de 3 gènes)**

L'AUC relative à la combinaison des niveaux d'expression des gènes MDK, LGALS3BP et CXCL10 (combinaison n°9 dans le tableau 7 ci-dessus) calculée sur la population d'étude complète de l'exemple 3 (n = 167 patients) est de 0,836 (*cf.* tableau 11 ci-dessus). Les dosages des concentrations protéiques ont été réalisés comme décrit en exemple 1 et tableau 44 ci-dessus. Par la méthode mROC, le seuil maximisant l'indice de Youden (δ) pour cette combinaison est de 2,164 (*cf.* tableau 9 ci-dessus). Pour le choix de ce seuil, les performances de la combinaison sont les suivantes :

Sensibilité (Se) = 82% ; spécificité (Spe) = 74% (*cf.* tableau 8 ci-dessus).

Un exemple de règle décisionnelle est la règle suivante :

$$Z = 0{,}029 \text{ x } \text{CXCL10}^t + 0{,}472 \text{ x } \text{LGALS3BP}^t - 0{,}319 \text{ x } \text{MDK}$$

(fonction Z9PROT ; *cf.* tableau 9 ci-dessus), où :

- CXCL10, LGALS3BP et MDK sont les valeurs de dosage des biomarqueurs BMQ, c'est-à-dire les valeurs de dosage des niveaux d'expression des gènes indiqués (en l'occurrence, concentration sérique des protéines),
- l'exposant t (ici porté par CXCL10 et LGALS3BP) indique que la valeur à appliquer dans la règle décisionnelle est la transformée Box-Cox (Box et Cox, 1964) de la valeur de dosage du niveau d'expression du gène considéré, afin de la normaliser selon la formule suivante :

$$\text{BMQ}^t = (\text{BMQ}^\lambda - 1)/\lambda.$$

Dans l'exemple de règle décisionnelle ci-dessus indiquée, les paramètres λ sont 0,41 pour CXCL10 et de 0,33 pour LGALS3BP.

Si Z ≥ 2,164 : le test diagnostic est positif (prédiction mROC = 1), le sujet est déclaré 'NR' (sujet pronostiqué non répondeur au traitement),

Si Z < 2,164 : le test est négatif (prédiction mROC = 0), le sujet est déclaré 'R' (sujet pronostiqué répondeur au traitement).

Un exemple de prédiction sur 20 sujets (patients humains) est donné dans le tableau 46 ci-dessous, qui présente les valeurs de dosage (BMQ) des niveaux d'expression sérique des gènes choisis. Il s'agit des mêmes 20 patients que ceux de l'exemple 2 ci-dessus.

Un ou plusieurs facteurs cliniques et/ou un ou plusieurs facteurs biologiques et/ou un ou plusieurs facteurs virologiques peuvent être combinés aux niveaux d'expression sérique de protéines sélectionnées conformément à l'invention, et conduire à une règle décisionnelle dont le pouvoir prédictif peut être encore supérieur à celui de la règle ci-dessus présentée (*cf.* exemple 1 ci-dessus).

**Tableau 46 : exemple d'application d'un modèle de classification basé sur la combinaison des niveaux d'expression sérique des gènes CXCL10, LGALS3BP et MDK (combinaison n°9 dans le tableau 7 ci-dessus)**

| n° du sujet test | modèle mROC δ = 2,164 | | | | | Statut R ou NR, tel que déterminé après traitement |
|---|---|---|---|---|---|---|
| | CXCL10 (ng/mL) | LGALS3BP (µg/mL) | MDK (ng/mL) | Z | Prédiction mROC | |
| 59 | 137,9 | 5,2 | 0,188 | 1,428 | R | R |
| 65 | 711,2 | 5,2 | 0,325 | 1,911 | R | R |
| 75 | 1036,3 | 23,8 | 1,018 | 3,462 | NR | NR |
| 83 | 1036,3 | 13,1 | 0,184 | 3,003 | NR | NR |
| 90 | 393,1 | 6,6 | 0,399 | 1,857 | R | R |
| 91 | 399,2 | 3,0 | 0,956 | 1,082 | R | R |
| 92 | 168,9 | 1,9 | 0,145 | 0,795 | R | R |
| 125 | 123,9 | 4,5 | 0,596 | 1,164 | R | R |
| 167 | 708,4 | 10,7 | 0,245 | 2,594 | NR | NR |

(suite)

| n° du sujet test | modèle mROC $\delta = 2{,}164$ | | | | | Statut R ou NR, tel que déterminé après traitement |
|---|---|---|---|---|---|---|
| | CXCL10 (ng/mL) | LGALS3BP (µg/mL) | MDK (ng/mL) | Z | Prédiction mROC | |
| 308 | 304,7 | 10,6 | 0,489 | 2,201 | NR | NR |
| 346 | 156,0 | 5,7 | 0,104 | 1,573 | R | R |
| 366 | 261,4 | 2,2 | 0,626 | 0,849 | R | R |
| 501 | 535,8 | 6,2 | 0,021 | 2,034 | R | R |
| 503 | 374,2 | 6,1 | 0,072 | 1,874 | R | R |
| 509 | 911,4 | 15,5 | 0,145 | 3,139 | NR | NR |
| 521 | 659,6 | 18,1 | 0,610 | 3,035 | NR | NR |
| 526 | 665,5 | 10,3 | 0,451 | 2,461 | NR | NR |
| 527 | 315,9 | 17,7 | 0,295 | 2,844 | NR | NR |
| 573 | 580,9 | 14,7 | 0,068 | 2,912 | NR | NR |
| 574 | 998,3 | 34,9 | 0,196 | 4,257 | NR | NR |

### EXEMPLE 5 : Protéines sériques (combinaison des niveaux d'expression de 2 gènes)

L'AUC relative à la combinaison des niveaux d'expression des gènes LGALS3BP et CXCL10 (combinaison n°15 dans le tableau 2 ci-dessus) calculée sur la population d'étude complète de l'exemple 3 (n = 167 patients) est de 0,831 (*cf.* tableau 6 ci-dessus).

Les dosages des concentrations protéiques ont été réalisés comme décrit en exemple 1 et tableau 44 ci-dessus.

Par la méthode mROC, le seuil maximisant l'indice de Youden ($\delta$) pour cette combinaison est de 2,169 (*cf.* tableau 4 ci-dessus).

Pour le choix de ce seuil, les performances de la combinaison sont les suivantes :

Sensibilité (Se) = 82% ; spécificité (Spe) = 72% (*cf.* tableau 3 ci-dessus).

Un exemple de règle décisionnelle est la règle suivante :

$$Z = 0{,}030 \text{ x } CXCL10^t + 0{,}447 \text{ x } LGALS3BP^t$$

(fonction Z15PROT, *cf.* tableau 4 ci-dessus), où :

- CXCL10 et LGALS3BP sont les valeurs de dosage des biomarqueurs BMQ, c'est-à-dire les valeurs de dosage des niveaux d'expression des gènes indiqués (en l'occurrence, concentration sérique des protéines),
- l'exposant t (ici porté par CXCL10 et LGALS3BP) indique que la valeur à appliquer dans la règle décisionnelle est la transformée Box-Cox (Box et Cox, 1964) de la valeur de dosage du niveau d'expression du gène considéré, afin de la normaliser selon la formule suivante :

$$BMQ^t = (BMQ^\lambda - 1)/\lambda.$$

Dans l'exemple de règle décisionnelle ci-dessus indiquée, les paramètres $\lambda$ sont 0,41 pour CXCL10 et de 0,33 pour LGALS3BP (*cf.* tableau 5 ci-dessus).

Si $Z \geq 2{,}169$ : le test diagnostic est positif (prédiction mROC = 1), le sujet est déclaré 'NR' (sujet pronostiqué non répondeur au traitement),

Si Z < 2,169 : le test est négatif (prédiction mROC = 0), le sujet est déclaré 'R' (sujet pronostiqué répondeur au traitement).

Un exemple de prédiction sur 20 sujets (patients humains) est donné dans le tableau 47 ci-dessous, qui présente les valeurs de dosage (BMQ) des niveaux d'expression sérique des gènes choisis. Il s'agit des mêmes 20 patients que ceux de l'exemple 2 ci-dessus.

Un ou plusieurs facteurs cliniques et/ou un ou plusieurs facteurs biologiques et/ou un ou plusieurs facteurs virologiques peuvent être combinés aux niveaux d'expression sérique de protéines sélectionnées conformément à l'invention, et conduire à une règle décisionnelle dont le pouvoir prédictif peut être encore supérieur à celui de la règle ci-dessus présentée.

**Tableau 47 : exemple d'application d'un modèle de classification basé sur la combinaison des niveaux d'expression sérique des gènes CXCL10 et LGALS3BP (combinaison n° 15 dans le tableau 2 ci-dessus)**

| n° du sujet test | modèle mROC $\delta = 2{,}169$ | | | | **Statut R ou NR,** tel que déterminé après traitement |
|---|---|---|---|---|---|
| | CXCL10 (ng/mL) | LGALS3BP (µg/mL) | Z | Prédiction mROC | |
| 59 | 137,9 | 5,2 | 1,450 | R | R |
| 65 | 711,2 | 5,2 | 1,993 | R | R |
| 75 | 1036,3 | 23,8 | 3,687 | NR | NR |
| 83 | 1036,3 | 13,1 | 3,000 | NR | NR |
| 90 | 393,1 | 6,6 | 1,944 | R | R |
| 91 | 399,2 | 3,0 | 1,379 | R | R |
| 92 | 168,9 | 1,9 | 0,841 | R | R |
| 125 | 123,9 | 4,5 | 1,321 | R | R |
| 167 | 708,4 | 10,7 | 2,615 | NR | NR |
| 308 | 304,7 | 10,6 | 2,291 | NR | NR |
| 346 | 156,0 | 5,7 | 1,564 | R | R |
| 366 | 261,4 | 2,2 | 1,048 | R | R |
| 501 | 535,8 | 6,2 | 2,008 | R | R |
| 503 | 374,2 | 6,1 | 1,861 | R | R |
| 509 | 911,4 | 15,5 | 3,111 | NR | NR |
| 521 | 659,6 | 18,1 | 3,141 | NR | NR |
| 526 | 665,5 | 10,3 | 2,550 | NR | NR |
| 527 | 315,9 | 17,7 | 2,841 | NR | NR |
| 573 | 580,9 | 14,7 | 2,856 | NR | NR |
| 574 | 998,3 | 34,9 | 4,190 | NR | NR |

**EXEMPLE 6 : Combinaison des niveaux d'expression dans le sérum de deux gènes (LGALS3BP et CXCL10) (combinaison n°15 dans le tableau 2 ci-dessus), combinée en outre à des facteurs cliniques et/ou des facteurs biologiques et/ou des facteurs virologiques.**

Un ou plusieurs facteurs cliniques et/ou un ou plusieurs facteurs biologiques et/ou un ou plusieurs facteurs virologiques peuvent être combinés aux niveaux d'expression sérique de protéines sélectionnées conformément à l'invention, et conduire à une règle décisionnelle dont le pouvoir prédictif peut être encore supérieur à celui de la règle ci-dessus présentée (*cf.* exemple 5).

**6a) Combinaison des niveaux d'expression dans le sérum des gènes LGALS3BP, CXCL10** (combinaison n°15 dans le tableau 2 ci-dessus), **combinée aux facteurs « âge à la date du prélèvement », « charge virale avant traitement » et « concentration en alanine aminotransférase ».**

Par exemple, la combinaison :

- des niveaux sériques de traduction des gènes LGALS3BP, CXCL10 (tableau 47 ci-dessus ; *cf* exemple 5 ci-dessus), et
- de la valeur du facteur clinique suivant :
  ◦ âge à la date du prélèvement, en l'occurrence âge à la date du prélèvement de sérum (Age), et
- de la valeur du facteur virologique suivant :
  ◦ charge virale avant traitement (CVavantTTT), et
- de la valeur du facteur biologique suivant :
  ◦ concentration en alanine aminotransférase (ALT ; concentration protéique dans le sérum),

conduit à une règle décisionnelle dont l'aire sous la courbe ROC (AUC), calculée sur la population d'étude complète de l'exemple 3 (n = 167 patients), est de 0,877 (*cf.* tableau 28 ci-dessus), alors qu'elle est de 0,831 (*cf.* exemple 5 ci-dessus), lorsque la combinaison des niveaux d'expression des gènes LGALS3BP, CXCL10 est utilisée seule, sans être combinée aux autres facteurs cliniques et/ou facteurs biologiques et/ou facteurs virologiques ci-dessus indiqués.

Par la méthode mROC (*cf.* exemple 1), le seuil maximisant l'indice de Youden pour cette combinaison est de -2,345 (*cf.* tableau 26 ci-dessus).

Pour le choix de ce seuil, les performances de la combinaison sont les suivantes :
Sensibilité (Se) = 82%, spécificité (Spe) = 77% (*cf.* tableau 25 ci-dessus).

Un exemple de règle décisionnelle est la règle suivante :

$$Z = 0{,}569 \text{ x } LGALS3BP^t + 0{,}033 \text{ x } CXCL10^t + 0{,}059 \text{ x } CVavantTTT^t - 0{,}899 \text{ x } Age^t -$$

$$0{,}538 \text{ x } ALT^t$$

(fonction Z15PROTsuppl ; *cf.* tableau 26 ci-dessus) où :

- LGALS3BP, CXCL10 sont les valeurs de dosage BMQ des biomarqueurs, c'est-à-dire les valeurs de dosage des niveaux d'expression des gènes indiqués (en l'occurrence, concentration sérique des protéines),
- CVavantTTT, Age et ALT sont les valeurs du facteur « charge virale avant traitement », du facteur « âge à la date du prélèvement » et du facteur « concentration en alanine aminotransférase » ci-dessus indiqués et où
- l'exposant t (ici porté par LGALS3BP, CXCL10, CVavantTTT, Age et ALT) indique la valeur à appliquer dans la règle décisionnelle est la transformée de Box-Cox (Box et Cox, 1964) de la valeur de dosage du biomarqueur BMQ considéré, afin de la normaliser selon la formule suivante :

$$BMQ^t = (BMQ^\lambda - 1)/\lambda.$$

Dans l'exemple de la règle décisionnelle ci-dessus indiquée, les paramètres $\lambda$ sont 0,41 pour CXCL10, 0,33 pour LGALS3BP, 0,09 pour Age, 0,2 pour CVavantTTT et -0,09 pour ALT (*cf.* tableau 27 ci-dessus).

Si $Z \geq$ -2,345 : le test diagnostic est positif (prédiction mROC = 1), le sujet est déclaré 'NR' (sujet pronostiqué non répondeur au traitement),

Si Z < -2,345 : le test est négatif (prédiction mROC = 0), le sujet est déclaré 'R' (sujet pronostiqué répondeur au traitement).

Un exemple de prédiction sur 20 sujets (patients humains) est donné dans le tableau 48 ci-dessous, qui présente des valeurs de dosage (BMQ) des niveaux d'expression sérique des gènes choisis.

**6b) Combinaisons des niveaux d'expression dans le sérum des gènes LGALS3BP, CXCL10** (combinaison n°15 dans le tableau 2 ci-dessus), **combinée aux facteurs « âge à la date du prélèvement », « charge virale avant traitement » et « concentration en gamma-glutamyl-transpeptidase ».**

Par exemple, la combinaison :

- des niveaux sériques de traduction des gènes LGALS3BP, CXCL10 (tableau 47 ci-dessus ; *cf.* exemple 5 ci-dessus) et
- de la valeur du facteur clinique suivant :
  ◦ âge à la date du prélèvement, en l'occurrence âge à la date du prélèvement de sérum (Age), et
- de la valeur du facteur virologique suivante :
  ◦ charge virale avant traitement CVavantTTT), et
- de la valeur du facteur biologique suivante :

∘ concentration en gamma glutamyl transpeptidase (GGT ; concentration protéique dans le sérum),

conduit à une règle décisionnelle dont l'aire sous la courbe ROC (AUC), calculée sur la population d'étude complète de l'exemple 3 (n = 167 patients), est de 0,872 (*cf* tableau 28 ci-dessus), alors qu'elle est de 0,831 (*cf.* exemple 5 ci-dessus), lorsque la combinaison des niveaux d'expression des gènes LGALS3BP, CXCL10 est utilisée seule, sans être combinée aux autres facteurs cliniques et/ou facteurs biologiques et/ou facteurs virologiques ci-dessus indiqués.

Par la méthode mROC (*cf.* exemple 1), le seuil maximisant l'indice de Youden pour cette combinaison est de 0,696 (*cf.* tableau 26 ci-dessus).

Pour le choix de ce seuil, les performances de la combinaison sont les suivantes : Sensibilité (Se) = 83%, spécificité (Spe) = 74% (*cf* tableau 25 ci-dessus).

Un exemple de règle décisionnelle est la règle suivante :

$$Z = 0{,}492 \text{ x } LGALS3BP^t + 0{,}018 \text{ x } CXCL10^t - 0{,}701 \text{ x } Age^t + 0{,}058 \text{ x } CVavantTTT^t + 0{,}202 \text{ x } GGT^t$$

(fonction Z15PROTsupp2 ; *cf.* tableau 26 ci-dessus) où :

- LGALS3BP, CXCL10 sont les valeurs de dosage BMQ des biomarqueurs, c'est-à-dire les valeurs de dosage des niveaux d'expression des gènes indiqués (en l'occurrence, concentration sérique des protéines),
- CVavantTTT, Age et GGT sont les valeurs du facteur « charge virale avant traitement », du facteur « âge à la date du prélèvement » et du facteur « concentration en gamma glutamyl transpeptidase » ci-dessus indiqués et où
- l'exposant t (ici porté par LGALS3BP, CXCL10, CVavantTTT, Age, GGT) indique la valeur à appliquer dans la règle décisionnelle est la transformée de Box-Cox (Box et Cox, 1964) de la valeur de dosage du biomarqueur BMQ considéré, afin de la normaliser selon la formule suivante :

$$BMQ^t = (BMQ^\lambda - 1)/\lambda.$$

Dans l'exemple de la règle décisionnelle ci-dessus indiquée, les paramètres $\lambda$ sont 0,41 pour CXCL10, 0,33 pour LGALS3BP, 0,09 pour Age, 0,2 pour CVavantTTT et -0,01 pour GGT (*cf.* tableau 27 ci-dessus).

Si $Z \geq 0{,}696$ : le test diagnostic est positif (prédiction mROC = 1), le sujet est déclaré 'NR' (sujet pronostiqué non répondeur au traitement),

Si $Z < 0{,}696$ : le test est négatif (prédiction mROC = 0), le sujet est déclaré 'R' (sujet pronostiqué répondeur au traitement).

Un exemple de prédiction sur 20 sujets (patients humains) est donné dans le tableau 49 ci-dessous, qui présente des valeurs de dosage (BMQ) des niveaux d'expression sérique des gènes choisis.

**6c) Combinaisons des niveaux d'expression dans le sérum des gènes LGALS3BP, CXCL10** (combinaison n°15 dans le tableau 2 ci-dessus), **combinée aux facteurs « charge virale avant traitement », « concentration en aspartate aminotransférase » et « concentration en phosphatase alcaline ».**

Par exemple, la combinaison :

- des niveaux sériques de traduction des gènes LGALS3BP, CXCL10 (tableau 47 ci-dessus ; *cf* exemple 5 ci-dessus), et
- de la valeur du facteur virologique :
  ∘ charge virale avant traitement (CVavantTTT), et
- des valeurs des (autres) facteurs biologiques suivants :

  o « concentration en aspartate aminotransférase » (AST ; concentration protéique dans le sérum),
  o « concentration en phosphatase alcaline » (PAL, concentration protéique dans le sérum)

conduit à une règle décisionnelle dont l'aire sous la courbe ROC (AUC), calculée sur la population d'étude complète de l'exemple 3 (n = 167 patients), est de 0,869 (*cf* tableau 28 ci-dessus), alors qu'elle est de 0,831 (*cf.* exemple 5 ci-dessus), lorsque la combinaison des niveaux d'expression des gènes LGALS3BP, CXCL10 est utilisée seule, sans être combinée aux autres facteurs biologiques et/ou facteurs virologiques ci-dessus indiqués.

Par la méthode mROC (*cf.* exemple 1), le seuil maximisant l'indice de Youden pour cette combinaison est de 3,862 (*cf.* tableau 26 ci-dessus).

Pour le choix de ce seuil, les performances de la combinaison sont les suivantes : Sensibilité (Se) = 86%, spécificité (Spe) = 77% (*cf* tableau 25 ci-dessus).

Un exemple de règle décisionnelle est la règle suivante :

$$Z = 0,499 \times \text{LGALS3BP}^t + 0,028 \times \text{CXCL10}^t + 0,06 \times \text{CVavantTTT}^t - 1,147 \times \text{AST}^t + 0,931 \times \text{PAL}^t,$$

(fonction Z15PROTsupp3 ; *cf.* tableau 26 ci-dessus) où :

- LGALS3BP, CXCL10 sont les valeurs de dosage BMQ des biomarqueurs, c'est-à-dire les valeurs de dosage des niveaux d'expression des gènes indiqués (en l'occurrence, concentration sérique des protéines)
- CVavantTTT, AST et PAL sont les valeurs du facteur « charge virale avant traitement », du facteur « concentration en aspartate aminotransférase » et du facteur « concentration phosphatase alcaline » ci-dessus indiqués et où
- L'exposant t (ici porté par LGALS3BP, CXCL10) indique la valeur à appliquer dans la règle décisionnelle est la transformée de Box-Cox (Box et Cox, 1964) de la valeur de dosage du biomarqueur BMQ considéré, afin de la normaliser selon la formule suivante :

$$\text{BMQ}^t = (\text{BMQ}^\lambda - 1)/\lambda.$$

Dans l'exemple de la règle décisionnelle ci-dessus indiquée, les paramètres $\lambda$ sont 0,41 pour CXCL10, 0,33 pour LGALS3BP, 0,2 pour CVavantTTT, -0,3 pour AST et -0,11 pour PAL (*cf.* tableau 27 ci-dessus).

Si $Z \geq 3,862$ : le test diagnostic est positif (prédiction mROC = 1), le sujet est déclaré 'NR' (sujet pronostiqué non répondeur au traitement),

Si $Z < 3,862$ : le test est négatif (prédiction mROC = 0), le sujet est déclaré 'R' (sujet pronostiqué répondeur au traitement).

Un exemple de prédiction sur 20 sujets (patients humains) est donné dans le tableau 50 ci-dessous, qui présente des valeurs de dosage (BMQ) des niveaux d'expression sérique des gènes choisis.

**6d) Combinaisons des niveaux d'expression dans le sérum des gènes LGALS3BP, CXCL10** (combinaison n°15 dans le tableau 2 ci-dessus), **combinée au facteur clinique « indice de masse corporelle ».**

Par exemple, la combinaison :

- des niveaux sériques de traduction des gènes LGALS3BP, CXCL10 (tableau 47 ci-dessus ; *cf* exemple 5 ci-dessus), et
- de la valeur du facteur clinique
  ∘ indice de masse corporelle (IMC),

conduit à une règle décisionnelle dont l'aire sous la courbe ROC (AUC), calculée sur la population d'étude complète de l'exemple 3 (n = 167 patients), est de 0,834 (*cf* tableau 28 ci-dessus), alors qu'elle est de 0,831 (*cf.* exemple 5 ci-dessus), lorsque la combinaison des niveaux d'expression des gènes LGALS3BP, CXCL10 est utilisée seule, sans être combinée au facteur clinique ci-dessus indiqué.

Par la méthode mROC (*cf.* exemple 1), le seuil maximisant l'indice de Youden pour cette combinaison est de 0,375 (*cf.* tableau 26 ci-dessus).

Pour le choix de ce seuil, les performances de la combinaison sont les suivantes : Sensibilité (Se) = 81%, spécificité (Spe) = 78% (*cf.* tableau 25 ci-dessus).

Un exemple de règle décisionnelle est la règle suivante :

$$Z = 0,451 \times \text{LGALS3BP}^t + 0,033 \times \text{CXCL10}^t - 0,535 \times \text{IMC}^t,$$

(fonction Z15PROTsupp4 ; *cf.* tableau 26 ci-dessus) où :

- LGALS3BP, CXCL10 sont les valeurs de dosage BMQ des biomarqueurs, c'est-à-dire les valeurs de dosage des niveaux d'expression des gènes indiqués (en l'occurrence, concentration sérique des protéines).

- IMC est la valeur du facteur indice de masse corporelle, et où
- l'exposant t (ici porté par LGALS3BP, CXCL10) indique la valeur à appliquer dans la règle décisionnelle est la transformée de Box-Cox (Box et Cox, 1964) de la valeur de dosage du biomarqueur BMQ considéré, afin de la normaliser selon la formule suivante :

$$BMQ^t = (BMQ^\lambda - 1)/\lambda.$$

Dans l'exemple de la règle décisionnelle ci-dessus indiquée, les paramètres $\lambda$ sont 0,41 pour CXCL10, 0,33 pour LGALS3BP, 0,08 pour IMC (*cf.* tableau 27 ci-dessus).

Si $Z \geq 0,375$ : le test diagnostic est positif (prédiction mROC = 1), le sujet est déclaré 'NR' (sujet pronostiqué non répondeur au traitement),

Si $Z < 0,375$ : le test est négatif (prédiction mROC = 0), le sujet est déclaré 'R' (sujet pronostiqué répondeur au traitement).

Un exemple de prédiction sur 20 sujets (patients humains) est donné dans le tableau 51 ci-dessous, qui présente des valeurs de dosage (BMQ) des niveaux d'expression sérique des gènes choisis.

**Tableau 48 : exemple d'application d'un modèle de classification basé sur la combinaison des niveaux d'expression sérique des gènes CXCL10, LGALS3BP et du facteur clinique « âge à la date du prélèvement » (Age), du facteur virologique « charge virale avant traitement » (CVavantTTT)) et du facteur biologique « concentration en alanine aminotransférase » (ALT), (exemple 6a).**

| n° du sujet test | modèle mROC $\delta$ = -2,345 | | | | | | | Statut R ou NR, tel que déterminé après traitement |
|---|---|---|---|---|---|---|---|---|
| | CXCL10 (ng/mL) | LGALS3BP (µg/mL) | Age | CVavantTTT (copies/mL. $10^3$) | ALT (U/L) | Z | Prédiction mROC | |
| 6 | 573,0 | 16,8 | 59 | 10450 | 69 | -1,078 | NR | NR |
| 45 | 268,3 | 2,1 | 44 | 3645 | 458 | -4,177 | R | R |
| 58 | 437,7 | 14,8 | 48 | 5079 | 93 | -1,475 | NR | NR |
| 59 | 137,9 | 5,1 | 51 | 1120 | 80 | -3,516 | R | R |
| 62 | 422,0 | 8,9 | 62 | 185 | 47 | -2,993 | R | R |
| 65 | 711,2 | 5,2 | 35 | 450 | 152 | -2,885 | R | R |
| 66 | 560,7 | 3,1 | 43 | 8132 | 71 | -2,655 | R | R |
| 73 | 330,1 | 4,1 | 55 | 7611 | 101 | -3,087 | R | R |
| 75 | 1036,3 | 23,8 | 53 | 2347 | 50 | -0,483 | NR | NR |
| 80 | 1068,4 | 11,7 | 37 | 12932 | 67 | -0,555 | NR | NR |
| 83 | 1036,3 | 13,1 | 51 | 3928 | 95 | -1,398 | NR | NR |
| 86 | 174,8 | 9,0 | 50 | 57 | 105 | -3,467 | R | R |
| 90 | 393,1 | 6,6 | 59 | 515 | 119 | -3,437 | R | R |
| 91 | 399,2 | 3,0 | 48 | 3902 | 79 | -3,232 | R | R |
| 92 | 168,9 | 1,9 | 35 | 3 | 128 | -4,828 | R | R |
| 167 | 708,4 | 10,7 | 49 | 12616 | 144 | -1,523 | NR | NR |
| 509 | 911,4 | 15,4 | 48 | 8779 | 243 | -1,216 | NR | NR |
| 517 | 911,4 | 15,8 | 48 | 11114 | 147 | -0,906 | NR | NR |
| 521 | 659,6 | 18,1 | 58 | 14432 | 166 | -1,081 | NR | NR |
| 527 | 315,9 | 17,7 | 47 | 3457 | 80 | -1,390 | NR | NR |

**Tableau 49 : exemple d'application d'un modèle de classification basé sur la combinaison des niveaux d'expression sérique des gènes CXCL10, LGALS3BP et du facteur clinique « âge à la date du prélèvement » (Age), du facteur virologique « charge virale avant traitement » (CVavantTTT) et du facteur biologique « concentration en gamma-glutamyl-transpeptidase » (GGT), (exemple 6b).**

| n° du sujet test | modèle mROC $\delta$ = 0,696 | | | | | | | Statut R ou NR, tel que déterminé après traitement |
| | CXCL10 (ng/mL) | LGALS3BP ($\mu$g/mL) | Age | CVavantTTT (copies/mL. $10^3$) | GGT (U/L) | Z | Prédiction mROC | |
|---|---|---|---|---|---|---|---|---|
| 6 | 573,0 | 16,8 | 59 | 10450 | 78 | 1,808 | NR | NR |
| 45 | 268,3 | 2,1 | 44 | 3645 | 34 | -0,459 | R | R |
| 58 | 437,7 | 14,8 | 48 | 5079 | 162 | 1,684 | NR | NR |
| 59 | 137,9 | 5,1 | 51 | 1120 | 18 | -0,481 | R | R |
| 62 | 422,0 | 8,9 | 62 | 185 | 46 | -0,151 | R | R |
| 65 | 711,2 | 5,2 | 35 | 450 | 46 | 0,195 | R | R |
| 66 | 560,7 | 3,1 | 43 | 8132 | 65 | 0,376 | R | R |
| 73 | 330,1 | 4,1 | 55 | 7611 | 31 | 0,060 | R | R |
| 75 | 1036,3 | 23,8 | 53 | 2347 | 135 | 2,161 | NR | NR |
| 80 | 1068,4 | 11,7 | 37 | 12932 | 132 | 2,210 | NR | NR |
| 83 | 1036,3 | 13,1 | 51 | 3928 | 82 | 1,492 | NR | NR |
| 86 | 174,8 | 9,0 | 50 | 57 | 287 | 0,096 | R | R |
| 90 | 393,1 | 6,6 | 59 | 515 | 21 | -0,369 | R | R |
| 91 | 399,2 | 3,0 | 48 | 3902 | 39 | -0,161 | R | R |
| 92 | 168,9 | 1,9 | 35 | 3 | 92 | -1,308 | R | R |
| 167 | 708,4 | 10,7 | 49 | 12616 | 378 | 1,909 | NR | NR |
| 509 | 911,4 | 15,4 | 48 | 8779 | 43 | 1,850 | NR | NR |
| 517 | 911,4 | 15,8 | 48 | 11114 | 266 | 2,332 | NR | NR |
| 521 | 659,6 | 18,1 | 58 | 14432 | 127 | 2,161 | NR | NR |
| 527 | 315,9 | 17,7 | 47 | 3457 | 127 | 1,687 | NR | NR |

**Tableau 50 : exemple d'application d'un modèle de classification basé sur la combinaison des niveaux d'expression sérique des gènes CXCL10, LGALS3BP, du facteur virologique « charge virale avant traitement » (CVavantTTT) et des facteurs biologiques « concentration en aspartate aminotransférase » (AST) et « concentration en phosphatase alcaline » (PAL), (exemple 6c).**

| n° du sujet test | modèle mROC $\delta$ = 3,862 | | | | | | Statut R ou NR, tel que déterminé après traitement |
| | CXCL10 (ng/mL) | LGALS3BP ($\mu$g/mL) | CVavantTTT (copies/mL. $10^3$) | AST (U/L) | PAL (U/L) | Z | Prédiction mROC | |
|---|---|---|---|---|---|---|---|---|
| 6 | 573,0 | 16,8 | 10450 | 50 | 40 | 4,973 | NR | NR |
| 45 | 268,3 | 2,1 | 3645 | 152 | 63 | 2,393 | R | R |
| 58 | 437,7 | 14,8 | 5079 | 62 | 51 | 4,533 | NR | NR |

(suite)

| n° du sujet test | modèle mROC δ = 3,862 | | | | | | | Statut R ou NR, tel que déterminé après traitement |
|---|---|---|---|---|---|---|---|---|
| | CXCL10 (ng/mL) | LGALS3BP (μg/mL) | CVavantTTT (copies/mL. 10³) | AST (U/L) | PAL (U/L) | Z | Prédiction mROC | |
| 59 | 137,9 | 5,1 | 1120 | 49 | 77 | 3,034 | R | R |
| 62 | 422,0 | 8,9 | 185 | 34 | 52 | 3,381 | R | R |
| 65 | 711,2 | 5,2 | 450 | 61 | 49 | 2,997 | R | R |
| 66 | 560,7 | 3,1 | 8132 | 39 | 83 | 3,750 | R | R |
| 73 | 330,1 | 4,1 | 7611 | 78 | 61 | 3,356 | R | R |
| 75 | 1036,3 | 23,8 | 2347 | 64 | 149 | 5,872 | NR | NR |
| 80 | 1068,4 | 11,7 | 12932 | 44 | 100 | 5,481 | NR | NR |
| 83 | 1036,3 | 13,1 | 3928 | 78 | 97 | 4,960 | NR | NR |
| 86 | 174,8 | 9,0 | 57 | 53 | 77 | 3,035 | R | R |
| 90 | 393,1 | 6,6 | 515 | 61 | 52 | 3,049 | R | R |
| 91 | 399,2 | 3,0 | 3902 | 64 | 72 | 3,116 | R | R |
| 92 | 168,9 | 1,9 | 3 | 29 | 68 | 1,633 | R | R |
| 167 | 708,4 | 10,7 | 12616 | 163 | 114 | 4,861 | NR | NR |
| 509 | 911,4 | 15,4 | 8779 | 154 | 56 | 4,860 | NR | NR |
| 517 | 911,4 | 15,8 | 11114 | 103 | 65 | 5,177 | NR | NR |
| 521 | 659,6 | 18,1 | 14432 | 83 | 62 | 5,345 | NR | NR |
| 527 | 315,9 | 17,7 | 3457 | 35 | 67 | 4,901 | NR | NR |

**Tableau 51 : exemple d'application d'un modèle de classification basé sur la combinaison des niveaux d'expression sérique des gènes CXCL10, LGALS3BP et du facteur clinique « indice de masse corporelle » (IMC) (exemple 6d).**

| n° du sujet test | modèle mROC δ = 0,375 | | | | | Statut R ou NR, tel que déterminé après traitement |
|---|---|---|---|---|---|---|
| | CXCL10 (ng/mL) | LGALS3BP (μg/mL) | IMC | Z | Prédiction mROC | |
| 6 | 573,0 | 16,8 | 19,6 | 1,314 | NR | NR |
| 45 | 268,3 | 2,1 | 22,0 | -0,784 | R | R |
| 58 | 437,7 | 14,8 | 25,2 | 0,879 | NR | NR |
| 59 | 137,9 | 5,1 | 23,7 | -0,421 | R | R |
| 62 | 422,0 | 8,9 | 27,4 | 0,293 | R | R |
| 65 | 711,2 | 5,2 | 29,6 | 0,022 | R | R |
| 66 | 560,7 | 3,1 | 19,4 | -0,179 | R | R |
| 73 | 330,1 | 4,1 | 23,4 | -0,312 | R | R |
| 75 | 1036,3 | 23,8 | 27,9 | 1,789 | NR | NR |
| 80 | 1068,4 | 11,7 | 31,6 | 0,908 | NR | NR |

(suite)

| n° du sujet test | modèle mROC δ = 0,375 | | | | | Statut R ou NR, tel que déterminé après traitement |
| | CXCL10 (ng/mL) | LGALS3BP (µg/mL) | IMC | Z | Prédiction mROC | |
|---|---|---|---|---|---|---|
| 83 | 1036,3 | 13,1 | 25,7 | 1,152 | NR | NR |
| 86 | 174,8 | 9,0 | 28,4 | -0,010 | R | R |
| 90 | 393,1 | 6,6 | 25,2 | 0,064 | R | R |
| 91 | 399,2 | 3,0 | 21,4 | -0,393 | R | R |
| 92 | 168,9 | 1,9 | 28,7 | -1,163 | R | R |
| 167 | 708,4 | 10,7 | 37,8 | 0,475 | NR | NR |
| 509 | 911,4 | 15,4 | 20,8 | 1,405 | NR | NR |
| 517 | 911,4 | 15,8 | 23,3 | 1,356 | NR | NR |
| 521 | 659,6 | 18,1 | 23,8 | 1,328 | NR | NR |
| 527 | 315,9 | 17,7 | 37,3 | 0,685 | NR | NR |

**RÉFÉRENCES BIBLIOGRAPHIQUES**

Anastasiadis et al. 2005 ; New globally convergent training scheme based on the résilient propagation algorithm. Neurocomputing 64: 253-270.

Asselah et al. 2008 ; "Liver gene expression signature to predict response to pegylated interferon plus ribavirin combination therapy in patients with chronic hepatitis C"; Gut 57: 516-524.

Box et Cox 1964 ; An analysis of transformations. Journal of the Royal Statistical Society, Series B 26: 211-243.

Breiman 2001 ; Random Forests. Machine Learning 45 : 5-32.

Chambers 2008 ; Software for data analysis : programming with R. Springer, New York, ISBN 978-0-387-75935-7.

Chen et al. 2005 ; Gastroenterology 128: 1437-1444.

Chen et al. 2010 ; Gastroenterology 138: 1123-1133.

Cole et al. 1983 ; Proc. Natl. Acad. Sci. USA 80: 2026-2030.

Cole et al. 1985 ; Monoclonal Antibodies And Cancer Therapy, Alan R. Liss, Inc., pp. 77-96.

Falissard 2005 ; Comprendre et utiliser les statistiques dans les sciences de la vie, Masson.

Hastie, Tibishirani et Friedman, 2009 ; "The Elements of Statistical Learning: Data Mining, Inference and Prediction", 2nd Edition, Springer.

Hidetsugu Saito et al. 2010 ; "On-treatment prédictions of success in peg-interferon/ribavirin treatment using a novel formula"; World J. Gastroenterol. 16(1): 89-97.

Intrator et Intrator 1993 ; Using Neural Nets for Interprétation of Nonlinear Models. Proceedings of the Statistical Computing Section, San Francisco: American Statistical Society (eds), pages 244-249.

Köhler and Milstein 1975 ; Nature 256: 495-497.

Kosbor et al. 1983 ; Immunology Today 4: 72.

Kramar et al. 1999 ; Critères ROC généralisés pour l'évaluation de plusieurs marqueurs tumoraux. Revue d'Epidémiologie et Santé Publique 47 :376-383.

Kramar et al. 2001 ; mROC: a computer program for combining tumour markers in predicting disease states. Computer methods and programs in biomedicine 66: 199-207.

Liaw et Wiener 2002 ; Classification and regression by Random Forest. R. News 2.3: 18-22.

Livak et Schmittgen 2001 ; Analysis of relative gene expression data using real-time quantitative PCR and the 2(-Delta Delta C(T)) Method. Methods 25: 402-408.

Reiser et Faraggi 1997 ; Confidence intervals for the generalized ROC criterion. Biometrics 53: 644-652.

Riedmiller 1994 ; Rprop - Description and Implementation Details. Technical Report. University of Karlsruhe.

Riedmiller et Braun 1993 ; A direct adaptive method for faster backpropagation learning: the RPROP algorithm. Proceedings of the IEEE International Conference on Neural Networks (ICNN), San Francisco, pages 586-591.

Schmitten et Livak 2008 ; Analyzing real-time PCR data by the comparative Ct method. Nature Protocols 3(6) : 1101-1108.

Shapiro 1999 ; The interpretation ofdiagnostic tests. Statistical Methods in Medical Research, 8: 113-134.

Su et Liu 1993 ; Linear combinations of multiple diagnostic markers. Journal of the American Statistical Association 88: 1350-1355.

Swets 1988 ; Measuring the accuracy of diagnostic systems. Science 240, 1285-1293.

Theodoridis et Koutroumbos 2009 ; Pattern Recognition. Academic Press, Elsevier.

US 4 376 110 (au nom de Hybritech Inc.).

Wahba, 1990 ; "Splines Models for Observational Data"; SIAM - Society for Industrial and Applied Mathematics.

Wahba et Wold, 1975 ; "A completely automatic French curve: Fitting spline functions by cross-validation"; Communications in Statistics, 4:1-17, 1975.

**Revendications**

1. **Une méthode *in vitro*** pour pronostiquer, avant traitement, si un sujet infecté par un ou des VHC a une forte probabilité d'être répondeur à un traitement anti-VHC qui comprendra l'administration d'interféron et l'administration de ribavirine ou d'une pro-drogue de la ribavirine, ou si, au contraire, ce sujet a une forte probabilité de ne pas être répondeur à ce traitement anti-VHC,
ladite méthode comprenant les étapes suivantes :

   i) procéder aux mesures suivantes :

   - dans un échantillon préalablement obtenu à partir dudit sujet, doser les niveaux auxquels des gènes de mammifère choisis sont transcrits ou traduits, lesdits gènes de mammifère choisis dont on dose les niveaux de transcription ou de traduction étant la combinaison de gènes suivante :

   (a)

   - au moins un gène parmi MBL2, LGALS3BP et IL8,
   et
   - au moins un gène parmi G1P2, CCL21 et CXCL10,
   le nombre total des gènes ainsi choisis étant de 2, 3, 4 ou 5,
   ou

(b)

- au moins un gène parmi MBL2, LGALS3BP et IL8, et
- au moins un gène parmi G1P2, CCL21 et CXCL10, et
- au moins un gène parmi AFP, CRP, CXCL11, CXCL6, CXCL9, FGF7, MDK, MMP2, SFN, TGFB2 et VEGFD,
le nombre total des gènes ainsi choisis étant de 3, 4 ou 5, et

- doser les niveaux de transcription ou de traduction de 0 à 4 gènes de mammifère autres que les gènes de mammifère choisis parmi MBL2, LGALS3BP, IL8, G1P2, CXCL10, CCL21, AFP, CRP, CXCL11, CXCL6, CXCL9, FGF7, MDK, MMP2, SFN, TGFB2, VEGFD, et

ii) comparer les valeurs des mesures obtenues pour ledit sujet à l'étape i), à leurs valeurs, ou à la distribution de leurs valeurs, dans des cohortes de référence pré-établies selon leur statut de répondeur ou non-répondeur au traitement anti-VHC, pour classer ledit sujet dans celle de ces cohortes de référence vis-à-vis de laquelle il a la plus forte probabilité d'appartenance, et où l'échantillon obtenu dudit sujet est prélevé ou collecté à partir : du foie, du parenchyme hépatique, du sang, du sérum, du plasma ou de l'urine.

2. La méthode de la revendication 1, dans laquelle l'étape i) comprend en outre une étape pour mesurer ou déterminer, pour ledit sujet, la valeur d'un ou plusieurs facteurs cliniques et/ou d'un ou plusieurs facteurs virologiques et/ou d'un ou plusieurs facteurs biologiques autres que les niveaux d'expression de gènes choisis parmi MBL2, LGALS3BP, IL8, G1P2, CXCL10, CCL21, AFP, CRP, CXCL11, CXCL6, CXCL9, FGF7, MDK, MMP2, SFN, TGFB2, VEGFD.

3. La méthode de la revendication 1 ou 2, dans laquelle la comparaison de l'étape ii) est faite en combinant les valeurs des mesures obtenues pour ledit sujet à l'étape i), dans un modèle de classification multivariée, qui compare ces valeurs à leurs valeurs, ou à la distribution de leurs valeurs, dans des cohortes de référence pré-établies selon leur statut de répondeur ou non-répondeur, pour classer ledit sujet dans celle de ces cohortes de référence vis-à-vis de laquelle il a la plus forte probabilité d'appartenance,
et/ou
dans laquelle la comparaison de l'étape ii) est faite en combinant les valeurs des mesures obtenues pour ledit sujet à l'étape i), dans un modèle de classification multivariée préalablement construit comme suit :

a) pour une population d'individus qui sont de la même espèce que ledit sujet, et qui sont infectés par un ou des VHC, déterminer pour chacun de ces individus, s'il répond ou ne répond pas à un traitement anti-VHC qui comprend l'administration d'interféron et de ribavirine, et classer ces individus en sous-populations distinctes selon qu'ils sont répondeurs ou qu'ils sont non-répondeurs à ce traitement, constituant ainsi des cohortes de référence établies selon la réponse ou non-réponse de ces individus au traitement anti-VHC ;
b) dans au moins un échantillon qui a été préalablement obtenu à partir de chacun desdits individus, dont la nature est identique à celle de l'échantillon dudit sujet, procéder aux mêmes mesures que celles faites pour ledit sujet à ladite étape i) ;
c) faire une comparaison inter-cohorte des valeurs des mesures obtenues à l'étape b), ou de la distribution de ces valeurs, pour construire un modèle de classification multivariée, qui induit un statut de répondeur au traitement anti-VHC ou un statut de non-répondeur à ce traitement, à partir de la combinaison desdites valeurs de mesures obtenues à l'étape b),

et/ou
dans laquelle la comparaison de l'étape ii) est réalisée par combinaison desdites valeurs de mesures obtenues à l'étape i) dans une fonction mathématique, notamment une fonction linéaire ou non linéaire, plus particulièrement une fonction linéaire, pour obtenir une valeur de sortie indicatrice du statut de répondeur ou de non-répondeur dudit sujet,
et/ou
dans laquelle la comparaison de l'étape ii) est réalisée par combinaison desdites valeurs obtenues à l'étape i) dans un modèle d'apprentissage automatique multivarié, par exemple un modèle de classification non paramétrique multivariée, un modèle heuristique multivarié, ou un modèle de prédiction probabiliste multivariée, pour obtenir une valeur de sortie indicatrice du statut de répondeur ou de non-répondeur dudit sujet.

**4.** La méthode selon l'une quelconque des revendications 1 à 3, dans laquelle le classement dudit sujet dans celle de ces cohortes de référence vis-à-vis de laquelle il a la plus forte probabilité d'appartenance est fait avec :

- une sensibilité (Se) d'au moins 70%, d'au moins 71%, d'au moins 72%, d'au moins 73%, ou d'au moins 74%, ou d'au moins 75%, ou d'au moins 76%, ou d'au moins 77%, ou d'au moins 78%, au d'au moins 79%, ou d'au moins 80%, ou d'au moins 81%, ou d'au moins 82% ; et/ou avec une

- spécificité (Sp) d'au moins 70%, d'au moins 71%, d'au moins 72%, d'au moins 73%, ou d'au moins 74%, ou d'au moins 75%, ou d'au moins 76%, ou d'au moins 77%, ou d'au moins 78%, ou d'au moins 79%, ou d'au moins 80%, ou d'au moins 81%, ou d'au moins 82%, ou d'au moins 83%, ou d'au moins 84%, ou d'au moins 85%, ou d'au moins 86%, ou d'au moins 87%, ou d'au moins 88%, ou d'au moins 89%, ou d'au moins 90%, ou d'au moins 91%, ou d'au moins 92%, ou d'au moins 92% ; et/ou avec

- une Valeur Prédictive Négative (VPN) d'au moins 78%, ou d'au moins 79%, ou d'au moins 80%, ou d'au moins 81%, ou d'au moins 82%, ou d'au moins 83%, ou d'au moins 84%, ou d'au moins 85%, ou d'au moins 86%, ou d'au moins 87%, ou d'au moins 88% ; et/ou avec

- une Valeur Prédictive Positive (VPP) d'au moins 63%, ou d'au moins 64%, ou d'au moins 65%, ou d'au moins 66%, ou d'au moins 67%, ou d'au moins 68%, ou d'au moins 69%, ou d'au moins 70%, ou d'au moins 71%, ou d'au moins 72%, ou d'au moins 73%, ou d'au moins 74%, ou d'au moins 75%, ou d'au moins 76%, ou d'au moins 77%, ou d'au moins 78%, ou d'au moins 79%, ou d'au moins 80%, ou d'au moins 81%, ou d'au moins 82%, ou d'au moins 83%, ou d'au moins 84%, ou d'au moins 85%, ou d'au moins 86%, ou d'au moins 87%, ou d'au moins 88%, ou d'au moins 89%, ou d'au moins 90%, ou d'au moins 91%, ou d'au moins 92%, ou d'au moins 93%, ou d'au moins 94%,

et/ou

dans laquelle ledit modèle de classification multivariée présente :

- une aire sous la courbe ROC (AUC) d'au moins 0,76, d'au moins 0,77, d'au moins 0,78, plus particulièrement d'au moins 0,79, encore plus particulièrement d'au moins 0,80, d'au moins 0,81, d'au moins 0,82, d'au moins 0,83, d'au moins 0,84, plus particulièrement d'au moins 0,85, encore plus particulièrement d'au moins 0,86, encore plus particulièrement d'au moins 0,87, et/ou

- une erreur LOOCV d'au plus 18%, d'au plus 17%, d'au plus 16%, d'au plus 15%, d'au plus 14%, d'au plus 13%, d'au plus 12%.

**5.** La méthode selon l'une quelconque des revendications 1 à 4, dans laquelle, à l'étape i),

• lesdits gènes choisis parmi :

(a)

- au moins un gène parmi MBL2, LGALS3BP, IL8, et
- au moins un gène parmi G1P2, CCL21, CXCL10,

sont :

- LGALS3BP et CXCL10 (combinaison n°15) ; ou
- IL8, CCL21, CXCL10, G1P2, LGALS3BP (combinaison n°37) ;

ou

• lesdits gènes choisis parmi :
(b)

- au moins un gène parmi MBL2, LGALS3BP, IL8, et
- au moins un gène parmi G1P2, CCL21, CXCL10, et
- au moins un gène parmi AFP, CRP, CXCL11, CXCL6, CXCL9, FGF7, MDK, MMP2, SFN, TGFB2, VEGFD,

sont :

- LGALS3BP, CXCL10 et MDK (combinaison n°9) ; ou
- LGALS3BP, IL8, CXCL10, CCL21 et MDK (combinaison n°24) ; ou

- CRP, G1P2, LGALS3BP, MBL2, TGFB2 (combinaison n°1) ; ou
- AFP, CXCL6, CXCL9, G1P2, MBL2 (combinaison n°2) ; ou
- AFP, FGF7, G1P2, MBL2, MMP2 (combinaison n°3) ; ou
- CXCL11, G1P2, IL8, MBL2, TGFB2 (combinaison n°4) ; ou
- G1P2, IL8, MBL2, SFN, TGFB2 (combinaison n°5) ; ou
- CCL21, FGF7, IL8, LGALS3BP, MBL2 (combinaison n°6) ; ou
- G1P2, LGALS3BP, MBL2, MDK, TGFB2 (combinaison n°7) ; ou
- G1P2, LGALS3BP, MBL2, MMP2, TGFB2 (combinaison n°8) ; ou
- G1P2, LGALS3BP, MBL2, SFN, TGFB2 (combinaison n°10) ; ou
- CXCL6, CXCL10, G1P2, MBL2, MMP2 (combinaison n°11) ; ou
- CXCL6, CXCL11, G1P2, MBL2, MMP2 (combinaison n°12) ; ou
- FGF7, G1P2, LGALS3BP, MBL2, TGFB2 (combinaison n°13) ; ou
- AFP, CXCL6, G1P2, IL8, MDK (combinaison n°14) ; ou
- CCL21, G1P2, LGALS3BP, MBL2, SFN (combinaison n°16) ; ou
- CXCL10, G1P2, LGALS3BP, MBL2, TGFB2 (combinaison n°17) ; ou
- CRP, CXCL6, G1P2, MBL2, SFN (combinaison n°18) ; ou
- CXCL10, CXCL11, G1P2, MBL2, MMP2 (combinaison n°19) ; ou
- CXCL11, G1P2, LGALS3BP, MBL2, MDK (combinaison n°20) ; ou
- G1P2, IL8, LGALS3BP, MBL2, TGFB2 (combinaison n°21) ; ou
- FGF7, G1P2, IL8, MDK, SFN (combinaison n°22) ; ou
- CCL21, FGF7, LGALS3BP, MBL2, MDK (combinaison n°23) ; ou
- CCL21, CXCL6, IL8, LGALS3BP, MDK (combinaison n°25) ; ou
- CCL21, FGF7, MBL2, MDK, VEGFD (combinaison n°26) ; ou
- CXCL6, IL8, CCL21, G1P2, MDK (combinaison n°30) ; ou
- CXCL6, IL8, CXCL10, G1P2, MDK (combinaison n°33) ; ou
- CCL21, CXCL10, G1P2, LGALS3BP, MDK (combinaison n°34) ; ou
- CXCL6, IL8, CCL21, G1P2, LGALS3BP (combinaison n°35) ; ou
- IL8, CXCL10, G1P2, LGALS3BP, MDK (combinaison n°38) ; ou
- CXCL6, IL8, G1P2, LGALS3BP, MDK (combinaison n°39) ; ou
- FGF7, G1P2, LGALS3BP, MBL2, MDK (combinaison n°27) ; ou
- CXCL10, FGF7, IL8, MDK, VEGFD (combinaison n°28) ; ou
- CCL21, CXCL6, CXCL10, LGALS3BP, MDK (combinaison n°29) ; ou
- IL8, CCL21, G1P2, LGALS3BP, MDK (combinaison n°31) ; ou
- IL8, CCL21, CXCL10, G1P2, MDK (combinaison n°32) ; ou
- CXCL6, IL8, CXCL10, G1P2, LGALS3BP (combinaison n°36) ; ou
- CXCL6, IL8, CCL21, CXCL10, G1P2 (combinaison n°40) ; ou
- CXCL6, CXCL10, G1P2, LGALS3BP, MDK (combinaison n°41) ; ou
- CXCL6, IL8, CCL21, CXCL10, LGALS3BP (combinaison n°42) ; ou
- CXCL6, CCL21, CXCL10, G1P2, LGALS3BP (combinaison n°43).

6. La méthode selon l'une quelconque des revendications 1 à 5, dans laquelle, à l'étape i), on dose les niveaux de traduction desdits gènes choisis.

7. La méthode selon la revendication 2, dans laquelle :

- ledit ou lesdits facteurs cliniques sont choisis parmi les facteurs suivants : sexe, âge à la date du prélèvement, âge du patient à la date de la contamination, âge du patient à la date du début du traitement, indice de masse corporelle, indice de sensibilité à l'insuline, diabète, consommation d'alcool, degré de stéatose, mode de contamination, activité Metavir, score de fibrose hépatique mesuré selon le système Metavir (score F Metavir) ou selon le système Ishak ; et/ou
- ledit ou lesdits facteurs virologiques sont choisis parmi les facteurs suivants : génotype viral, durée de l'infection, charge virale avant traitement, charge virale mesurée chez le patient à la date du début du traitement, charge virale mesurée chez le patient à la date du prélèvement ; et/ou
- ledit ou lesdits facteurs biologiques autres que les niveaux d'expression de gènes choisis parmi MBL2, LGALS3BP, IL8, G1P2, CXCL10, CCL21, AFP, CRP, CXCL11, CXCL6, CXCL9, FGF7, MDK, MMP2, SFN, TGFB2, VEGFD sont choisis parmi les facteurs suivants : concentration en haptoglobine, concentration en apolipoprotéine A1, teneur en bilirubine totale, concentration en gamma glutamyl transpeptidase, concentration en aspartate aminotransférase, concentration en alanine aminotransférase, teneur en plaquettes, taux de prothrombine, teneur en cholestérol HDL, teneur en cholestérol total, concentration en ferritine, teneur glycémique, concentration en peptide C, teneur en insuline, concentration en triglycérides, teneur en albumine, coefficient de saturation en fer de la transferrine, concentration en phosphatase alcaline,

et/ou
dans laquelle :

- ledit ou lesdits facteurs cliniques comprennent le score de fibrose hépatique mesuré selon le système Metavir (score F Metavir) ou selon le système Ishak, et/ou âge à la date du prélèvement (Age), par exemple, âge à la date de la PBH, âge à la date de la cytoponction hépatique, âge à la date du prélèvement de sang, de sérum, de plasma ou d'urine, et/ou indice de masse corporelle (IMC) ; et/ou
- ledit ou lesdits facteurs virologiques comprennent le génotype viral et/ou la charge virale avant traitement ; et/ou
- ledit ou lesdits facteurs biologiques autres que les niveaux d'expression de gènes choisis parmi MBL2, LGALS3BP, IL8, G1P2, CXCL10, CCL21, AFP, CRP, CXCL11, CXCL6, CXCL9, FGF7, MDK, MMP2, SFN, TGFB2, VEGFD comprennent la concentration en gamma glutamyl transpeptidase et/ou la concentration en phosphatase alcaline et/ou la concentration en alanine aminotransférase et/ou la concentration en aspartate aminotransférase.

8. La méthode selon la revendication 7, qui comprend :

- le fait de déterminer si le score de fibrose hépatique dudit sujet est un score qui, selon le système de score Metavir, est d'au moins F1, plus particulièrement d'au moins F2 ; et/ou
- le fait de déterminer si le ou les VHC dont est infecté ledit sujet comprennent un VHC de génotype 1, 4, 5 ou 6, plus particulièrement de génotype 1 ou 4.

9. La méthode selon l'une quelconque des revendications 1 à 8, dans laquelle ledit échantillon préalablement obtenu à partir dudit sujet est :

- un échantillon de fluide biologique intracorporel préalablement prélevé dudit sujet, tel qu'un échantillon de sang, de sérum, de plasma, ou un échantillon d'urine dudit sujet, ou
- un échantillon contenant des protéines et/ou polypeptides et/ou peptides extraits ou purifiés dudit échantillon biologique.

10. **Article manufacturé** comprenant des réactifs en préparation combinée pour leur utilisation simultanée, séparée ou étalée dans le temps, lesdits réactifs étant constitués de réactifs qui détectent de manière spécifique chacun des produits de transcription ou de traduction de 2 à 9 gènes humains, lesdits 2 à 9 gènes humains comprenant lesdits gènes choisis conformément à l'étape i) de l'une quelconque des revendications 1 à 9,
dans lequel lesdits réactifs sont des acides nucléiques qui s'hybrident de manière spécifique à l'ARN desdits gènes choisis conformément à l'une quelconque des revendications 1 à 9, et/ou à l'ADNc obtenu par transcription inverse de ces ARN, ou sont des protéines, polypeptides ou peptides qui se lient de manière spécifique aux protéines codées par lesdits gènes choisis, et en particulier dans lequel lesdits réactifs sont des amorces d'amplification et/ou des sondes acides nucléiques, ou sont des anticorps ou fragments d'anticorps ou des aptamères protéiques, polypeptidiques ou peptidiques,
et
dans lequel lesdits réactifs sont contenus dans un ou des tubes, ou dans les puits d'une plaque d'amplification d'acide nucléique destinée à recevoir un échantillon contenant des acides nucléiques et un mélange réactionnel d'amplification d'acide nucléique, ou dans les puits d'une plaque de titrage protéique, plus particulièrement une plaque de microtitrage protéique, par exemple une plaque ELISA, ou sur des microbilles ou sur une puce pour la détection et la quantification d'acides nucléiques, protéines, polypeptides ou peptides.

11. Article manufacturé selon la revendication 10 pour mettre en oeuvre la méthode de la revendication 7, comprenant en outre d'autres réactifs en préparation combinée pour leur utilisation simultanée, séparée ou étalée dans le temps, lesdits autres réactifs permettant de détecter, doser ou déterminer :

- un ou plusieurs facteur(s) virologique(s) choisi(s) parmi les facteurs suivants: génotype viral, durée de l'infection, charge virale avant traitement, charge virale mesurée chez le patient à la date du début du traitement, charge virale mesurée chez le patient à la date du prélèvement ; et/ou
- un ou plusieurs facteur(s) biologique(s) autre(s) que les niveaux d'expression de gènes choisis parmi MBL2, LGALS3BP, IL8, G1P2, CXCL10, CCL21, AFP, CRP, CXCL11, CXCL6, CXCL9, FGF7, MDK, MMP2, SFN, TGFB2, VEGFD, tel(s) qu'un ou plusieurs facteur(s) biologique(s) choisi(s) parmi: concentration en haptoglobine, concentration en apolipoprotéine A1, teneur en bilirubine totale, concentration en gamma glutamyl transpeptidase, concentration en aspartate aminotransférase, concentration en alanine aminotransférase, teneur en plaquettes, taux de prothrombine, teneur en cholestérol HDL, teneur en cholestérol total, concentration en ferritine, teneur glycémique, concentration en peptide C, teneur en insuline, concentration en triglycérides, teneur en albumine, coefficient de saturation en fer de la transferrine, concentration en phosphatase alcaline, dans lequel lesdits réactifs sont des acides nucléiques qui s'hybrident de manière spécifique à l'ARN desdits gènes choisis conformément à l'une quelconque des revendications 1 à 10, et/ou à l'ADNc obtenu par transcription inverse de ces ARN, ou sont des protéines, polypeptides ou peptides qui se lient de manière spécifique aux protéines codées par lesdits gènes choisis, et en particulier dans lequel lesdits réactifs sont des amorces d'amplification et/ou des sondes acides nucléiques, ou sont des anticorps ou fragments d'anticorps ou des aptamères protéiques, polypeptidiques ou peptidiques,

et

dans lequel lesdits réactifs sont contenus dans un ou des tubes, ou dans les puits d'une plaque d'amplification d'acide nucléique destinée à recevoir un échantillon contenant des acides nucléiques et un mélange réactionnel d'amplification d'acide nucléique, ou dans les puits d'une plaque de titrage protéique, plus particulièrement une plaque de microtitrage protéique, par exemple une plaque ELISA, ou sur des microbilles ou sur une puce pour la détection et la quantification d'acides nucléiques, protéines, polypeptides ou peptides.

12. **Utilisation d'un article manufacturé** selon la revendication 10 ou 11, pour la mise en oeuvre d'une méthode selon l'une quelconque des revendications 1 à 9.

**Patentansprüche**

1. In-Vitro-Verfahren zur Prognose vor der Behandlung, ob ein oder mehrere HCVinfizierter Patient eine hohe Wahrscheinlichkeit hat, auf eine Anti-HCV-Behandlung anzusprechen, die die Verabreichung von Interferon und die Verabreichung von Ribavirin oder einer Prodrug von Ribavirin umfasst, oder ob im Gegenteil dieser Patient eine hohe Wahrscheinlichkeit hat, nicht auf diese Anti-HCV-Behandlung anzusprechen, wobei das Verfahren die folgenden Schritte umfasst:

i) Durchführen folgender Maßnahmen:

- Zumessen der Niveaus, bei denen ausgewählte Säugergene transkribiert bzw. translatiert werden, in einer Probe, die zuvor von dem Patienten gewonnen wurde, wobei die ausgewählten Säugergene, deren Transkriptions- bzw. Translationsniveaus zugemessen werden, die folgende Genkombination sind:

(a)

- zumindest ein Gen aus MBL2, LGALS3BP und IL8,
und
- zumindest ein Gen aus G1P2, CCL21 und CXCL10,
wobei die Gesamtzahl der so ausgewählten Gene 2, 3, 4 oder 5 ist,
oder

(b)

- zumindest ein Gen aus MBL2, LGALS3BP und IL8,
und
- zumindest ein Gen aus G1P2, CCL21 und CXCL10,
und
- zumindest ein Gen aus AFP, CRP, CXCL11, CXCL6, CXCL9, FGF7, MDK, MMP2, SFN, TGFB2 und VEGFD,
wobei die Gesamtzahl der so ausgewählten Gene 3, 4 oder 5 ist, und

- Zumessen der Transkriptions- bzw. Translationsniveaus von 0 bis 4 anderen Säugergenen als den Säugergenen, die ausgewählt sind aus MBL2, LGALS3BP, IL8, G1P2, CXCL10, CCL21, AFP, CRP, CXCL11, CXCL6, CXCL9, FGF7, MDK, MMP2, SFN, TGFB2, VEGFD, und

ii) Vergleichen der für den Patienten in Schritt i) erhaltenen Messwerte mit ihren Werten bzw. mit der Verteilung ihrer Werte in vordefinierten Referenzkohorten entsprechend ihrem Status des Ansprechens bzw. Nicht-Ansprechens auf die Anti-HCV-Behandlung, um den Patienten in diejenige dieser Referenzkohorten einzuordnen, gegenüber welcher er die höchste Zugehörigkeitswahrscheinlichkeit hat, wobei die von dem Patienten gewonnene Probe entnommen bzw. erhalten wird aus: Leber, Leberparenchym, Blut, Serum, Plasma oder Urin.

2. Verfahren nach Anspruch 1, wobei der Schritt i) ferner einen Schritt des Messens bzw. Bestimmens von dem Wert eines oder mehrerer anderer klinischer Faktoren und/oder eines oder mehrerer anderer virologischer Faktoren und/oder eines oder mehrerer anderer biologischer Faktoren als die Genexpressionsniveaus der Gene, ausgewählt aus MBL2, LGALS3BP, IL8, G1P2, CXCL10, CCL21, AFP, CRP, CXCL11, CXCL6, CXCL9, FGF7, MDK, MMP2, SFN, TGFB2, VEGFD, für den Patienten umfasst.

3. Verfahren nach Anspruch 1 oder 2, wobei der Vergleich von Schritt ii) durch Kombination der in Schritt i) für den Patienten erhaltenen Messwerte in einem multivariaten Klassifikationsmodell erfolgt, das diese Werte mit ihren Werten bzw. der Verteilung ihrer Werte in vordefinierten Referenzkohorten je nach deren Ansprech- bzw. Nichtansprech-Status vergleicht, um den Patienten in diejenige dieser Referenzkohorten einzuteilen, gegenüber welcher er die höchste Zugehörigkeitswahrscheinlichkeit hat,
und/oder
wobei der Vergleich von Schritt ii) durch Kombination der in Schritt i) für den Patienten erhaltenen Messwerte in einem multivariaten Klassifikationsmodell erfolgt, das zuvor wie folgt erstellt wird:

a) bei einer Population von Personen, die von derselben Spezies wie der Patient sind und die mit ein oder mehrere HCV infiziert sind, wird für jede dieser Personen bestimmt, ob sie auf eine Anti-HCV-Behandlung anspricht oder nicht, die die Verabreichung von Interferon und Ribavirin umfasst, und diese Personen werden in Subpopulationen eingeteilt, die sich je nachdem, ob sie auf diese Behandlung ansprechen oder nicht, unterscheiden und somit Referenzkohorten darstellen, die nach Ansprechen oder Nichtansprechen dieser Personen auf die Anti-HCV-Behandlung erstellt werden;
b) bei zumindest einer Probe, die zuvor von jeder dieser Personen gewonnen wurde und deren Beschaffenheit identisch zu der der Probe des Patienten ist, werden die gleichen Maßnahmen ergriffen, die für den Patienten in Schritt i) erfolgt sind;
c) es wird ein Kohortenvergleich der Werte der in Schritt b) erhaltenen Messwerte oder der Verteilung dieser Werte durchgeführt, um ein multivariantes Klassifikationsmodell zu erstellen, das einen Ansprech- bzw. Nichtansprech-Status auf die Anti-HCV-Behandlung angibt, und zwar ausgehend von der Kombination dieser in Schritt b) erhaltenen Messwerte,

und/oder
wobei der Vergleich von Schritt ii) durch Kombination der in Schritt i) erhaltenen Messwerte in einer mathematischen Funktion, insbesondere in einer linearen oder nicht linearen, insbesondere in einer linearen Funktion erfolgt, um einen Ausgangswert zu erhalten, der den Ansprech- bzw. Nichtansprechstatus des Patienten angibt,
und/oder
wobei der Vergleich von Schritt ii) durch Kombination der in Schritt i) erhaltenen Messwerte in einem multivariaten maschinellen Lernmodell, beispielsweise einem multivariaten nichtparametrischen Klassifikationsmodell, einem multivariaten heuristischen Modell oder einem multivariaten probabilistischen Vorhersagemodell erfolgt, um einen Ausgangswert zu erhalten, der den Ansprech- bzw. Nichtansprech-Status des Patienten angibt.

4. Verfahren nach einem der Ansprüche 1 bis 3, wobei die Einteilung des Patienten in diejenige der Referenzkohorten, gegenüber welcher er die höchste Zugehörigkeitswahrscheinlichkeit hat, erfolgt mit:

- einer Sensitivität (Se) von zumindest 70 %, von zumindest 71 %, von zumindest 72 %, von zumindest 73 %, oder von zumindest 74 %, oder von zumindest 75 %, oder von zumindest 76 %, oder von zumindest 77 %, oder von zumindest 78 %, oder von zumindest 79 %, oder von zumindest 80 %, oder von zumindest 81 %, oder von zumindest 82 %; und/oder mit
- einer Spezifität (Sp) von zumindest 70 %, von zumindest 71 %, von zumindest 72 %, von zumindest 73 %, oder von zumindest 74 %, oder von zumindest 75 %, oder von zumindest 76 %, oder von zumindest 77 %, oder von zumindest 78 %, oder von zumindest 79 %, oder von zumindest 80 %, oder von zumindest 81 %, oder von zumindest 82 %, oder von zumindest 83 %, oder von zumindest 84 %, oder von zumindest 85 %, oder von zumindest 86 %, oder von zumindest 87 %, oder von zumindest 88 %, oder von zumindest 89 %, oder von zumindest 90 %, oder von zumindest 91 %, oder von zumindest 92 %, oder von zumindest 92 %; und/oder mit
- einem negativen Vorhersagewert (VPN) von zumindest 78 %, oder von zumindest 79 %, oder von zumindest 80 %, oder von zumindest 81 %, oder von zumindest 82 %, oder von zumindest 83 %, oder von zumindest 84 %, oder von zumindest 85 %, oder von zumindest 86 %, oder von zumindest 87 %, oder von zumindest 88 %; und/oder mit
- einem positiven Vorhersagewert (VPP) von zumindest 63 %, oder von zumindest 64 %, oder von zumindest 65 %, oder von zumindest 66 %, oder von zumindest 67 %, oder von zumindest 68 %, oder von zumindest 69 %, oder von zumindest 70 %, oder von zumindest 71 %, oder von zumindest 72 %, oder von zumindest 73 %, oder von zumindest 74 %, oder von zumindest 75 %, oder von zumindest 76 %, oder von zumindest 77 %, oder von zumindest 78 %, oder von zumindest 79 %, oder von zumindest 80 %, oder von zumindest 81 %, oder von zumindest 82 %, oder von zumindest 83 %, oder von zumindest 84 %, oder von zumindest 85 %, oder von zumindest 86 %, oder von zumindest 87 %, oder von zumindest 88 %, oder von zumindest 89, oder von zumindest 90 %, oder von zumindest 91 %, oder von zumindest 92 %, oder von zumindest 93 %, oder von zumindest 94 %,

und/oder
wobei das multivariante Klassifikationsmodell aufweist:

- ein Cut-Off in der ROC-Kurve (AUC) von zumindest 0,76, von zumindest 0,77, von zumindest 0,78, insbesondere von zumindest 0,79, besonders bevorzugt von zumindest 0,80, von zumindest 0,81, von zumindest 0,82, von zumindest 0,83, von zumindest 0,84, insbesondere von zumindest 0,85, besonders bevorzugt von zumindest 0,86, noch bevorzugter von zumindest 0,87, und/oder
- einen LOOCV-Fehler von höchstens 18 %, von höchstens 17 %, von höchstens 16 %, von höchstens 15 %, von höchstens 14 %, von höchstens 13 %, von höchstens 12 %.

5.   Verfahren nach einem der Ansprüche 1 bis 4, wobei in Schritt i)

   • die Gene, die ausgewählt sind aus:

      (a)

         - zumindest einem Gen aus MBL2, LGALS3BP, IL8, und
         - zumindest einem Gen aus G1P2, CCL21, CXCL10,

   sind:

      - LGALS3BP und CXCL10 (Kombination Nr. 15); oder
      - IL8, CCL21, CXCL10, G1P2, LGALS3BP (Kombination Nr. 37);

   oder
   • die Gene, die ausgewählt sind aus:
   (b)

      - zumindest einem Gen aus MBL2, LGALS3BP, IL8 und
      - zumindest einem Gen aus G1P2, CCL21, CXCL10 und
      - zumindest einem Gen aus AFP, CRP, CXCL11, CXCL6, CXCL9, FGF7, MDK, MMP2, SFN, TGFB2, VEGFD,

   sind:

      - LGALS3BP, CXCL10 und MDK (Kombination Nr. 9); oder
      - LGALS3BP, IL8, CXCL10, CCL21 und MDK (Kombination Nr. 24); oder
      - CRP, G1P2, LGALS3BP, MBL2, TGFB2 (Kombination Nr. 1); oder
      - AFP, CXCL6, CXCL9, G1P2, MBL2 (Kombination Nr. 2); oder
      - AFP, FGF7, G1P2, MBL2, MMP2 (Kombination Nr. 3); oder
      - CXCL11, G1P2, IL8, MBL2, TGFB2 (Kombination Nr. 4); oder

- G1P2, IL8, MBL2, SFN, TGFB2 (Kombination Nr. 5); oder
- CCL21, FGF7, IL8, LGALS3BP, MBL2 (Kombination Nr. 6); oder
- G1P2, LGALS3BP, MBL2, MDK, TGFB2 (Kombination Nr. 7); oder
- G1P2, LGALS3BP, MBL2, MMP2, TGFB2 (Kombination Nr. 8); oder
- G1P2, LGALS3BP, MBL2, SFN, TGFB2 (Kombination Nr. 10); oder
- CXCL6, CXCL10, G1P2, MBL2, MMP2 (Kombination Nr. 11); oder
- CXCL6, CXCL11, G1P2, MBL2, MMP2 (Kombination Nr. 12); oder
- FGF7, G1P2, LGALS3BP, MBL2, TGFB2 (Kombination Nr. 13); oder
- AFP, CXCL6, G1P2, IL8, MDK (Kombination Nr. 14); oder
- CCL21, G1P2, LGALS3BP, MBL2, SFN (Kombination Nr. 16); oder
- CXCL10, G1P2, LGALS3BP, MBL2, TGFB2 (Kombination Nr. 17); oder
- CRP, CXCL6, G1P2, MBL2, SFN (Kombination Nr. 18); oder
- CXCL10, CXCL11, G1P2, MBL2, MMP2 (Kombination Nr. 19); oder
- CXCL11, G1P2, LGALS3BP, MBL2, MDK (Kombination Nr. 20); oder
- G1P2, IL8, LGALS3BP, MBL2, TGFB2 (Kombination Nr. 21); oder
- FGF7, G1P2, IL8, MDK, SFN (Kombination Nr. 22); oder
- CCL21, FGF7, LGALS3BP, MBL2, MDK (Kombination Nr. 23); oder
- CCL21, CXCL6, IL8, LGALS3BP, MDK (Kombination Nr. 25); oder
- CCL21, FGF7, MBL2, MDK, VEGFD (Kombination Nr. 26); oder
- CXCL6, IL8, CCL21, G1P2, MDK (Kombination Nr. 30); oder
- CXCL6, IL8, CXCL10, G1P2, MDK (Kombination Nr. 33); oder
- CCL21, CXCL10, G1P2, LGALS3BP, MDK (Kombination Nr. 34); oder
- CXCL6, IL8, CCL21, G1P2, LGALS3BP (Kombination Nr. 35); oder
- IL8, CXCL10, G1P2, LGALS3BP, MDK (Kombination Nr. 38); oder
- CXCL6, IL8, G1P2, LGALS3BP, MDK (Kombination Nr. 39); oder
- FGF7, G1P2, LGALS3BP, MBL2, MDK (Kombination Nr. 27); oder
- CXCL10, FGF7, IL8, MDK, VEGFD (Kombination Nr. 28); oder
- CCL21, CXCL6, CXCL10, LGALS3BP, MDK (Kombination Nr. 29); oder
- IL8, CCL21, G1P2, LGALS3BP, MDK (Kombination Nr. 31); oder
- IL8, CCL21, CXCL10, G1P2, MDK (Kombination Nr. 32); oder
- CXCL6, IL8, CXCL10, G1P2, LGALS3BP (Kombination Nr. 36); oder
- CXCL6, IL8, CCL21, CXCL10, G1P2 (Kombination Nr. 40); oder
- CXCL6, CXCL10, G1P2, LGALS3BP, MDK (Kombination Nr. 41); oder
- CXCL6, IL8, CCL21, CXCL10, LGALS3BP (Kombination Nr. 42); oder
- CXCL6, CCL21, CXCL10, G1P2, LGALS3BP (Kombination Nr. 43).

6. Verfahren nach einem der Ansprüche 1 bis 5, wobei in Schritt i) die Translationsniveaus der ausgewählten Gene zugemessen werden.

7. Verfahren nach Anspruch 2, wobei

- der bzw. die klinischen Faktoren ausgewählt werden aus den nachfolgenden Faktoren: Geschlecht, Alter zum Zeitpunkt der Probenahme, Alter des Patienten zum Zeitpunkt der Infizierung, Alter des Patienten zu Beginn der Behandlung, Body-Mass-Index, Insulin-Sensitivitäts-Index, Diabetes, Alkoholkonsum, Steatosegrad, Übertragungsweg, Metavir-Aktivität, Leberfibrose-Score, gemessen nach dem Metavir-System (Metavir-F-Score) oder nach dem Ishak-System; und/oder
- der bzw. die virologischen Faktoren ausgewählt werden aus den folgenden Faktoren: viraler Genotyp, Infektionsdauer, Viruslast vor der Behandlung, beim Patienten gemessene Viruslast zu Beginn der Behandlung, beim Patienten gemessene Viruslast zum Zeitpunkt der Probenahme; und/oder
- der bzw. die anderen biologischen Faktoren als die Genexpressionsniveaus der Gene, ausgewählt aus MBL2, LGALS3BP, IL8, G1P2, CXCL10, CCL21, AFP, CRP, CXCL11, CXCL6, CXCL9, FGF7, MDK, MMP2, SFN, TGFB2, VEGFD, ausgewählt werden aus den folgenden Faktoren: Konzentration an Haptoglobin, Konzentration an Apolipoprotein A1, Gesamtbilirubingehalt, Konzentration an Gamma-Glutamyltranspeptidase, Konzentration an Aspartataminotransferase, Konzentration an Alaninaminotransferase, Thrombozytengehalt, Prothrombingehalt, HDL-Cholesteringehalt, Gesamtcholesteringehalt, Ferritin-Konzentration, glykämischer Gehalt, Peptid-C-Konzentration, Insulingehalt, Triglyceridkonzentration, Albumingehalt, Transferrin-Eisensättigungsfaktor, Konzentration an alkalischer Phosphatase, und/oder

wobei

- der bzw. die klinischen Faktoren den Leberfibrose-Score, gemessen nach dem Metavir-System (F Metavir-Score) oder nach dem Ishak-System, und/oder das Alter zum Zeitpunkt der Entnahme (Age), z. B. das Alter zum Zeitpunkt der PBH, das Alter zum Zeitpunkt der Leberzytopunktur, das Alter zum Zeitpunkt der Entnahme von Blut, Serum, Plasma oder Urin und/oder den Body-Mass-Index (BMI) umfassen; und/oder
- der bzw. die virologischen Faktoren den viralen Genotyp und/oder die Viruslast vor der Behandlung umfassen; und/oder
- der bzw. die anderen biologischen Faktoren als die Genexpressionsniveaus, ausgewählt aus MBL2, LGALS3BP, IL8, G1P2, CXCL10, CCL21, AFP, CRP, CXCL11, CXCL6, CXCL9, FGF7, MDK, MMP2, SFN, TGFB2, VEGFD, die Konzentration an Gammaglutamyl-Transpeptidase und/oder die Konzentration an alkalischer Phosphatase und/oder die Konzentration an Alaninaminotransferase und/oder die Konzentration an Aspartataminotransferase umfassen.

8. Verfahren nach Anspruch 7, wobei es umfasst:

- Bestimmen, ob der Leberfibrose-Score des Patienten ein Score ist, der nach dem Metavir-Score-System zumindest F1, insbesondere zumindest F2, beträgt; und/oder
- Bestimmen, ob das bzw. die HCV, womit der Patient infiziert ist, ein HCV des Genotyps 1, 4, 5 oder 6, insbesondere des Genotyps 1 oder 4, enthält/enthalten.

9. Verfahren nach einem der Ansprüche 1 bis 8, wobei die zuvor von dem Patienten gewonnene Probe

- eine Probe intrakorporaler biologischer Flüssigkeit ist, die zuvor von dem Patienten entnommen wurde, wie etwa eine Probe von Blut, Serum, Plasma oder eine Urinprobe des Patienten, oder
- eine Probe ist, die Proteine und/oder Polypeptide oder Peptide enthält, die aus der biologischen Probe extrahiert oder aufgereinigt wurden.

10. Fertigungsgegenstand, enthaltend Reagenzien in einer kombinierten Zubereitung zur gleichzeitigen, getrennten oder zeitlich gestaffelten Verwendung, wobei die Reagenzien aus Reagenzien bestehen, die jedes der Transkriptions- bzw. Translationsprodukte von 2 bis 9 menschlichen Genen spezifisch nachweisen, wobei die 2 bis 9 menschlichen Gene die gemäß Schritt i) nach einem der Ansprüche 1 bis 9 ausgewählten Gene umfassen, wobei die Reagenzien Nukleinsäuren sind, die spezifisch mit der RNA der nach einem der Ansprüche 1 bis 9 ausgewählten Gene und/oder mit der durch reverse Transkription dieser RNAs erhaltenen cDNA hybridisieren, oder Proteine, Polypeptide oder Peptide sind, die sich auf spezifische Weise mit den durch die ausgewählten Gene codierten Proteinen binden, und wobei insbesondere die Reagenzien Amplifikationsprimer und/oder Nukleinsäuresonden oder Antikörper oder Antikörperfragmente oder Protein-, Polypeptid- oder Peptid-Aptamere sind, und wobei die Reagenzien in einem oder mehreren Röhrchen oder in den Vertiefungen einer Nukleinsäureamplifikationsplatte zur Aufnahme einer Probe, die Nukleinsäuren und ein Nukleinsäureamplifikationsreaktionsgemisch enthält, oder in den Vertiefungen einer Protein-Titrationsplatte, insbesondere einer Protein-Mikrotiterplatte, beispielsweise einer ELISA-Platte, oder auf Mikrokügelchen oder auf einem Biochip zum Nachweis und zur Quantifizierung von Nukleinsäuren, Proteinen, Polypeptiden oder Peptiden enthalten sind.

11. Fertigungsgegenstand nach Anspruch 10 zum Durchführen des Verfahrens nach Anspruch 7, ferner enthaltend weitere Reagenzien in kombinierter Zubereitung zur gleichzeitigen, getrennten oder zeitlich gestaffelten Verwendung, wobei die weiteren Reagenzien das Nachweisen, Zumessen bzw. Bestimmen gestatten von

- einem bzw. mehreren virologischen Faktoren, ausgewählt aus den folgenden Faktoren: viraler Genotyp, Infektionsdauer, Viruslast vor der Behandlung, beim Patienten gemessene Viruslast zum Zeitpunkt des Beginns der Behandlung, beim Patienten gemessene Viruslast zum Zeitpunkt der Probenahme; und/oder
- einem bzw. mehreren anderen biologischen Faktoren als die Genexpressionsniveaus der Gene, ausgewählt aus MBL2, LGALS3BP, IL8, G1P2, CXCL10, CCL21, AFP, CRP, CXCL11, CXCL6, CXCL9, FGF7, MDK, MMP2, SFN, TGFB2, VEGFD, wie etwa einem oder mehreren biologischen Faktoren, ausgewählt aus: Haptoglobinkonzentration, Konzentration an Apolipoprotein A1, Gesamtbilirubingehalt, Konzentration an Gamma-Glutamyltranspeptidase, Konzentration an Aspartataminotransferase, Konzentration an Alaninaminotransferase, Thrombozytengehalt, Prothrombingehalt, HDL-Cholesteringehalt, Gesamtcholesteringehalt, Ferritin-Konzentration, glykämischer Gehalt, Peptid-C-Konzentration, Insulingehalt, Triglyceridkonzentration, Albumingehalt, Transferrin-Eisensättigungsfaktor, Konzentration an alkalischer Phosphatase,

wobei die Reagenzien Nukleinsäuren sind, die spezifisch mit der RNA der nach einem der Ansprüche 1 bis 10 ausgewählten Gene und/oder mit der durch reverse Transkription dieser RNAs erhaltenen cDNA hybridisieren, oder Proteine, Polypeptide oder Peptide sind, die sich auf spezifische Weise mit den durch die ausgewählten Gene codierten Proteinen binden, und wobei insbesondere die Reagenzien Amplifikationsprimer und/oder Nukleinsäuresonden oder Antikörper oder Antikörperfragmente oder Protein-, Polypeptid- oder Peptid-Aptamere sind, und wobei die Reagenzien in einem oder mehreren Röhrchen oder in den Vertiefungen einer Nukleinsäureamplifikationsplatte zur Aufnahme einer Probe, die Nukleinsäuren und ein Nukleinsäureamplifikationsreaktionsgemisch enthält, oder in den Vertiefungen einer Protein-Titrationsplatte, insbesondere einer Protein-Mikrotiterplatte, beispielsweise einer ELISA-Platte, oder auf Mikrokügelchen oder auf einem Biochip zum Nachweis und zur Quantifizierung von Nukleinsäuren, Proteinen, Polypeptiden oder Peptiden enthalten sind.

**12.** Verwendung eines Fertigungsgegenstands nach Anspruch 10 oder 11 zum Durchführen eines Verfahrens nach einem der Ansprüche 1 bis 9.

**Claims**

**1.** An *in vitro* **method** for predicting, prior to treatment, whether a subject infected with one or more HCVs has a high probability of being a responder to an anti-HCV treatment which will comprise the administration of interferon and the administration of ribavirin or a prodrug of ribavirin or whether, in contrast, that subject has a high probability of not responding to that anti-HCV treatment,
said method comprising the following steps:

i) making the following measurements:

- in a sample which has already been obtained from said subject, measuring the levels to which selected mammalian genes have been transcribed or translated, said selected mammalian genes the levels of transcription or translation of which have been measured being a combination of the following genes:

(a)

- at least one gene from among MBL2, LGALS3BP and IL8,
and
- at least one gene from among G1P2, CCL21 and CXCL10,
the total number of the genes selected thereby being 2, 3, 4 or 5,
or

(b)

- at least one gene from among MBL2, LGALS3BP and IL8,
and
- at least one gene from among G1P2, CCL21 and CXCL10,
and
- at least one gene from among AFP, CRP, CXCL11, CXCL6, CXCL9, FGF7, MDK, MMP2, SFN, TGFB2 and VEGFD,
the total number of the genes selected thereby being 3, 4 or 5, and

- measuring the levels of transcription or translation from 0 to 4 mammalian genes other than the mammalian genes selected from MBL2, LGALS3BP, IL8, G1P2, CXCL10, CCL21, AFP, CRP, CXCL11, CXCL6, CXCL9, FGF7, MDK, MMP2, SFN, TGFB2, VEGFD, and

ii) comparing the values for the measurements obtained for said subject in step i) with their values, or the distribution of their values, in reference cohorts which have been pre-established as a function of their status of responder or non-responder to anti-HCV treatment, in order to classify said subject into that of those reference cohorts to which it has the highest probability of belonging, and wherein the sample obtained from said subject has been collected or removed from: liver, hepatic parenchyma, blood, serum, plasma or urine.

**2.** The method according to claim 1, **characterized in that** step (i) further comprises a step of measuring or determining, for said subject, the value of one or more clinical factors and/or of one or more virological factors and/or of one or more biological factors other than the levels of expression of genes selected from MBL2, LGALS3BP, IL8, G1P2, CXCL10, CCL21, AFP, CRP, CXCL11, CXCL6, CXCL9, FGF7, MDK, MMP2, SFN, TGFB2, VEGFD.

3. The method according to claim 1 or 2, in which the comparison of step ii) is carried out by combining the measurement values obtained for said subject in step i) into a multivariate classification model which compares those values with their values, or the distribution of their values, in reference cohorts which have been pre-established as a function of their status of responder or non-responder, in order to classify said subject into that of those reference cohorts to which it has the highest probability of belonging,
and/or
in which the comparison of step ii) is made by combining measurement values obtained for said subject in step i) into a pre-constructed multivariate classification model as follows:

   a) for a population of individuals who are of the same species as said subject, and who are infected with one or more HCVs, determining for each of those individuals whether or not that individual responds to an anti-HCV treatment which comprises the administration of interferon and of ribavirin, and classifying those individuals into distinct sub-populations as a function of whether they are responders or whether they are non-responders to that treatment, thus constituting reference cohorts established as a function of the response or non-response of those individuals to anti-HCV treatment;
   b) in at least one sample which has previously been obtained from each of said individuals, the nature of which is identical to that of the sample from said subject, making the same measurements as those carried out for said subject in said step i);
   c) making an inter-cohort comparison of the values for the measurements obtained in step b), or of the distribution of those values, in order to construct a multivariate classification model which infers a status of responder to anti-HCV treatment or a status of non-responder to that treatment, starting from the combination of said values for the measurements obtained in step b),
   and/or

in which the comparison of step ii) is made by combining said measurement values obtained in step i) into a mathematical function, in particular a linear or non-linear function, more particularly a linear function, in order to obtain an output value which is indicative of the status of responder or of non-responder of said subject, and/or
in which the comparison of step ii) is made by combining said values obtained in step i) into a multivariate machine learning model, for example a multivariate non-parametric classification model, a multivariate heuristic model, or a multivariate probabilistic prediction model, in order to obtain an output value which is indicative of the status of responder or of non-responder of said subject.

4. The method according to any one of claims 1 to 3, in which the classification of said subject into that of said reference cohorts to which it has the highest probability of belonging is made with:

   - a sensitivity (Se) of at least 70%, at least 71%, at least 72%, at least 73%, or at least 74%, or at least 75%, or at least 76%, or at least 77%, or at least 78%, or at least 79%, or at least 80%, or at least 81%, or at least 82%; and/or with a
   - specificity (Sp) of at least 70%, at least 71%, at least 72%, at least 73%, or at least 74%, or at least 75%, or at least 76%, or at least 77%, or at least 78%, or at least 79%, or at least 80%, or at least 81%, or at least 82%, or at least 83%, or at least 84%, or at least 85%, or at least 86%, or at least 87%, or at least 88%, or at least 89%, or at least 90%, or at least 91%, or at least 92%, or at least 92%; and/or with
   - a negative predictive value (NPV) of at least 78%, or at least 79%, or at least 80%, or at least 81%, or at least 82%, or at least 83%, or at least 84%, or at least 85%, or at least 86%, or at least 87%, or at least 88%;

and/or with

   - a positive predictive value (PPV) of at least 63%, or at least 64%, or at least 65%, or at least 66%, or at least 67%, or at least 68%, or at least 69%, or at least 70%, or at least 71%, or at least 72%, or at least 73%, or at least 74%, or at least 75%, or at least 76%, or at least 77%, or at least 78%, or at least 79%, or at least 80%, or at least 81%, or at least 82%, or at least 83%, or at least 84%, or at least 85%, or at least 86%, or at least 87%, or at least 88%, or at least 89%, or at least 90%, or at least 91%, or at least 92%, or at least 93%, or at least 94%,

and/or
in which said multivariate classification model has:

- an area under the ROC curve (AUC) of at least 0.76, at least 0.77, at least 0.78, more particularly at least 0.79, still more particularly at least 0.80, at least 0.81, at least 0.82, at least 0.83, at least 0.84, more particularly of at least 0.85, still more particularly of at least 0.86, still more particularly of at least 0.87, and/or
- a LOOCV error of at most 18%, at most 17%, at most 16%, at most 15%, at most 14%, at most 13%, at most 12%.

5. The method according to any one of claims 1 to 4 in which, in step i),

• said genes selected from:

(a)

- at least one gene from among MBL2, LGALS3BP, IL8, and
- at least one gene from among G1P2, CCL21, CXCL10,

are:

- LGALS3BP and CXCL10 (combination No.15); or
- IL8, CCL21, CXCL10, G1P2, LGALS3BP (combination No.37);

or
• said genes selected from:
(b)

- at least one gene from among MBL2, LGALS3BP, IL8, and
- at least one gene from among G1P2, CCL21, CXCL10, and
- at least one gene from among AFP, CRP, CXCL11, CXCL6, CXCL9, FGF7, MDK, MMP2, SFN, TGFB2, VEGFD,

are:

- LGALS3BP, CXCL10 and MDK (combination No.9); or
- LGALS3BP, IL8, CXCL10, CCL21 and MDK (combination No.24); or
- CRP, G1P2, LGALS3BP, MBL2, TGFB2 (combination No.1); or
- AFP, CXCL6, CXCL9, G1P2, MBL2 (combination No.2); or
- AFP, FGF7, G1P2, MBL2, MMP2 (combination No.3); or
- CXCL11, G1P2, IL8, MBL2, TGFB2 (combination No.4); or
- G1P2, IL8, MBL2, SFN, TGFB2 (combination No.5); or
- CCL21, FGF7, IL8, LGALS3BP, MBL2 (combination No.6); or
- G1P2, LGALS3BP, MBL2, MDK, TGFB2 (combination No.7); or
- G1P2, LGALS3BP, MBL2, MMP2, TGFB2 (combination No.8); or
- G1P2, LGALS3BP, MBL2, SFN, TGFB2 (combination No.10); or
- CXCL6, CXCL10, G1P2, MBL2, MMP2 (combination No.11); or
- CXCL6, CXCL11, G1P2, MBL2, MMP2 (combination No.12); or
- FGF7, G1P2, LGALS3BP, MBL2, TGFB2 (combination No.13); or
- AFP, CXCL6, G1P2, IL8, MDK (combination No.14); or
- CCL21, G1P2, LGALS3BP, MBL2, SFN (combination No.16); or
- CXCL10, G1P2, LGALS3BP, MBL2, TGFB2 (combination No.17); or
- CRP, CXCL6, G1P2, MBL2, SFN (combination No.18); or
- CXCL10, CXCL11, G1P2, MBL2, MMP2 (combination No.19); or
- CXCL11, G1P2, LGALS3BP, MBL2, MDK (combination No.20); or
- G1P2, IL8, LGALS3BP, MBL2, TGFB2 (combination No.21); or
- FGF7, G1P2, IL8, MDK, SFN (combination No.22); or
- CCL21, FGF7, LGALS3BP, MBL2, MDK (combination No.23); or
- CCL21, CXCL6, IL8, LGALS3BP, MDK (combination No.25); or
- CCL21, FGF7, MBL2, MDK, VEGFD (combination No.26); or

- CXCL6, IL8, CCL21, G1P2, MDK (combination No.30); or
- CXCL6, IL8, CXCL10, G1P2, MDK (combination No.33); or
- CCL21, CXCL10, G1P2, LGALS3BP, MDK (combination No.34); or
- CXCL6, IL8, CCL21, G1P2, LGALS3BP (combination No.35); or
- IL8, CXCL10, G1P2, LGALS3BP, MDK (combination No.38); or
- CXCL6, IL8, G1P2, LGALS3BP, MDK (combination No.39); or
- FGF7, G1P2, LGALS3BP, MBL2, MDK (combination No.27); or
- CXCL10, FGF7, IL8, MDK, VEGFD (combination No.28); or
- CCL21, CXCL6, CXCL10, LGALS3BP, MDK (combination No.29); or
- IL8, CCL21, G1P2, LGALS3BP, MDK (combination No.31); or
- IL8, CCL21, CXCL10, G1P2, MDK (combination No.32); or
- CXCL6, IL8, CXCL10, G1P2, LGALS3BP (combination No.36); or
- CXCL6, IL8, CCL21, CXCL10, G1P2 (combination No.40); or
- CXCL6, CXCL10, G1P2, LGALS3BP, MDK (combination No.41); or
- CXCL6, IL8, CCL21, CXCL10, LGALS3BP (combination No.42); or
- CXCL6, CCL21, CXCL10, G1P2, LGALS3BP (combination No.43).

6. The method according to any one of claims 1 to 5 in which, in step i), the levels of translation of said selected genes are assayed.

7. The method according to claim 2, in which:

- said clinical factor(s) are selected from the following factors: sex, age at the date of sampling, age of patient at the date of contamination, age of patient at the treatment start date, body mass index, insulin sensitivity index, diabetes, alcohol consumption, degree of steatosis, mode of contamination, Metavir activity, hepatic fibrosis score measured using the Metavir system (Metavir F score) or using the Ishak system; and/or
- said virological factor(s) are selected from the following factors: viral genotype, duration of infection, viral load before treatment, viral load assayed for the patient at the treatment start date, viral load assayed for the patient at the date of sampling; and/or
- said biological factor(s) other than the levels of expression of genes selected from MBL2, LGALS3BP, IL8, G1P2, CXCL10, CCL21, AFP, CRP, CXCL11, CXCL6, CXCL9, FGF7, MDK, MMP2, SFN, TGFB2, VEGFD are selected from the following factors: concentration of haptoglobin, concentration of apolipoprotein A1, total quantity of bilirubin, concentration of gamma glutamyl transpeptidase, concentration of aspartate aminotransferase, concentration of alanine aminotransferase, platelet count, quantity of prothrombin, quantity of HDL cholesterol, total quantity of cholesterol, concentration of ferritin, level of glycaemia, concentration of peptide C, quantity of insulin, concentration of triglycerides, quantity of albumin, transferrin saturation, concentration of alkaline phosphatase,

and/or

- in which:
- said clinical factor(s) comprise the hepatic fibrosis score measured using the Metavir system (Metavir F score) or using the Ishak system, and/or age at the date of sampling (Age), for example, age at the date of HBP, age at the date of hepatic cytopuncture, age at the date of sampling blood, serum, plasma or urine, and/or body mass index (BMI); and/or
- said virological factor(s) comprise the viral genotype and/or the viral load before treatment; and/or
- said biological factor(s) other than the levels of expression of genes selected from MBL2, LGALS3BP, IL8, G1P2, CXCL10, CCL21, AFP, CRP, CXCL11, CXCL6, CXCL9, FGF7, MDK, MMP2, SFN, TGFB2, VEGFD comprise the concentration of gamma glutamyl transpeptidase and/or the concentration of alkaline phosphatase and/or the concentration of alanine aminotransferase and/or the concentration of aspartate aminotransferase.

8. The method according to claim 7, which comprises:

- determining whether the hepatic fibrosis score of said subject is a score which, in the Metavir score system, is at least F1, more particularly at least F2; and/or
- determining whether the HCV or HCVs with which said subject is infected comprises an HCV of genotype 1, 4, 5 or 6, more particularly of genotype 1 or 4.

9. The method according to any one of claims 1 to 8, in which said sample which has been obtained in advance from of said subject is:

- a sample of intracorporal biological fluid which has been taken from said subject, such as a sample of blood, serum, plasma, or a sample of urine from said subject, or
- a sample containing proteins and/or polypeptides and/or peptides extracted or purified from said biological sample.

10. A **manufactured article** comprising reagents in a combined preparation for their simultaneous, separate or sequential use, said reagents being constituted by reagents which specifically detect each of the transcription or translation products of 2 to 9 human genes, said 2 to 9 human genes comprising said selected genes according to step i) of any one of claims 1 to 9,
in which said reagents are nucleic acids which hybridize specifically to the RNA of said selected genes according to any one of claims 1 to 9, and/or to the cDNA obtained by reverse transcription of these RNA, or are proteins, polypeptides or peptides which specifically bind to the proteins encoded by said selected genes, and in particular in which said reagents are amplification primers and/or nucleic acid probes, or are antibodies or fragments of antibodies or protein, polypeptide or peptide aptamers,
and
in which said reagents are contained in one or more tubes, or in the wells of a nucleic acid amplification plate for receiving a sample containing nucleic acids and a reaction mixture for nucleic acid amplification, or in the wells of a protein titration plate, more particularly a protein microtitration plate, for example an ELISA plate, or on microbeads or on a chip for the detection and quantification of nucleic acids, proteins, polypeptides or peptides.

11. A manufactured article according to claim 10 for carrying out the method according to claim 7, further comprising other reagents in a combined preparation for their simultaneous, separate or sequential use, said other reagents enabling to detect, measure or determine:

- one or more virological factor(s) selected from the following factors: viral genotype, duration of infection, viral load before treatment, viral load assayed for the patient at the treatment start date, viral load assayed for the patient at the date of sampling; and/or
- one or more biological factor(s) other than the levels of expression of genes selected from MBL2, LGALS3BP, IL8, G1P2, CXCL10, CCL21, AFP, CRP, CXCL11, CXCL6, CXCL9, FGF7, MDK, MMP2, SFN, TGFB2, VEGFD, such as one or more biological factor(s) selected from: concentration of haptoglobin, concentration of apolipoprotein A1, total quantity of bilirubin, concentration of gamma glutamyl transpeptidase, concentration of aspartate aminotransferase, concentration of alanine aminotransferase, platelet count, quantity of prothrombin, quantity of HDL cholesterol, total quantity of cholesterol, concentration of ferritin, level of glycaemia, concentration of peptide C, quantity of insulin, concentration of triglycerides, quantity of albumin, transferrin saturation, concentration of alkaline phosphatase,

in which said reagents are nucleic acids which hybridize specifically to the RNA of said selected genes according to any one of claims 1 to 10, and/or to the cDNA obtained by reverse transcription of these RNA, or are proteins, polypeptides or peptides which specifically bind to the proteins encoded by said selected genes, and in particular in which said reagents are amplification primers and/or nucleic acid probes, or are antibodies or fragments of antibodies or protein, polypeptide or peptide aptamers,
and
in which said reagents are contained in one or more tubes, or in the wells of a nucleic acid amplification plate for receiving a sample containing nucleic acids and a reaction mixture for nucleic acid amplification, or in the wells of a protein titration plate, more particularly a protein microtitration plate, for example an ELISA plate, or on microbeads or on a chip for the detection and quantification of nucleic acids, proteins, polypeptides or peptides.

12. **Use of a manufactured article** according to claim 10 or 11, for carrying out a method according to any one of claims 1 to 9.

**FIGURE 1A**

**FIGURE 1B**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4376110 A
- US 4683202 A
- US 4683195 A
- US 4965188 A
- US 6569627 B

- FR 1151031
- US 61440980 B
- FR 1155004
- US 61494470 B

**Littérature non-brevet citée dans la description**

- **STEFAN FRITSCH ; FRAUKE GUENTHER.** *Neural-net, http://www.r-project.org*
- **ANASTASIADIS et al.** New globally convergent training scheme based on the résilient propagation algorithm. *Neurocomputing,* 2005, vol. 64, 253-270
- **ASSELAH et al.** Liver gene expression signature to predict response to pegylated interferon plus ribavirin combination therapy in patients with chronic hepatitis C. *Gut,* 2008, vol. 57, 516-524
- **BOX ; COX.** An analysis of transformations. *Journal of the Royal Statistical Society, Series B,* 1964, vol. 26, 211-243
- **BREIMAN.** Random Forests. *Machine Learning,* 2001, vol. 45, 5-32
- **CHAMBERS.** Software for data analysis : programming with R. Springer, 2008
- **CHEN et al.** *Gastroenterology,* 2005, vol. 128, 1437-1444
- **CHEN et al.** *Gastroenterology,* 2010, vol. 138, 1123-1133
- **COLE et al.** *Proc. Natl. Acad. Sci. USA,* 1983, vol. 80, 2026-2030
- **COLE et al.** Monoclonal Antibodies And Cancer Therapy. Alan R. Liss, Inc, 1985, 77-96
- **FALISSARD.** Comprendre et utiliser les statistiques dans les sciences de la vie. Masson, 2005
- **HASTIE ; TIBISHIRANI ; FRIEDMAN.** The Elements of Statistical Learning: Data Mining, Inference and Prediction. Springer, 2009
- **HIDETSUGU SAITO et al.** On-treatment prédictions of success in peg-interferon/ribavirin treatment using a novel formula. *World J. Gastroenterol.,* 2010, vol. 16 (1), 89-97
- Using Neural Nets for Interprétation of Nonlinear Models. **INTRATOR ; INTRATOR.** Proceedings of the Statistical Computing Section. 1993, 244-249
- **KÖHLER ; MILSTEIN.** *Nature,* 1975, vol. 256, 495-497

- **KOSBOR et al.** *Immunology Today,* 1983, vol. 4, 72
- **KRAMAR et al.** Critères ROC généralisés pour l'évaluation de plusieurs marqueurs tumoraux. *Revue d'Epidémiologie et Santé Publique,* 1999, vol. 47, 376-383
- **KRAMAR et al.** mROC: a computer program for combining tumour markers in predicting disease states. *Computer methods and programs in biomedicine,* 2001, vol. 66, 199-207
- **LIAW ; WIENER.** *Classification and regression by Random Forest,* 2002, 18-22
- **LIVAK ; SCHMITTGEN.** Analysis of relative gene expression data using real-time quantitative PCR and the 2(-Delta Delta C(T)) Method. *Methods,* 2001, vol. 25, 402-408
- **REISER ; FARAGGI.** Confidence intervals for the generalized ROC criterion. *Biometrics,* 1997, vol. 53, 644-652
- Rprop - Description and Implementation Details. **RIEDMILLER.** Technical Report. University of Karlsruhe, 1994
- **RIEDMILLER ; BRAUN.** A direct adaptive method for faster backpropagation learning: the RPROP algorithm. *Proceedings of the IEEE International Conference on Neural Networks (ICNN),* 1993, 586-591
- **SCHMITTEN ; LIVAK.** Analyzing real-time PCR data by the comparative Ct method. *Nature Protocols,* 2008, vol. 3 (6), 1101-1108
- **SHAPIRO.** The interpretation of diagnostic tests. *Statistical Methods in Medical Research,* 1999, vol. 8, 113-134
- **SU ; LIU.** Linear combinations of multiple diagnostic markers. *Journal of the American Statistical Association,* 1993, vol. 88, 1350-1355
- **SWETS.** Measuring the accuracy of diagnostic systems. *Science,* 1988, vol. 240, 1285-1293
- **THEODORIDIS ; KOUTROUMBOS.** Pattern Recognition. Academic Press. Elsevier, 2009

- **WAHBA.** Splines Models for Observational Data. *SIAM - Society for Industrial and Applied Mathematics,* 1990

- **WAHBA ; WOLD.** A completely automatic French curve: Fitting spline functions by cross-validation. *Communications in Statistics,* 1975, vol. 4, 1-17

SEQUENCE LISTING

<110> BIO-RAD PASTEUR INSTITUT NATIONAL DE LA SANTE ET DE LA RECHERCHE MEDICALE ARIANA PHARMACEUTICALS S.A. ASSISTANCE PUBLIQUE - HOPITAUX DE PARIS CENTRE NATIONAL DE LA RECHERCHE SCIENTIFIQUE

<120> Combinaison de biomarqueurs pour le pronostic d'une réponse ou non-réponse à un traitement anti-VHC

<130> B09074B - FP/BA

<150> FR1151031
<151> 2011-02-09

<150> US61440980
<151> 2011-02-09

<150> FR1155004
<151> 2011-06-08

<150> US61494470
<151> 2011-06-08

<160> 36

<170> PatentIn version 3.3

<210> 1
<211> 22
<212> DNA
<213> Homo sapiens

<400> 1
ggcacgtatc aaaaagtggc tg          22

<210> 2
<211> 25
<212> DNA
<213> Homo sapiens

<400> 2
atttcaccat tggtcaggaa gaact          25

<210> 3
<211> 24

<212> DNA
<213> Homo sapiens

<400> 3
gaggcagcga actcatcttt gcca        24

<210> 4
<211> 21
<212> DNA
<213> Homo sapiens

<400> 4
ccgccagcat cttcaccgtc a        21

<210> 5
<211> 19
<212> DNA
<213> Homo sapiens

<400> 5
gggcagcgag atgcagcac        19

<210> 6
<211> 20
<212> DNA
<213> Homo sapiens

<400> 6
ccactcagcg cactcgctcc 20

<210> 7
<211> 20
<212> DNA
<213> Homo sapiens

<400> 7
tgacccctcc gaggctcttc        20

<210> 8
<211> 22
<212> DNA
<213> Homo sapiens

<400> 8
atgtcaccat cgttcacgcc tt        22

<210> 9
<211> 25
<212> DNA
<213> Homo sapiens

<400> 9
ctgactctaa gtggcattca aggag        25

<210> 10
<211> 25
<212> DNA
<213> Homo sapiens

<400> 10
ggttgattac taatgctgat gcagg          25


<210> 11
<211> 25
<212> DNA
<213> Homo sapiens


<400> 11
cacagtggta cctgaggatc gataa          25


<210> 12
<211> 23
<212> DNA
<213> Homo sapiens


<400> 12
gccactgtcc tgatttccat gat          23


<210> 13
<211> 24
<212> DNA
<213> Homo sapiens


<400> 13
caccggaagg aaccatctca ctgt          24


<210> 14
<211> 23
<212> DNA
<213> Homo sapiens


<400> 14
tccttggcaa aactgcacct tca          23


<210> 15
<211> 21
<212> DNA
<213> Homo sapiens


<400> 15
agagtgcctg aacaacggat t          21


<210> 16
<211> 19
<212> DNA
<213> Homo sapiens


<400> 16
ccattcgcct tctgctctt          19


<210> 17
<211> 24
<212> DNA
<213> Homo sapiens


<400> 17
ctccatccca gctatcctgt tctt          24

```
<210> 18
<211> 23
<212> DNA
<213> Homo sapiens

<400> 18
tctgcacata gctctgcctg aga          23

<210> 19
<211> 25
<212> DNA
<213> Homo sapiens

<400> 19
gtttacgcgt tacgctgaga gtaaa          25

<210> 20
<211> 21
<212> DNA
<213> Homo sapiens

<400> 20
cgttcttcag ggaggctacc a          21

<210> 21
<211> 21
<212> DNA
<213> Homo sapiens

<400> 21
actgcggttt tctcgaatcc a          21

<210> 22
<211> 20
<212> DNA
<213> Homo sapiens

<400> 22
ggtatccatc gccatgctcc          20

<210> 23
<211> 22
<212> DNA
<213> Homo sapiens

<400> 23
cgacaagaag cgcatcattg ac          22

<210> 24
<211> 22
<212> DNA
<213> Homo sapiens

<400> 24
ctgttggcga tctcgtagtg ga          22

<210> 25
<211> 24
```

<212> DNA
<213> Homo sapiens

<400> 25
gtgtgctaca gttgttcaag gctt        24

<210> 26
<211> 25
<212> DNA
<213> Homo sapiens

<400> 26
ctcaatatct gccactttca ctgct        25

<210> 27
<211> 24
<212> DNA
<213> Homo sapiens

<400> 27
acccgaactt tccaagccat aact        24

<210> 28
<211> 26
<212> DNA
<213> Homo sapiens

<400> 28
ccacatccag gactagtttc tggatt        26

<210> 29
<211> 24
<212> DNA
<213> Homo sapiens

<400> 29
cctcgtacat ttccaaacag ctct        24

<210> 30
<211> 24
<212> DNA
<213> Homo sapiens

<400> 30
tggcaagcac ttacaacctg tatg        24

<210> 31
<211> 23
<212> DNA
<213> Homo sapiens

<400> 31
gacgtgacca tggagaagct gtt        23

<210> 32
<211> 24
<212> DNA
<213> Homo sapiens

<400> 32
aagccttcct cgacatgtct gtct          24

<210> 33
<211> 24
<212> DNA
<213> Homo sapiens

<400> 33
atccacctac aatccttgaa agac          24

<210> 34
<211> 26
<212> DNA
<213> Homo sapiens

<400> 34
tccattcttc agtgtagcaa tgattt          26

<210> 35
<211> 20
<212> DNA
<213> Homo sapiens

<400> 35
ggcgacctgg aagtccaact          20

<210> 36
<211> 20
<212> DNA
<213> Homo sapiens

<400> 36
ccatcagcac cacagccttc          20